(19)

Europäisches
Patentamt
European
Patent Office
Office européen
des brevets

(11)  **EP 1 929 297 B1**

(12)  # EUROPEAN PATENT SPECIFICATION

(45) Date of publication and mention
of the grant of the patent:
**21.11.2012 Bulletin 2012/47**

(51) Int Cl.:
***A61K 45/00*** [(2006.01)]     ***A61K 47/00*** [(2006.01)]
***C12Q 3/00*** [(2006.01)]

(21) Application number: **06815711.4**

(22) Date of filing: **29.09.2006**

(86) International application number:
**PCT/US2006/037924**

(87) International publication number:
**WO 2007/041245 (12.04.2007 Gazette 2007/15)**

(54) **BIOMARKERS FOR MULTIPLE SCLEROSIS AND METHODS OF USE THEREOF**

BIOMARKER FÜR MULTIPLE SKLEROSE UND VERWENDUNGSVERFAHREN DAFÜR

MARQUEURS BIOLOGIQUES DE LA SCLEROSE EN PLAQUES ET METHODES D'UTILISATION
DE CEUX-CI

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **29.09.2005 US 722172 P**

(43) Date of publication of application:
**11.06.2008 Bulletin 2008/24**

(60) Divisional application:
**12161896.1 / 2 517 727**

(73) Proprietors:
 • **Caprion Proteomics USA, LLC**
  **Menlo Park, CA 94025 (US)**
 • **Biogen Idec MA Inc.**
  **Cambridge, MA 02142 (US)**

(72) Inventors:
 • **KANTOR, Aaron, B.**
  **San Carlos, CA 94070 (US)**
 • **GOELZ, Susan**
  **Portland, OR 97202 (US)**
 • **DENG, Jun**
  **Cupertino, CA 95014 (US)**
 • **LIN, Hua**
  **Fremont, CA 94539 (US)**
 • **BECKER, Christopher**
  **Palo Alto, CA 94301 (US)**

(74) Representative: **Pilkington, Stephanie Joan
Potter Clarkson LLP
The Belgrave Centre
Talbot Street
Nottingham NG1 5GG (GB)**

(56) References cited:
**US-B2- 6 753 135**

 • **KANTOR ET AL: "Identification of short-term
pharmacodynamic effects of interferon-beta-1a
in multiple sclerosis subjects with broad- based
phenotypic profiling" JOURNAL OF
NEUROIMMUNOLOGY, ELSEVIER SCIENCE
PUBLISHERS BV, XX, vol. 188, no. 1-2, 27 July
2007 (2007-07-27), pages 103-116, XP022206090
ISSN: 0165-5728**
 • **BOYLAN M T ET AL: "Interferon-beta1a
administration results in a transient increase of
serum amyloid A protein and C-reactive protein:
comparison with other markers of inflammation."
IMMUNOLOGY LETTERS 15 JAN 2001, vol. 75, no.
3, 15 January 2001 (2001-01-15), pages 191-197,
XP002558377 ISSN: 0165-2478**
 • **STÜRZEBECHER S ET AL: "Pharmacodynamic
comparison of single doses of IFN-beta1a and
IFN-beta1b in healthy volunteers." JOURNAL OF
INTERFERON & CYTOKINE RESEARCH : THE
OFFICIAL JOURNAL OF THE INTERNATIONAL
SOCIETY FOR INTERFERON AND CYTOKINE
RESEARCH NOV 1999, vol. 19, no. 11, November
1999 (1999-11), pages 1257-1264, XP002558378
ISSN: 1079-9907**

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**(Cont. next page)**

- ALAM JOHN ET AL: "Comparative pharmacokinetics and pharmacodynamics of two recombinant human interferon beta-1a (IFN-beta-1a) products administered intramuscularly in healthy male and female volunteers" PHARMACEUTICAL RESEARCH (NEW YORK), vol. 14, no. 4, 1997, pages 546-549, XP002558379 ISSN: 0724-8741
- SHAPIRO SARAH ET AL: "The 'Immunological-Synapse' at its APC side in relapsing and secondary-progressive multiple sclerosis: Modulation by interferon-beta." JOURNAL OF NEUROIMMUNOLOGY, vol. 144, no. 1-2, November 2003 (2003-11) , pages 116-124, XP002558380 ISSN: 0165-5728
- HARTRICH, L. ET AL.: 'Dynamics of immune cell trafficking in interferon-beta treated multiple sclerosis patients.' J. NEUROIMMUNOL. vol. 139, 2003, pages 84 - 92, XP008126515
- STURZEBECHER, S. ET AL.: 'Expression profiling identifies responder and non-responder phenotypes to interferon-beta in multiple sclerosis.' BRAIN. vol. 126, 2003, pages 1419 - 1429, XP008126516
- HONG, J. ET AL.: 'Gene expression profiling of relevant biomarkers for treatment evaluation in 1 multiple sclerosis.' J. NEUROIMMUNOL. vol. 152, 2004, pages 126 - 139, XP002566863
- CHEN, M. ET AL.: 'Glatiramer acetate-reactive T cells produce brain-derived neurotrophic factor.' J. NEUROL SCI. vol. 215, 2003, pages 37 - 44, XP008076627
- MOLNARFI, N. ET AL.: 'Opposite effects of interferon-beta on cytokine homeostasis in LPS- and T cell cantact-activated human monocytes.' J. NEUROIMMUNOL. vol. 146, 2004, pages 76 - 83, XP008126471
- ALLIE, R. ET AL.: 'Bystander Modulation of Chemokine Receptor Expression on Peripheral Blood T Lymphocytes Mediated by Glatiramer Therapy.' ARCH, NEUROL. vol. 62, June 2005, pages 889 - 894, XP008126474
- BUTTMANN M ET AL: "Interferon-beta induces transient systemic IP-10/CXCL10 chemokine release in patients with multiple sclerosis", JOURNAL OF NEUROIMMUNOLOGY, ELSEVIER SCIENCE PUBLISHERS BV, XX, vol. 156, no. 1-2, 1 November 2004 (2004-11-01), pages 195-203, XP004591416, ISSN: 0165-5728, DOI: DOI: 10.1016/J.JNEUROIM.2004.07.016

**Description**

Field of the Invention

The present invention relates to the identification and use of biomarkers that are useful monitoring interferon (IFN)-beta therapy in patients with multiple sclerosis (MS).

Background of the Invention

Multiple sclerosis (MS) is a chronic neurological and inflammatory disease of the central nervous system (CNS). In people affected by MS, patches of damage called plaques or lesions appear in seemingly random areas of the CNS white matter. At the site of a lesion, a nerve insulating material, myelin, is lost in a process known as demyelination. Inflammation, demyelination, oligodendrocyte death, membrane damage and axonal death all contribute to the symptoms of MS. An unpredictable disease of the central nervous system, multiple sclerosis (MS) can range from relatively benign to somewhat disabling, to devastating, as communication between the brain and other parts of the body is disrupted. Many investigators believe MS to be an autoimmune disease, whereby the immune system destroys the nerve-insulating myelin. Such assaults may be linked to a yet unknown environmental trigger, such as a virus, diet, or allergy.

MS is a complex syndrome and may not represent a single disease entity. Based on accumulating data from immunological studies of patients with MS and animal model data, autoimmune dysregulation has been viewed as the primary contributor to tissue damage. There is widespread support for the view that MS is an immune-mediated disease, based largely on: 1) the genetic linkage to immune-related molecules, primarily MHC class II; 2) the presence of CNS inflammatory infiltrates including CD4[+] T cells, CD8[+] T cells, B cells, plasma cells, macrophages; 3) magnetic resonance imaging (MRI) indicative of the loss of integrity of the blood-brain barrier (BBB); 4) the response to immune-modulating therapies such as IFN-$\beta$; 5) similarities to the animal model of experimental autoimmune encephalomyelitis (EAE); and 6) the presence of oligoelonal bands and elevated immunoglobulin in the cerebrospinal fluid (CSF).

In accordance with this evidence, one model of MS immunopathology suggests that activated antoreactive T cells recognize myelin antigens in the periphery, cross the CNS endothelium, leading to a cascade of events that culminates in white matter inflammation and tissue destruction. Inside the CNS, release of local cytokines, chemokines, and matrix motallo-proteins support the recruitment of subsequent waves of infiltrating effector cells, including monocytes and B cells. Mechanisms of myelin destruction and axonal damage are likely to be multiple and include direct effects of pro-inflammatory cytokines, complement-fixing antibodies, antigen-specific and non-specific cytotoxicity, and apoptosis.

While it is broadly appreciated that many MS cases involve immune-mediated pathogenesis, neurodegenerative processes not necessarily associated with inflammation appear to be principal in others. Theories that minimize inflammation as a primary event hold that MS is at least partially a genetically determined disorder characterized by metabolic neurodegeneration. Studies of the mechanisms of axonal damage and neurodegeneration in MS are relatively new, though recognition of axonal damage as a prominent pathological feature in MS lesions is increasing.

It is likely that both neurodegeneration and inflammation contribute to various extents, both among and within individuals with MS. The significance of both inflammatory and non-inflammatory mechanisms in MS pathology was shown by Lassman and colleagues who catalogued lesions and demonstrated the existence of four fundamentally different patterns of demyelination in humans. While two patterns (I and II) showed close similarities to T-cell-mediated or T-cell plus antibody-mediated autoimmune encephalomyelitis, the other patterns (III and IV) were highly suggestive of a primary oligodendrocyte dystrophy, reminiscent of demyelination induced by a virus or toxin rather than one involving the immune system.

One known therapy for MS includes treatment with interferon beta. Interferons (IFNs) are natural proteins produced by the cells of the immune systems of most animals in response to challenges by foreign agents such as viruses, bacteria, parasites and tumor cells. Interferons belong to the large class of glycoproteins known as cytokines. Interferon beta has 165 amino acids. Interferons alpha and beta are produced by many cell types, including T-cells and B-cells, macrophages, fibroblasts, endothelial cells, osteoblasts and others, and stimulate both macrophages and NK cells. Interferon gamma is involved in the regulation of immune and inflammatory responses. It is produced by activated T-cells and Th1 cells. Several different types of interferon are now approved for use in humans. Interferon alpha (including forms interferon alpha-2a, interferon alpha-2b, and interferon alfacon-1) was approved by the United States Food and Drug Administration (FDA) as a treatment for Hepatitis C. There are two currently FDA-approved types of interferon beta. Interferon beta 1a (Avonex®) is identical to interferon beta found naturally in humans, and interferon beta 1b (Betaseron®) differs from interferon beta found naturally in humans in that it contains a serine residue in place of a cysteine residue at position 17. Interferon gamma has been approved only for Chronic Granulomatous Disease. Off-label uses of interferon beta have included AIDS, cutaneous T-cell lymphoma. Acute Hepatitis C (non-A. non-B), Kaposi's sarcoma, malignant melanoma, and metastatic renal cell carcinoma.

Stürzebecher et al (1999) J. Interferon & Cytokine Res. 19: 1257-64 describes a pharmacodynamic comparison of the effects of a single dose of IFN-beta1a and IFN-beta1b in healthy volunteers.

Shapiro et al (2003, Journal of Neuroimmunology 144: 116-124) examined the expression of CD40, CD54, CD80, CD86 and HLA-DR on the cell-surface of CD14(+) peripheral blood monocytes from relapsing-remitting and secondary-progressive MS patients, prior to and during 1 year of IFN-β1a (Rebif) therapy. Biomarker levels were evaluated 3, 6, 9 and 12 months after initiation of treatment, IFN-β therapy led to a transient increase in expression of HLA-DR, CD40 and CD86 in patients, reflecting drug-target interaction.

Boylan et al (2001, Immunology letter 75: 191-197) discloses that following IFN-β1a (Avonex) administration to MS patient HLA-DR and CD86 are transiently upregulated on monocytes 24 hours and 72 hours post-injection relative to pre-injection levels. This effect was observed at initiation of treatment as well as at week 12 of treatment.

A physician may diagnose MS in some patients soon after the onset of the illness. In others, however, doctors may not be able to readily identify the cause of the symptoms, leading to years of uncertainty and multiple diagnoses punctuated by baffling symptoms that mysteriously wax and wane. The vast majority of patients are mildly affected, but in the worst cases, MS can render a person unable to write, speak, or walk. MS is a disease with a natural tendency to remit spontaneously, for which there is no universally effective treatment. No single laboratory test is yet available to prove or rule out MS, nor does a cure exist. Therefore, there is a great need in the art for improved methods to identify compositions for the treatment of multiple sclerosis, improved methods for identifying a dose of a composition useful for the treatment of multiple sclerosis, and improved methods for monitoring treatment of a subject with multiple sclerosis. There is also a need in the art for improved methods to diagnose multiple sclerosis, and improved methods to diagnose relapse or disease progression in subjects that are being treated for multiple sclerosis.

Summary of the Invention

The present invention provides a method of monitoring interferon (IFN)-beta therapy in a human subject, the subject having been administered an IFN-beta therapeutic composition, the method comprising evaluating in a biological sample whether administration of the IFN-beta therapeutic composition causes a transient change in a monocyte-associated variable, wherein the monocyte-associated variable is monocyte expression of an HLA Cass II HLA-DP, wherein the subject is a multiple sclerosis (MS) patient un established IFN-beta therapy, aud wherein a transieut change in the monocyte-associated variable indicates efficacions treatment of multiple sclerosis.

In some embodiments, the change in the monocyte-assodated variable is an increase and occurs within about 1 to about 2 days after administration of the therapeutic composition.

In other embodiments, the change in the monocyte-associated variable is reversed at about 6 days after administration of the therapeutic composition.

In some embodiments, two or more, three or more, or four or more monocyte-associated variables are evaluated, and wherein the further monocyte-associated variables are selected from the group consisting of absolute monocyte count; monocyte/leukocyte ratio; and monocyte expression of CCR5, CD11b, CD38, CD40. CD54, CD64, CD69, CD86, TLR2, or TLR4; or MCP2 (monocyte chemoattractant protein 2).

In some embodiments the change comprises an increase in monocyte expression of HLA-DP protein of at least 10%.

In some embodiments the change comprises an increase in two or more, or three or more HLA Class II, the HLA Class II further comprising DR, DQ or PAN (HLA-DP, DR and DQ).

In some embodiments the expression of the HLA Class II is an increase in expression and wherein the increase is of at least about 30%, at least about 40%, at least about 50%, at least about 60%, at least about 65%, at least about 70%, or at least about 75%.

In some embodiments the expression of the HLA Class II is an increase in expression and wherein the increase is of between about 30% and about 120%, or between about 50% and about 75%.

In some embodiments the change in the monocyte-associated variable is detected by cytometry, immunoassay, mass spectrometry, or a method of quantifying nucleic acids.

In some embodiments the therapeutic composition comprises an interferon beta variant, homologue, or fragment thereof, wherein said variant, homologue, or fragment has a similar activity as interferon beta 1a The interferon beta homologue may be derivatized by pegylation.

In some embodiments therapeutic composition comprises AVONEX® or BETASERON®.

In some embodiments the expression of the HLA Class 11 molecule has an effect size of at least about 0.7, at least about 0.8, at least about 0.9, at least about 1, at least about 1.1, at least about 1.2, at least about 1.3, at least about 1.4, at least about 1.5, at least about 1.6, at least about 1.7, at least about 1.8, at least about 19, at least about 2, at least about 2.1, at least about 2.2, at least about 2.3, at least about 2.4, at least about 2.5, at least about 2.6, or at least about 28.

Brief Description of the Drawings

Figure 1 shows subject visits and sample collection scheme for the main study.

Figure 2 Shows subject visits and sample collection scheme for Naive subjects.

Figures 3A and 3B shows distribution of differences for within-group comparisons. For each comparison, differences of means is for all variables with a p-value < 0.05. Figure 3A shows cell count differences for the Naïve, Stable and Breakthrough groups short-term post injection. The Healthy repeat measure only had 7 differences and is not included. Figure 3B shows intensity diffecences.

Figure 4 shows the distribution of CVs for cytometry cell count and intensity variables. Data are shown for 1969 variables (960 count, 986 intensity) from the Healthy group at TL Forty variables with CVs > 200 are not shown.

Figure5A and 5B shows distribution of differences for between group comparisons. For each comparison, differences of means is for all variables with a p-value < 0.05. Figure 5A shows cell counts and Figure 5B shows intensity differences.

Figure 6A and 6B show variables over the duration of the study. Figure 6A shows CD8 T cell counts during the course of the study and Figure 6B shows CD44 Intensity on CD 4 T cells. Data are shown for T1 samples.

Figure 7 shows a schematic diagram detailing the fractionation and mass spectrometric analysis used in this present study. This study used serum as the starting material. Also used in this study was a three-fraction (partial) DeepLook™ analysis, a 2-dimensional separation of tryptic peptides that uses a first step of off-line strong-cation-exchange (SCX) chromatography and then on-line reverse-phase chromatography

Figure 8 shows a schematic of the workflow in metabolite identification process. The first step after determining the statistics for differential expression, internal library linking, is a matching process to molecules previously identified in our laboratories.

Figure 9 shows components identified in the 1 D serum proteome per accession number. Y axis is occurrence.

Figure 10 shows components identified in the 2D serum proteome per accession number.

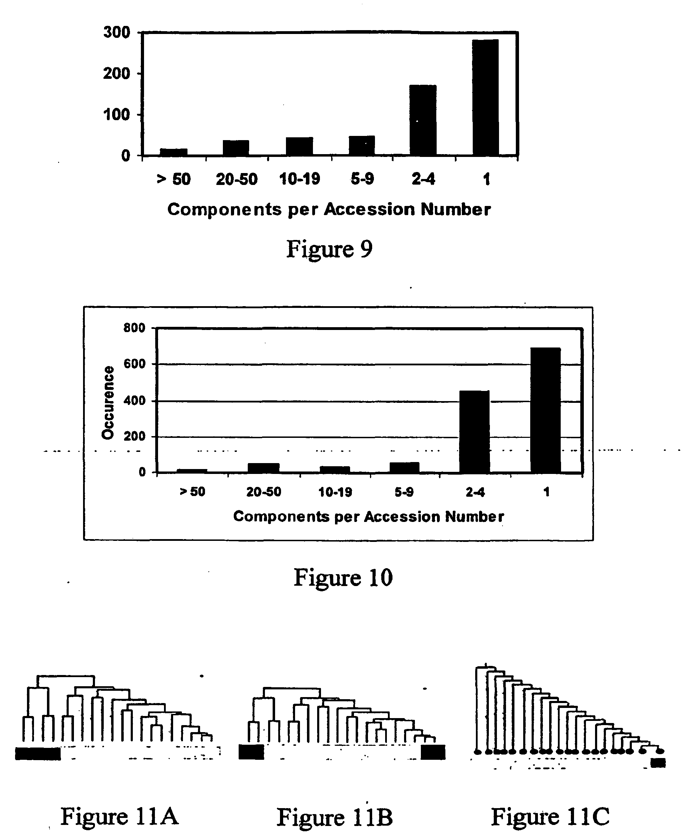

Figure 11A-C show dendrograms that ideally segregates members of two groups. The bars beneath the tree indicate the members of the two groups. The dots indicate the highest monochromatic nodes in the trees. In the ideal case (Figure 11A), there are two monochromatic nodes which are immediately under the root node and so the measure is given by D= (4* 1 + 16* 1)/20 = 1. For a dendrogram that segregates samples in a somewhat less than ideal manner (Figure 11B) the distance is given by D = (2*2 + 2*2 + 3*2 + 2*3 + 1*4 + 2*5 + 3*6 + 1*6 +1*7 +1*8 + 2*9) /20 = 4.55. In this case there are a few monochromatic nodes at higher branches in the tree but many are lower in the tree. In the worst scenario (Figure 11 C) there are 20 monochromatic nodes that are each at the lowest nodes in the tree, the leaf nodes. The measure is given by D = (19 +$\sum$i=1to19 i )/20 =11.45.

Figure 12 shows data presentation. In an Effect Size plot (upper), the direction of triangle represents the trend. In a box and whiskers plot (lower): Center line=Median, bottom box = 25%, top box = 75%, bottom whisker = 10% top whisker = 90%, dots = all other points.

Figure 13 shows select analyte that show short-term pharmacodynamic changes post Avonex treatment. Results are for within group comparisons of T2 (34 hr) vs the steady- stat (repeat) measure). Effect size is the mean difference between the two comparison groups divided by the weighted standard deviation

Figure 14 shows interferon-inducible protein MxA is strongly increased short term post injection. (Var: IA-0051 (ng/mL))

Figures 15A and 15B shows MxA by sample. Figure 15A shows Stable and Breakthrough cohorts. Figure 15B shows Naive cohorts. The left panel shosws subjects with initial visit only, and the right panel shows subjects with 3 and/or 6 month visits. (MxA measurements were only made for 10 repeats within in each group.) Subject PAL035 was the only one to develop neutralizing antibodies. The two subjects which initially enrolled as Stable subjects and returned as Breakthrough) subjects are highlighted.

Figure 16 shows total leukocyte counts are decreased short-term after Avonex dosing in all groups. Assay is based on the expression of the pan leukocyte marker CD45. (Var: CYT-15673)

Figure17 shows neutrophil counts are decreased short-term after Avonex dosing in all groups. Assay is based on the expression of the pan leukocyte marker CD16. (Var: CYT-15668)

Figure 18 shows CD4 T Cell counts are decreased short term after Avonex dosing in all groups. Variable is the average of multiple assays based on CD3 and CD4. (Var: CYT-15635)

Figure 19 shows CD8 T Cell counts are decreased short-term after Avonex dosing in all groups. Variable is the average from x assays based on CD8 expression. (Var: CYT-15645)

Figure 20 shows B Cell counts are decreased short term after Avonex dosing in all groups. Variable is the average from several assays based on CD20 expression. (Var: CYT-15633)

Figure 21 shows monocyte counts are increased short-term after Avonex dosing in all groups. Variable is the average from several assays based on CD14 expression. (Var: CYT-15662) .

Figure 22 shows monocytes as a fraction of total leukocytes are increased short-term post injection. (Var: CYT-15665)

Figure 23 shows monocyte related variables show the strongest and most prevalent short-term pharmacodynamic effect.

Figure 24 shows the fraction of monocytes expressing HLA-DP is increased short-term post injection. (Var: CYT-16489)

Figure 25 shows HLA-PAN Class II on monocytes increased 60 to 80% short-term post injection. (Var: CYT-18196) ,

Figure 26 shows CD38 expression on total monocytes increases short-term after Avonex dosing in all groups. (Var: CYT-17374)

Figure 27 shows the fraction CD38 positive B cells increases short-term after Avonex. (Var: CYT-15948)

Figure 28 shows CD38 expression on total B cells increases short-term after Avonex dosing in all groups. (Var: CYT- 17386)

Figure 29 shows CD40 intensity on monocytes is increased short-term after Avonex dosing in all groups. (Var: CYT-17410)

Figure 30 shows CD86 intensity on monocytes is increased short-term after Avonex dosing in all groups. (Var: CYT-17986)

Figure 31 shows CD54 expression on monocytes is increased short-term after Avonex dosing in all groups. (Var: CYT-17539)

Figure 32 shows sICAM-1 (sCD54) not changed short term after Avonex injection. (Var: IA-0012)

Figure 33 shows CD64 expression on monocytes is increased short-term after Avonex dosing in all groups. (Var: CYT-17749)

Figure 34 shows TRL4 expression on monocytes is increased short-term after Avonex dosing in all groups. Monocytes are identified with CD33 in this assay. (Var: CYT- 18646)

Figure 35 shows TRL2 expression on monocytes is increased short-term after Avonex dosing in all groups. Monocytes are identified with CD33 in this assay. (Van: CYT- 18640)

Figure 36 shows the fraction of CD69 monocytes is increased short-term post Avonex dosing. (Var: CYT- 16261)

Figure 37 shows MCP-2 is increased short term after Avonex dosing in all groups. (Var: IA-17)

Figure 38 shows IP-10 is increased short term after Avonex dosing in all groups. (Var: IA-15 (pg/mL))

Figure 39 shows IL_1RA is increased short term after Avonex dosing in all groups. (Var: IA-27)

Figure 40 shows CRP is increased short term after Avonex dosing in all groups. (Var: IA-5)

Figure 41 shows ACT, $\alpha$-1-anti-chymotrypsin (Pep) is increased short term after Avonex dosing in all groups. (Var: SE-LC-PROT 158973)

Figures 42-45 show Apolipoprotein H (Beta-2-Glycoprotein I) decreases short term after Avonex. Figure 42 shows results for peptide 1. Figure 42 shows results for peptide 2. Figure 42 shows results for peptide 3. (Var: SE-LC-PROT 161593, 161904, 16189)

Figure 45 shows total leukocytes are not different among the groups. (Var: CYT-15673)

Figure 46 shows total monocytes are not different among the groups. (Var: CYT-15662)

Figure 47 shows B cells counts are lower in Naive subjects. (Var: CYT-15633)

Figure 48 shows Avonex treated subjects have lower CD8 T cell counts. (Var: CYT-15645)

Figure 49 shows Platelets are lower in the stable and breakthrough groups. (Var: CYT-15670)

Figure 50 shows NK cells are lower in subjects on Avonex. (Var: CYT- 15666)

Figure 51 shows CD38 B Cells are lower in Naive subjects. Variable is the fraction of B cells which are CD38 positive. (Var -CYT 15948)

Figure 52 shows CD38 intensity on B cells is the highest on Avonex treated subjects. (Var -CYT 17386)

Figure 53 shows the fraction of CD69 positive monocytes is higher in the Healthy group than the Naïve group. (Var: CYT-16261)

Figure 54 and 55 shows CD56 on NK cells is highest in the Avonex treated groups. (Figure 54 Var: CYT- 17596 Cell population: CD3nCD56p; Figure 55 Var: CYT- 17581 Cell population: CD2pCD3nCD56p).

Figure 56 shows NKB1 on NK cells is lowest in the Healthy group (Var: CYT-18610)

Figure 57 shows IFN-$\gamma$ production ex vivo is greater in CD8 T Cell for MS Groups. (Var: CYT- 16615, fraction of CD8 T cells)

Figure 58 shows IL-2 production ex vivo is greater in CD8 T Cell for MS Groups. (Var: CYT- 16731 fraction of CD8 T cells)

Figure 59 shows IL-2 production ex vivo is greater in CD4 T Cell for MS Groups. (Var: CYT- 16694 fraction of CD4 T cells)

Figure 60 shows TNF$\alpha$ production ex vivo is greater in B Cells for MS Groups (Var: CYT- 16914 fraction of B cells)

Figure 61 shows Naive and memory T cell subsets. CD4 and CD8 T cells subsets can be identified based on the level of expression of CD45RA and CD62L as shown in the diagram. Box mimics the gating strategy for the cytometry plots.

Figure 62 shows CD4 T cell naïve memory subsets differ among the cohorts Variables are the % of CD4 T cells. Values in boxes above are mean ± SD.

Figure 63 shows CD8 T cell naïve memory subsets differ among the cohortsVariables are the % of CD8 T cells. Values in boxes above are mean ± SD.

Figure 64 shows soluble CD14 was lower in Healthy subjects. Var: IA-3 (pg/mL)

Figure 65 shows MIP1$\alpha$ (Macrophage Inflammatory Protein 1 a) is higher in healthy subjects Var: IA-20 (pg/mL).

Figure 66 shows MIP1$\beta$ (Mocrophage Inflammatory Protein 1 b) is higher in healthy subjects Var. IA-21 (pg/mL).

Figure 67 shows MCP-1 is lower in MS subjects. (Var. IA-16)

Figure 68 shows MCP-2 is lowest in the Naive group. (Var. IA-17)

Figure 69 shows sIL6R. (Var IA-39)

Figure 70 shows MMP9 is lower in MS subjects. (Van IA-44)

Figure 71 shows TIMP1 is lower in MS subjects. (Var: IA-I)

Figure 72 shows MMP9/TIMP1is lowest for treated subjects (Var: Ad hoc).

Figure 73 shows Soluble Vascular Cell Adhesion Molecule-1 (Var: IA-0014 (pg/mL))

Figure 74 shows MXA and IP10 are higher in the Breakthrough group than the Stable Group. (Var. IA 51, 15)

Figure 75 shows Breakthrough subjects tend to have higher levels of MXA than Stable subjects. The two subjects that initially enrolled as Stable subjects and returned as Breakthrough subjects are highlighted in bold yellow.

Figure 76 shows MIP]$\beta$is lower in Breakthrough group than Stable group at all time points. (Var: IA-21).

Figure 77 shows $\beta$-NGF -Nerve Growth Factor is higher in Breakthrough vs Stable at all time points.

Description

The present invention includes methods of monitoring IFN-beta treatment of a subject for multiple sclerosis. These methods are generally accomplished by phenotyping subjects, e.g., measuring for the presence, absence, increase, decrease or other changes in particular biomarkers and/or characteristics of these biomarkers in response to administration of compositions in accordance with the present invention. These biomarkers and/or characteristics can be detected by any method for analysis of amount or expression of these markers, including without limitation, cytometry, immunoassay, mass spectrometry, and methods for quantifying nucleic acids.

Methods of the present invention are based on a study that included cross-sectional pharmacodynamic Phase IV molecular phenotyping of subjects identified as "Healthy," "Naïve," "Stable," or "Breakthrough." The four subject cohorts were "Healthy" composed of healthy individuals without MS or acute systemic disease; "Naïve" composed of MS patients with no previous interferon therapy, who were to commence AVONEX® (Interferon-$\beta$-1a) treatment (some Naive subjects returned for additional visits at 3 and 6 months after beginning treatment); "Stable" composed of MS patients on AVONEX® treatment for at least 18 months who were clinically Stable; and "Breakthrough" composed of MS patients on AVONEX® treatment for at least 12 months with a history of clinical relapses or disease progression. Stable and Breakthrough subjects continued to receive their regular weekly doses of AVONEX®. Sample collection included two baseline samples, in the absence of drug for Healthy and Naive subjects; two steady-state samples, 6 days post injection for Stable, Breakthrough subjects and Naive subjects at 3 and 6-month visits; one sample from all MS subjects (Naive, Stable and Breakthrough) short-term post treatment, 34 hours after injection; and one sample 6 days post drug for Naïve subjects. The repeat samplings, at equivalent time points, within each group provided controls for the analysis.

In the study, broad-based molecular phenotyping was used. The methods enabled tracking of thousands of analytical variables and included microvolume laser scanning cytometry for specific cellular assays, immunoassays for specific soluble proteins, and mass-spectrometry based differential proteomic and metabolomic profiling.

Statistical comparisons were made in the study with appropriate linear mixed effect models and variables showing differences ranked by univariate p-value levels in consideration of multiple comparisons with thousands variables. Within cohort comparisons reflect both short-term and long-term effects of treatment. This class of markers indicates drug-target interactions and downstream consequences and is useful for, among others, evaluating dosing and monitoring treatment. Between cohort comparisons reflect differences that relate to, among others, disease pathophysiology, disease progression and efficacy of treatment.

For the "within cohort" comparisons, the study focused on determining the following: the level of false positive 'hits' from repeat samplings at equivalent time points, the differences between samples taken short-term (34 hr) and long-term (6 days) after injection, and whether the pharmacodynamic differences are the same for the Naive subjects following initiation of AVONEX® treatment For the "between cohort" comparisons, the study focused on determining whether there are differences between Naive and Healthy subjects in the absence of drug treatment that reflect disease, differences between AVONEX® treated and Healthy subjects that could reflect either disease or treatment, differences between AVONEX® treated and Naïve subjects that could reflect either treatment or disease progression, and differences that reflect treatment response that could distinguish Stable and Breakthrough subjects.

Analysis of the study showed that comparisons made at two-equivalent time points that would not be expected to show biological differences did indeed yield relatively few differences, i.e. there are few false positives for cytometry, immunoassay and serum proteomics for all four cohorts, providing confidence for results in other comparisons. A very strong short-term pharmacodynamic effect was observed for treatment with AVONEX® in all cohorts. Hundreds of differences were observed across multiple platforms at a univariate p-value <0.001. Many of the differences were substantial with effect sizes (mean difference/standard deviation) greater than one. Consistent differences were often observed in the Naïve, Stable and Breakthrough cohorts. Generally, Naive subjects showed the most and strongest differences when treatment was initiated. Analysis of MxA, an interferon inducible gene product and a well-established IFN beta marker shows a strong increase short-term post injection in all cohorts.

Within Cohort Comparisons. The study found that most major blood cell populations . (neutrophils, B cells, CD4 T cells, CD8 T cells) decreased in absolute cell counts short-term post injection in all AVONEX® cohorts. However, the striking exception was monocytes, which increased short-term in all cohorts. The increase in the fraction of monocytes per total leukocytes was very dramatic, ranging from 50 to 75% and is a useful pharmacodynamic assay. Additionally, many monocyte-associated cellular variables showed differences short-term post injection. For example, HLA Class II expression was up regulated on monocytes. This was a strong effect, which was consistent across cohorts, ranging from 30 to 120%. This effect was consistent for HLA- DP, DQ, DR and PAN (i.e., all three markers). In contrast, B cells showed a small increase (0-10%) in HLA class II expression with less consistency across cohorts. Other short-term changes included HLA Class I, measured with a pan marker recognizing A, B and C proteins, which was increased 20-40% on total leukocytes. Additionally, monocyte expression of CCR5, CD11b, CD38, CD40, CD54, CD64, CD69 CD86, TLR2, and TLR4, all increased short-term post injection. MCP2 (Monocyte Chemoattractant Protein 2) also increased substantially short-term post injection in all cohorts. Additionally, molecules IP-10 (INF Inducible Protein 1, CXCL- 10) increased short-term post injection in all cohorts; IL,-1RA increased short-term post injection in all cohorts; IL-18 binding protein, detected in the urine, was increased up to two-fold short-term post injection; IL-18 in the serum had a modest short-term increase for the Naïve group; and two nervous system related proteins, β-NGF and BDNF, changed in the Naïve group at first treatment. Further, some acute phase proteins increased short-term post injection. The strongest effects were observed early in AVONEX® treatment for the Naïve group. CRP, ACT, ceruloplasmin, haptoglobin, alpha-1-acid glycoprotein, orosomucoid and serum amyloid A were all increased. Multiple components of the complement cascade increased modestly short-term post injection. Soluble VCAM showed a modest increase short-term post injection. The study showed that some analytes showed a short-term effect only for the Naïve subjects first going on drug. Finally, LIF and β-NGF increased and BDNF and MMP decreased short-term.

**Between Cohort Comparisons.**

Long-term changes. The study found longer term changes and differences in then AVONEX® cohorts. In summary, the study showed that NK cells were lower with drug treatment: Stable, Breakthrough < Naive, Healthy. The study also showed that NK levels decreased for the Naïve group at 3 and 6 months; that CD56 on NK cells increased with drug treatment: Stable, Breakthrough > Naïve, Healthy; that MCP2 was higher in Avonex treated groups: Stable; Breakthrough > Naïve, Healthy; that CD8 T cells decreased with drug treatment: Stable, Breakthrough < Naïve, Healthy; and that B cells increased to Healthy levels with drug: Naïve < Stable, Breakthrough, Healthy. The study also found that the percent of CD38 positive B cells was higher in Stable and Breakthrough subjects than Naive subjects; showed redistribution of naïve and memory CD4 and CD8 T cell subsets between Naïve and Avonex treated subjects; that Apo H was lower with drug treatment for the Stable group: Stable < Naive; that Inter-alpha globulin inhibitor H1 was lower with drug treatment for the Stable group: Stable < Naive; that complement component C1s was lower and C2 higher with drug treatment for the Stable Group; that Mac2 binding protein was higher in the Avonex treated groups; and that Attractin-2 was lower in the Avonex treated groups.

Healthy v. MS. Comparisons between the Naïve and Healthy cohort (in the absence of AVONEX®) showed differences between multiple sclerosis and healthy populations. The study found hundreds of differences across multiple platforms at a univariate p-value <0.01. In general, magnitude and effect size of the differences are smaller than observed for the short-term drug effect in the within group comparisons. Consistent differences were observed in the Naive, Stable and Breakthrough cohorts. The study showed that most major blood cell populations (neutrophils, monocytes CD4 T cells, CD8 T cells) were not different between Naive and Healthy subjects. The study showed that B cells were lower in the Naïve group compared to Healthy, but this effect was not observed in the Stable or Breakthrough groups. The study also showed that the fraction of B cells that express CD38 was also lower in the Naive group compared to Healthy, and that NKB1 on NK cells was lowest in the Healthy group. The study also showed that IFNγ and IL-2 production was greater in CD8 T cells and IL-2 production was greater in CD4 T cells for all MS Groups following ex vivo stimulation. Redistribution of naive and memory T cell subsets between Naive and Healthy subjects was also observed. Soluble CD14 and soluble VCAM were higher in all MS groups compared to Healthy, while MIP-1a, IL-6R and MCP-1 are lower in all MS groups.

Stable v. Breakthrough groups. Comparisons between the Stable and Breakthrough cohorts (during treatment with AVONEX®) showed differences between Stable and Breakthrough populations and markers for efficacy of treatment. Generally, it was found that subtle, smaller differences were observed for these comparisons. The study found that MxA was higher in the Breakthrough group at both 6 days and 34 hours post injection. The study also showed that IP-10 was higher in the Breakthrough group at both 6 days and 34 hours post injection, and that MIP-1-β was lower in the Breakthrough group at both 6 days and 34 hours post injection. The study also showed that β-NGF (Nerve Growth Factor) was higher in Breakthrough group at all time points; that complement components (C1s, C1q) were higher in the Breakthrough group at both 6 days and 34 hours post injection; that Prothrombin was higher in the Breakthrough group at both 6 days and 34 hours post injection. Additionally, it was found that soluble VCAM was highest in the Breakthrough group, which was significantly different than the Stable group: Breakthrough > Stable, Naive > Healthy. Alpha-1 antichymotrypsin was, highest in the Breakthrough group; CALNUC was lowest in the Breakthrough group; Gelsolin was higher in the Breakthrough group; Alpha two plasmin inhibitor was higher in the Breakthrough group; Fetuin-A, which is detected in the urine, was lowest in the Breakthrough group; and AMBP, which is detected in the urine, was lowest in the Breakthrough group.

According to the present invention, the term "multiple sclerosis." (MS) is used to describe the art-recognized disease characterized by inflammation, demyelination, oligodendrocyte death, membrane damage and axonal death. MS can be more particularly categorized as either relapsing/remitting MS (observed in 85-90% of patients) or progressive MS. In some embodiments, MS can be characterized as one of four main varieties as defined in an international survey of neurologists (Lublin and Reingold, 1996, Neurology 46(4):907-11), which are namely, relapsing/remitting MS, secondary progressive MS, progressive/relapsing MS, or primary progressive MS (PPMS).

As used herein, the terms "patient", "a subject who has MS", "a patient who has MS", "an MS subject", "an MS patient", and similar phrases, are intended to refer to subjects who have been diagnosed with MS. The terms "Healthy subject", "non-MS subject", "a subject who does not have MS", "a patient who does not have MS", and similar phrases, are intended to refer to a subject who has not been diagnosed with MS. A Healthy subject has no other acute systemic disease. The term "Naïve", "Naïve cohort" and "Naïve subject" refers to subjects with MS previously naïve to interferon therapy, who commence AVONEX® treatment in the study discussed in the Examples. Naive subjects were diagnosed with MS within six months prior to starting the study discussed in the Examples. The term "Stable", "Stable cohort", "Stable subject", refers to MS patients on AVONEX® for at least 18 months prior to the starting the study discussed in the Examples, with no history of Breakthrough disease (clinically Stable) as evidenced by no relapses for one year. The term "Breakthrough", "Breakthrough cohort", "Breakthrough subject", refers to MS patients on AVONEX® treatment for at least 12 months with a history of Breakthrough disease as evidenced by two relapses in the last 15 months or one relapse in the last 15 months and EDSS progression.

As used herein, the term "biological sample" includes a sample of any cell type or from any tissue or body fluid, body fluids including, but not limited to: cerebrospinal fluid (CSF), serum, plasma, blood, urine, prostatic fluid, saliva or fluid from any suitable tissue. In a preferred embodiment, the biological sample is blood or any component of blood (e.g., serum, plasma, etc.).

The data gathered by the inventors and referenced herein, useful for the methods of the present invention, was generated using AVONEX®, i.e., Interferon beta 1a (SwissProt Accession No. P01574 and gi:50593016) The sequence of interferon beta is:

MTNKCLLQIALLLCFSTTALSMSYNLLGFLQRSSNFQCQKLLWQLNGRLEYCLKDRM
NFDIPEEIKQLQQFQKEDAALTIYEMLQNIFAIFRQDSSSTGWNETIVENLLANVYHQIN
HLKTVLEEKLEKEDFTRGKLMSSLHLKRYYGRILHYLKAKEYSHCAWTIVRVEILRNF
YFINRLTGYLRN (SEQ ID NO:1).

Further information can be found at the Bioinformatic Harvester website (containing a search and ranking of greater than 16 protein resources for about 80,000 human proteins). However, the data and biomarkers generated using AVONEX® are not unique to that composition. Many of the methods of the present invention are based on the principle that compositions having biological activity that is substantially similar to that of AVONEX® will elicit a similar biomarker response as AVONEX® when administered in a similar manner. Such other compositions include, e.g., other interferons and fragments, homologues, and natural variants thereof with substantially similar biological activity. Accordingly, these compositions can be used in methods of the present invention. Such compositions include, for example, type I interferons (alpha, beta, omega, epsilon and kappa), type II interferons (gamma) and interferon lambda, and additional naturally occurring subtypes and/or isoforms of interferon, produced by different cell types. Preferred compositions of the present invention include interferon beta variants, homologues, and fragments which have substantially similar biological activity as interferon beta Ia as measured by, for example, the specific activity of the molecule compared to the antiviral activity of the reference standard of recombinant human Interferon beta or Interferon beta 1 a.

Additionally, homologues of compounds and compositions referenced above (e.g., interferon beta 1 b (BETASERON®) which contains a serine residue in place of a cysteine residue at position 17) with substantially similar biological activity are encompassed by the compositions used in the present invention. As used herein, the term "homologue" is used to refer to a polypeptide which differs from a naturally occurring polypeptide by one or more minor modifications or mutations to the naturally occurring polypeptide, but which maintains the overall basic protein and side chain structure of the naturally occurring form (i.e., such that the homologue is identifiable as being related to the wild-type polypeptide). Such changes include, but are not limited to: changes in one or a few amino acid side chains; changes one or a few amino acids, including deletions (e.g., a truncated version of the protein or peptide) insertions and/or substitutions; changes in stereochemistry of one or a few atoms; and/or minor derivatizations, including but not limited to: PEGylalion (polyethylene glycol modifications), methylation, famesylation, geranyl geranylation, glycosylation, carboxymethylation, phosphorylation, acetylation, myristoylation, prenylation, palmitation, and/or amidation. A homologue can have either enhanced, decreased, or substantially similar properties as compared to the naturally occurring polypeptide. Homologues can include synthetically produced homologues, naturally occurring allelic variants of a given protein or domain, or homologous sequences from organisms other than the organism from which the reverence polypeptide was derived. Test compositions to be screened also includes known organic compounds such as peptides (e.g., products of peptide libraries), oligonucleotides, carbohydrates, synthetic organic molecules (e.g., products of chemical combinatorial libraries), and antibodies. Compounds may also be identified using rational drug design relying on the structure of the product of a gene or polynucleotide. Such methods are known to those of skill in the art and involve the use of threo-dimensional imaging software programs. For example, various methods of drug design useful to design or select mimetics or other therapeutic compounds useful in the present invention are disclosed in Maulik et al., 1997, Molecular Biolechnology: Therapeutic Applications and Strategies. Wiley-Liss, Inc,

Other compositions of the present invention include mimetics. As used herein, a mimetic refers to any peptide or non-peptide compound that is able to mimic the biological action of a naturally occurring peptide, often because the mimetic has a basic structure that mimics the basic structure of the naturally occurring peptide and/or has the salient biological properties of the naturally occurring peptide. Mimetics can include, but are not limited to: peptides that have substantial modifications from the prototype such as no side chain similarity with the naturally occurring peptide (such modifications, for example, may decrease its susceptibility to degradation); anti-idiotypic and/or catalytic antibodies, or fragments thereof, non-proteinaceous portions of an isolated protein (e.g., carbohydrate structures); or synthetic or natural organic molecule, including nucleic acids and drugs identified through combinatorial chemistry, for example. Such mimetics can be designed, selected and/or otherwise identified using a variety of methods known in the art.

A mimetic can be obtained, for example, from molecular diversity strategies (a combination of related strategies allowing the rapid construction of large, chemically diverse molecule libraries), libraries of natural or synthetic compounds, in particular from chemical or combinatorial libraries (i.e, libraries of compounds that differ in sequence or size but that have the similar building blocks) or by rational, directed or random drug design. See for example, Meulik d *al., supra.*

In a molecular diversity strategy, large compound libraries are synthesized, for example, from peptides, oligonucleotides, carbohydrates and/or synthetic organic molecules, using biological, enzymatic and/or chemical approaches. The critical parameters in developing a molecular diversity strategy include subunit diversity, molecular size, and library diversity. The general goal of screening such libraries is to utilize sequential application of combinatorial selection to obtain high-affinity ligands for a desired target, and then to optimize the lead molecules by either random or directed design strategies. Methods of molecular diversity are described in detail in Maulik, *et al., ibid.*

Maulik *et al.* also disclose, for example, methods of directed design, in which the user directs the process of creating novel molecules from a fragment library of appropriately selected fragments; random design, in which the user uses a genetic or other algorithm to randomly mutate fragments and their combinations while simultaneously applying a selection criterion to evaluate the fitness of candidate ligands; and a grid-based approach in which the user calculates the interaction energy between three dimensional receptor structures and small fragment probes, followed by linking together of favorable probe sites.

Designing a compound for testing according to the present disclosure can include creating a new chemical compound or searching databases of libraries of known compounds (e.g., a compound listed in a computational screening database containing three dimensional structures of known compounds). Designing can also be performed by simulating chemical compounds having substitute moieties at certain structural features. The step of designing can include selecting a chemical) compound based on a known function of the compound. A preferred step of designing comprises computational screening of one or more databases of compounds in which the three dimensional structure of the compound is known and is interacted (e.g., docked, aligned, matched, interfaced) with the three dimensional structure of a target by computer (e.g. as described by Humblet and Dunbar, Animal Reports in Medicinal Chemistry, vol. 28, pp. 275-283, 1993, M Venuti, ed., Academic Press). Methods to synthesize suitable chemical compounds are known to those of skill in the art and depend upon the structure of the chemical being synthesized. Methods to evaluate the bioactivity of the synthesized compound depend upon the bioactivity of the compound (e.g., inhibitory or stimulatory).

Candidate test compositions identified or designed by the methods described herein can be synthesized using techniques known in the art, and depending on the type of compound. Synthesis techniques for the production of non-protein compounds, including organic and inorganic compounds are well known in the art For example, for smaller peptides, chemical synthesis methods are preferred. For example, such methods include well known chemical procedures, such as solution or solid-phase peptide synthesis, or semi- synthesis in solution beginning with protein fragments coupled through conventional solution methods. Such methods are well know in the art and may be found in general texts and articles in the area such as: Merrifield, 1997, Methods Enzymol. 289:3-13; Wade et al., 1993, Australas Biotechnol. 3(6):332-336. Wong et al., 1991. Experientia 47(11-12):1123-1129; Carey et al., 1991, Ciba Found Symp. 158:187-203; Plane et al., 1990, Biologicals 18(3):147-157; Bodanszky, 1985, Int. J. Pept. Protein Res. 25(5):449-474; or H. Dugas and C. Penney, BIOORGANIC CHEMISTRY. (1981) at pages 54-92.

For example, peptides may be synthesized by solid-phase methodology utilizing a commercially available peptide synthesizer and synthesis cycles supplied by the manufacturer. One skilled in the art recognizes that the solid phase synthesis could also be accomplished using the FMOC strategy and a TF A/scavenger cleavage mixture. A compound that is a protein or peptide can also be produced using recombinant DNA technology and methods standard in the art, particularly if larger quantities of a protein are desired.

As used herein, the terms "test composition", "test compound". "putative inhibitory compound" or "putative regulatory compound" refer to compositions having an unknown or previously unappreciated regulatory activity in a particular process. As such, the term "identify" with regard to methods to identify compounds is intended to include all compositions, the usefulness of which as a regulatory compound for the purposes of regulating the expression or activity of a target biomarker or otherwise regulating some activity that may be useful in the study or treatment ofMS is determined by a method of the present invention.

The terms "biomarker" or "marker", as used herein, can refer to a cell, particularly a blood cell, a ratio of particular cells, a cell-associated polypeptide or protein, a soluble polypeptide or metabolite described herein or to a polynucleotide (including a gene) that encodes a polypeptide identified by the invention. In addition, the terms "biomarker" or "marker" can be generally used to refer to any portion or component of such a cell, portion a component indicating the ratio of particular cells, cell-associated polypeptides, soluble polypeptides or polynucleotides that can identity or correlate with the cell, ratio of particular cells, full-length polypeptide or polynucleotide, for example, in an assay of the invention. Biomarkers also include any components or portions of cells, precursors and successors of polypeptides and polynucleotides of the invention, as well as polypeptides and polynucleotides substantially homologous to polypeptides and polynucleotides of the invention Accordingly, a biomarker useful in the present invention is any cell, cell ratio, polynucleotide, polypeptide or metabolite, the expression or occurrence of which is regulated (up or down) in a subject with a condition (e.g., MS) as compared to a normal control.

Selected sets of one, two, three, and more of the biomarkers of this invention (up to the number equivalent to all of the biomarkers, including any intervening number, in whole number increments, e.g., 1, 2, 3, 4, 5, 6.-) can be used as end-points for methods of the present invention. In one embodiment, larger numbers of the biomarkers identified herein are used in methods of the invention, since the accuracy of the method may improve as the number of biomarkers screened increases. Specifically, methods of the present invention include evaluating whether administration of the composition causes a change, preferably either a transient change and/or a long term change, in two or more of the biomarkers; in three or more of the biomarkers; in four or more of the biomarkers; in five or more of the biomarkers, in six or more of the biomarkers, etc.

Of the cells analyzed, several thousand molecular ions quantified and several hundred proteins that were profiled, the present inventors have identified multiple biomarkers, (i) the expression of which are regulated differentially in subjects with MS (both Stable and Breakthrough) as compared to subjects without MS, (ii) the expression of which are regulated differentially in subjects with Stable MS compared to subjects with Breakthrough MS, (iii) that cause short-term changes upon drug therapy, and (iv) that cause longer-term changes upon drug therapy among the different cohort populations. More particularly, the biomarkers can be grouped into the following main categories, as described more fully in the Examples section: (1) biomarkers that are selectively (i.e., exclusively or uniquely) upregulated in the serum, urine, or cerebrospinal fluid (CSF) of Naive, Stable and/or Breakthrough subjects as compared to Healthy controls; (2) biomarkers that are selectively downregulated in the serum, urine or CSF of Naive, Stable and/or Breakthrough MS as compared to Healthy controls; (3) biomarkers that are selectively upregulated in the serum, urine, or cerebrospinal fluid (CSF) of Healthy controls as compared to Naïve, Stable and/or Breakthrough subjects treated with AVONEX® (4) biomarkers that are selectively downregulated in the serum, urine or CSF of Healthy controls as compared to Naive, Stable and/or Breakthrough subjects treated with AVONEX®; (5) biomarkers that are selectively upregulated in the serum, urine, or CSF of subjects with Breakthrough MS as compared to Stable controls; (6) biomarkers that are selectively downregulated in the serum, urine, or CSF of subjects with Breakthrough MS as compared to Stable controls; (7) biomarkers that are selectively upregulated in the serum, urine, or CSF of Naive subjects as compared to Healthy controls; (8) biomarkers that are selectively downregulated in the serum, urine, or CSF of Naive subjects as compared to Healthy controls; (9) biomarkers that are selectively upregulated in the serum, urine, or CSF short term after AVONEX® treatments in Naive, Stable, and Breakthrough subjects; (10) biomarkers that are selectively downregulated in the serum, urine, or CSF short term after AVONEX® treatments for Naïve, Stable, and Breakthrough subjects; (11) biomarkers that are selectively upregulated in the serum, urine, or CSF long term after AVONEX® treatments for Naive, Stable, and Breakthrough subjects; and (12) biomarkers that are selectively dowmegulated in the serum, urine, or CSF long term after AVONEX® treatments for Naive, Stable, end Breakthrough subjects.

As used herein, in one embodiment, compositions and/or biomarkers comprising peptides can be substantially homologous or homologues to another composition or to the biomarker, respectively. Two polypeptides are "substantially homologous" or "homologues" when there is at least 70% homology, at least 80% homology, at least 90% homology, at least 95% homology or at least 99% homology between their amino acid sequences, or when polynucleotides encoding the polypeptides are capable of forming a stable duplex with each other. As used herein, unless otherwise specified, reference to a percent (%) identity refers to an evaluation of homology which is performed using: (1) a BLAST 2.0 Basic BLAST homology search using blastp for amino acid searches, blastn for nucleic acid searches, and blastX for nucleic acid searches and searches of translated amino acids in all 6 open reading frames, all with standard default parameters, wherein the query sequence is filtered for low complexity regions by default (described in Altschul, S.F., Madden, T.L., Schaaffer, A.A., Zhang, J., Zhang, Z., Miller, W. & Lipman, DJ. (1997) "Gapped BLAST and PSI-BLAST: a new generation of protein database search programs." Nucleic Acids Res. 25:3389; (2) a BLAST 2 alignment (using the parameters described below); (3) and/or PSI-BLAST with the standard default parameters (Position- Specific Iterated BLAST). It is noted that due to some differences in the standard parameters between BLAST 2.0 Basic BLAST and BLAST 2, two specific sequences might be recognized as having significant homology using the BLAST 2 program, whereas a search performed in BLAST 20 Basic BLAST using one of the sequences as the query sequence may not identify the second sequence in the top matches. In addition, PSI-BLAST provides an automated, easy-to-use version of a "profile" search, which is a sensitive way to look for sequence homologues. The program first performs a gapped BLAST database search. The PSI- BLAST program uses the information from any significant alignments returned to construct a position-specific score matrix, which replaces the query sequence for the next round of database searching. Therefore, it is to be understood that percent identity can be determined by using any one of these programs.

As used herein, a "fragment" of a polypeptide refers to a single or a plurality of amino acid residues comprising an amino acid sequence that has at least 5 contiguous amino acid residues, at least 10 contiguous amino acid residues, at least 20 contiguous amino acid residues, at least 30 contiguous amino acid residues, at least 40 contiguous amino acid residues, at least 50 contiguous amino acid residues, at least 60 contiguous amino acid residues, at least 70 contiguous amino acid residues, at least 80 contiguous amino acid residues, at least 90 contiguous amino acid residues, or at least 100 contiguous amino acid residues of a sequence of the polypeptide, or any number of residues between 5 and 100, in whole number increments.

As used herein, a polypeptide is referred to as "isolated" when it has been removed from its natural milieu (i.e., that has been subject to human manipulation), and can include purified polypeptides, partially purified polypeptides, synthetically produced polypeptides, and recombinantly produced polypeptides, for example. As such, "isolated" does not reflect the extent to which the polypeptide has been purified.

In some embodiments, a biomarker of the invention is a member of a biological pathway. As used herein, the term "precursor" or "successor" refers to molecules that precede or follow the biomarker. Thus, once a biomarker is identified as a member of one or more biological pathways, the present invention can include additional members of the biological pathway that come before (are upstream of or a precursor of) or follow (are downstream of) the biomarker. Such identification of biological pathways and their members is within the skill of one in the art.

Polypeptide and metabolite markers may be isolated by any suitable method known in the art. Native polypeptide and metabolite markers can be purified from natural sources by standard methods known in the art such as chromatography, centrifugation, differential solubility or immunoassay. In one embodiment, polypeptide and metabolite markers may be isolated from a serum sample using, for example, the chromatographic methods disclosed herein or affinity purification using substrate-bound antibodies that specifically bind to the marker. Metabolite markers may be synthesized using the techniques of organic and inorganic chemistry. Given the amino acid sequence or the corresponding DNA, cDNA, or mRNA that encodes them, polypeptides markers may be synthesized using recombinant or chemical methods. For example, polypeptide markers can be produced by transforming a host cell with a nucleotide sequence encoding the polypeptide marker and cultured under conditions suitable for expression and recovery of the encoded protein from the cell culture. (See, e.g., Hunkapiller *et al*., Nature 310:105-111,1984).

The present disclosure also includes the use as biomarkers of polynucleotides that encode any of the polypeptides identified by the methods disclosed herein or that encode any other polypeptide that can be identified as differentially expressed in subjects with MS using the identification method disclosed herein, or that encode a molecule that comprises such a polypeptide or a polypeptide having substantial homology with a component set forth herein.

In accordance with the present invention, an isolated polynucleotide, or an isolated nucleic acid molecule, is a nucleic acid molecule that has been removed from its natural milieu (i.e., that has been subject to human manipulation), its natural milieu being the genome or chromosome in which the nucleic acid molecule is found in nature. As such, "isolated" does not necessarily reflect the extent to which the polynucleotide has teen purified, but indicates that the molecule does not include an entire genome or an entire chromosome in which the nucleic acid molecule is found in nature. Polyoucleotides useful in the present invention include a portion of a gene (sense or nonsense strand) that is suitable for use as a hybridization probe or FCR primer for the identification of a full-length gene (or portion thereof) in a given sample (e.g., a CSF or serum sample), a gene, or any portion of a gene, as well as a reporter gene.

The minimum size of a polynucleotide of the present invention is a size sufficient to encode a polypeptide having a desired biological activity, sufficient to form a probe or oligonucleotide primer that is capable of forming a stable hybrid with the complementary sequence of a polynucleotide encoding the natural polypeptide, or to otherwise be used as a target in an assay, in a diagnostic assay, or in any therapeutic method discussed herein. The minimum size of a polynucleotide that is-used as an oligonucleotide probe or primer is at least about 5 nucleotides in length, and preferably ranges from about 5 to about 50 or about 500 nucleotides or greater (1000, 2000, etc.), including any length in between, in whole number increments (i.e., 5, 6, 7, 8, 9, 10,...33, 34,...256, 257,...500...1000...). "Hybridization" has the meaning that is well known in the art, that is, the formation of a duplex structure by two single-stranded nucleic acids due to complementary base pairing.

In one embodiment of the present invention, any amino acid sequence described herein can be produced with from at least one, and up to about 20, additional heterologous amino acids flanking each of the C- and/or N-terminal ends of the specified amino acid sequence. The resulting protein or polypeptide can be referred to as "consisting essentially of the specified amino acid sequence. According to the present invention, the heterologous amino acids are a sequence of amino acids that are not naturally found (i.e., not found in nature, *in vivo*) flanking the specified amino acid sequence, or that are not related to the function of the specified amino acid sequence, or that would not be encoded by the nucleotides that flank the naturally occurring nucleic acid sequence encoding the specified amino acid sequence as it occurs in the gene, if such nucleotides in the naturally occurring sequence were translated using standard codon usage for the organism from which the given amino acid sequence is derived. Similarly, the phrase "consisting essentially of", when used with reference to a nucleic acid sequence herein, refers to a nucleic acid sequence encoding a specified amino acid sequence that can be flanked by from at least one, and up to as many as about 60, additional heterologous nucleotides at each of the 5' and/or the 3' end of the nucleic acid sequence encoding the specified amino acid sequence. The heterologous nucleotides are not naturally found (*i.e.,* not found in nature, *in vivo*) flanking the nucleic acid sequence encoding the specified amino acid sequence as it occurs in the natural gene or do not encode a protein that imparts any additional function to the protein or changes the function of the protein having the specified amino acid sequence.

One embodiment of the invention relates to a plurality of polynucleotides for the detection of the expression of biomarkers that are differentially regulated in serum or CSF of subjects with MS. The plurality of polynucleotides consists of, or consists essentially of, at least two polynucleotide probes that are complementary to RNA transcripts, or nucleotides derived therefrom, of at least one polynucleotide, the polypeptide encoded by which has been identified herein as being differentially regulated in the serum or CSF of subjects with MS. As such, the plurality of polynucleotides is distinguished from previously known nucleic acid arrays and primer sets. The plurality of polynucleotides within the above-limitation includes at least two or more polynucleotide probes (e.g., at least 2, 3, 4, 5, 6, and so on, in whole integer increments, up to all of the possible probes) that are complementary to RNA transcripts, or nucleotides derived therefrom, of at least one polynucleotide, and preferably, at least 2 or more polynucleotides, encoding polypeptides identified by the present invention. Such polynucleotides are selected from any of the polynucleotides encoding a polypeptide listed in the tables provided herein and can include any number of polynucleotides, in whole integers (e.g., 1, 2, 3, 4,..) up to all of the polynucleotides represented by a biomarker described herein, or that can be identified in MS subjects using the methods described herein. Multiple probes can also be used to detect the same polynucleotide or to detect different splice variants of the same gene. In one aspect, each of the polynucleotides in the plurality is at least 5 nucleotides in length.

The invention also includes antibodies, or antigen binding fragments thereof, that specifically bind to a polypeptide marker, a metabolite marker or a polynucleotide marker, in particular that bind to a component described herein or any other component that can be identified using the methods described herein. The invention also involves antibodies that specifically bind to a polypeptide having substantial homology with a polypeptide identified herein.

The invention involves antibodies, or antigen binding fragments thereof, that specifically bind to a polypeptide or metabolite of the invention having (i) a mass-to-charge value and (ii) an RT value of about the values stated, respectively, for a marker described herein.

The invention also involves antibodies, or antigen binding fragments thereof, that specifically bind to a component that is a fragment, modification, precursor or successor of a polypeptide or metabolite described herein.

The present invention also involves a plurality of antibodies, or antigen binding fragments thereof, for the detection of biomarkers, the expression of which is differentially regulated in the serum, urine or CSF of subjects as described herein, In addition, the plurality of antibodies, or antigen binding fragments thereof, comprises antibodies, or antigen binding fragments thereof, that selectively bind to a biomarker provided herein.

According to the present invention, a plurality of antibodies, or antigen binding fragments thereof, refers to at least 2, at least 3, at least 4, at least 5, at least 6, at least 7, at least 8, at least 9, at least 10, and so on, in increments of one, up to any suitable number of antibodies, or antigen binding fragments thereof, including antibodies representing all of the biomarkers described herein.

According to the present invention, the phrase "selectively binds to" refers to the ability of an antibody or antigen binding fragment thereof to preferentially bind to specified proteins. More specifically, the phrase "selectively binds" refers to the specific binding of one protein to another (e.g., an antibody or antigen binding fragment thereof to an antigen), wherein the level of binding, as measured by any standard assay (e.g., an immunoassay), is statistically significantly higher than the background control for the assay. For example, when performing an immunoassay, controls typically include a reaction well/tube that contain antibody or antigen binding fragment alone (i.e., in the absence of antigen), wherein an amount of reactivity (e.g., non-specific binding to the well) by the antibody or antigen binding fragment thereof in the absence of the antigen is considered to be background. Binding can be measured using a variety of methods standard in the art including enzyme immunoassays (e.g., ELISA), immunoblot assays, etc.).

As used herein, the term "specifically binding," refers to the interaction between binding pairs such as an antibody and an antigen with an affinity constant of at most $10^{-6}$ moles/liter, at most $10^{-7}$ moles/liter, or at most $10^{-8}$ moles/liter.

Limited digestion of an immunoglobulin with a protease may produce two fragments. An antigen binding Segment is referred to as an Fab, an Fab', or an $F(ab')_2$ fragment. A fragment lacking the ability to bind to antigen is referred to as an Fc fragment. An Fab fragment comprises one arm of an immunoglobulin molecule containing a L chain ($V_L + C_L$ domains) paired with the $V_H$ region and a portion of the $C_H$ region (CH1 comain). An Fab' fragment corresponds to an Fab fragment with part of the hinge region attached to the CH1 domain. An $F(ab')_2$ fragment corresponds to two Fab' fragments that are normally covatently linked to each other through a di-sulfide bond, typically in the hinge regions.

Isolated antibodies of the present invention can include serum containing such antibodies, or antibodies that have been purified to varying degrees. Whole antibodies of the present invention can be polyclonal or monoclonal. Alternatively, functional equivalents of whole antibodies, such as antigen binding fragments in which one or more antibody domains are truncated or absent (e.g., Fv, Fab, Fab', or $F(ab)_2$ fragments), as well as genetically- engineered antibodies or antigen binding fragments thereof, including single chain antibodies or entibodies that can bind to more than one epitope (e.g., bi-specific antibodies), or antibodies that can bind to one or more different antigens (e.g., bi-or multi-specific antibodies), may also be employed in the invention.

Binding partners i (e.g., antibodies and antigen binding fragments thereof, or other peptides) useful in any embodiment of the present invention may be conjugated to detectable markers. Detectable labels suitable for use in the present invention include any composition detectable by spectroscopic, photochemical, biochemical, immunochemical, electrical, optical or chemical means, examples of which have been described above. Useful labels in the present invention include biotin for staining with labeled streptavidin conjugate, magnetic beads (e.g., Dynabeads™), fluorescent dyes (e.g., cyanine, tandem, fluorescein, texas red, rhodamine, green fluorescent protein, yellow fluorescent protein and the like), radiolabels (e.g., $^3H$, $^{125}I$. $^{35}S$, $^{14}C$, or $^{32}P$), enzymes (e.g., horse radish peroxidase, alkaline phosphatase and others commonly used in an ELISA), and colorimetric labels such as colloidal gold or colored glass or plastic (e.g., polystyrene, polypropylene, latex, etc.) beads.

As used herein, detecting a biomarker of the present invention can also include detecting transcription of the gene encoding a biomarker protein and/or to detecting translation of the biomarker protein. To detect a biomarker refers to the act of actively determining the level of and/or expression of a biomarker. This can include determining whether the biomarker expression is upregulated as compared to a control, downregulated as compared to a control, or substantially unchanged as compared to a control. Therefore, the step of detecting expression does not require that expression of the biomarker actually is upregulated or downregulated, but rather, can also include detecting no expression of the biomarker or detecting that the expression of the biomarker has not changed or is not different (i.e., detecting no significant expression of the biomarker or no significant change in expression of the biomarker as compared to a control).

The present disclosure includes administering the test composition to a human subject.

Routes of administration are known in the art, and include intravenous, intraperitoneal, subcutaneous, intramuscular, intragastric, intranasal, intratracheal, inhalational, intracerebral, and dermal routes of administration. A preferred route of administration is intramuscular. Amounts of the composition to administer can be determined by one of skill in the art, and include clinically acceptable amounts to administer based on the specific activity of the composition and its pharmacodynamic profile. For example, AVONEX® is typically administered at 30 microgram once a week via intramuscular injection. Other forms of interferon beta Ia, specifically REB IF®, is administered, for example, at 22 microgram three times a week or 44 micrograms once a week, via subcutaneous injection.

Where the composition is AVONEX® (Interferon beta Ia), the transient change in the biomarker preferably occurs within 12 to 48 hours, most preferably at about 34 hours post administration of the composition. For other compositions, the time period for a "transient" change can be determined by one of skill in the art, either empirically and/or by prediction based on the pharmacodynamic profile (including, for example, half life and/or specific activity) of the composition. A "transient" change can be defined in multiple ways. For example, a transient change can refer to differences that appear "short-term" post-injection but have substantially returned to previous levels (pre-injection levels) on a longer term basis (a "baseline" level). A transient change may occur in Naïve subjects and may also occur in subjects previously treated with a composition upon each new treatment or injection of the composition. A baseline level includes both levels of a biomarker that appear prior to any treatments with a given composition or levels that occur post treatments. Levels of a biomarker posttreatment that are used as a "baseline" level are preferably determined during a time period where levels of that biomarker are relatively stable, i.e., do not change in a substantive or significant manner. In a preferred embodiment, a transient change is a difference that appears and is detectable at 34 hours post injection but is no longer apparent and therefore is not detectable at 6 days post injection. Preferably, a transient change of the invention is reversed at about 6 days after administration of the test composition. Appropriate time periods for measurement of transient changes can be determined by one of skill in the art, and can include determining appropriate time periods for measurement of "baseline" levels and appropriate time periods for measurement of "transient" levels. For non-human subjects and animal models, appropriate time periods for measurement of transient changes can be determined by one of skill in the art.

A preferred biomarker of the present invention includes a monocyte-associated variable. A monocyte-associated variable can refer to a monocyte count, a monocyte/leukocyte ratio, an individual antigen occurring on monocytes, or a soluble factor such as cytokines or metabolites which are associated with monocytes and/or biologically related to monocytes. Generally, for monocyte-associated variable which is an individual antigen or a soluble factor, such as cytokines or metabolites which are associated with monocytes, the most useful variables are ones whose changes are not merely directly proportional to the number of monocytes, rather, the changes are larger than what could be attributed to the change in the monocyte parent population, i.e., are upregulated or downregulated.

Monocytes may be quantitated by any methods known in the art. In a preferred method, monocytes may be quantitated by absolute count (cells/$\mu$l blood). Other appropriate methods to quantitate monocytes or any other cell population is to measure a variable that tracks with the cell population, e.g., expression and/or intensity of individual antigens or soluble factors identified by immunoassay and/or mass spectrometry that are biologically related to and therefore proportional to the cell population. In preferred embodiments, the magnitude of the change as measured by any method is at least about 10%, at least about 15%, at least about 20%, at least about 23%, at least about 25%, at least about 30%, at least about 35%, and at least about 38%. In other preferred embodiments, the magnitude of the change as measured by any method has an effect size (difference of means divided by the weighted standard deviation) of at least about 0.2, at least about 0.4, at least about 0.6, at least about 0.8, at least about 1, and at least about 1.2.

Another monocyte-associated variable includes a ratio of monocytes to total number of white blood cells counted, or any subset of white blood cells. In a preferred embodiment, a preferred ratio is the monocyte/leukocyte ratio. Cell populations, e.g., monocytes, leukocytes, and white blood cells can all be quantitated by methods known in the art and as discussed above. The present inventors have found that total leukocytes decrease about 20% post dosing transiently for all cohorts in the study, specifically, that neutrophils decrease about 19-33%, B cells decrease about 22-26%, CD4 T-cells decrease about 8-12%, and CD8 T cells decrease about 12-16%. In light of the transient decrease in total leukocytes and the transient increase in monocytes, the monocyte/leukocyte ratio generally has increased changes and increased effect size compared to absolute monocyte counts. In preferred embodiments, the magnitude of the change in the monocyte/leukocyte ratio is an increase and is at least about 20%, at least about 30%, at least about 40%, at least about 50%, at least about 55%, at least about 60%, at least about 65%, and even more preferably at least about 70% and at least about 75%, and most preferably between about 50% and about 75%. In other preferred embodiments, the magnitude of the change in the monocyte/leukocyte ratio has an effect size of at least about 0.7, at least about 1, at least about 1.2, at least about 1.4, at least about 1.5, and more preferably at least about 1.7, at least about 2, at least about 2.2, and at least about 2.5.

Another monocyte-associated variable includes monocyte expression of HLA Class II molecules. In preferred embodiments, the magnitude of the change is an increase and is at least about 10%, at least about 20%, at least about 30%, at least about 40%, at least about 50%, at least about 55%, at least about 60%, at least about 65%, and even more preferably at least about 70% and at least about 75%, and most preferably between about 50% and about 75%. A preferred change is an increase, which is at least about 30% and preferably between about 30% and about 120%. Preferred HLA Class II molecules include HLA-DP, DR, DQ or PAN. A preferred effect size is at least about 0.7, at least about 0.8, at least about 0.9, at least about 1, at least about 1.1, at least about 1.2, at least about 1.3, at least about 1.4, at least about 1.5, at least about 1.6, at least about 1.7, at least about 1.8, at least about 1.9, at least about 2, at least about 2.1, at least about 2.2, at least about 23, at least about 2.4, at least about 2.5, at least about 2.6, and at least about 2.8. Other preferred monocyte-associated variables include CCR5, CDIIb, CD38, CD40, CD54, CD64, CD69, CD86, TLR2, or TLR4; and MCP2 (monocyte chemoattractant protein 2). In preferred embodiments, the magnitude of the change is an increase and is at least about 10%, at least about 20%, at least about 30%, at least about 40%, at least about 50%, at least about 55%, at least about 60%, at least about 65%, and even more preferably at least about 70% and at least about 75%, and most preferably between about 50% and about 75%. A preferred change is an increase, which is at least about 30% and preferably between about 30% and about 120%. Preferred effect sizes of expression of any these monocyte-associated variables is preferably at least about 0.1, at least about 0.2, at least about 0.3, at least about 0.4, at least about 0.4, at least about 0.5, at least about 0.6, at least about 0.7, at least about 0.8, at least about 0.9, at least about 1, at least about 1.1, at least about 1.2, at least about 13, at least about 1.4, at least about 1.5, at least about 1.6, at least about 1.7, at least about 1.8, at least about 1.9, at least about 2, at least about 2.1, at least about 2.2, at least about 2.3, at least about 2.4, at least about 2.5, at least about 2.6, and at least about 2.8. The values for the change in any biomarker of the present invention may be combined or related with values for any other biomarker and/or otherwise mathematically manipulated for use in methods of the present invention.

Expression of genes/transcripts and/or proteins encoded by the genes represented by biomarkers of the invention is measured by any of a variety of known methods in the art. In general, expression of a nucleic acid molecule (e.g., DNA or RNA) can be detected by any suitable method or technique of measuring or detecting gene or polynucleotide sequence or expression. Such methods include, but are not limited to, polymerase chain reaction (PCR), reverse transcriptase-PCR (RT-PCR), in situ PCR, quantitative PCR (q-PCR), in situ hybridization, Southern blot, Northern blot, sequence analysis, microarray analysis, detection of a reporter gene, or other DNA/RNA hybridization platforms. For RNA expression, preferred methods include, but are not limited to: extraction of cellular mRNA and Northern blotting using labeled probes that hybridize to transcripts encoding all or part of one or more of the genes of this invention; amplification ofmRNA expressed from one or more of the genes represented by biomarkers of this invention using gene-specific primers, polymerase chain reaction (PCR)5 quantitative PCR (q-PCR), and reverse transcriptase-polymerase chain reaction (RT-PCR), followed by quantitative detection of the product by any of a variety of means; extraction of total RNA from the cells, which is then labeled and used to probe cDNAs or oligonucleotides encoding all or part of the genes of this invention, arrayed on any of a variety of surfaces; in situ hybridization, and detection of a reporter gene. The term "quantifying" or "quantitating" when used in the context of quantifying transcription levels of a gene can refer to absolute or to relative quantification. Absolute quantification may be accomplished by inclusion of known concentration (s) of one or more target nucleic acids and referencing the hybridization intensity of unknowns with the known target nucleic acids (e.g. through generation of a standard curve). Alternatively, relative quantification can be accomplished by comparison of hybridization signals between two or more genes, or between two or more treatments to quantify the changes in hybridization intensity and, by implication, transcription level.

Methods to measure biomarkers of this invention, include, but are not limited to: Western blot, immunoblot, enzyme-linked immunosorbant assay (ELISA), radioimmunoassay (RIA), immunoprecipitation, surface plasmon resonance, chemiluminescence, fluorescent polarization, phosphorescence, immunohistochemical analysis, liquid chromatography mass spectrometry (LC-MS), matrix-assisted laser desorption/ionization time-of-flight (MALDI- TOF) mass spectrometry, microcytometry, microarray, microscopy, fluorescence activated cell sorting (FACS), flow cytometry, laser scanning cytometry, hematology analyzer and assays based on a property of the protein including but not limited to DNA binding, ligand binding, or interaction with other protein partners.

The present invention provides a method to monitor treatment of a human subject for multiple sclerosis as specified in the claims. This method includes the steps of administering an IFN beta therapeutic composition to the subject, and evaluating whether administration of the therapeutic composition causes a transient change in monocyte-associated variable biomarker. Alternatively, this method can include evaluating whether the magnitude of a transient change decreases over time. In this embodiment, a decrease in the magnitude of a transient change can be predictive of a relapse. Such a change can be caused, for example, by the development of neutralizing antibodies to the therapeutic composition. A transient change in the biomarker indicates efficacious treatment of multiple sclerosis.

A preferred transient change is a transient change that is substantially similar to a transient change that is observed for any of the therapeutically acceptable forms of interferon beta at dosages which are clinically appropriate. A preferred transient change would be, for example, at least one transient change that is substantially similar to at least one transient change that is observed upon administration of AVONEX® at 30 micrograms once a week (via intramuscular injection). Such a preferred transient change predicts or indicates efficacious treatment of multiple sclerosis. More specifically, a preferred transient change is a transient change that is substantially similar to at least one transient change that is associated with subjects experiencing efficacious treatment of multiple sclerosis and/or relapsing forms of multiple sclerosis. A preferred transient change, which indicates or predicts efficacious treatment of MS, preferably includes a transient change where at least one biomarker is within about 50%, within about 60%, within about 70%, within about 75%, within about 80%, within about 85%, within about 90%, and more preferably within about 95% of a transient change identified herein. In alternative embodiments, the transient change is evaluated for at least two biomarkers, at least three biomarkers, at least four biomarkers, and at least five or more biomarkers. Preferred transient changes for any of the biomarkers according to this embodiment include effect sizes of expression of any these biomarkers of preferably at least about 0.1, at least about 0.2, at least about 0.3, at least about 0.4, at least about 0.5, at least about 0.6, at least about 0.7, at least about 0.8, at least about 0.9, at least about 1, at least about 1.1, at least about 1.2, at least about 1.3, at least about 1.4, at least about 1.5, at least about 1.6, at least about 1.7, at least about 1.8, at least about 1.9, at least about 2, at least about 2.1, at least about 2.2, at least about 2.3, at least about 2.4, at least about 2.5, at least about 2.6, and at least about 2.8. In preferred embodiments, the magnitude of the change is an increase and is at least about 10%, at least about 20%, at least about 30%, at least about 40%, at least about 50%, at least about 55%, at least about 60%, at least about 65%, and even more preferably at least about 70% and at least about 75%, and preferably between about 50% and about 75%. A preferred change is an increase, which is at least about 30% and most preferably between about 30% and about 120%.

A preferred transient change may be a transient change or transient changes that have been identified, e.g., identified herein for AVONEX®. Additionally, a preferred transient change may be chosen by *de novo* quantitation of the magnitude of response of the herein described biomarkers for a particular composition. Such *de novo* quantitation is useful for, for example, different formulations of compositions such as interferon beta formulations, variants, homologues of the present invention which have or could be predicted to have differing pharmacodynamic profiles from AVONEX®.

The present disclosure also, provides assay kits that are suitable for the performance of any method described herein and/or the detection of any of the biomarkers that are described herein. The assay kit preferably contains at least one reagent that is suitable for detecting the expression or activity of a biomarker of the present invention in a test sample (e.g., serum or CSF), and preferably includes a probe, PCR primers, an antibody or antigen binding fragment thereof, peptides, binding partners, aptamers, enzymes, enzyme substrates and small molecules that bind to or otherwise identify a biomarker of the invention. The kit can include any reagent needed to perform a diagnostic method envisioned herein or to perform a target-based assay envisioned herein. The kit can contain a means for detecting a control marker characteristic of a cell type in the test sample. The kit can also include suitable reagents for the detection of and/or for the labeling of positive or negative controls, wash solutions, dilution buffers and the like. The kit can also include a set of written instructions for using the kit and interpreting the results.

The means for detecting of the assay kit of the present invention can be conjugated to a detectable tag or detectable label. Such a tag can be any suitable tag which allows for detection of the reagents used to detect the gene or protein of interest and includes, but is not limited to, any composition or label detectable by spectroscopic, photochemical, electrical, optical or chemical means.

In addition, the means for detecting of the assay kit of the present disclosure can be immobilized on a substrate. Such a substrate can include any suitable substrate for immobilization of a detection reagent such as would be used in any of the previously described methods of detection. Briefly, a substrate suitable for immobilization of a means for detecting includes any solid support, such as any solid organic, biopolymer or inorganic support that can form a bond with the means for detecting without significantly affecting the activity and/or ability of the detection means to detect the desired target molecule. Exemplary organic solid supports include polymers such as polystyrene, nylon phenol-formaldehyde resins, and acrylic copolymers (e.g., polyacrylemide).

The following examples are provided for the purpose of illustration and are not intended to limit the scope of the present invention.

## Examples

### Example 1. Methods

### STUDY DESIGN

The design was multi-center, open-label, assessor blinded and prospective. Four groups of subjects were included.

- HEALTHY (H): Healthy individuals without MS or acute systemic disease.
- NAÏVE (N): MS patients previously naive to interferon therapy, who will commence AVONEX® treatment in this study. Subjects were diagnosed with MS within six months prior to starting the study.
- STABLE (S): MS patients on AVONEX® treatment for at least 18 months with no history of breakthrough disease (clinically stable) as evidenced by no relapses for one year.
- BREAKTHROUGH (B): MS patients on AVONEX® treatment for at least 12 months with a history of breakthrough disease as evidenced by two relapses in the last 15 months or one relapse in the last 15 months and EDSS progression.

Treatment and sample collection scheme

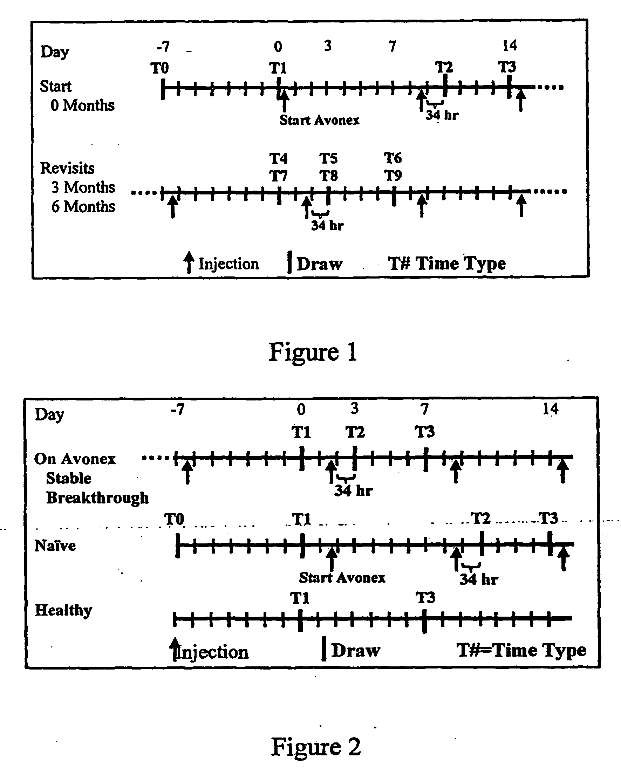

The treatment and sample collection scheme is shown in Figure 1. During this study, MS subjects continued to receive their regular doses of AVONEX®, once weekly, or began AVONEX®, once weekly as indicated by the arrows. Sample collection includes: a) two baseline samples, in the absence of drug for Healthy and Naive subjects, b) two steady-state samples, 6 days post drug for Stable, Breakthrough groups, c) one sample subjects short-term post treatment, 34 hours after injection for Naive, Stable and Breakthrough subjects and d) one sample 6 days post drug for Naive subjects.

Some of the Naive subjects returned for additional visits at three and six months after beginning AVONEX®, treatment. The sample collection scheme for these subjects is shown in **Figure 2**

**Sample collection, demographics and MS history**

The original goal of enrolling 35 subjects per cohort was met for the Healthy, Naive and Stable groups. Only 16 subjects were enrolled for the Breakthrough group. The number of subjects and samples by cohort is detailed in Table 1. It should be noted that two subjects who were originally enrolled as Stable subjects, later met the criteria for Breakthrough subjects and were re-enrolled. Among Naive subjects, ten returned for three-month visits and seven for the sixth-month visits. Overall the study included 413 subject visits.

**Table 1: Subjects and Sample Collection by Cohort**

| Group | Subjects | Samples by Time Type | | | | | | |
| | | Main Study | | | | Naïve Revisit | | |
| | | T0 | T1 | T2 | T3 | T4-6 | T7-9 | Total |
|---|---|---|---|---|---|---|---|---|
| *Healthy* | 35 | | 35 | | 35 | | | 70 |
| **Naïve** | 35 | 34 | 35 | 35 | 35 | 10x3 | 7x3 | 190 |
| **Stable** | 35 | | 35 | 35 | 35 | | | 105 |
| **Breakthrough** | 16 | | 16 | 16 | 16 | | | 48 |
| **Total** | 121 | 34 | 121 | 86 | 121 | 30 | 21 | 413 |

Demographic information for the subjects is summarized in Table 2. Like many autoimmune disorders, MS disproportionately affects women with a ratio of women to men of about 2 to 1. The gender representation for cohorts ranged from 74 to 88% with the Breakthrough group being the highest and the Healthy group the lowest. Mean ages for the groups range from 37 to 47 years

**TABLE 2: Cohorts by Age and gender**

| | Healthy | Naïve | Stable | Breakthrough |
|---|---|---|---|---|
| **N** | 35 | 35 | 35 | 16 |
| **Gender (%F)** | 74 | 83 | 80 | 88 |
| **Age*** | 37 (12) | 41 (8) | 47(9) | 38 (9) |
| * Mean (SD) | | | | |

A summary of MS history is provided in **Table 3.** Relapse history was consistent with the enrollment criteria, with the mean number of relapses for Breakthrough > Naive > Stable at one and three years. Similarly, the mean time since last relapse was much greater for Stable subjects compared with Breakthrough and Naïve subjects. The Naive group had the shortest time since onset of disease, which is also consistent with the enrollment criteria.

**Table 3: Multiple Sclerosis Cohorts Disease History**

| | Naïve | Stable | Breakthrough |
|---|---|---|---|
| **Relapses-1 year*** | 1.3 (0.6) | 0.1 (0.4) | 2.1 (1.1) |
| **Relapses-3 years*** | 1.7 (0.9) | 0.6 (0.9) | 3.9 (2.8) |
| **Time since onset (Yr)*** | 5.0 (6.4) | 11.6 (6.4) | 10 (5.6) |

(continued)

|  | Naïve | Stable | Breakthrough |
|---|---|---|---|
| Time since last relapse (Mo)* | 6.4 (11) | 52 (30) | 7.5 (16) |
| * Mean (SD) | | | |

## ANALYTICAL METHODS

Analytical methods enable a broad molecular phenotyping of cells, soluble proteins and small molecules in subject sample (Table 4). The integrated platform enables both hypothesis-based and discovery-based strategies (55, 57). For the hypothesis-based strategies, the analytes are known *a priori.* Here we used antibody-based assays for analysis of cells and soluble factors. The discovery-based approaches do not require *a prior* knowledge about the analytes. Differential proteomic profiling with mass spectrometry was used to identify differences between study groups. Identification of the differences was done *post hoc.*

**TABLE 4: Summary of analytical methods**

| <u>Hypothesis-based</u> | <u>Discovery-based</u> |
|---|---|
| • Analytes known *a priori* | • No *a priori* knowledge needed |
| • Specific | • Differential profiling |
| • Sensitive | • Less sensitive |
| • Antibody based | • Mass Spectrometry based |
| | |
| • Cellular Assays on SurroScan™ | • Proteomics |
|     • Cell Surface (64) |     • Serum - Basic |
|     • Stimulation and intracellular staining (32) |     • Serum - DeepLook |
| • Immunoassays |     • Urine |
|     • Serum assays (50) | • Metabolomics |
|     • Blood cell preparation - MXA |     • Serum |
| |     • Urine |

## Cellular Assays

The protocol included 64 three-color cell surface assays performed by microvolume laser scanning cytometry (MLSC) on SurroScan™ system (SurroMed/PPD Biomarker) (34, 56, 121). The assays are well suited for evaluating immune and inflammatory processes. Monoclonal antigen-specific antibodies were purchased from various commercial vendors and developed into cellular assays. Three different fluorophores, Cy5, Cy5.5 (78, 105) and the tandem dye Cy7-APC (9, 93), were coupled to individual monoclonal antibodies specific for different cellular antigens in each assay. Each fluorophore is measured in a separate detection channel. The antibody-dye reagents were combined into pre-made cocktails and all assays were homogeneous, without removal of unreacted antibody reagents. Aliquots of whole or red blood cell-lysed blood from EDTA collection tubes were added to 96-well micro-titer plates containing the appropriate reagent cocktails, incubated in the dark at room temperature for 20 minutes, diluted with an appropriate buffer and loaded into Flex32™ capillary arrays (SurroMed/PPD Biomarker) and analyzed with SurroScan™. Images were converted to a list-mode data format with in-house software (80). Fluorescence intensities were compensated for spectral overlap of the dyes so values are proportional to antigen density.

The protocol also included a panel of 32 stimulation assays with intracellular staining for specific cytokines. Peripheral blood mononuclear cells (PBMC) were prepared by the Ficoll method from blood collected in Heparin tubes. This method removes any drug present in the blood. Cells were cultured in three conditions 1) no stimulation control, 2) stimulation with Phorbol 12-myristate 13-acetate (PMA), a protein kinase C activator, and ionomycin, a calcium ionophore for lymphocytes and 3) stimulation with lipopolysaccharide (LPS-E) for monocytes. Cytokine secretion was stopped with monensin to enable intracellular staining for cytokines. There were multiple steps and washes to the method, which yields ratios of cell populations.

We developed and established quality and baseline measures with twenty blood bank samples for the 96 different three-color cellular assays used in this study. Standard template gates were established using these results plus additional staining controls for all individual reagents and two-color combinations. Template gates were established using FlowJo™ cytometry analysis software (Tree Star, Inc., Ashland, OR) customized for PPD Biomarker/SurroMed to enable upload of gates to our Oracle database. Gating information was stored in the database and applied to the scan data for each assay using SurroGate™ database-driven cytometry analysis software in order to generate the resulting cell count and antigen intensity data.

The assay panel allowed the enumeration of major cell populations: granulocytes, eosinophils, monocytes, CD4 and CD8 T cells, B cells, and NK cells. A summary of the target antigens for each of the major cell populations is provided in Table 5. It allows finer phenotyping of cell types based on the expression of the activation antigens, co-stimulatory molecules, adhesion molecules, antigen receptors, cytokine receptors, etc. These assays monitor cell counts of more than 200 different cell populations, plus the relative levels of the different cell surface antigens on specific populations. The specific assays, including representative plots and descriptive statistical results from the blood bank samples, are included in Assay Baseline Results.

*Table 5: Target Antigens for Cellular Assays*

| Major Cells | Major Marker | Subsetting Antigen |
|---|---|---|
| *T Cells* | All: CD2, CD3, CD5 Helper: CD4 T Cytotoxic: CD8 T | CCR5, CD6, CD7, CD11b, CD25, CD26, CD27, CD28, CD29, CD38, CD44 CD45RA, CD49d, CD54, CD56, CD57, CD60, CD62L, CD69, CD71, CD86, CD89, CD94, CD95, CD101, CD127, CD134, CD150, TCR $\alpha\beta$, TCR$\gamma\delta$, HLA-DR, NKB1, IL2, IL 4, IL10, IFN$\gamma$, TNF$\alpha$ |
| **B Cells** | CD19, CD20 | CCR5, CD5, CD25, CD38, CD40, CD44, CD54, CD62L, CD69, CD71, CD72, CD80, CD86, CD95, HLA-DP, DQ, DR, PAN, TNF$\alpha$ |
| **NK Cells** | CD56, NKB1 | CD2, CD7, CD8, CD26, CD57, CD94 |
| *Granulocytes* **Eosinophils** | CD15, CD16 | CD11b, CD32, CD45, CD64, CD66b, CD89, CD101, CD119, HLA-DR |
| **Monocytes** | CD14 | CCR5, CD4, CD11b, CD25, CD32, CD33, CD38, CD40, CD44, CD45, CD54, CD62L, CD64, CD69, CD71, CD80, CD86, CD89, CD101, CD119, HLA-DP, DQ, DR, PAN, TLR2, TLR4, IL10, TNF$\alpha$ |
| **Platelets, etc.** | CD41a; CD45 | CD62P; HLA-ABC |

Cytometry variables. Our hypothesis tests included 1949 variables from cell-counts and cell surface antigen intensities. Multiple measures of the same cell population (e.g., CD4 T cells) were combined into a single average for the analysis. Variables for this reduced variable list are designated as S 1 in the cytometry result tables. Overall results are reported for 784 S1 and 1165 S2 variables. The S2 variables are informative, but may be redundant with S1 variables.

**Cytometry data collection.**

Cytometry results are reported for the full set of 413 samples (Table 1). This includes the 362 samples from the main study and 51 samples from Naive subjects who returned at three and six months. Template gates were used to enumerate the cell populations of interest in all of the assays. Invalid assays and those that do not support the template gates were flagged. An analyst visually reviewed all assay results prior to data upload. In this study 413 subject samples were analyzed with 96 assays (64 cell surface and 32 stimulations) for a total 39,648 assays as specified in the work plan **(Table 6).**

**Table 6: Cellular assay data**

| | Assays per Sample | Assay X Sample | Invalid | Non Standard Gates |
|---|---|---|---|---|
| **Cell Surface assays** | 64 | 26,432 | 1.04% | 6.3% |
| **Stimulation/Intracellular assays** | 32 | 13,216 | 1.66% | 19.4% |
| **Total** | 96 | 39,648 | 1.25% | 10.67% |

Among the assays 1.3% were invalid due to technical difficulties and were excluded from the statistical analysis. An additional 11% required non-standard gates. These results were used in the statistical analysis. Cell counts from non-standard gates are generally not affected, but cell surface expression results may have a larger variation.

Cytometry data analysis

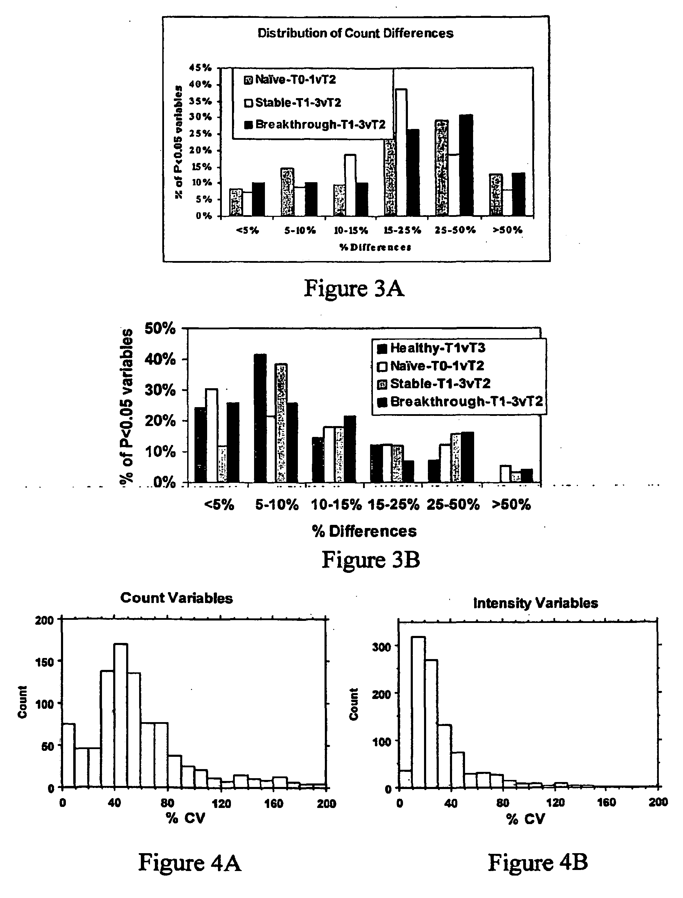

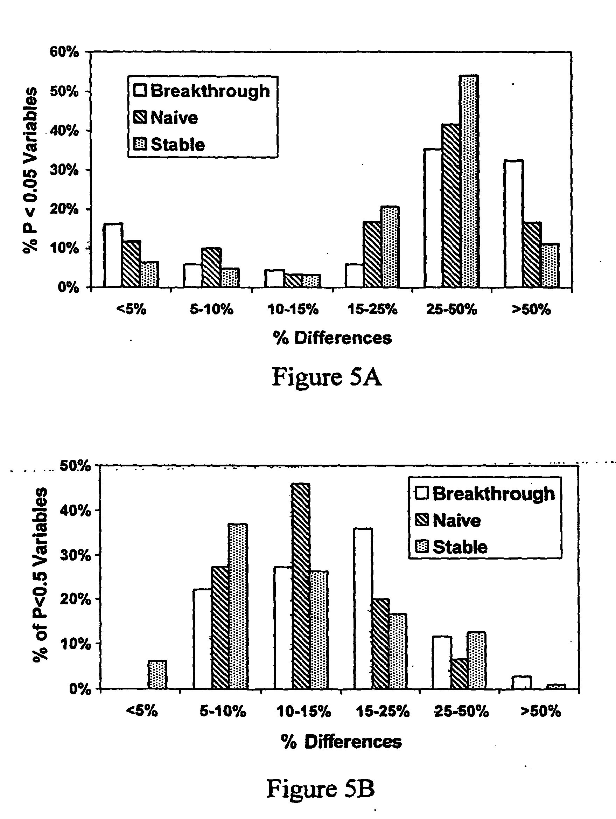

Comparative statistics were used to evaluate differences among subject samples (See Statistical method below). It was informative to consider the magnitude of the difference observed in the comparisons and to separate these for cell count and intensity measures. The distribution of differences of means was plotted for all variables with a p-value < 0.5 in the given comparisons in Figures 3 and 5. For within group comparisons, all samples that were being compared in paired tests were collected within two weeks of each other. Long-term aspects of the collection were of major concern. The p-value distributions for counts and intensities show different shapes. Counts are more approximately normally distributed with a peak in the 15-25% range. Intensities are skewed to the lower end of differences (<10%). Median CVs were 2-fold lower for intensities (24%) than counts (50%). Distributions are shown in Figure 4. This is one reason that small intensity differences come up on the p-value hit list.

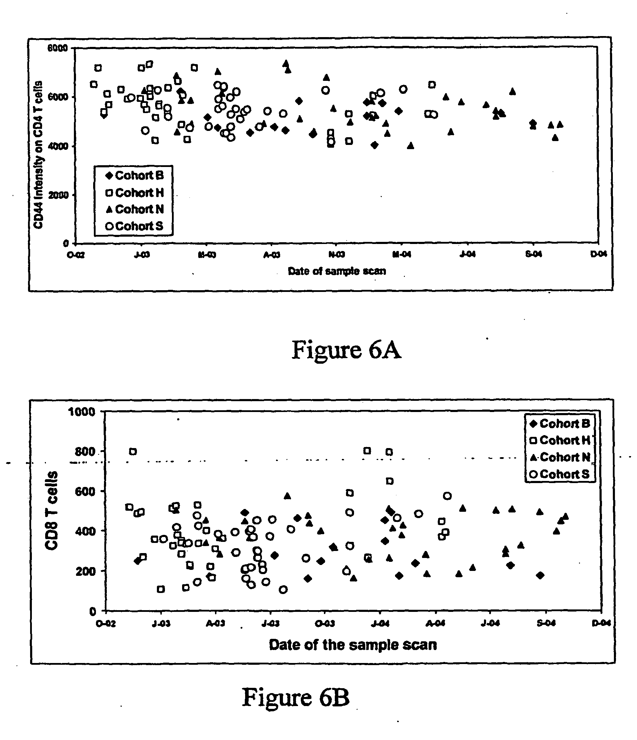

Collection of samples for the different cohorts varied substantially over the course of the study. Healthy and Stable subjects were collected early with a predominant set for the PAL site. Completion of the Naïve subjects was much later. For between group comparisons, cell count p-value distributions show an approximately normal distribution with a peak in the 25-50% range. Intensities p-value distributions show an approximately normal distribution with a peak in the 10-20% range (Figure 5). This is higher than for the within group comparisons. In general counts do not drift over time in this study, whereas intensities drifted several percent. Examples of changes in counts and intensities over time are shown in Figure 6 for CD8 T cell counts and CD44 intensity on CD4 T cells. In considering p-value variable hits for cytometry it important to consider the specific comparison and the magnitude of the difference.

**Immunoassays**

MXA whole blood assay. Intracellular MxA expression is exclusively induced by type I interferons and viruses. It is induced to high levels with IFN treatment and can be monitored in whole blood (83). Whole blood was processed within six hours of collection. Two preparation methods were used since the selection of antibody reagents for the immunoassay was not determined at the start of the protocol: 1) NP-40 lysis and gentle protein denaturation to preserve non-linear epitopes and 2) SDS/Urea lysis and harsh protein denaturation to maximize inhibition of endogenous enzymes (62, 82, 113). A select group of samples were sent to Andrew Pachner at UMDNJ, Newark, NJ. Sample measurements were analyzed using the NP-40 preparations.

The sample set for the MXA assay was different from all other platforms (Table 7). A total of 245 samples were analyzed. The assay was not run on the Healthy group. Repeat samples were only run for a subset from the Naive, Stable and Breakthrough Groups. Samples were also run for returning Naive subjects, without the repeat time points (T5, T6, T8, T9).

**Table 7: Samples for MXA assay**

|  | Stable | Breakthrough | Naïve | Total |
|---|---|---|---|---|
| T0 | NA | NA | 10 | 10 |
| T1 | 10 | 10 | 10 | 30 |
| T2 | 34 | 16 | 35 | 85 |
| T3 | 35 | 16 | 35 | 86 |
| T5 |  |  | 10 | 10 |
| T6 |  |  | 10 | 10 |
| T8 |  |  | 7 | 7 |
| T9 |  |  | 7 | 7 |
| Total | 79 | 42 | 124 | 245 |

Serum Immunoassays. A panel of 50 serum immunoassays was measured for the study samples (Table 8). Serum aliquots were prepared within six hours of sample collection. Levels of analytes were measured using Search-Light® Proteomic Arrays by the vendor (Pierce Biotechnology, Woburn, MA). This multiplexed mini-array system uses sandwich-type ELISA with chemiluminescent readout in a 96-well microtiter plate format (77). Analytes were grouped by serum dilution and compatibility, with up to 12 different capture antibodies spotted per well. The bound proteins were detected with a biotinylated detection antibody, followed by the addition of streptavidin-horseradish peroxidase and chemiluminescent substrate (30). Luminescent signal intensities were recorded with a cooled CCD camera using whole plate imaging. Signal is proportional to the amount of each analyte present in the original standard or sample. Concentrations are extrapolated from a standard curves and results were subjected for statistical analysis.

**Table 8: Serum Immunoassays**

| Panel 1 | Panel 2 | Panel 3 | Panel 4 | Panel 5 |
|---|---|---|---|---|
| TIMP1 | PAI1-total | IL1a | LIF | CD14 |
| TIMP2 | VCAM | IL1b | IP-10 | CD40L |
| CRP | IL6R | IL2 | MCP1 | NT-pBNP |
| E-Selectin | MMP2 | IL4 | MCP2 | PAI-1act |
| ICAM1 | BDNF | IL6 | MCP4 | MMP1 |
| L-Selectin | MMP9 | IL8 | MIG | MMP3 |
| RANTES | CNTF | IL10 | Mip1a | MMP8 |
| | IFNa | IL12p40 | Mip1b | MMP10 |
| | IFNg | IL12p70 | IL1Ra | MMP13 |
| | | IL15 | IL2R | NT3 |
| | | IL18 | β-NGF | OPN |
| | | TNFa | | |

The sample set for the serum immunoassays were the main study samples from all four cohorts for a total of 362.

**Mass Spectrometry**

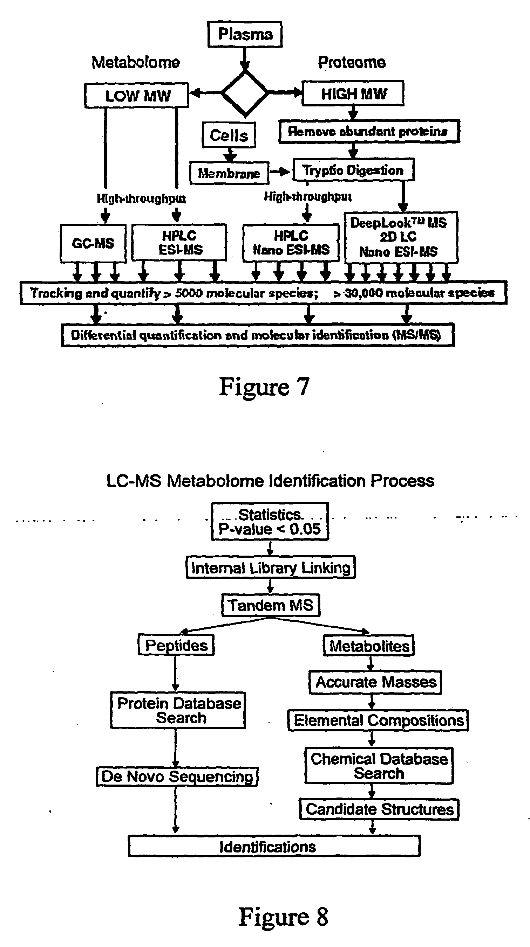

The serum samples were subjected to mass spectrometric analysis for differential expression of proteins and metabolites, and identification of components in the fluid (10, 94, 95, 122, 130). For each sample, the material was analyzed for low and high molecular weight components. First the biological fluid was separated based on molecular weight range. A schematic diagram of the processing is shown in Figure 7. Molecules were tracked and quantified molecules for their differential expression.

**Proteome**

The proteomic, high-molecular-weight (HMW) fraction has the six most abundant proteins (albumin, IgG, IgA, haptoglobin, transferrin and antitrypsin) substantially depleted by an affinity resin to increase the effective dynamic range of the measurements. The remaining proteins were denatured, disulfide bonds reduced, and sulfhydryl groups carboxymethylated prior to digestion by modified trypsin. During this process, low molecular weight molecules were excluded during a buffer exchange step with a 5-kDa cut-off filter.

For part of this study an abbreviated form of the DeepLook™ analysis methodology was employed for serum samples where three fractions of peptides were obtained by off-line (fraction collected) strong-cation-exchange (SCX) chromatography. (A more complete DeepLook™ analysis normally would include as many as eight fractions to increase the dynamic range of the measurement.)

The tryptic peptides were then profiled (individual molecules tracked across samples and their differential expression quantified) by liquid chromatography-electrospray ionization-mass spectrometry (LC-ESI-MS) on high-resolution (R > 5,000) time-of-flight (TOF) instruments using a capillary chromatography column. The on-line chromatography used was reverse-phase chromatography for one-dimensional (I-D) chromatography with a water/acetonitrile 100-minute gradient, and 0.1% formic acid added to aid in ionization efficiency and chromatographic behavior.

Software was used to track and quantify molecules for their differential expression. This includes joining results from the three SCX chromatography fractions in the DeepLook experiment. See the section on quantification strategy.

Identification of proteins occurs via identification of peptides. Peptides of interest (significantly changing in expression level) were linked to tandem mass spectrometry (MS/MS) experiments on quadrupole-time-of-flight (Q-TOF) and ion-trap mass spectrometers using extra or similar sample material. The resulting MS/MS spectra contain fragmentation patterns with characteristic peptide backbone cleavages. Each MS/MS raw spectrum from an isolated precursor ion was compared using commercially available software with *in silico* protein digestion and fragmentation using NCBI's RefSeq database to find a match, and hence identification. A match-quality score is reported. This identification approach also applies to peptides found in the LC-MS low-molecular-weight fraction. In some instances, *de novo* sequencing was also employed (no database matching) using one of several commercial software packages.

**Metabolome**

The metabolomic, low-molecular-weight (LMW) fraction was obtained from approximately one hundred microliters of the serum by first removing proteins by precipitation with the addition of an organic solution. The supernatant containing the LMW fraction was transferred from the solution by pipetting. This LMW material was further divided into two fractions.

One fraction was for the volatile or volatilizable small molecule components analyzed by gas chromatography-electron-impact ionization-mass spectrometry (GC-EI-MS). Volatilization was enhanced by trimethylsilyl derivatization of active hydrogens. The carrier gas was helium.

The second fraction was for analysis of nonvolatile components by liquid chromatography-electrospray ionization-mass spectrometry (LC-ESI-MS) using reverse phase (RP) chromatography. In this LC-MS analysis, low molecular weight free-floating peptides were detected in addition to metabolites. This LC-ESI-MS arrangement, with reverse-phase capillary HPLC, was similar to that used for the tryptic peptides from the proteomic fraction. For LC-MS, high-resolution (R > 5,000) time-of-flight mass spectrometers were used for profiling, while for GC-MS a quadrupole mass spectrometer was used with unit mass resolution although accurate mass by TOF-MS is available for GC-MS on select samples to aid with identifications.

For molecular identification of the volatile low molecular weight molecules, electron-impact ionization provides characteristic fingerprint fragmentation patterns that can lead to identification if the molecule has been analyzed previously in pure form and entered into a database such as that provided by National Institute of Standards and Technology (NIST) or PPD Biomarker's own database. Otherwise, use of accurate mass to constrain the elemental composition is also useful, and finally, tandem mass spectrometry (MS/MS) is available with a triple-quadrupole instrument. For those molecules tracked and deemed to be of interest due to their significant differential expression, even if an initial attempt at identification was not successful (no molecular name given in the results) it often is possible to later obtain identification with further effort, sometimes requiring isolation from the complex mixture.

For identification of the LMW fraction studied by LC-MS, a primary tool in the case of peptides is MS/MS (described above, for the case of digested proteins). Of importance especially for metabolites is accurate mass determination (usually to within a few mDa) to constrain elemental composition, and use of data sources such as the *Dictionary of Natural Products, Merck Index,* and the NIST and KEGG databases to infer identity. Comparison of mass spectrum and chromatographic retention time with pure compound, if available, can provide a definitive identification. Again, MS/MS is a primary tool for metabolites as well because it can be used to corroborate identity or provide insight into an unknown's structure. Ultimately, if necessary, the molecule of interest can be purified and subjected to NMR analysis. A schematic of the standard identification logic is shown in **Figure 8.**

**Quantification strategy.** One approach to quantification of LC-MS data, applicable to large numbers of proteins/peptides and metabolites for the purpose of differential expression measurements and discovery of biomarkers has been discussed (10, 122). In this situation, many or most monitored proteins are unanticipated at the time of laboratory study, thus eliminating the possibility of prior investigation of relative sensitivity factors (RSFs). Furthermore, methods based on introducing a known amount of a chemically analogous extraneous substance as an internal standard (i.e. "spiking" of a standard reference material) are not practical, whether the analog is chemically identical and isotopically labeled (the isotope dilution method) or based on chemical similarity.

The differential quantification method used here relies on the changes in analyte signal intensities directly reflecting their concentrations in one sample relative to another. Samples are not mixed nor are the samples otherwise manipulated beyond that required for the LC-MS analysis itself. The sample preparation and LC-MS conditions need to be carefully controlled, however, for optimal results, and frequent quality control samples are analyzed to assure stable, reproducible performance. Generally similar samples were compared in the method, such as plasma from different human subjects.

This quantification technology employed overall spectral intensity normalization by employing signals of molecules that do not change concentration from sample to sample. In this way, a simple correction was applied for any drift over time in overall LC-MS instrument response and/or differences in sample concentrations. Analysis includes normalization by determining the median of the ratios for a large number of molecular components, requiring no operator intervention, as well as spectral smoothing, baseline subtraction, noise evaluation, isotopic analysis, peak identification, intensity evaluation, inter-scan evaluation to construct chromatographic peaks, inter-file (inter-sample) evaluation to establish molecular components for analysis, normalization (mentioned above), and finally, quantification for the thousands of components. When spectra are sparse and simple, spectral analysis similarly is simple.

Quantification for GC-MS was done by referencing the intensity of all molecular components to one or two isotopically labeled and spiked components in the complex mixture. The simpler chromatography and ionization, relative to LC-MS, made this a feasible approach for quantification. Peak identification was performed via the AMDIS program published by National Institutes of Standards and Technology. This program deconvoluted electron-impact ionization mass spectra over chromatographic time and components are tracked using a library. Each entry in the library representing a distinct molecular component was constrained by a tight chromatographic elution time window and mass fingerprint pattern.

**Proteomics library**

Peptide identification for the serum proteome was very good. For the 1D proteome the 6544 components were matched to 591 different accession numbers and 65% of the total components were identified. This number was greater for those components with the lowest p-values reaching 92% for components with a p< 0.001 in the Naive (T0,T1) vs T2 comparison **(Table 9).** About half of the accession numbers were identified with two or more components. The distribution of components per accession number is given in Figure 9. For the 2D serum proteome the 16297 components were matched to 1304 different accession numbers and 42% of the total components were identified. About half of the accession numbers were identified with two or more components **Figure 10.** For the 1D urine proteome the 4492 components were matched to 307 different accession numbers and 16% of the total components were identified. Identification for the metabolome was much more challenging and is <10% for the various platforms.

**Table 9: Proteome Component Identification**

| P-level | Number Components | Number with ID | % With ID* |
|---------|-------------------|----------------|------------|
| P<0.001 | 244 | 225 | 92 |
| P<0.01 | 526 | 445 | 85 |
| All | 6544 | 4243 | 65 |
| * From one comparison - Naïve (T0,T1) vs T2 | | | |

**Mass spectrometry data collection**

Subjects and samples for the mass spectrometry analysis are based on collections through April 30, 2004. In general, 308 samples from 107 subjects were available (Table 10). This is less than the 362 samples from 121 subjects available from the main study for cytometry and immunoassays. For some platforms additional samples may be missing or excluded.

**TABLE 10: Samples for mass spectrometry analysis**

| Group | Subjects | Samples by Time-Type | | | | Total Samples |
|-------|----------|------|------|------|------|---------------|
| | | T0 | T1 | T2 | T3 | |
| **Healthy** | 35 | NA | 35 | NA | 35 | 70 |
| **Naïve** | 23 | 22 | 22 | 23 | 23 | 91 |
| **Stable** | 35 | NA | 35 | 35 | 35 | 105 |
| **Breakthrough** | 14 | NA | 14 | 14 | 14 | 42 |
| **Total** | 107 | 22 | 107 | 72 | 107 | **308** |

A problem in the sample preparation, variable tryptic digestion, was identified for the Serum LC-Proteome. A metric, based on the digestion of specific proteins, was developed to monitor this process. Based on the metric, 82% (254/308) of the samples remain in the analysis. A breakdown is given in Table 11.

**TABLE 11: Serum-LC-Protcome**

| Group | Samples by Time-Type | | | | Total Samples |
|---|---|---|---|---|---|
| | T0 | T1 | T2 | T3 | |
| **Healthy** | NA | 27 | NA | 28 | 55 |
| **Naïve** | 18 | 22 | 19 | 22 | 81 |
| **Stable** | NA | 28 | 27 | 22 | 77 |
| **Breakthrough** | NA | 13 | 14 | 14 | 41 |
| **Total** | 18 | 90 | 60 | 86 | 254 |

The DeepLook analysis of the serum proteome was a cross sectional comparison of subjects at T1. All main study samples are included. The number of subjects is given in Table 12.

**TABLE 12: Serum Samples for Deep Look - Proteomics**

| Group | Subjects/Samples* |
|---|---|
| Healthy | 35 |
| Naïve | 35 |
| Stable | 35 |
| Breakthrough | 16 |
| Total | 121 |
| * All samples are from T1 | |

### Mass spectrometry analysis

For these data sets, components were quantified at an occurrence threshold of 25% that means for a given comparison, the component had to appear in at least 25% of the samples to be reported. Each component is a distinct molecular ion, and their tally does not include all the observed isotopes. **Table 13** reports the median and mean CV's were about 23-30%, for the both the 1D and 2D serum proteome, which compared well with other human serum and plasma studies. Median CVs were about 40% for the serum LC metabolome. CVs for urine LC metabolome and serum GC metabolome were substantially higher. Based on these results the urine GC metabolome data would be very noisy and comparative statistics were excluded.

**Table 13: CV Statistics for Mass Spectrometry platform**

| | 1D SERUM-PROT | | 1D URINE PROT | | SERUM LC MET | | 2D SERUM PROT | | SERUM GC MET | |
|---|---|---|---|---|---|---|---|---|---|---|
| Components | 6544 | | 4492 | | 4129 | | 16297 | | 234 | |
| Sample Set* | Med | Mean | Med | Mean | Med | Mean | Med | Mean | Med | Mean |
| Healthy T1 | 29.4 | 33.1 | 62.7 | 80.3 | 39.4 | 47.1 | 28.0 | 33.2 | 68.0 | 72.1 |
| Naive T1 | 29.5 | 33.0 | 66.0 | 76.1 | 41.8 | 50.4 | 28.9 | 33.6 | 69.5 | 75.7 |
| Naive T2 | 29.9 | 33.1 | 56.0 | 65.7 | 41.3 | 49.7 | NA | NA | 81.1 | 80.7 |
| Naive T3 | 31.0 | 34.7 | 61.1 | 70.1 | 43.2 | 52.9 | NA | NA | 73.0 | 76.6 |

### STATISTICAL METHODS

The statistical analysis included two approaches 1) Classical statistics using ANOVA and linear mixed models and 2) Clustering and classification methods. The latter two were considered more exploratory.

The data set for this study was broad, i.e., there are many more variables (thousands) than subjects (hundreds). All variables are analyzed in order to identify potential new biomarkers. Consideration of the multiple comparison problem was needed. All statistical comparisons displayed in output sheets were ranked by univariate p-values.

As an additional guide, the false discovery rate (FDR), was controlled as proposed by Benjamini and Hochberg (11). This method was chosen over the more conservative step-down method of Holm (15, 50) to control the family-wise error rate in order to increase the power of the analysis. The adjusted values were not presented in the output files. For each variable, higher unadjusted p-values (up to 0.05) and consistency in multiple comparisons considered.

**Linear Mixed Effect Models**

Details on the statistical methods are presented in the statistics plan. Linear mixed effect models were used to enable consideration of time components and repeat measures. The LME package (6) was used for the R statistical environment (88) to complete the analyses. R is an open-source implementation of the S statistical language that has been developed and maintained by the international statistical community and is freely available from the R-project website. The exact model specification is described in more detail in each of the sections below.

**Within group comparisons**

Our analysis of outcomes within treatment groups was primarily to confirm beliefs about similarity and differences at various time points. Naive subjects have two baseline pretreatment measurements. A healthy subject should always have similar measurements across time points. Stable and Breakthrough subjects are assumed to be at steady-state in their treatment plans and so measurements six-days post-injection should be roughly the same. **Table 14** indicates the comparisons and the expected results. The differences that we expect are only for those outcomes that the treatment affects.

| Table 14: Within treatment group comparisons | | | |
|---|---|---|---|
| **Treatment group** | **Reference** | **Test** | **Expect** |
| Healthy | T1 | T3 | Same |
| Stable | T1 | T3 | Same |
| Stable | T1, T3 | T2 | Differences |
| Breakthrough | T1 | T3 | Same |
| Breakthrough | T1, T3 | T2 | Differences |
| Naive | T0 | T1 | Same |
| Naïve | T0, T1 | T3 | Differences |
| Naïve | T0, T1 | T2 | Differences |
| Naïve | T3 | T2 | Differences |

While we expect no findings of interest among the steady-state comparisons in this analysis, we carried out these tests to check for irregularities in measurements between time points and as a candidate baseline to assess the number of potential false positives.

Differences between a subject's reference measurements help estimate the baseline within subject variation. We do not expect all of the subjects to have measurements that shift in the same direction over the reference period. If we are confident that no systematic differences should exist between time point T1 and T3 for Healthy, Stable and Breakthrough subjects and T0 and T1 for Naive subjects, on average, then any testing procedure that detects differences is generating false positives at a rate that we can estimate from this set of data.

We fit models of the form

$$y_i = \alpha_{j(i)} + \beta t_i + \varepsilon_i$$

$$\alpha_j \sim N(\alpha, \tau^2)$$

$$\varepsilon_i \sim N(0,\sigma^2)$$

Here $y_i$ is the $i$th outcome measurement. The $i$th outcome measurement comes from subject $j(i)$ at time point $t_i$. $\alpha_j$ is the random intercept for each subject. More concretely, if observations $y_1$ and $y_2$ are measurements for subject 1 then $j(1)=1$ and $j(2)=1$ so that a $\alpha_1$ appears in the first equation for both of the first two observations, inducing correlation between them. For the reference time point we will set $t_i=0$ and for the test time point $t_i=1$. For those comparisons with multiple measures at steady-state, such as stable T1,T3 compared with T2, both of the steady-state measurements are coded as $t_i=0$ modeling them as repeated measures under the same conditions. $\beta$ represents the expected difference between measures at the test and the reference time points. Our primary interest is in estimates of this parameter. $\tau^2$ and $\sigma^2$ are variance terms. Repeated measures on the same subject will have correlation $\tau^2/(\tau^2+\sigma^2)$.

For the last three Naive analyses described in Table 14, we expended the first equation to allow for differences at three different time points.

$$y_i = \alpha_{j(i)} + \beta_1 t_i + \beta_2 u_i + \varepsilon_i$$

Here $t_i=1$ only for time point T2 and $u_i=1$ only for time points T3. The model treats measures at T0 and T1 as repeated measures under the same conditions. Contrasts involving $\beta_1$ and $\beta_2$ test for changes in the outcome over time. Fitting the three time points simultaneously can improve precision for estimating the variance components.

**Comparisons between Healthy and Multiple Sclerosis groups**

In this section we compare the Healthy with the MS groups, N (naïve), S (stable), and B (breakthrough). We expect differences between each of the multiple sclerosis groups and the healthy subjects; however, the Stable group is expected to be the most like the Healthy group (Table 15).

**Table 15: Between Group Comparisons -Multiple Sclerosis Groups vs Healthy**

| Reference | Test | Expect | Comment |
|---|---|---|---|
| Healthy (T1, T3) | Stable (T1, T3) | Differences | Closest MS group to Healthy |
| Healthy (T1, T3) | Breakthrough (T1, T3) | Differences | |
| Healthy (T1, T3) | Naïve (T0, T1) | Differences | |

For this analysis we fit models of the form

$$y_i = \alpha_{j(i)} + \beta g_i + \varepsilon_i$$

$$\alpha_j \sim N(\alpha,\tau^2)$$

$$\varepsilon_i \sim N(0,\sigma^2_{g(j)})$$

As opposed to the within treatment group comparisons previously described, we coded measures from the reference group as $g_i=0$ and measures from the test group as $g_i=1$. Since each of the time points represent steady-state time points, each measure is effectively modeled as a repeated measure under the same conditions. $\beta$ represents the expected difference between measures in the test and the reference group. Note also that this model allows for different residual variances by treatment group, where $g(j)$ indicates to which treatment group subject j belongs.

**Comparisons among the Avonex treatment groups.**

This section evaluates differences among multiple sclerosis subjects and different stages of treatment or with different responses to treatment. The Naïve group is expected to be more similar to the Breakthrough group than the Stable Group prior to Avonex treatment Table 16). Post treatment, the Naive Group may become more similar to the Stable group even at the early stage of treatment evaluated in this study.

Table 16: Between Group Comparisons -Avonex Treatment Groups

| Reference | Test | Expect | Comment |
|---|---|---|---|
| Stable (T1, T3) | Breakthrough (T1, T3) | Differences, may be subtle | T2 post-injection may give stronger effect than steady state results |
| Stable (T2) | Breakthrough (T2) | | |
| Stable (MD) | Breakthrough (MD) | | Magnitude Delta (T2-<T1, T3>)/ (<T1,T3>) |
| Stable (T1, T3) | Naïve (T0, T1) | Differences | Drug vs No Drug |
| Stable (T2) | Naïve (T2) | | |
| Stable (T1, T3) | Naïve (T3) | | |
| Breakthrough (T1, T3) | Naïve (T0, T1) | Differences | Drug vs No Drug |
| Breakthrough (T2) | Naïve (T2) | | |
| Breakthrough (T1, T3) | Naïve (T3) | | |

This analysis replicated the model used for comparing the Healthy and Multiple Sclerosis Groups previously described.

**Comparisons between Naive subjects at 0, 3 and 6 months**

Some of the Naïve subjects returned for an additional set of blood draws at 3 and 6 months after the start of treatment. Subjects are assumed to be at steady-state in their treatment plans and so measurements six-days post-injection should be roughly the same. Table 17 indicates the comparisons and the expected results. The additional comparisons at 3 and 6 months parallel the comparisons done for the Stable and Breakthrough groups in Table 5. The differences that are expected are only for those outcomes that the treatment affects.

Table 17: Within treatment group comparisons

| Treatment group | Reference | Test | Expect |
|---|---|---|---|
| Naïve - 0M | T0 | T1 | Same |
| Naïve - 0M | T0, T1 | T3 | Differences |
| Naïve - 0M | T0, T1 | T2 | Differences |
| Naïve - 0M | T3 | T2 | Differences |
| Naïve-3M | T04 | T06 | Same |
| Naïve-3M | (T04, T06) | T05 | Differences |
| Naïve-6M | T07 | T09 | Same |
| Naïve-6M | (T07, T09) | T08 | Differences |

A linear mixed effect model was used to look at variable changes over time. Only samples at steady-state, pre-treatment and six days post dosing were used. Sample time points are listed in Table 18.

**TABLE 18: Linear mixed model**

| Group-Time | Naïve 0M | Naïve 0M | Naïve 3M | Naïve-6M |
|---|---|---|---|---|
| **Drug** | No Drug | Early (6D) | Drug (Steady State) | Drug (Steady State) |
| **Time Types** | (T00, T01) | T03 | (T04, T06) | (T07, R09) |

For this analysis we fit a model of the form

$$Y_{it} = \alpha_i + \beta_1 * I(t=T0,T1) + \beta_2 * I(t=T3) + \beta_3 * I(t=T4,T6) + \beta_4 * I(t=T7,T9) + \varepsilon_i$$

where i indexes the subject, *t* specifies the time point, $\varepsilon_i \sim N(0,\sigma^2)$, and $\sim \alpha_i \sim N(0,\tau^2)$.

In this equation, there are terms for each of the four time phases in which the βs measure differences compared with the no drug phase. For example, $\beta_2$ is the expected difference between an individual's measurements at T03 compared with measurements at T0, or T1 (the no drug measurements). How likely it is that a given β truly does differ from zero (that is whether or not there is in fact that specific phase effect) is measured by its corresponding phase p-value. In addition to the phase p-value, p-values are reported for all six group-time pairs.

## Discriminant analysis

Discriminant analysis is used to determine which variables discriminate between two or more defined groups and is done in two steps. In the first step a discriminant function model is built by from variables which act as predictors of the two groups. In the second step, the above model is used to predict to which group a case belongs as well as the probability of that prediction being the correct classification. In our examples, the two groups are two different cohorts and the variables are analytes. For example, if there are two variables, a linear equation of the type:

$$y = a + b1 * x1 + b2 * x2$$

is fit to the data. In this equation, a is a constant, *b1* and *b2* are the regression coefficients, and *x1* and *x2* are the intensities corresponding to the two analyte variables selected. The above model is used to predict cohort classification scores for a subject sample and the cohort to which the subject sample is predicted to belong to is the one with the highest classification score. The probability that the sample is correctly predicted, the posterior probability, is computed from the Mahalonobis distance which is a measure of the distance between two points in the space defined by two or more correlated variables. This distance can be thought of as a generalization of the Euclidean distance that also takes into account for the degree of relatedness between all of the variables in the model.

We performed linear discriminant analyses using one and two variables on the following six pairs of cohorts: 1) B(T1,T3) v. N(T0,T1), 2) H(T1,T3) v. B(T1,T3),3) H(T1,T3) v. N(T0,T1), 4) S(T1,T3) v. B(T1,T3),5) H(T1,T3) v. S (T1,T3), and 6) S(T1,T3)v. N(T0,T1). Linear discriminant analyses were first performed on each analyte variable that has a p-value of at least 0.01 in the above univariate comparisons. Individual analyses were also performed for each possible pair of p-value selected variables. Analyte variables were selected from the data generated from the immunoassay, cytometry, and serum 1D proteome.

We used the lda and predict.lda packages in the MASS library within the R statistical environment (88) to complete the analyses. For more details on linear discriminant analysis, as well as the R functions lda and predict.lda, the user is referred to Modem Applied Statistics with S (119)

The results of the linear discriminant analysis for the two group comparisons in Table 19 below show that in many cases while one variable can discriminate fairly well between the between the two groups, the percentage of samples correctly classified does not dramatically improve upon addition of a second variable. Although, the percentage does not significantly rise, the confidence with which many of the classifications is made does rise as does the number of lda sets with a correct prediction of >75%. The percent high confidence column indicates the percentage of samples that are classified into their respective group with at least an 80% posterior probability.

**Table 19: Summary of Results of Linear Discriminant Analyses**

| Comparison | # Vars | Top 10 sets of Identified Variables | % Highest Confidence | Max %Correct Predicted | # with % Correct Predicted >75 |
|---|---|---|---|---|---|
| S(T1,T3)v H (T1,T3) | 1 | LC159122<br>CYT18391 | 10.0 | 77.9 | 2 |
| | 2 | CYT 17008, LC160885<br>CYT 17581, LC160885<br>LC159289, LC161611<br>CYT17008, LC161662<br>CYT 17008, LC159289 | 46.4 | 84.3 | 1000 |

(continued)

| Comparison | # Vars | Top 10 sets of Identified Variables | % Highest Confidence | Max %Correct Predicted | # with % Correct Predicted >75 |
|---|---|---|---|---|---|
| | | IA 3, CYT17008<br>CYT 17578, LC160885<br>CYT 16096, LC161482<br>IA 3, CYT17581<br>CYT16590, CYT17257 | | | |
| S(T1,T3) v N (T0,T1) | 1 | CYT17578 | 32.6 | 73.9 | 0 |
| | 2 | CYT 17257,CYT17581<br>CYT15854, CYT15947<br>CYT17257, CYT17579<br>CYT17578, CYT18282<br>CYT16911, CYT17581<br>CYT16910,CYT17581<br>CYT17581,CYT18042<br>CYT17008,CYT17328<br>CYT15946,CYT17008<br>CYT17008,CYT18285 | 52.6 | 83.9 | 789 |
| H(T1,T3) v N (T0,T1) | 1 | CYT17046<br>CYT17058<br>CYT17370<br>CYT17011<br>CYT17700<br>CYT17367 | 31.4 | 78.1 | 6 |
| | 2 | CYT 17011,CYT17937<br>CYT17691, CYT17937<br>CYT17817, CYT17937<br>CYT17649, CYT17937<br>CYT17046, CYT17937<br>CYT17937, CYT17982<br>CYT17937, CYT17979<br>CYT17370, CYT17937<br>CYT17367, CYT17937<br>CYT16996, CYT1797 | 62.0 | 83.7 | >1000 |
| S(T1,T3) v B (T1,T3) | 1 | LC160788 | 18.4 | 75.5 | 1 |
| | 2 | CYT17257, LC158384<br>CYT17257, LC158113<br>CYT17257, LC161745<br>IA51,LC160788<br>CYT17257,LC161043<br>CYT17257,LC160967<br>CYT17257,LC160923<br>CYT17257,LC159467<br>CYT17257,LC159289<br>LC160788,LC163495 | 48.5 | 85.5 | 562 |
| H(T1,T3) v BT1,T3) | 1 | IA14<br>LC161911 | 2.9 | 83.3 | 16 |

(continued)

| Comparison | # Vars | Top 10 sets of Identified Variables | % Highest Confidence | Max %Correct Predicted | # with % Correct Predicted >75 |
|---|---|---|---|---|---|
| | | IA3 CYT18385 LC159970 LC160763 CYT17392 CYT18736 CYT18364 LC159799 | | | |
| | 2 | IA21, CYT17392 CYT18751, LC159799 CYT18250, LC159799 IA3, CYT16852 CYT17008, LC160761 IA3, CYT18250 IA3, CYT16805 IA3, CYT16803 CYT18331, LC161631 IA3, CYT17008 | 47.1 | 91.2 | >1000 |
| B(T1,T3) v N (T0,T1) | 1 | LC159799 | 19.2 | 74.0 | 0 |
| | 2 | CYT15855, LC159799 CYT15873, LC159970 CYT15873, LC159368 CYT15854, LC159799 CYT15873, LC162551 CYT15873, LC159799 CYT15825, CYT15855 CYT15825, CYT15854 CYT16609, LC161069 CYT16609, LC159799 | 36.1 | 83.3 | 195 |

## Clustering

The hierarchical clustering analysis procedure begins by measuring each of a set of n objects on k variables. For example, if one clusters subject samples, then the n objects are the subject samples and the k variables are the analyte variables. Likewise, if one clusters the analyte variables, then the n objects are the analyte variables and the k variables correspond to the subject samples. In order to perform the clustering, a measure of the degree of similarity or distance between each pair of objects must be specified. Then the objects are clustered into subgroups based on the inter-object similarities, or distances, as calculated by the specified metric. Clustering proceeds in the following four steps:

• Start by assigning each object to its own cluster, so that if you have n objects, you now have n clusters, each containing just one item. Let the distances (similarities) between the clusters equal the distances (similarities) between the objects they contain.
• Find the closest (most similar) pair of clusters and merge them into a single cluster, so that now you have one less cluster.
• Compute distances (similarities) between the new cluster and each of the old clusters.
• Repeat steps 2 and 3 until all objects are clustered into a single cluster of size n.

In our analyses, step 3 was performed with complete link clustering. Complete-link clustering considers the distance between one cluster and another cluster to be equal to the longest distance from any member of one cluster to any member of the other cluster. We used the Euclidean distance as our distance metric and our input data consisted of z-score normalized intensity values. Z-score normalized intensity values are computed independently for each analyte. For any given analyte, for each sample, the z-score is calculated by subtracting the mean intensity value from the sample intensity and dividing the difference by the standard deviation of the intensity values. We used the hclust package for the R statistical environment (88) to complete the analyses.

We performed clustering analyses on the following four data sets: 1) H(T1) v. N(T1), 2) H(T1,T3) v. N(T0,T1), 3) S(T1) v B(T1), and 4) N(T2) v S(T1) and B(T1). The univariate p-values used as cutoffs for variable selection as well as the number of variables selected are shown in Table 20, **Table 21,** and **Table 22** below. Variable lists for each result are provided electronically (file name P7003-ClusterVarList.xls). Data generated from the immunoassay, cytometry, and ID serum proteome and 2D serum proteome were used for data sets 1, 3, and 4 while data generated from the immunoassay, cytometry, and liquid chromatography platforms were used for data set 2. For each of the above data sets, the following steps were performed. First the p-value selected analyte variables are clustered as described above. A reduced set of variables is selected from the analyte variables by first assigning each of the analyte variables to whichever cluster it falls into based on a specified tree branch cut height. Different values of the tree branch cut heights are shown in the results section. The value of each reduced variable is determined as the median value of all analyte variables falling within its cluster. Having generated a set of values for the reduced variables, the samples are then clustered.

To determine the quality of fit associated with any given dendrogram, one can consider the following measure of dendrogram performance in separating members of one or more groups. The measure is based on the number of monochromatic nodes in a dendrogram where a monochromatic node in a dendrogram is defined as the highest node in a tree for which all its descendant leaf nodes belong to the same group, as shown by being the same color (here, cohort) in the plots. For each leaf, we computed the number of branches that must be traversed along the path from the root to the leaf before a monochromatic node is encountered. Averaging this quantity over all leaves gives our performance measure. As shown in the left panel of Figure 11, in an ideal case, the two nodes immediately under the root node are monochromatic, resulting in the best possible performance measure of 1. In the middle panel of Figure 11 is shown a slightly worse example in which there are several distinct monochromatic nodes, with some occurring as far down the tree as possible at the leaf nodes. In this case, for the 20 samples, the measure is given by D = (2*2 + 2*2 + 3*2 + 2*3 + 1*4 + 2*5 + 3*6 + 1*6 + 1*7 +1*8 + 2*9 )/20 = 4.55. In the right panel the worst case is shown with a tree that has every leaf as a monochromatic node; that is all monochromatic nodes are as low in the tree as they can possibly be. This worst case occurs with a perfectly unbalanced tree that has only the leaf nodes being monochromatic. For the tree with 20 leaves below, the result is 11.45. In general for a tree with N leaves, the measure is given by D = $((N-1) + \sum_{i=1\,to\,N-1} i)/20$ = $(N+1)/2 - (1/N)$.

**Table 20: Healthy vs. Naïve**

| Comparison | Data Set | NLP | # Variables | Filter | #Variables after filter | Fit Metric |
|---|---|---|---|---|---|---|
| 1 | H(T1) v N(T1) | 4 | 59 | none | 59 | 6.11 |
| 2 | B(T1) v N(T1) | 4 | 59 | 6.58 | 17 | 5.44 |
| 3 | H(T1) v N(T1) | 4 | 59 | 8.22 | 11 | 5.51 |
| 4 | H(T1) v N(T1) | 4 | 59 | 9.87 | 5 | 5.84 |
| 5 | H(T1) v N(T1) | 3 | 126 | none | 126 | 6.47 |
| 6 | H(T1) v N(T1) | 3 | 126 | 6.58 | 41 | 6.25 |
| 7 | H(T1) v N(T1) | 3 | 126 | 8.22 | 30 | 6.64 |
| 8 | H(T1) v N(T1) | 3 | 126 | 9.87 | 15 | 6.87 |
| 9 | H(T1) v N(T1) | 3 | 126 | 11.51 | 6 | 6.14 |
| 10 | H(T1,T3) v N(T0,T1) | 3.5 | 83 | none | 83 | 6.87 |
| 11 | H(T1,T3) v N(T0,T1) | 3.5 | 83 | 6.94 | 30 | 6.64 |
| 12 | H(T1,T3) v N(T0,T1) | 3.5 | 83 | 11.57 | 17 | 6.59 |
| 13 | H(T1.T3) v N(T0,T1) | 3.5 | 83 | 13.89 | 9 | 6.71 |
| 14 | ·H(T1,T3) v N(T0,T1) | 3 | 125 | none | 125 | 6.64 |
| 15 | H(T1,T3) v N(T0,T1) | 3 | 125 | 9.26 | 40 | 6.81 |
| 16 | H(T1,T3) v N(T0,T1) | 3 | 125 | 11.57 | 30 | 7.09 |

(continued)

| Comparison | Data Set | NLP | # Variables | Filter | #Variables after filter | Fit Metric |
|---|---|---|---|---|---|---|
| 17 | H(T1,T3) v N(T0.T1) | 3 | 125 | 13.89 | 16 | 6.7 |

**Table 21: Stable vs. Breakthrough**

| Comparison | Data Set | NLP | # Variables | Filter | #Variables afterFilter | Fit Metric |
|---|---|---|---|---|---|---|
| 18 | S(T1) v B(T1) | 4 | 113 | none | 113 | 6.31 |
| 19 | S(T1) v B(T1) | 4 | 113 | 5.34 | 35 | 6.13 |
| 20 | S(T1) v B(T1) | 4 | 113 | 6.68 | 24 | 5.62 |
| 21 | S(T1) v B(T1) | 4 | 114 | 8.01 | 14 | 5.23 |
| 22 | S(T1) v B(T1) | 3 | 369 | none | 369 | 6 |
| 23 | S(T1) v B(T1) | 3 | 369 | 6.86 | 105 | 5.35 |
| 24 | S(T1) v B(T1) | 3 | 369 | 8.23 | 56 | 5.39 |
| 25 | S(T1) v B(T1) | 3 | 369 | 9.6 | 24 | 6.86 |

**Table 22: Naïve vs. Stable and Breakthrough**

| Comparison | Data Set | NLP | # Vars | Filter | #Variables after filter | Fit Metric |
|---|---|---|---|---|---|---|
| 26 | N(T2) v S(T1)and B(T1) | 3 | 41 | none | 41 | 5.62 |
| 27 | N(T2) v S(T1) and B(T1) | 3 | 41 | 7.21 | 20 | 5.72 |
| 28 | N(T2) v S(T1) and B(T1) | 3 | 41 | 9.02 | 15 | 7.06 |
| 29 | N(T2) v S(T1) and B(T1) | 3 | 41 | 10.82 | 11 | 7.23 |
| 30 | N(T2) v S(T1) and B(T1) | 1.3 | 436 | none | 436 | 6.55 |
| 31 | N(T2) v S(T1) and B(T1) | 1.3 | 436 | 9.02 | 148 | 7.37 |
| 32 | N(T2) v S(T1)andB(T1) | 1.3 | 436 | 10.82 | 95 | 7.30 |
| 33 | N(T2) v S(T1) and B(T1) | 1.3 | 436 | 12.63 | 38 | 6.63 |
| 34 | N(T2) v S(T1) and B(T1) | 1.3 | 436 | 14.43 | 10 | 7.02 |

**Data presentation**

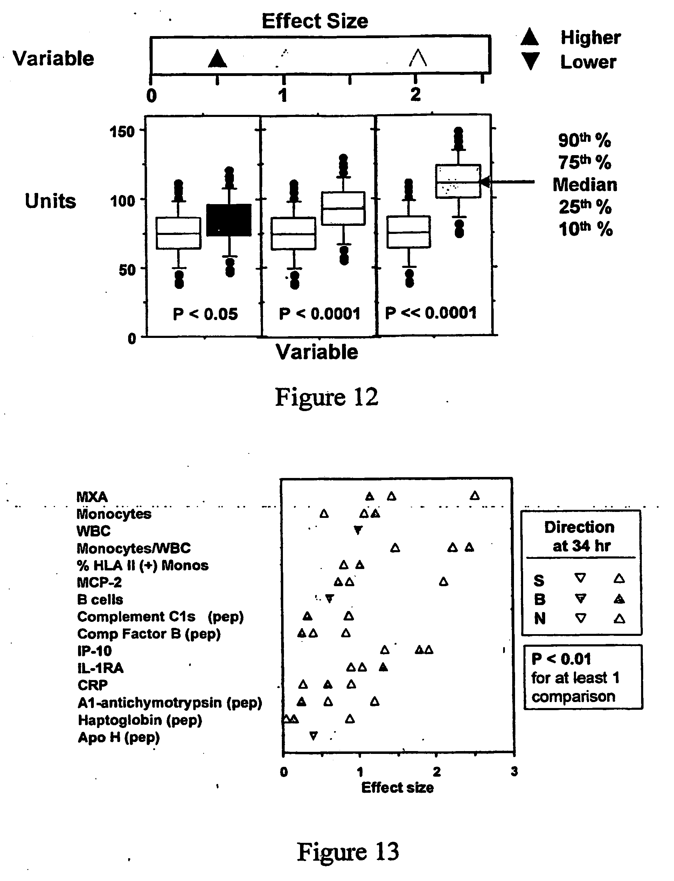

Examples of two types of data presentation are provided in Figure 12. The top graph shows an effect size plot. The effect size for two groups being compared is the difference of means divided by the weighted standard deviation. It is a good initial measure of potential to use as a diagnostic marker. The bottom graph shows the distribution of samples as a box and whiskers plot. Typically two or more groups are compared. This example is based on calculations using normal distributions and comparisons are all relative to the group shown. Thus, with an effect size of one, the boxes of the two populations are almost separated. This is a strong difference, with a p-value of < 0.0001.

Comments on considering data

In this study thousands of variables were measured and evaluated in multiple different comparisons. The variables were chosen based on the following:

Univariate p-value
Consistency across more than one comparison where appropriate
Consistency among related variables within a comparison
Magnitude of the difference between the comparison groups

Effect-size (mean difference/standard deviation of the measure
Identified variables for the mass spectrometry platform

**Example 2**

**WITHIN GROUP COMPARISONS**

**IDENTIFICATION OF PHARMACODYNAMIC RESPONSE MARKERS SHORT-TERM POST DOSING**

Biological response markers transiently change following dosing with IFN-β in multiple sclerosis patients and healthy controls. This class of markers indicates drug-target interactions and down stream consequences and is useful for evaluating dosing (12). Here we use broad differential profiling across all platforms to evaluate potential pharmacodynamic markers. This study focuses on effects short-term (34 hours) post injection.

Key Questions. Within group statistical comparisons are listed in **Table 23.** We ask three questions: 1) Are there differences short-term (34 hr) after injection compared with six days after injection? 2) What levels of false positives are observed among repeat samplings? and 3) Are there pharmacodynamic differences among the groups? Comparisons where a pharmacodynamic effect is expected at T2 are highlighted. Repeat samplings were made for subjects both on drug (Stable and Breakthrough, both six days post injection) and subjects not on drug (Healthy and Naïve prior to first injection). *A priori,* differences are not expected for these control comparisons above the p-value thresholds. The most and strongest differences are expected in the Naive group when the subjects first begin to receive the drug. Two comparisons to pre-drug are presented for this group, one at 34 hours post injection (T2) and one at six days post injection (T3).

**Table 23: Within Group Statistical Comparisons**

| Comparison Class | Cohort | Reference | Test | Expect |
|---|---|---|---|---|
| **Pharmacodynamic** | Stable | T1, T3 | T2 | Differences |
| | Breakthrough | T1, T3 | T2 | Differences |
| **PD Early** | Naïve | T3 | T2 | Differences |
| **PD Early, No Drug** | Naïve | T0, T1 | T2 | Most Differences |
| **Repeat (control)** | | | | |
| **6 day post injection** | Stable | T1 | T3 | Same |
| | Breakthrough | T1 | T3 | Same |
| **Repeat (control)** | | | | |
| **No drug** | Healthy | T1 | T3 | Same |
| | Naive | T0 | T1 | Same |
| **Drug v No Drug** | Naive | T0, T1 | T3 | Difference |

**Summary Statistics**

A strong pharmacodynamic effect is observed for multiple cohorts and multiple platforms. A summary of the number of hits in this biomarker screen is presented in Table 24 by analytical platform and ranked by p-value. Note that the number of samples is not the same for each group and each comparison. Considering the cytometry results first, there were many more hits for the T2 within group comparisons (hundreds for each comparison at p <0.001) than the repeat comparisons (0-5 for each comparisons at p< 0.001). Among the repeat comparisons the number of hits was approximately what is expected to appear by chance for 2000 variables at each p-value level, assuming all of the variables are independent This indicates that the analysis was well controlled. Among the pharmacodynamic comparisons, the most hits were observed for each p-value level for the Naïve group when comparing short-term post drug (T2) to pre drug (T0, T1).

For the serum assays, including immunoassays, proteome, and LC-metabolome, many more hits for the T2 within group comparisons vs the repeat measures were also observed. For all three platforms, the number of hits for the repeat comparisons was approximately what is expected to appear by chance. For the urine assays, the results were less dramatic. For the urine proteome, a large number of hits are indicated for the Stable T1vT3 repeat comparison, making conclusions about the T2 results more difficult to interpret. For the urine LC-metabolome, the repeat measure control yielded an appropriate number of hits, but the relative level of T2 differences is more modest than the other platforms. The most differences in the number of hits in observed for the Naïve comparisons.

It is instructive to consider the consistency of the pharmacodynamic effect short-term post injection across multiple comparisons. Again, considering cytometry results first, a total of 998 variables (51 %) had at least one hit at a p-value of <0.05 among the four comparisons. **Table 25** provides a summary of the number of variables that are significant in more the one comparison. For the pharmacodynamic comparisons, 29% (572/1949) of the variables are significant in at least two or more comparisons at the $p < 0.05$ level. In contrast, for the repeat comparison, only 1% (20/1949) of the variables were significant at in at least two or more comparisons at the $p < 0.05$ level. This further supports the conclusion the observed pharmacodynamic differences are meaningful.

The pharmacodynamic effect was consistent for the immunoassays, with 35% (18/51) of the variables significant in at least two or more comparisons at the $p < 0.05$ level, compared with none for the repeat comparisons. The pharmacodynamic effect is consistent but less dramatic for the serum proteome, with 11% (735/6544) of the variables significant in at least two or more comparisons at the $p < 0.05$ level, compared with 3.3% for the repeat comparisons.

Summary statistics for the Naive subjects who returned for additional visits at three and six months are provided for cytometry. There were substantially fewer samples at three and six months than the initial visits. **Table 26** summarizes the analysis of the short-term pharmacodynamic effects. There were many more hits for the T2 comparisons than the repeat comparisons within each time set. The results are consistent with those observed for the Stable and Breakthrough groups, further supporting the conclusion the observed short-term pharmacodynamic differences were meaningful. Long-term effects, evaluated with the linear mixed effect model are summarized in **Table 27.** Both the overall phase p-value and all six group-time p-values are reported. Overall, 40% (786/1949 phase p-value <0.05) of the variables change with time during the six-month Avonex treatment. The most differences between two time groups are observed for the 6-month vs pre-treatment comparisons (43%).

Detailed lists of all the variables used to generate these summary results are provided in the electronic Results Tables, which are described below.

**Table 24: Summary statistics for within group comparisons.**

| | Repeat | | | | Early Drug | Pharmacodynamic-Short-term | | | |
|---|---|---|---|---|---|---|---|---|---|
| Cohort | H | N | S | B | N | N | N | S | B |
| Comparison | T1vT3 | T0vT1 | T1vT3 | T1vT3 | T0-1vT3 | T0-1vT2 | T3vT2 | T1-3vT2 | T1-3vT2 |
| Drug | No | No | Yes | Yes | No vs Yes | No vs Yes | Yes | Yes | Yes |
| Subjects-CYT | 35 | 35 | 35 | 16 | 35 | 35 | 35 | 35 | 16 |
| Subjects-Mass | 35 | 23 | 35 | 14 | 23 | 23 | 23 | 35 | 14 |
| Expect | Same | | | | Differences | Differences | | | |
| Cytometry - 1949 Variables | | | | | | | | | |
| P<0.0001 | 0 | 0 | 0 | 0 | 23 | 252 | 166 | 155 | 103 |
| P<0.001 | 0 | 0 | 5 | 0 | 43 | 321 | 247 | 227 | 152 |
| P<0.01 | 25 | 6 | 24 | 4 | 82 | 467 | 388 | 331 | 267 |
| P<0.05 | 156 | 52 | 121 | 41 | 221 | 716 | 596 | 491 | 451 |
| Immunoassays - 51 Variables | | | | | | | | | |
| P<0.0001 | 0 | 0 | 0 | 0 | 3 | 8 | 6 | 4 | 4 |
| P<0.001 | 0 | 0 | 0 | 0 | 4 | 9 | 6 | 0 | 5 |
| P<0.01 | 0 | 0 | 0 | 0 | 10 | 14 | 9 | 7 | 6 |
| P<0.05 | 2 | 3 | 0 | 1 | 12 | 19 | 16 | 14 | 12 |

(continued)

| Proteome-LC-Serum-6544 Variables | | | | | | | | |
|---|---|---|---|---|---|---|---|---|
| P<0.0001 | 0 | 0 | 0 | 0 | 48 | 112 | 19 | 13 | 5 |
| P<0.001 | 2 | 4 | 6 | 4 | 84 | 244 | 79 | 40 | 23 |
| P<0.01 | 54 | 59 | 91 | 73 | 232 | 526 | 257 | 182 | 104 |
| P<0.05 | 301 | 383 | 466 | 480 | 624 | 1100 | 746 | 520 | 368 |
| Metabolome -LC - Serum -4129 Variables | | | | | | | | |
| P<0.0001 | 0 | 0 | 0 | 0 | 1 | 17 | 8 | 2 | 4 |
| P<0.001 | 0 | 2 | 2 | 1 | 4 | 50 | 28 | 10 | 18 |
| P<0.01 | 28 | 30 | 47 | 17 | 44 | 128 | 117 | 62 | 94 |
| P<0.05 | 184 | 201 | 236 | 119 | 278 | 328 | 351 | 311 | 300 |
| Metabolome - GC - Serum - 234 Variables | | | | | | | | |
| P<0.0001 | 0 | 0 | 0 | 0 | 0 | 0 | 0 | 0 | 0 |
| P<0.001 | 0 | 0 | 0 | 0 | 0 | 0 | 0 | 0 | 0 |
| P<0.01 | 2 | 2 | 2 | 0 | 1 | 1 | 2 | 4 | 3 |
| P<0.05 | 10 | 9 | 5 | 2 | 7 | 10 | 16 | 19 | 7 |
| Proteome - LC - Urine - 4492 Variables | | | | | | | | |
| P<0.0001 | 0 | 0 | 4 | 1 | 0 | 3 | 1 | 6 | 0 |
| P<0.001 | 1 | 1 | 45 | 2 | 2 | 38 | 9 | 13 | 3 |
| P<0.01 | 9 | 16 | 214 | 25 | 34 | 163 | 83 | 56 | 30 |
| P<0.05 | 100 | 148 | 652 | 138 | 246 | 563 | 314 | 237 | 233 |
| Metabolome- LC - Urine - 3270 Variables[1] | | | | | | | | |
| P<0.0001 | 0 | 0 | 0 | 1 | 0 | 2 | 2 | 0 | 0 |
| P<0.001 | 0 | 0 | 1 | 2 | 2 | 10 | 7 | 2 | 2 |
| P<0.01 | 11 | 7 | 12 | 24 | 37 | 75 | 64 | 23 | 26 |
| P<0.05 | 123 | 99 | 108 | 139 | 155 | 284 | 256 | 127 | 169 |

**Table 25: Consistency across multiple comparisons**

| Number of variables that are consistent among two to four different within group comparisons | | | | | | |
|---|---|---|---|---|---|---|
| | REPEAT | | | PHARMACODYNAMIC(T2) | | |
| Number of comparisons* | 4 | ≥3 | ≥2 | 4 | ≥3 | ≥2 |
| CYT-998 PD (T2) variables with hits at p<0.05 in≥1 comparison | | | | | | |
| P<0.0001 | 0 | 0 | 0 | 66 | 103 | 169 |
| P<0.001 | 0 | 0 | 0 | 97 | 156 | 226 |
| P<0.01 | 0 | 0 | 0 | 148 | 257 | 354 |
| P<0.05 | 0 | 1 | 20 | 247 | 349 | 572 |
| IA -22 PD (T2) variables with hits at p<0.05 in ≥1 comparison | | | | | | |
| P<0.0001 | 0 | 0 | 0 | 3 | 5 | 5 |
| P<0.001 | 0 | 0 | 0 | 4 | 5 | 5 |
| P<0.01 | 0 | 0 | 0 | 4 | 5 | 5 |
| P<0.05 | 0 | 0 | 0 | 5 | 8 | 18 |
| SE-PR 2223 PD (T2) variables with hits at p<0.05 in ≥1 comparison | | | | | | |
| P<0.0001 | 0 | 0 | 0 | 1 | 5 | 28 |
| P<0.001 | 0 | 0 | 0 | 7 | 15 | 67 |
| P<0.01 | 0 | 0 | 7 | 18 | 56 | 230 |
| P<0.05 | 0 | 21 | 220 | 47 | 193 | 735 |
| * Based on a maximum of 4 comparisons for both the repeat and pharmacodynamic (T2). Calculate separately at each p-value level | | | | | | |

**Table 26: NAIVE Cohort at 0, 3 and 6 months -Short term effect**

| Category | Repeat | | | Pharmacodynamic | | | |
|---|---|---|---|---|---|---|---|
| Comparison | N T0vT1 | N T4vT6 | N T7vT9 | N T0-1vT2 | N T3vT2 | N T4-6vT5 | N T79vT8 |
| Drug | No | Yes | Yes | No vs Yes | Yes | Yes | Yes |
| Subjects | 35 | 10 | 7 | 35 | 35 | 10 | 7 |
| Cytometry - 1949 Variables | | | | | | | |
| P<0.0001 | 0 | 0 | 0 | 119 | 65 | 41 | 22 |
| P<0.001 | 0 | 4 | 0 | 149 | 88 | 58 | 52 |
| P<0.01 | 4 | 12 | 2 | 226 | 144 | 117 | 108 |
| P<0.05 | 25 | 60 | 22 | 328 | 241 | 191 | 196 |

**Table 27: NAÏVE Cohort at 0, 3 and 6 months - Long-term effect - Linear effects model**

| Category | Phase | Pre drug vs. Steady State | | | Drug: Late vs. Early | | 3M vs. 6M |
|---|---|---|---|---|---|---|---|
| Comparison | All Times | T0-1vT3 | T0-1vT4-6 | T0-1vT7-9 | T3vT4-6 | T3vT7-9 | T4-6vT7-9 |
| Drug | No vs Yes | No vs Yes | No vs Yes | No vs Yes | Yes | Yes | Yes |
| Subjects | 34, 35, 10, 7 | 34, 35 | 34,10 | 34, 7 | 35, 10 | 35, 7 | 10, 7 |

(continued)

| Cytometry -1949 Variables | | | | | | | |
|---|---|---|---|---|---|---|---|
| P<0.0001 | 181 | 26 | 41 | 243 | 18 | 136 | 53 |
| F<0.001 | 315 | 50 | 129 | 370 | 54 | 273 | 109 |
| P<0.01 | 521 | 94 | 311 | 587 | 180 | 473 | 234 |
| P<0.05 | 786 | 254 | 539 | 835 | 385 | 731 | 384 |

## Specific Short-term Pharmacodynamic Results

A set of specific variables showing pharmacodynamic effects has been selected for discussion below. Selections are based on 1) univariate p-value, 2) consistency in more than one comparison 3) the magnitude of the differences 4) effect size (mean difference/weighted standard deviation and 5) biological relationships and 6) identification for mass spectrometry variables.

There are many analytes that show very strong effect size differences (>1) short-term after Avonex treatment Figure 13. Often the Naïve group comparison (blue) with the pre-treatment time point shows the biggest effect. Some of these specific analytes and others are considered in detail.

## MxA

Myxovirus resistance protein A (MxA) intracellular expression is specifically induced by type-I IFNs in many cell types and peaks 24-48 hours post IFN-β injection (108, 120). It has been used as a biological response marker to IFN treatment (1). It is included as an expected result to anchor this study. It also provides a means to evaluate individual subjects.

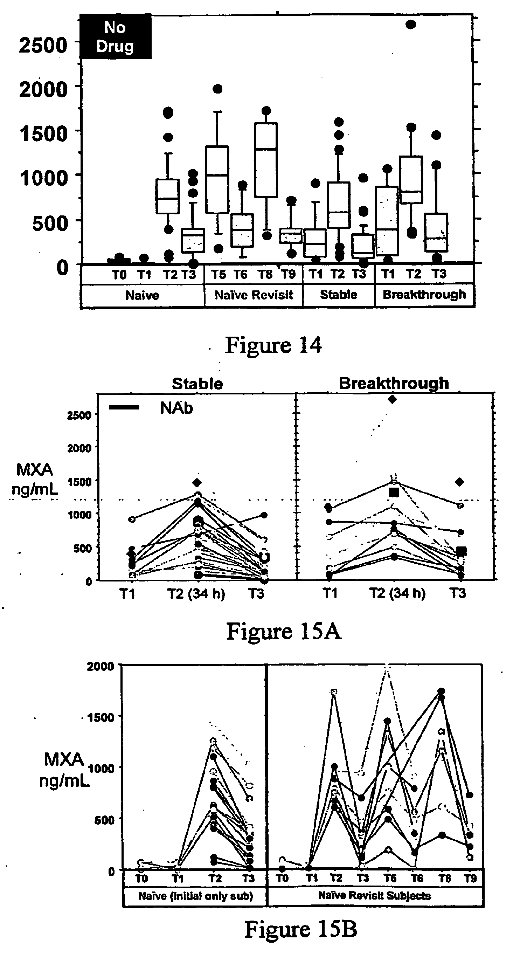

MxA shows a very strong pharmacodynamic effect in all treatment cohorts **(Figure 14,** Table 36). The protein is very low prior to treatment (Naive, T0, T1) and elevated at all times after treatment. The increase from no drug to short-term post injection is more than 20-fold and the difference between 1.5 and 6 days post injection is 2-3 fold for the various comparisons. MxA results on a sample-by-sample basis are shown in Figure 15. In general, the pharmacodynamic effect is consistent across many samples. Neutralizing antibodies to IFN-β block drug binding to its receptor and decrease the MxA response (24, 33, 70, 83, 117). Only one subject in this study had neutralizing antibodies. The subject, PAL035, who was in the Stable group, was among those with the lowest levels of MxA at both 34-hr and 6-day post injection (bold red line in Figure 15). A relative increase at T2 was still observed for this subject

### Table 28: Interferon-inducible protein MxA is strongly increased short term post injection

| | N | | S | B |
|---|---|---|---|---|
| | T2 v T0, T1 | T2 v T3 | T2 v T1, T3 | T2 v T1, T3 |
| P* | 15.31 | 11.15 | 11.25 | 6.71 |
| ES** | 2.5 | 1.4 | 1.4 | 0.9 |
| MR^ | 27.01 | 2.41 | 2.88 | 2.21 |
| *Neg log p-value, **Effect size ^ Mean ratio | | | | |

## Major cell populations

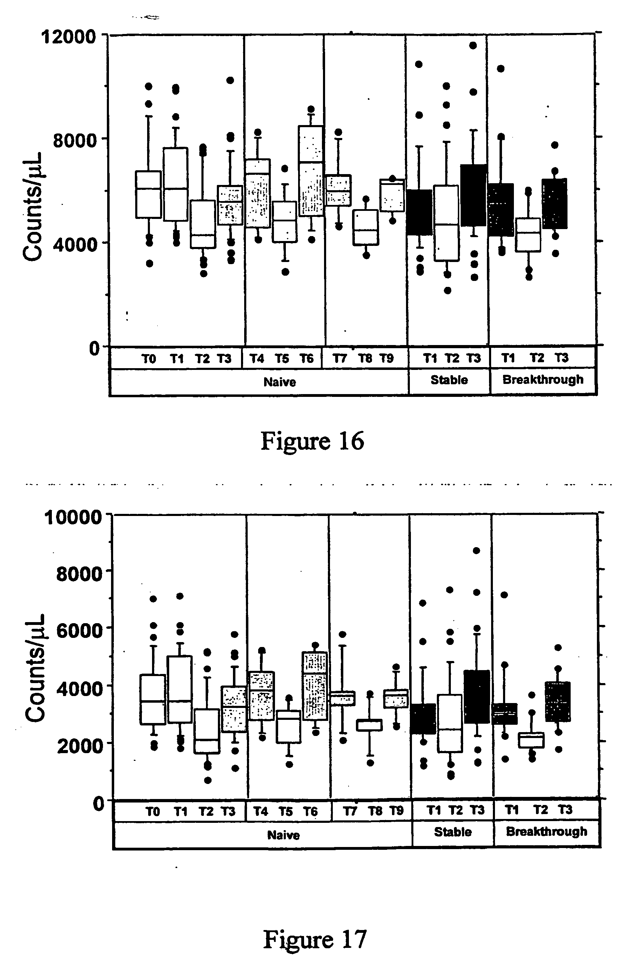

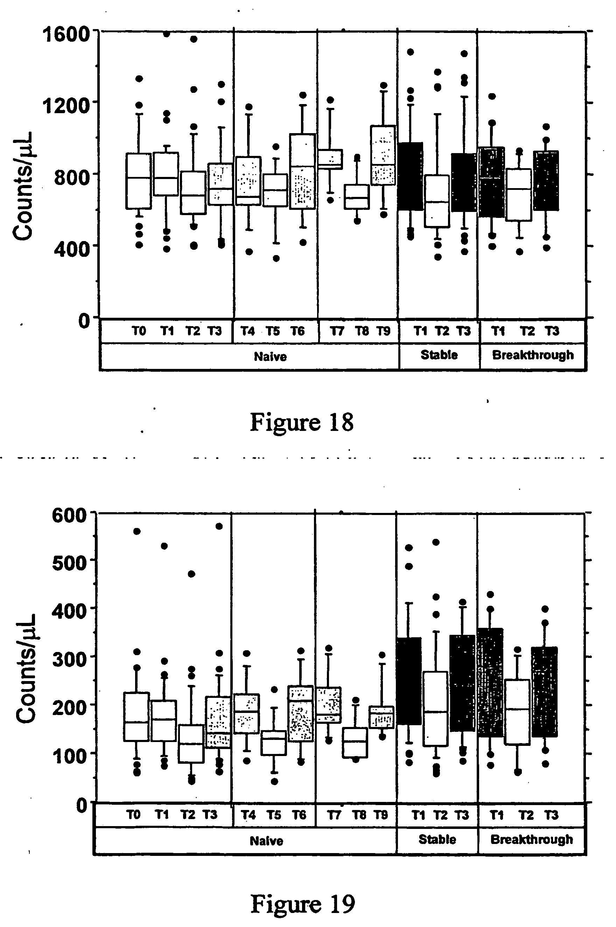

Total leukocytes are decreased with interferon-beta treatment (42, 92, 96). Less information is available on the pharmacodynamics of specific cell populations. We begin with the major cell populations and saw the absolute count (cells/uL of blood) decrease for many specific cell populations short-term post Avonex. Total leukocytes were about 20% lower short-term after dosing in all groups **(Figure 16, Table 29).** Among subsets neutrophils, CD4 and CD8 T cells, and B cells were all significantly decreased **(Figures 17-20, Table 30, Table 31, Table 32, Table 33).** These trends were consistent for the Stable, Breakthrough and Naive groups, both at initial dosing and follow up. The magnitude of the decrease was greatest for neutrophils (19-33%) and B cells (22-26%) and modest for the CD4 T cells (8-12%) and CD8 T cells (12-16%). Each trend applies across multiple comparisons. The biggest and most significant change was usually for the Naive comparison to the pre-drug samples. Among other major populations, eosinophils showed a decreasing trend, although with less statistical significance and NK cells were unchanged (Results not shown).

The decrease in these major cell population counts was transient and returns to steady stat level for Stable, Breakthrough and Naive groups six days after dose. There were fewer major cell population differences among control and MS groups (See between group section). These pharmacodynamic effects short-term post dose were generally consistent with reported observations. IFNβ-1a (Rebif) caused dose-related reduction of total leukocytes and neutrophils. The reduction of leukocyte counts occurred in the first six months of the treatment and the counts gradually recovered back to pre-treatment level (92). Long-term treatment of MS with IFNβ caused a generalized lymphopenia but not affecting the profiles of T-cell subsets (49). The reduction of T cells by IFNβ treatment might be due to 1) increased apoptosis (42, 44), 2) decreased cell proliferation (79, 97); and 3) enhanced redistribution to lymph nodes and spleen (52, 61).

Table 29: (Var: CYT-15673)

|  | N | | S | B |
|---|---|---|---|---|
|  | T2 v T0, T1 | T2 v T3 | T2 v T1, T3 | T2 v T1, T3 |
| **P*** | 10.25 | 3.33 | 2.09 | 3.83 |
| **ES**** | 0.90 | 0.56 | 0.39 | 0.99 |
| **MR^** | 0.78 | 0.86 | 0.85 | 0.77 |
| *Neg log p-value, **Effect size<br>^ Mean ratio T2/Other | | | | |

Table 30: (Var: CYT-15668)

|  | N | | S | B |
|---|---|---|---|---|
|  | T2 v T0, T1 | T2 v T3 | T2 v T1, T3 | T2 v T1, T3 |
| **P*** | 10.42 | 4.18 | 1.72 | 4.13 |
| **ES**** | 0.96 | 0.66 | 0.39 | 1.12 |
| **MR^** | 0.67 | 0.76 | 0.81 | 0.67 |
| *Neg log p-value, **Effect size<br>^ Mean ratio T2/Other | | | | |

Table 31: (Var:CYT-15635)

|  | N | | S | B |
|---|---|---|---|---|
|  | T2 v T0, T1 | T2 v T3 | T2 v T1, T3 | T2 v T1, T3 |
| **P*** | 2.89 | 2.18 | 5.44 | 2.51 |
| **ES**** | 0.29 | 0.29 | 0.36 | 0.30 |
| **MR^** | 0.91 | 0.92 | 0.88 | 0.92 |
| *Neg log p-value, **Effect size<br>^ Mean ratio T2/Other | | | | |

Table 32: (Var: CYT- 15645)

|  | N | | S | B |
|---|---|---|---|---|
|  | T2 v T0, T1 | T2 v T3 | T2 v T1, T3 | T2 v T1, T3 |
| **P*** | 9.03 | 4.40 | 8.44 | 5.67 |
| **ES**** | 0.54 | 0.38 | 0.43 | 0.41 |

(continued)

|  | N | | S | B |
|---|---|---|---|---|
|  | T2 v T0, T1 | T2 v T3 | T2 v T1, T3 | T2 v T1, T3 |
| MR^ | 0.84 | 0.88 | 0.84 | 0.84 |
| *Neg log p-value, **Effect size<br>^ Mean ratio T2/Other | | | | |

Table 33: (Var: CYT- 15633)

|  | N | | S | B |
|---|---|---|---|---|
|  | T2 v T0, T1 | T2 v T3 | T2 v T1, T3 | T2 v T1, T3 |
| P* | 13.62 | 8.35 | 7.42 | 5.78 |
| ES** | 0.55 | 0.47 | 0.47 | 0.62 |
| MR^ | 0.74 | 0.78 | 0.76 | 0.75 |
| *Neg log p-value, *Effect size<br>^ Mean ratio T2/Other | | | | |

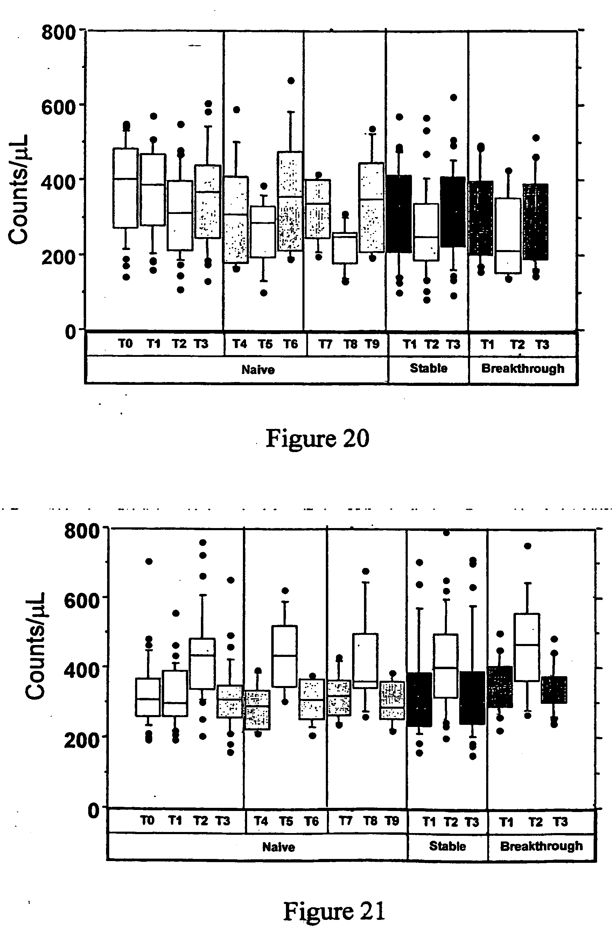

**The striking exception to the decreasing trend was monocytes.** Monocytes were dramatically increased short-term post injection7 **(Figure 21, Table 34).** The magnitude of the increase ranges from 23 to 38% and the effect size ranges from 0.6 to 1.2. The results were consistent among the different comparisons. The increase in monocyte counts was transient and back to steady stat levels six days after dose.

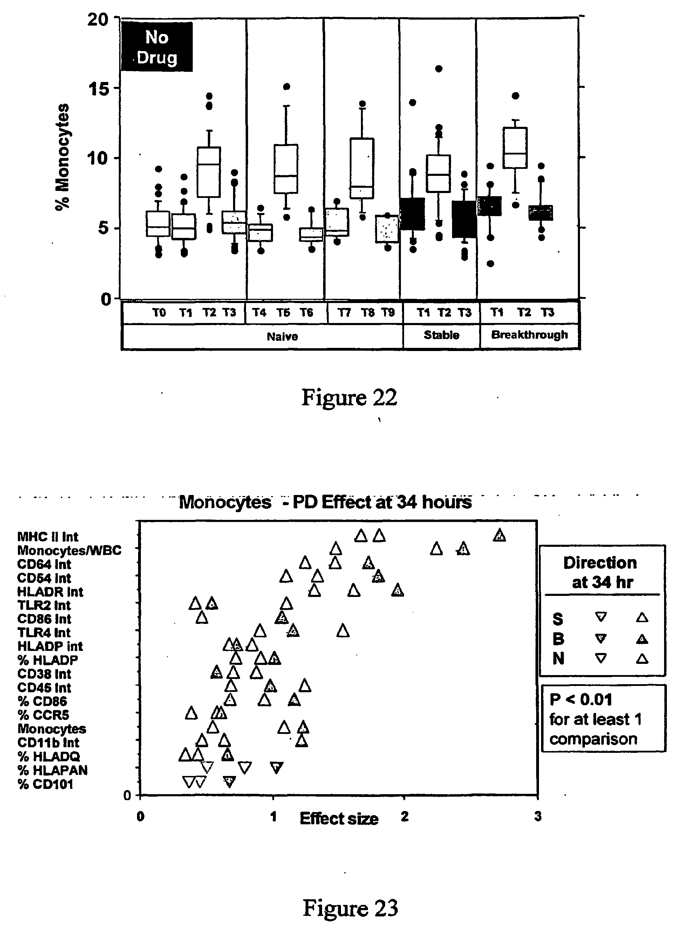

A more dramatic difference was apparent for the monocyte/leukocyte ratio, since monocytes increase and total leukocytes decrease **(Figure 22, Table 35).** The magnitude of the increase ranged from 50 to 75 % and the effect size ranges from 1.5 to 2.5. The results are consistent among the different comparisons with the strongest effect observed in the comparison within the Naïve group before drug.

Table 34: (Var: CYT-15662)

|  | N | | S | B |
|---|---|---|---|---|
|  | T2 v T0, T1 | T2 v T3 | T2 v T1, T3 | T2 v T1, T3 |
| P* | 15.71 | 14.28 | 5.84 | 5.57 |
| ES** | 1.08 | 0.97 | 0.55 | 1.23 |
| MR^ | 1.35 | 1.38 | 1.23 | 1.33 |
| *Neg log p-value, **Effect size<br>^ Mean ratio T2/Other | | | | |

Table 35: (Var: CYT-15665)

|  | N | | S | B |
|---|---|---|---|---|
|  | T2 v T0, T1 | T2 v T3 | T2 v T1, T3 | T2 v T1, T3 |
| P* | 27.34 | 20.80 | 14.46 | 11.19 |
| ES** | 2.24 | 1.50 | 1.49 | 2.45 |
| MR^ | 1.75 | 1.65 | 1.52 | 1.64 |
| *Neg log p-value, **Effect size<br>^ Mean ratio T2/Other | | | | |

**Cell population subsets**

Many of the cytometry variables that showed a pharmacodynamic effect short-term after injection are subsets of the major cell populations. Most of these subset changes track with their parent populations. For example, many subsets of B cells, CD4 and CD8 T cells and neutrophils change in proportion to the parent population. Therefore these changes are not considered independent findings and are discussed further. However, some of the subset changes do not track with their parent populations. The ratios of these subsets to their parent population showed significant changes short-term after dose. Many monocyte subset changes belong to this category and are discussed in the sections below. We also discuss differences in the level of expression (intensity) of individual antigens on specific cell types. In addition, we discuss some of the soluble factors differences identified by immunoassay and mass spectrometry that may be biologically related to the cell population.

**Monocyte subsets and related markers**

The strongest and most prevalent short-term pharmacodynamic effects are observed on monocytes. Many monocyte-related markers show significant differences short-term post injection. The effect size plot in **Figure 23**, identifies some of these with p-values <0.01. Note two variables, MHC class II expression and the fraction of monocytes, have effect sizes near or greater than two. These and other variables are discussed in detail.

**HLA-Class II.**

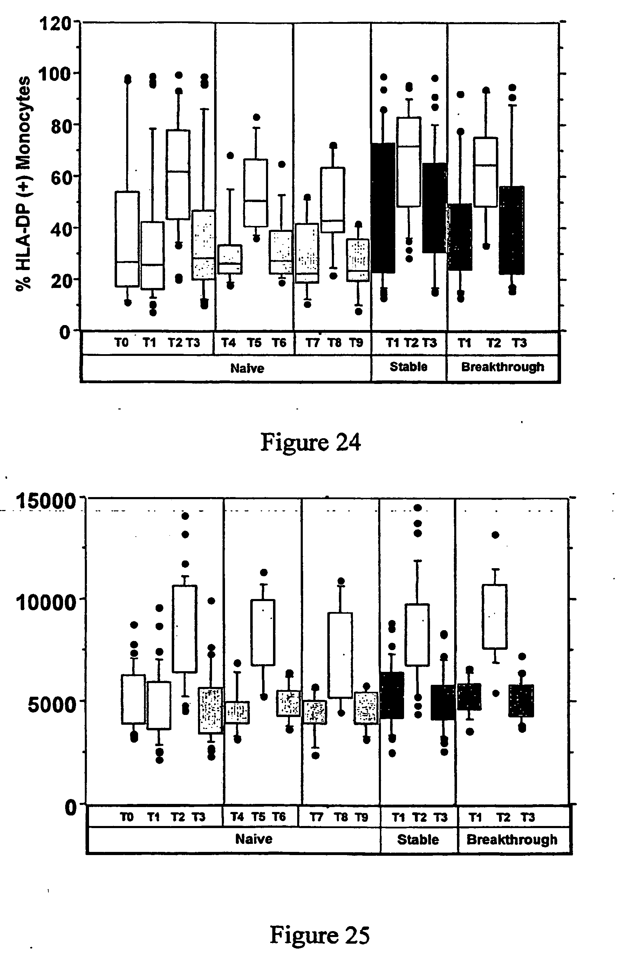

Class II major histocompatibility antigens were increased on monocytes short-term after injection. **Table 36** provides p-values, effect size and mean ratio for the three major antigens, DP, DQ and DR as well as results with a pan HLA marker that recognizes all three molecules. As a percent of monocytes the largest increase and best effect size was observed for HLA-DP, which has a range of 20 to 80% positive cells in the assay. **Figure 24** and **Table 37** show consistent short-term increase in HLA-DP expressing monocytes in all treatment groups. Most monocytes are HLA-DR and HLA-PAN positive in the assays, so changes were less apparent as a percent of monocytes. It is helpful to look at intensity values for the four antigens.

The expression of all four HLA Class II markers increased on monocytes-short-term post injection. This is one of the strongest and most consistent pharmacodynamic results. Intensities are up around two-fold for HLA-DP, DR and PAN, with effect sizes generally greater than 1. Distributions are shown in **Figure 25** and **Table 38**. The increase for HLA-DQ, is modest, ranging from 30 to 50%. The effect of IFN-beta on class II expression on monocytes is complex. Both in vivo and in vitro results show that IFN-beta increases class II molecule expression on monocytes (5,43,106,111). However in the presence of IFN-gamma, IFN-beta inhibits IFN-gamma induced class II expression on monocytes (65). Monocytes from active MS patients express lower levels of class II molecules than normal monocytes. The deficient expression of HLA-DR on MS monocytes causes reduced suppressor function in activated autologous T cells and may be an important abnormality in the immunopathogenesis of MS (7, 8). Thus the increase of class II expression on monocytes by IFN-beta may contribute to the immunomodulatory effect in MS.

The HLA Class II antigens were also expressed on B cells. For comparison these results are also presented in **Table 36**. In general there is a small increase (10%) in HLA expression on B cells. In vitro treatment with IFN-beta increases (86) or has no effect (71) on B cells.

HLA Class I, measured with a pan marker recognizing HLA-A, B and C, was increased 20-40% on total leukocytes. This result agrees with previous reports (2, 106).

**Table 36: MHC class II expression on Monocytes and B cells**

| CYT ID | Cell type | Variable | [-log(P-values)] | | | | Effect Size | | | | Mean Ratio* | | | |
|---|---|---|---|---|---|---|---|---|---|---|---|---|---|---|
| | | | N T0-1 V T2 | N T3 V T2 | ST1-3 V T2 | B T1-3 V T2 | N T0-1 V T2 | NT3 V T2 | ST1-3 V T2 | B T1-3 V T2 | N T0-1 V T2 | NT3 V T2 | ST1-3 V T2 | B T1-3 V T2 |
| 16489 | Monocytes | % HLA-DP | 20.1 | 7.4 | 11.5 | 8.7 | 0.9 | 1.1 | 0.7 | 1.0 | 1.6 | 1.6 | 1.3 | 1.6 |
| 16501 | Monocytes | % HLA-DQ | 10.5 | 6.5 | 6.7 | 6.3 | 0.3 | 0.4 | 0.4 | 0.7 | 1.4 | 1.4 | 1.4 | 1.7 |
| 16513 | Monocytes | % HLA-DR | 7.9 | 4.0 | 1.4 | 3.4 | 0.7 | 0.6 | 0.3 | 1.1 | 1.1 | 1.0 | 1.0 | 1.1 |
| 16548 | Monocytes | % HLA-PAN | 7.2 | 5.2 | 2.2 | 3.0 | 0.8 | 1.1 | 0.5 | 1.0 | 1.1 | 1.0 | 1.0 | 1.0 |
| 18121 | Monocytes | Int - HLA-DP | 18.0 | 9.3 | 18.3 | 7.7 | 1.0 | 0.8 | 1.2 | 1.4 | 2.1 | 2.2 | 1.8 | 2.1 |
| 18136 | Monocytes | Int- HLA-DQ | 8.0 | 7.7 | 4.5 | 5.2 | 0.3 | 0.3 | 0.4 | 0.5 | 1.3 | 1.4 | 1.3 | 1.5 |
| 18151 | Monocytes | Int - HLA-DR | 24.3 | 10.2 | 143 | 8.9 | 1.6 | 1.2 | 1.3 | 2.0 | 1.8 | 1.9 | 1.6 | 1.9 |
| 18196 | Monocytes | Int - HLA-PAN | 30.0 | 26.3 | 19.4 | 9.5 | 1.8 | 1.5 | 1.7 | 2.8 | 1.8 | 1.8 | 1.6 | 1.8 |
| 18118 | B cells | Int - HLA-DP | 4.1 | 3.1 | 5.0 | 2.9 | 0.3 | 0.3 | 0.3 | 0.2 | 1.1 | 1.1 | 1.1 | 1.1 |
| 18133 | B cells | Int - HLA-DQ | 4.4 | 3.1 | 1.1 | 1.4 | 0.3 | 0.3 | 0.1 | 0.3 | 1.1 | 1.1 | 1.0 | 1.1 |
| 18148 | B cells | Int - HLA-DR | 6.2 | 3.8 | 0.7 | 1.9 | 0.5 | 0.4 | 0.1 | 0.4 | 1.1 | 1.1 | 1.0 | 1.1 |
| 18193 | B cells | Int - HLA-PAN | 5.5 | 1.8 | 1.2 | 1.8 | 0.5 | 0.2 | 0.2 | 0.3 | 1.1 | 1.1 | 1.0 | 1.1 |
| 18109 | WBC | Int - HLA ABC | 24.4 | 15.9 | 9.1 | 10.9 | 1.92 | 1.21 | 1.10 | 2.03 | 1.42 | 1.32 | 1.20 | 1.32 |
| *T2/Steady State | | | | | | | | | | | | | | |

Table 37: (Var: CYT- 16489)

|  | N | | S | B |
|---|---|---|---|---|
|  | T2 v T0, T1 | T2 v T3 | T2 v T1, T3 | T2 v T1, T3 |
| P* | 20.1 | 15.5 | 11.5 | 8.7 |
| ES** | 0.91 | 1.1 | 0.73 | 1.0 |
| MR^ | 1.62 | 1.60 | 1.35 | 1.57 |
| *Neg log p-value, **Effect size<br>^ Mean ratio T2/other | | | | |

Table 38: (Var: CYT- 18196)

|  | N | | S | B |
|---|---|---|---|---|
|  | T2 v T0, T1 | T2 v T3 | T2 v T1, T3 | T2 v T1, T3 |
| P* | 30.04 | 26.35 | 19.38 | 9.55 |
| ES** | 1.81 | 1.48 | 1.65 | 2.78 |
| MR^ | 1.77 | 1.81 | 1.63 | 1.77 |
| *Neg log p-value, **Effect size<br>^ Mean ratio - T2/Other | | | | |

**Other markers on monocytes.**

The intensities of CD38, CD40, CD54, CD64, CD69 CD86, TLR2, and TLR4 on monocytes were significantly increased short-term after Avonex injection in all three MS groups. The strongest effect was observed in the Naïve group initial after dose. The intensities of these markers on monocytes are presented. The increase of CD38 expression shortly after injection is also observed on B cells, CD4 T cells and CD8 T cells.

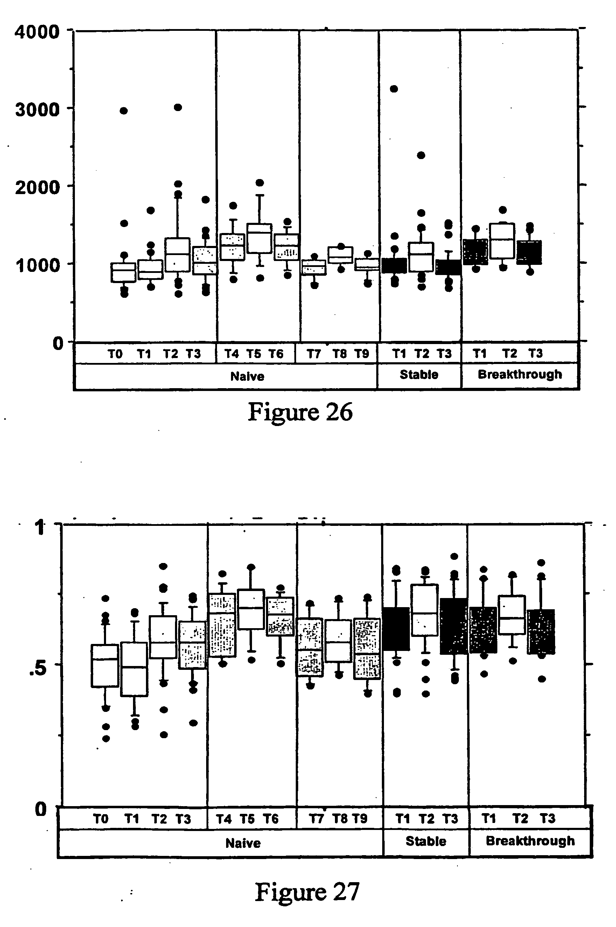

CD38 results are shown in Figures 26-28 and Table 39, Table 40, and Table 41 for both monocytes and B cells. CD38 has multiple enzymatic activities. It functions as a positive and negative regulator of cell activation and proliferation and is involved in lymphocyte-endothelial cell adhesion (31, 32). The quantitative measurement of CD38 expression on CD8 T cells has been used in predicting prognosis of AIDS (16). The expression level of CD38 on B cells may have prognostic value in B cell chronic lymphocytic leukemia (69). In this study, CD38 expression on monocytes, B cells, and T cells was significantly up-regulated by IFN-beta.

Table 39: (Var: CYT-17374)

|  | N | | S | B |
|---|---|---|---|---|
|  | T2 v T0, T1 | T2 v T3 | T2 v T1, T3 | T2 v T1, T3 |
| P* | 16.25 | 7.12 | 4.29 | 3.22 |
| ES** | 0.83 | 0.47 | 0.51 | 0.61 |
| MR^ | 1.44 | 1.25 | 1.19 | 1.21 |
| *Neg log p-value, **Effect size<br>^ Mean ratio - T2/Other | | | | |

Table 40: (Var: CYT-15948)

|  | N | | S | B |
|---|---|---|---|---|
|  | T2 v T0, T1 | T2 v T3 | T2 v T1, T3 | T2 v T1, T3 |
| P* | 11.80 | 0.57 | 1.99 | 2.42 |

(continued)

| | N | | S | B |
|---|---|---|---|---|
| | T2 v T0, T1 | T2 v T3 | T2 v T1, T3 | T2 v T1, T3 |
| ES** | 0.82 | 0.13 | 0.28 | 0.43 |
| MR^ | 1.19 | 1.03 | 1.05 | 1.07 |
| *Neg log p-value, **Effect size ^ Mean ratio T2/Other | | | | |

Table 41: (Var: CYT-17386)

| | N | | S | B |
|---|---|---|---|---|
| | T2 V T0, T1 | T2 v T3 | T2 v T1, T3 | T2 v T1, T3 |
| P* | 9.89 | 3.02 | 3.35 | 3.98 |
| ES** | 0.98 | 0.42 | 0.64 | 0.65 |
| MR^ | 1.40 | 1.19 | 1.18 | 1.17 |
| *Neg log p-value, **Effect size ^ Mean ratio T2/Other | | | | |

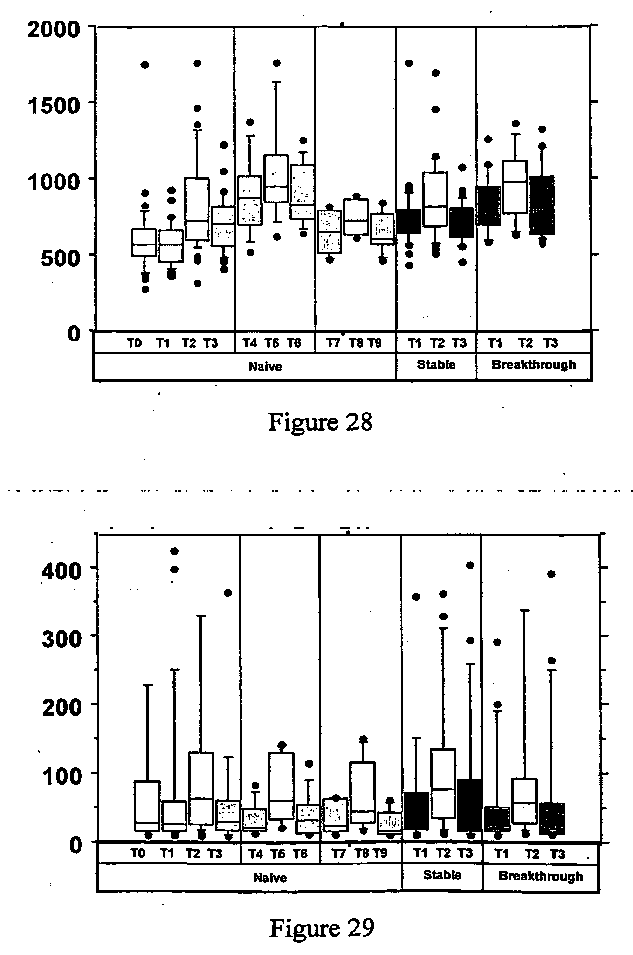

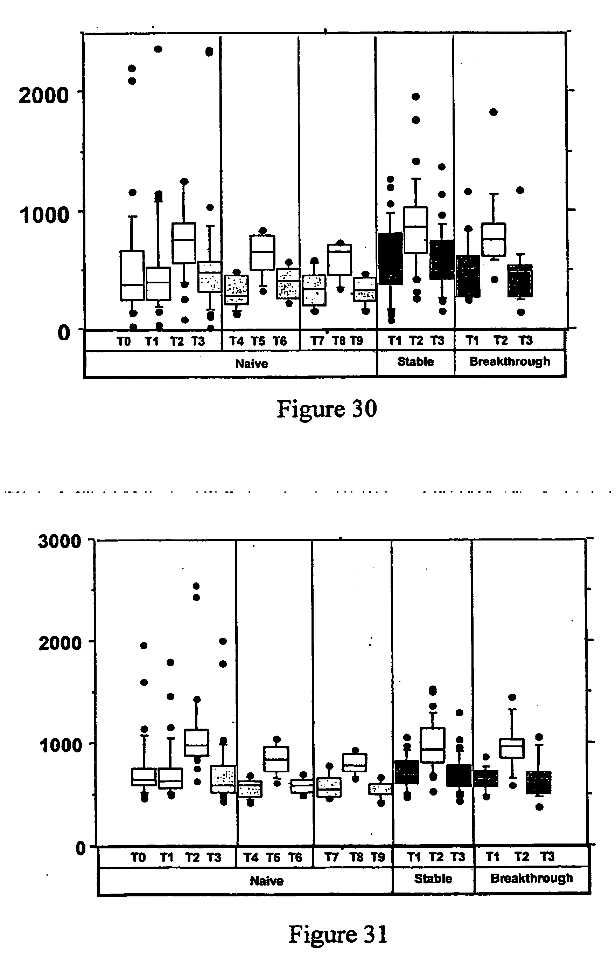

**CD40 and CD86** results for monocytes are shown in **Figures 29 and 30** and **Table 42**, and **Table 43**. They are involved in T cell co-stimulation. CD40 and CD86 expression on monocytes, but not on B cells was increased shortly after IFN-beta injection.

Table 42: (Var: CYT-17410)

| | N | | S | B |
|---|---|---|---|---|
| | T2 v T0, T1 | T2 v T3 | T2 V T1, T3 | T2 v T1, T3 |
| P* | 2.48 | 1.90 | 2.18 | 2.11 |
| ES** | 0.29 | 0.21 | 0.19 | 0.35 |
| MR^ | 1.85 | 1.85 | 1.33 | 1.61 |
| *Neg log p-value, **Effect size ^ Mean ratio T2/Other | | | | |

Table 43: (Var: CYT-17986)

| | N | | S | B |
|---|---|---|---|---|
| | T2 V T0, T1 | T2 v T3 | T2 v T1, T3 | T2 v T1, T3 |
| P* | 14.45 | 9.58 | 13.23 | 7.79 |
| ES** | 0.68 | 0.49 | 0.93 | 1.21 |
| MR^ | 1.69 | 1.56 | 1.50 | 1.67 |
| *Neg log p-value, **Effect size ^ Mean ratio T2/Other | | | | |

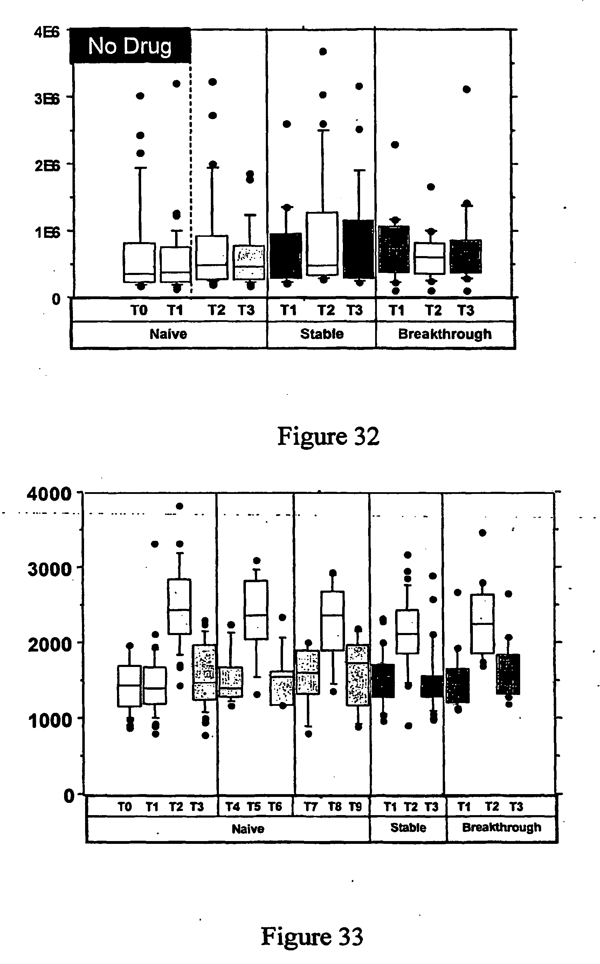

**CD54**, ICAM-1, is fundamental for adhesion of leukocytes to endothelial cells. A soluble form of ICAM-1 (sICAM-1) exists in human serum and is used as marker for disease activity in MS. Higher levels of sICAM-1 have been detected in MS patients benefiting from IFN-beta treatment (**41, 114**). We found that CD54 (mICAM-1) expression on monocytes was increased short term after IFN-beta treatment (**Figure 31** and **Table 44**). This increase is consistent among all three MS groups. However the serum levels of sICAM-1 did not change substantially short-term post dosing Break through (**Figure 32** and **Table 45**). Serum levels of sICAM-1 in the Breakthrough group were decreased 20% short-term after IFN-beta injection with a modest p-value.

Table 44: (Var: CYT-17539)

|  | N | | S | B |
|---|---|---|---|---|
|  | T2 v T0, T1 | T2 v T3 | T2 v T1, T3 | T2 v T1, T3 |
| **P*** | 23.1 | 18.9 | 15.8 | 8.7 |
| **ES**** | 0.96 | 0.84 | 1.1 | 1.7 |
| **MR^** | 1.59 | 1.59 | 1.41 | 1.57 |
| *Neg log p-value, **Effect size<br>^ Mean ratio T2/Other | | | | |

Table 45: (Var: IA-0012)

|  | N | | S | B |
|---|---|---|---|---|
|  | T2 v T0, T1 | T2 v T3 | T2 v T1, T3 | T2 V T1, T3 |
| **P*** | 0.72 | 0.78 | 0.48 | 1.33 |
| **ES**** | 0.16 | 0.23 | 0.14 | 0.22 |
| **MR^** | 1.18 | 1.23 | 1.17 | 0.81 |
| *Neg log p-value, **Effect size<br>^ Mean ratio T2/Other | | | | |

**CD64** was increased on monocytes short-term post injection in all groups (**Figure 33**, Table 46). It is a high affinity Fc gamma receptor expressed on monocytes, macrophages, and granulocytes at very low levels. It is involved in phagocytosis, respiratory burst, antibody-dependent cell-mediated cytotoxicity (ADCC) and cytokine secretion.

**Table 46**: (Var: CYT-17749)

|  | N | | S | B |
|---|---|---|---|---|
|  | T2 v T0, T1 | T2 v T3 | T2 v T1, T3 | T2 v T1, T3 |
| **P*** | 22.5 | 14.3 | 13.4 | 10.5 |
| **ES**** | 1.22 | 0.82 | 1.42 | 1.74 |
| **MR^** | 1.75 | 1.55 | 1.41 | 1.49 |
| *Neg log p-value, **Effect size<br>^ Mean ratio T2/Other | | | | |

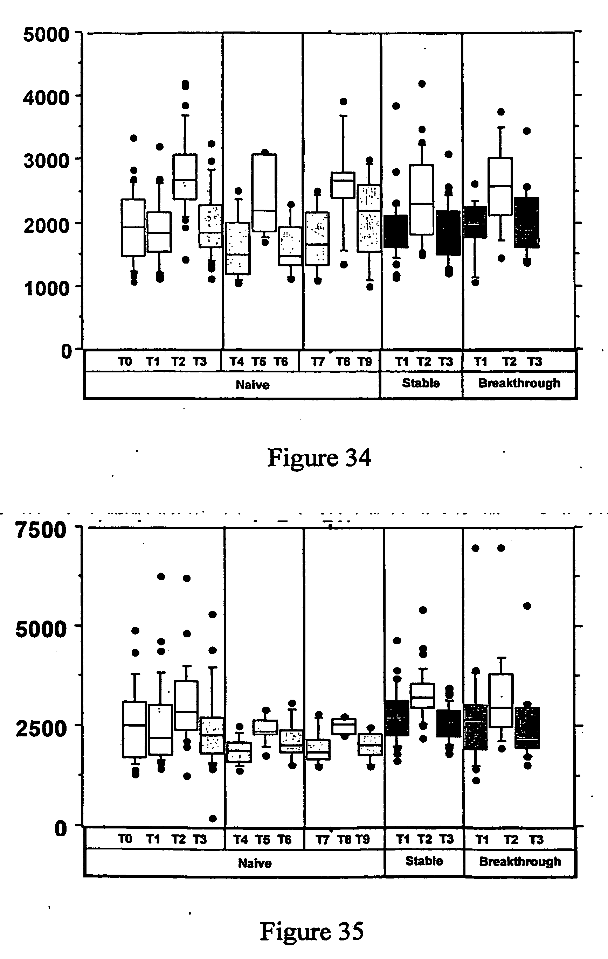

**TLR2 and TLR4** on monocytes were both increased short-term post injection in all groups (**Figures 34-35** and **Table 47**, and **Table 48**). They are highly expressed on monocytes and are involved in the innate immune response to microbes. The increase of TLR2 and TLR4 on monocytes after Avonex injection that we observed was transient and returned to pretreatment levels six days after injection. The responses are consistent across all three MS groups.

Table 47: (Var: CYT- 18646)

|  | N | | S | B |
|---|---|---|---|---|
|  | T2 v T0, T1 | T2 v T3 | T2 v T1, T3 | T2 v T1, T3 |
| **P*** | 11.90 | 9.54 | 4.74 | 5.22 |
| **ES**** | 1.44 | 1.22 | 0.92 | 1.39 |

(continued)

|  | N | | S | B |
|---|---|---|---|---|
|  | T2 v T0, T1 | T2 v T3 | T2 v T1, T3 | T2 v T1, T3 |
| MR^ | 1.48 | 1.51 | 1.28 | 1.40 |
| *Neg log p-value, **Effect size<br>^ Mean ratio T2/Other | | | | |

Table 48: (Var: CYT-18640)

|  | N | | S | B |
|---|---|---|---|---|
|  | T2 v T0, T1 | T2 v T3 | T2 v T1, T3 | T2 v T1, T3 |
| P* | 6.07 | 4.91 | 12.95 | 5.59 |
| ES** | 0.42 | 0.33 | 1.11 | 0.55 |
| MR^ | 1.27 | 1.27 | 1.23 | 1.24 |
| *Neg log p-value, **Effect size<br>^ Mean ratio T2/Other | | | | |

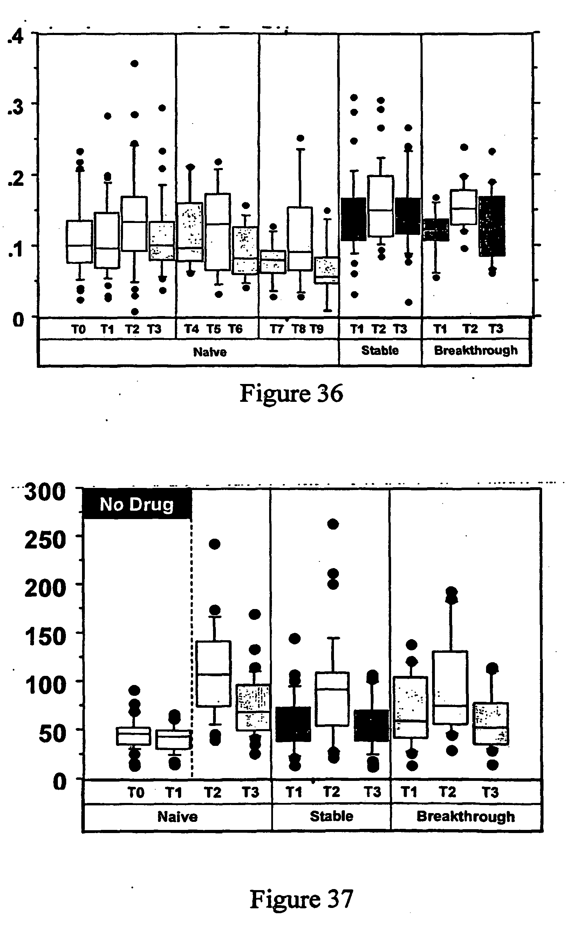

**CD69** is expressed very early during the activation of lymphocytes and monocytes. It is increased on monocytes about 20% short term post dosing. The result had a p-value < 0.05 for the Naïve, and Breakthrough groups, but not the Stable-group. **Figure 36** and **Table 49**

**Table 49**: (Var: CYT- 16261)

|  | N | | S | B |
|---|---|---|---|---|
|  | T2 V T0, T1 | T2 v T3 | T2 v T1, T3 | T2 v T1, T3 |
| P* | 2.16 | 1.50 | 1.22 | 2.27 |
| ES** | 0.45 | 0.33 | 0.33 | 0.79 |
| MR^ | 1.25 | 1.22 | 1.13 | 1.26 |
| *Neg log p-value, **Effect size<br>^ Mean ratio T2/Other | | | | |

## Monocyte related cytokine

Serum levels of monocyte chemoattractant protein-2 (MCP-2), IFN-inducible protein 10 (IP-10), IL-1 receptor antagonist (IL-1RA), and sVCAM were significantly increased short term after Avonex injection. The increase was transient and their levels were back to steady state in Stable and Breakthrough groups. For naive group their levels were reduced at day 6 after injection but maintain at higher levels than before treatment (**Figures 37-39** and **Table 50, Table 51**, and **Table 52**).

**MCP-2** belongs to monocyte chemotactic protein family and specifically attracts monocytes, but not neutrophils. It is secreted by various cell types, including fibroblasts, epithelial cells, and leukocytes.

Table 50: (Var: IA-17)

|  | N | | S | B |
|---|---|---|---|---|
|  | T2 v T0, T1 | T2 v T3 | T2 v T1, T3 | T2 v T1, T3 |
| P* | 23.9 | 9.4 | 8.4 | 3.4 |
| ES** | 2.1 | 0.89 | 0.88 | 0.59 |

(continued)

|  | N | | S | B |
|---|---|---|---|---|
|  | T2 v T0, T1 | T2 v T3 | T2 v T1, T3 | T2 v T1, T3 |
| MR^ | 2.52 | 1.51 | 1.55 | 1.45 |
| *Neg log p-value, **Effect size ^ Mean ratio T2/Other | | | | |

Table 51: (Var: IA-15 (pg/mL))

|  | N | | S | B |
|---|---|---|---|---|
|  | T2 v T0, T1 | T2 v T3 | T2 v T1, T3 | T2 v T1, T3 |
| P* | 21.84 | 10.39 | 11.86 | 6.91 |
| ES** | 1.92 | 1.00 | 1.33 | 1.45 |
| MR^ | 2.70 | 1.74 | 1.86 | 1.76 |
| *Neg log p-value, **Effect size ^Mean ratio T2/Other | | | | |

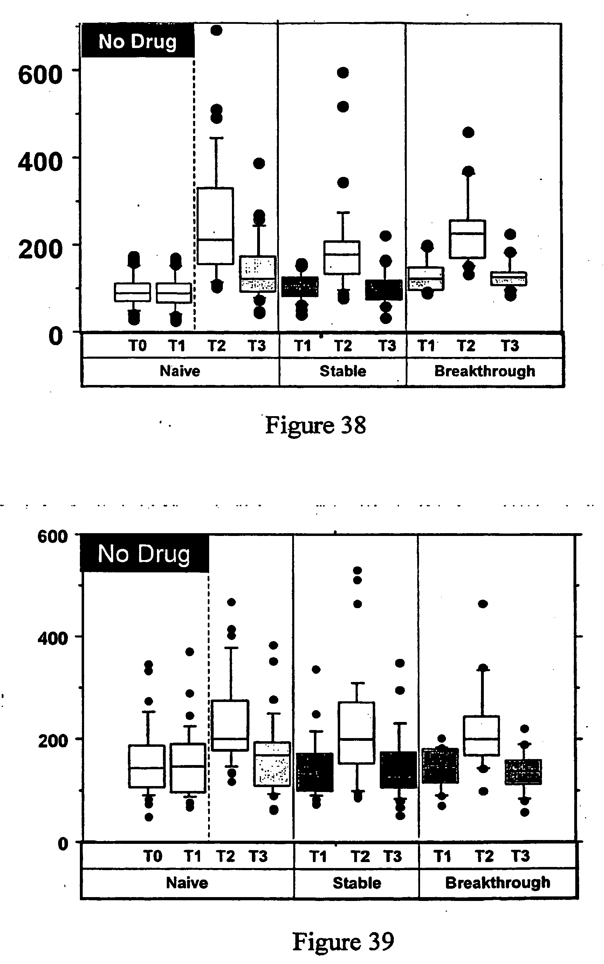

**IL-1 receptor antagonist (IL-1-Ra)** was increased about 50% short- term after Avonex dosing for all MS groups (**Figure 39, Table 52**). IL1-Ra is a member of IL-1 family and binds to competitively to IL-1RI without inducing signal transduction, and thus inhibits IL-1a and IL-1β actions.

Table 52: (Var: IA-27)

|  | N | | S | B |
|---|---|---|---|---|
|  | T2 v T0, T1 | T2 v T3 | T2 v T1, T3 | T2 v T1, T3 |
| P* | 17.28 | 12.36 | 11.39 | 5.09 |
| ES** | 1.04 | 0.87 | 0.90 | 1.06 |
| MR^ | 1.49 | 1.42 | 1.50 | 1.55 |
| *Neg log p-value, **Effect size ^ Mean ratio - T2/Other | | | | |

Interleukin-18 binding protein precursor (IL-18BP) (Accession No. 095998 and NP_766630.1). IL-18BP levels in urine were up-regulated short-term post Avonex injection. Two peptides were identified, both with identity scores >40 (Table 53). The increase was about two- fold and was strongest and most in the Naïve group at their initial visit. IL18 in the serum increased about 30% in the Naïve Group (p-value <0.001) short-term post injection. It does not change significantly for the established Avonex groups.

Table 53: IL18BP

|  | N T0-1vT2 | N T3vT2 | S T1-3vT2 | B T1-3vT2 | N T0-1vT2 | N T3vT2 | S T1-3vT2 | B T1-3vT2 |
|---|---|---|---|---|---|---|---|---|
| VAR: UR-LC-PT-0000408715 | | | | UR-LC-PT-0000408795 | | | | |
| P | 9.08 | 6.66 | 9.94 | 2.82 | 3.24 | 1.03 | 0.90 | 2.41 |
| ES | 1.40 | 0.89 | 1.20 | 1.05 | 1.18 | 0.39 | 0.34 | 0.88 |
| MR | 2.19 | 2.01 | 1.68 | 1.60 | 2.25 | 1.39 | 1.93 | 1.88 |
| *Neg log p-value, **Effect size ^ Mean ratio - T2/Other | | | | | | | | |

**Acute phase reactants**

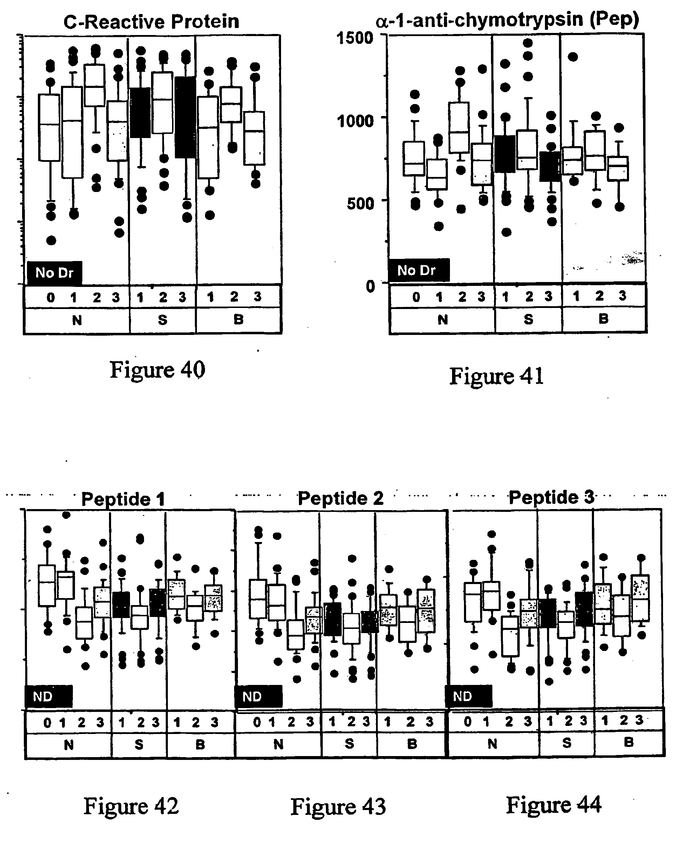

Some acute phase reactants showed a short-term pharmacodynamic effect. Results are shown for CRP and an ACT peptide (**Figures 40, 41** and Table 54, and Table 55). The strongest effect was observed in the Naive group, when subjects first go on Avonex. Similar results were observed for peptides from other acute phase: ceruloplasmin (Ferroxidase), haptoglobin, $\alpha$-1-acid glycoprotein (Orosmucoid) serum amyloid A.

Table 54: (Var: IA-5)

|  | **N** |  | **S** | **B** |
|---|---|---|---|---|
|  | T2 v T0, T1 | T2 v T3 | T2 v T1, T3 | T2 v (T1, T3 |
| **P\*** | 7.51 | 7.00 | 1.37 | 4.79 |
| **ES\*\*** | 0.89 | 0.95 | 0.26 | 0.48 |
| **MR^** | 2.40 | 2.84 | 1.34 | 1.85 |
| *Neg log p-value, **Effect size<br>^ Mean ratio - T2/Other | | | | |

Table 55: (Var: SE-LC-PROT 158973

|  | **N** |  | **S** | **B** |
|---|---|---|---|---|
|  | T2 v T0, T1 | T2 V T3 | T2 v T1, T3 | T2 v T1, T3 |
| **P\*** | 6.74 | 3.63 | 0.91 | 1.87 |
| **ES\*\*** | 1.20 | 0.82 | 0.24 | 0.58 |
| **MR^** | 1.29 | 1.21 | 1.06 | 1.11 |
| *Neg log p-value, **Effect size<br>^ Mean ratio - T2/Other | | | | |

**Apolipoproteins**

Multiple apolipoproteins show short-term pharmacodynamic effects. ApoH (Beta-2-Glycoprotein I) was identified with two accession numbers (NBHU and P02749). Results for three consistent peptides are shown in **Figures 42** and **Table 56**). Apo H was decreased 10 to 30% short-term Avonex treatment. The untreated Avonex group had the highest level of ApoH and the difference 34 hours post injection was greatest for this group.

Table 56: (Var: SE-LC-PROT 161593,161904,16189)

Pep1: KCSYTEDAQCIDGTIEVPK (SEQ ID NO:2),
Pep 2: ATFGCHDGYSLDGPEEIECTK (SEQ ID NO:3),
Pep 3: TFYEPGEEITYSCKPGYVSR (SEQ ID NO:4)

|  | **N** | | **S** | **B** | **N** | | **S** | **B** | **N** | | **S** | **B** |
|---|---|---|---|---|---|---|---|---|---|---|---|---|
|  | T2 v T0, T1 | T2 v T3 | T2 v T1, T3 | T2 v T1, T3 | T2 v T0, T1 | T2 v T3 | T2 v T1, T3 | T2 v T1, T3 | T2 v T0, T1 | T2 v T3 | T2 v T1, T3 | T2 v T1, T3 |
| **P\*** | 13.93 | 3.63 | 2.80 | 3.32 | 12.22 | 2.55 | 2.34 | 4.86 | 14.23 | 4.37 | 3.16 | 4.33 |
| **ES\*\*** | 1.39 | 0.66 | 0.33 | 0.72 | 1.13 | 0.50 | 0.27 | 0.59 | 1.52 | 0.91 | 0.39 | 0.61 |
| **MR^** | 0.71 | 0.84 | 0.92 | 0.88 | 0.73 | 0.86 | 0.93 | 0.87 | 0.70 | 0.82 | 0.91 | 0.86 |
| *Neg log p-value, **Effect size ^ Mean ratio - T2/Other | | | | | | | | | | | | |

**EXAMPLE 3**

**BETWEEN GROUP COMPARISONS**

**IDENTIFICATION OF MARKERS FOR DISEASE AND LONG-TERM DRUG**

This section focuses on differences among the four study cohorts: Healthy, Naive, Stable and Breakthrough. Results reflect longer-term differences that may be related to disease pathophysiology, disease progression, the presence or absence of drug or efficacy of treatment. The effects were expected to be more modest than those observed for the short-term pharmacodynamic effect discussed above.

**Key Questions**. The key cross group comparisons are listed in **Table 57**

The first three statistical comparison to considered in Table 57 compare Healthy and MS subjects. These comparisons were done using the repeated measures at T1 and T3 for each for the Healthy, Stable and Breakthrough groups and repeat measure, before any drug treatment, at T01 and T1 for the Naive group.

● Were there differences between Naive and Healthy subjects that reflect disease?

This is of primary interest because it is the only comparison between MS and Healthy subjects made in the absence of drug. Differences may have a direct relation to disease pathophysiology.

• Were there differences between Avonex treated and Healthy subjects?

Comparisons between Stable or Breakthrough and Healthy subjects were more ambiguous because they can be related to either disease or the presence of drug. One might also expect to observe a trend for some markers relative to the healthy group indicating a shift to a healthy phenotype, with N>B>S.
The final four statistical comparisons to consider in Table 57 make comparisons among MS groups.

● Are there differences between Avonex treated and Naive subjects?

Statistical comparisons were done using the repeat (steady-state) measures for the Avonex treated groups (T1, T3) and the Naive group (T0,T1). The meaning of differences in these comparisons is potentially ambiguous. Differences could reflect the consequences of IFN-β treatment, the presence or absence of drug or disease progression. The Naive group has the shortest history of disease (5 years vs 10 years for the treated groups) and an intermediate level of relapses between the Stable (low) and Breakthrough group (high, see cohort disease history). Consideration of common differences with the MS vs Healthy comparisons may help in interpreting the results.

● Are there differences that distinguish Stable and Breakthrough subjects and reflect treatment response?

This is a very subtle comparison. All subjects have MS and have been on Avonex treatment for a considerable period of time. Differences between the two are potential biomarkers for responders and non-responders. Between group statistical comparisons are done using the both the steady-state (T1, T3) taken six days post injection and the short term sample (T2) taken 34 hours post injection.
There are four additional cross-group comparisons: Stable vs Naïve at 34 hrs, Breakthrough vs Naive at 34 hrs, Stable vs Naive at 6 days, Breakthrough vs Naive at 6 days

**Table 57: Between group comparisons**

| Comparison Class | Group 1 | Group 2 | Expect |
|---|---|---|---|
| MS (early) vs H No drug Disease marker | Healthy (T1,T3) | Naïve (T0,T1) | Differences |
| MS (later) vs H or | Healthy (T1,T3) | Breakthrough (T1,T3) | Differences |
| Drug v No Drug | Healthy (T1,T3) | Stable (T1,T3) | Differences |
| Drug vs No Drug | Stable (T1,T3) | Naive (T0,T1) | Differences |
| Treatment, MS progression | Breakthrough (T1,T3) | Naive (T0,T1) | Differences |

(continued)

| Comparison Class | Group 1 | Group 2 | Expect |
|---|---|---|---|
| MS on Drug | Stable (T1, T3) | Breakthrough (T1,T3) | Differences |
| Responder vs Non-responder | Stable (T2) | Breakthrough (T2) | Differences |

**Summary Statistics - Between Group comparisons**

Differences were observed for multiple comparisons between cohorts on multiple platforms. A summary of the number of hits in this biomarker screen is presented in **Table 58** by analytical platform and ranked by p-value. Note that the number of samples is not the same for each group and each comparison.

The cytometry platform showed many more hits in the between group comparisons than expected by chance. The most differences were observed for the MS vs Healthy comparisons and the Stable vs. Naive comparison (148 to 285 at the p<0.01 level vs 20 by chance for independent measures). The level is a bit less the observed for the short-term pharmacodynamic effect (267 to 467 at the p <0.01 level, **Table 24**). Fewer differences were observed for the three additional comparisons with the Breakthrough group. This may be affected at least in part, by the smaller sample size for the breakthrough group and consequently the lower power for the comparison.

Serum immunoassays also showed more hits then expected by chance. The most hits occured in the Healthy vs MS comparisons.

The mass spectrometry results usually showed more differences than expected by chance for independent variables. Most differences were generally observed for the Healthy vs MS comparisons. Note the subject numbers and hence power, is lower for the mass spectrometry analyses than for the cytometry and immunoassays.

**Stable 58: Summary statistics: Between Group Comparisons**

| | Healthy vs. MS | | | MS Subjects Drug vs No Drug | | Stable vs Breakthrough | |
|---|---|---|---|---|---|---|---|
| | H v N SS* | H v S SS | H v B SS | S v N SS | B v N SS | S v B SS (6 D) | S v B (34 hr) |
| Subjects-CYT, IA | 35, 35 | 35, 35 | 35, 16 | 35,3 5 | 16, 35 | 35, 16 | 35, 16 |
| Subjects-MASS | 35, 23 | 35, 35 | 35, 14 | 35, 23 | 14, 23 | 35, 14 | 35, 14 |
| **Cytometry-1949 Variables** | | | | | | | |
| *P<0.0001* | 58 | 15 | 13 | 12 | 4 | 9 | 0 |
| P<0.001 | 122 | 46 | 60 | 58 | 11 | 32 | 0 |
| P<0.01 | 285 | 148 | 209 | 216 | 44 | 56 | 30 |
| P<0.05 | 527 | 371 | 452 | 496 | 201 | 225 | 110 |
| **Immunoassays-51 Variables** | | | | | | | |
| P<0.0001 | 1 | 2 | 2 | 1 | 0 | 0 | 0 |
| P<0.001 | 3 | 6 | 4 | 0 | 2 | 0 | 0 |
| P<0.01 | 7 | 9 | 5 | 4 | 4 | 2 | 1 |
| P<0.05 | 12 | 16 | 7 | 6 | 6 | 5 | 8 |
| **Proteome -Serum - 6544 Variables** | | | | | | | |
| P<0.0001 | 0 | 0 | 7 | 3 | 0 | 4 | 4 |
| P<0.001 | 2 | 11 | 22 | 19 | 1 | 23 | 21 |
| P<0.01 | 52 | 103 | 124 | 154 | 34 | 152 | 124 |

(continued)

| Proteome -Serum - 6544 Variables | | | | | | | |
|---|---|---|---|---|---|---|---|
| P<0.05 | 309 | 429 | 456 | 593 | 245 | 518 | 563 |

| Metabolome LC -Serum - 4129 Variables | | | | | | | |
|---|---|---|---|---|---|---|---|
| P<0.0001 | 0 | 19 | 4 | 1 | 0 | 3 | 1 |
| P<0.001 | 5 | 59 | 52 | 3 | 1 | 18 | 12 |
| P<0.01 | 61 | 153 | 203 | 42 | 33 | 87 | 103 |
| P<0.05 | 263 | 460 | 531 | 192 | 181 | 317 | 330 |

| Metabolome- GC - Serum-234 Variables | | | | | | | |
|---|---|---|---|---|---|---|---|
| | Healthy vs. MS | | | MS Subjects Drug vs No Drug | | Stable vs Breakthrough | |
| | H v N SS* | H v S SS | H v B SS | S v N SS | B v N SS | S v B SS (6 D) | S v B (34 hr) |
| Subjects-CYT, IA | 35, 35 | 35, 35 | 35, 16 | 35, 35 | 16, 35 | 35, 16 | 35, 16 |
| Subjects-MASS | 35, 23 | 35, 35 | 35, 14 | 35, 23 | 14, 23 | 35, 14 | 35, 14 |
| P<0.0001 | 0 | 0 | 5 | 0 | 0 | 2 | 0 |
| P<0.001 | 0 | 0 | 8 | 0 | 1 | 5 | 4 |
| P<0.01 | 9 | 0 | 18 | 9 | 3 | 20 | 15 |
| P<0.05 | 27 | 4 | 36 | 40 | 12 | 38 | 32 |

| Proteome- Urine 4492 variables | | | | | | | |
|---|---|---|---|---|---|---|---|
| P<0.0001 | 0 | 0 | 29 | 1 | 5 | 14 | 2 |
| P<0.001 | 3 | 4 | 80 | 1 | 20 | 50 | 13 |
| P<0.01 | 27 | 36 | 272 | 29 | 112 | 192 | 85 |
| P<0.05 | 184 | 218 | 645 | 165 | 419 | 480 | 296 |

| Metabolome-LC-Urine-3270 Variables | | | | | | | |
|---|---|---|---|---|---|---|---|
| P<0.0001 | 2 | 2 | 1 | 1 | 0 | 0 | 4 |
| P<0.001 | 16 | 11 | 2 | 5 | 0 | 7 | 8 |
| P<0.01 | 72 | 102 | 39 | 42 | 29 | 37 | 35 |
| P<0.05 | 273 | 391 | 203 | 170 | 217 | 190 | 156 |

| *SS = Steady state, the repeat time point for any cohort: Naïve T0, T1 (before drug), Healthy: T1, T3 (not on drug), Stable, Breakthrough, T1, T3 (on drug) |
|---|

Summary statistics for the DeepLook serum proteome cross group comparisons are presented in **Table 59**. Analysis was done with one T1 sample for all groups. Subject numbers were greater for the Naive and Breakthrough group than in the comparisons above. Many more differences than expected by chance were observed for five of the six comparisons (352 to 1273 vs 160 by chance at the p-value 0.01 level). The exception is the Healthy vs Naive comparison, which only had 39 differences at this level.

**Table 59: Summary statistics: DeepLook Proteome (-Serum - 16,297 Variable)- Between Group Comparisons**

| | Healthy vs. MS Subjects | | | MS Subjects | | |
|---|---|---|---|---|---|---|
| | No Drug | Drug vs No Drug | | Drug vs No Drug | | Drug |
| Subjects- DL | 35, 35 | 35, 35 | 35, 16 | 35, 35 | 16, 35 | 35, 16 |

(continued)

|  | H v N | H v S | H v B | S v N | B v N | S v B |
|---|---|---|---|---|---|---|
| **P<0.0001** | 0 | 43 | 162 | 60 | 121 | 101 |
| **P<0.001** | 1 | 128 | 438 | 140 | 344 | 317 |
|  | **Healthy vs. MS Subjects** | | | **MS Subjects** | | |
|  | **No Drug** | **Drug vs No Drug** | | **Drug vs No Drug** | | **Drug** |
| P<0.01 | 39 | 526 | 1273 | 353 | 1026 | 1125 |
| P<0.05 | 284 | 1596 | 2694 | 1118 | 2266 | 2641 |

### Specific Between Group Results

Specific results from the between group comparisons are discussed below. Variables that were different in multiple cross group comparisons are generally discussed first, with Healthy vs MS groups and drug vs no-drug being major themes as well as some differences among the MS cohorts. Additional specific differences between the stable and Breakthrough cohorts are discussed toward the end. Comparisons are grouped by platform and biological content.

### Major cell populations

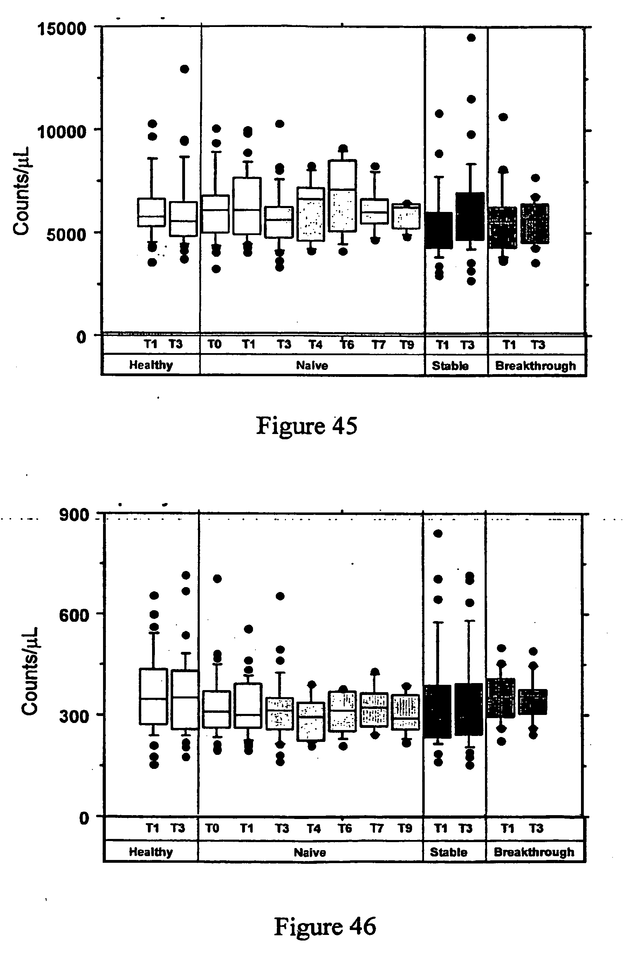

There were fewer differences among the major cell populations in the cross group comparison than observed for the short-term pharmacodynamic effect discussed in Example 2. The absolute counts of total leukocytes, neutrophils, eosinophils and CD4 T cells, were not significantly different among four groups at steady state. A box and whisker plots are shown for total leukocytes in **Figure 45** (also see **Table 62).** Plots from cytometry include results for the Naive subjects before treatment (T0,T1) after the second injection (T3) and at three (T4, T6) and six months (T7,T9). Between-group statistical comparisons were not made for these subjects because the number of continuing subjects was low (an overlapping set of 10 at 3 months and 7 at 6 months).. Monocytes, which showed the most dramatic short-term pharmacodynamic effect, were also not significantly different among the groups **(Figure 46** (also see Table 63)).

**Table 60: Major cell populations - Between Group comparisons**

| | | [-log(p-value)] | | | | | | Mean ratios | | | | | |
|---|---|---|---|---|---|---|---|---|---|---|---|---|---|
| | | HvMS | | | vN | | | HvMS | | | vN | | |
| CYT ID | cell Type | HvN | HvS | HvB | SvN | BvN | SvB | HvN | HvS | HvB | SvN | BvN | SvB |
| 15673 | *WBC* | 0.03 | 0.34 | 0.60 | 0.41 | 0.78 | 0.11 | 1.00 | 0.94 | 0.91 | 1.07 | 1.10 | 0.97 |
| 15656 | Granulocytes | 0.00 | 0.14 | 0.50 | 0.19 | 0.69 | 0.20 | 1.00 | 0.93 | 0.89 | 1.07 | 1.12 | 0.96 |
| 15668 | Neutrophils | 0.38 | 0.00 | 0.15 | 0.36 | 0.72 | 0.13 | 1.08 | 1.00 | 0.96 | 1.07 | 1.12 | 0.96 |
| 15652 | Eosinophils | 0.24 | 0.98 | 0.82 | 0.92 | 0.59 | 0.05 | 0.88 | 0.68 | 0.67 | 1.30 | 1.31 | 1.00 |
| 15633 | B cells | 1.81 | 0.46 | 0.12 | 2.52 | 1.76 | 0.19 | 0.76 | 1.12 | 1.04 | 0.68 | 0.73 | 0.93 |
| 15635 | CD4 T cells | 0.08 | 0.10 | 0.11 | 0.02 | 0.21 | 0.22 | 1.03 | 1.02 | 0.97 | 1.00 | 1.06 | 0.95 |
| 15645 | CD8 T cells | 0.36 | 1.61 | 1.53 | 1.24 | 1.56 | 0.30 | 0.94 | 0.80 | 0.73 | 1.18 | 1.28 | 0.92 |
| 15671 | T cells | 0.03 | 0.23 | 0.60 | 0.31 | 0.86 | 0.37 | 1.01 | 0.96 | 0.89 | 1.05 | 1.13 | 0.93 |
| 15666 | NK cells | 0.48 | 3.07 | 2.47 | 2.12 | 2.00 | 0.30 | 0.90 | 0.70 | 0.64 | 1.28 | 1.39 | 0.92 |
| 15665 | Mono/WBC | 1.30 | 0.04 | 0.54 | 1.03 | 2.35 | 0.53 | 0.89 | 0.99 | 1.08 | 0.90 | 0.83 | 1.09 |
| 15662 | Monocytes | 0.92 | 0.36 | 0.14 | 0.20 | 0.59 | 0.14 | 0.90 | 0.93 | 0.97 | 0.96 | 0.92 | 1.04 |
| 15670 | Platelets | 0.04 | 1.27 | 1.31 | 1.42 | 1.47 | 0.14 | 1.01 | 0.88 | 0.86 | 1.15 | 1.17 | 0.98 |

Table 61: Var: CYT- 15673

|  | NvH | SvH | BvH | SvN | BvN | SvB |
|---|---|---|---|---|---|---|
| P* | 0.03 | 0.34 | 0.60 | 0.41 | 0.78 | 0.11 |
| ES** | 0.02 | 0.17 | 0.30 | 0.20 | 0.37 | 0.09 |
| ^MR | 1.00 | 0.94 | 0.91 | 1.07 | 1.10 | 0.97 |

*Neg log p-value, **Effect size
^Mean ratio: MS/H; N/S,B; B/S

Table 62: Var: CYT- 15662

|  | NvH | SvH | BvH | SvN | BvN | SvB |
|---|---|---|---|---|---|---|
| P* | 0.92 | 0.36 | 0.14 | 0.20 | 0.59 | 0.14 |
| ES** | 0.35 | 0.18 | 0.10 | 0.10 | 0.31 | 0.11 |
| ^MR | 0.90 | 0.93 | 0.97 | 0.96 | 0.92 | 1.04 |

*Neg log p-value, **Effect size
^ Mean ratio: MS/H; N/S,B; B/S

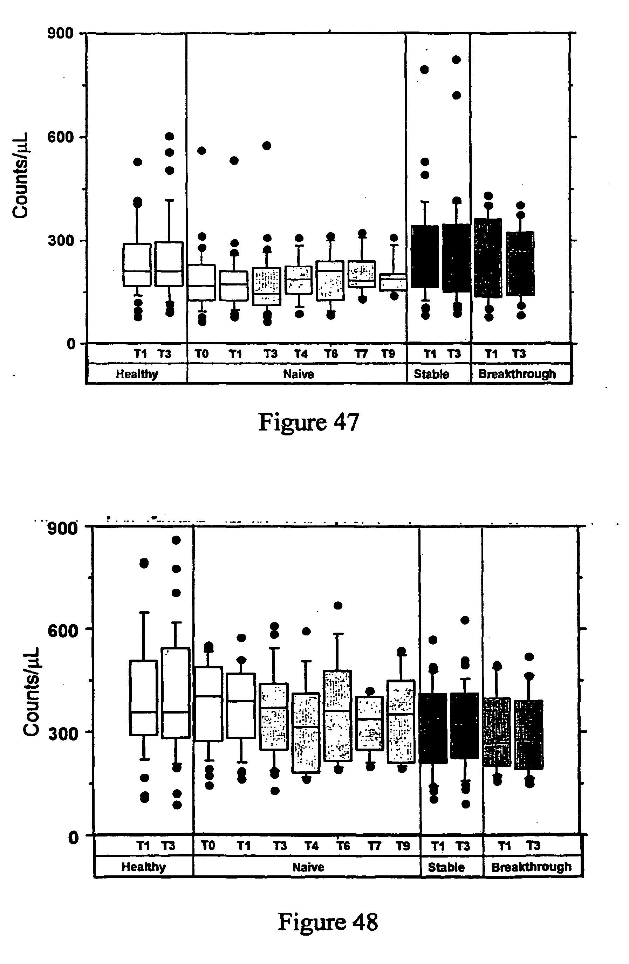

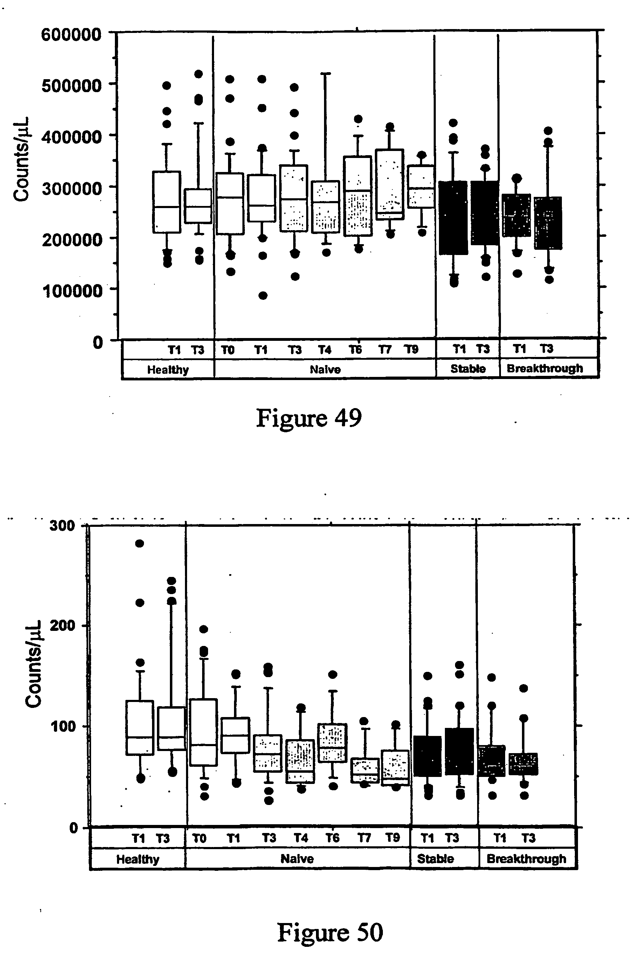

Some major cell populations did show differences among the cohorts **(Table 59,** above). **B cell** counts were lower in Naive subjects pre-treatment. Avonex causes a decrease in B cell counts shortly after injection. However, after long-term Avonex treatment, B cell counts in Stable and Breakthrough subjects are back to the level comparable to that in Healthy subjects **(Figure 47** and **Table 63).** Counts appear to be increasing at three and six months for the Naive subjects continuing on drug, but had not reached the level of the Stable and Breakthrough groups. Avonex treated subjects had lower **CD8 T cell** counts than untreated subjects **(Figure 48 and Table 64).** There were no differences in CD8 T cell counts between Naive and Healthy subjects. Similarly, total platelets were lower in long-term Avonex treated subjects **(Figure 49 and Table 65).** There were no differences in platelet counts between Naïve and Healthy subjects.
**NK cells** were lower in Avonex treated subjects than untreated subjects by 30-40% **(Figure 50 and Table 66).** The decreasing trend was observed for the Naive subjects that continued at three and six months. The result is supported by a phase p-value from the mixed effect model of $<10^{-12}$. INF-beta treatment of relapsing-remitting MS patients induces a rapid and marked reduction in number of NK cells. This reduction occurs one month after initiation of the IFN-beta treatment reaches the peak at three months and then sustains. In the subjects who developed anti-IFN-beta antibodies NK cell counts are back to pretreatment level (84). It is also reported that NK cell functional activity is inversely related to the total number of active lesion on MRI (58). Our results on NK cells suggest that NK cells might be one of the immunological targets of INF-beta based treatment.

Table 63: (Var: CYT- 15633)

|  | NvH | SvH | BvH | SvN | BvN | SvB |
|---|---|---|---|---|---|---|
| P* | 1.81 | 0.46 | 0.12 | 2.52 | 1.76 | 0.19 |
| ES** | 0.57 | 0.22 | 0.09 | 0.71 | 0.73 | 0.14 |
| ^MR | 0.76 | 1.12 | 1.04 | 0.68 | 0.73 | 0.93 |

*Neg log p-value, **Effect size
^Mean ratio: MS/H; N/S,B; B/S

Table 64: (Var: CYT- 15645)

|  | NvH | SvH | BvH | SvN | BvN | SvB |
|---|---|---|---|---|---|---|
| P* | 0.36 | 1.61 | 1.53 | 1.24 | 1.56 | 0.30 |
| ES** | 0.16 | 0.54 | 0.66 | 0.49 | 0.71 | 0.20 |

(continued)

|  | NvH | SvH | BvH | SvN | BvN | SvB |
|---|---|---|---|---|---|---|
| ^MR | 0.94 | 0.80 | 0.73 | 1.18 | 1.28 | 0.92 |

*Neg log p-value, **Effect size
^Mean ratio MS/H; N/S,B; B/S

Table 65: (Var: CYT- 15670)

|  | NvH | SvH | BvH | SvN | BvN | SvB |
|---|---|---|---|---|---|---|
| P* | 0.04 | 1.27 | 1.31 | 1.42 | 1.47 | 0.14 |
| ES** | 0.03 | 0.41 | 0.49 | 0.44 | 0.52 | 0.07 |
| ^MR | 1.01 | 0.88 | 0.86 | 1.15 | 1.17 | 0.98 |

*Neg log p-value, **Effect size
^ Mean ratio MS/H; N/S,B; B/S

Table 66: (Var: CYT- 15666)

|  | NvH | SvH | BvH | SvN | BvN | SvB |
|---|---|---|---|---|---|---|
| P* | 0.48 | 3.07 | 2.47 | 2.12 | 2.00 | 0.30 |
| ES** | 0.24 | 0.75 | 0.83 | 0.60 | 0.75 | 0.19 |
| ^MR | 0.90 | 0.70 | 0.64 | 1.28 | 1.39 | 0.92 |

*Neg log p-value, **Effect size
^Mean ratio MS/H; N/S,B; B/S

## B cell subsets and antigen intensities

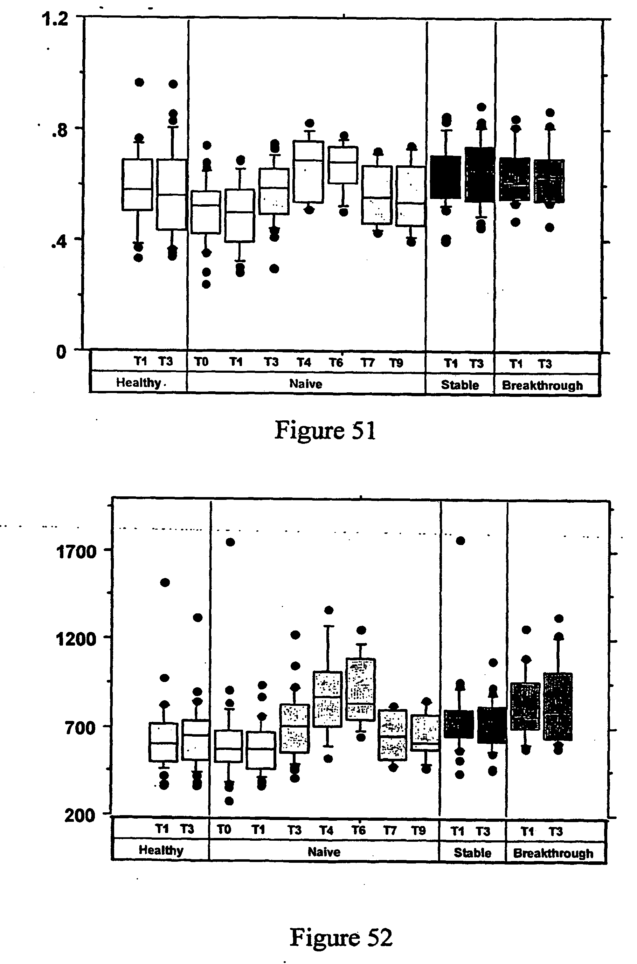

The fraction of B cells expressing cell surface **CD38** was lower in Naive than in Healthy subjects **(Figure 51 and Table 67).** After long-term Avonex treatment they were back to Healthy group or higher levels in Stable and Breakthrough subjects. It was higher in the Breakthrough group than the Stable and untreated groups. CD38 cell surface intensity on B cells in Naive subjects was similar to Healthy controls. Six days after the second Avonex dose CD38 intensity was already increased **(Figure 52 and Table 68).** We also observed a short-term pharmacodynamic increase in CD38 as discussed in the within group comparisons. It was increased further at the 3-month visit and returned to almost pre-treatment level at the 6-month visit. The CD38 intensity level at 6 months Naive subjects was also similar to that in Stable subjects at steady state, suggesting that there was a transient intermediate term increase in CD38 intensity and then it returned to pretreatment level.

Table 67: (Var -CYT 15948)

|  | NvH | SvH | BvH | SvN | BvN | SvB |
|---|---|---|---|---|---|---|
| P* | 2.12 | 1.47 | 0.71 | 6.92 | 4.25 | 0.18 |
| ES** | 0.62 | 0.50 | 0.38 | 1.30 | 1.20 | 0.13 |
| ^MR | 0.86 | 1.11 | 1.09 | 0.77 | 0.79 | 0.98 |

*Neg log p-value, **Effect size
^ Mean ratio: MS/H; N/S,B; B/S

Table 68: (Var -CYT 17386)

|  | NvH | SvH | BvH | SvN | BvN | SvB |
|---|---|---|---|---|---|---|
| P* | 0.50 | 1.66 | 2.60 | 3.51 | 4.09 | 1.05 |
| ES** | 0.22 | 0.53 | 0.94 | 0.75 | 1.13 | 0.48 |
| ^MR | 0.93 | 1.15 | 1.29 | 0.81 | 0.72 | 1.12 |
| *Neg log p-value, **Effect size<br>^ Mean ratio: MS/H; N/S,B; B/S | | | | | | |

### Monocyte antigens.

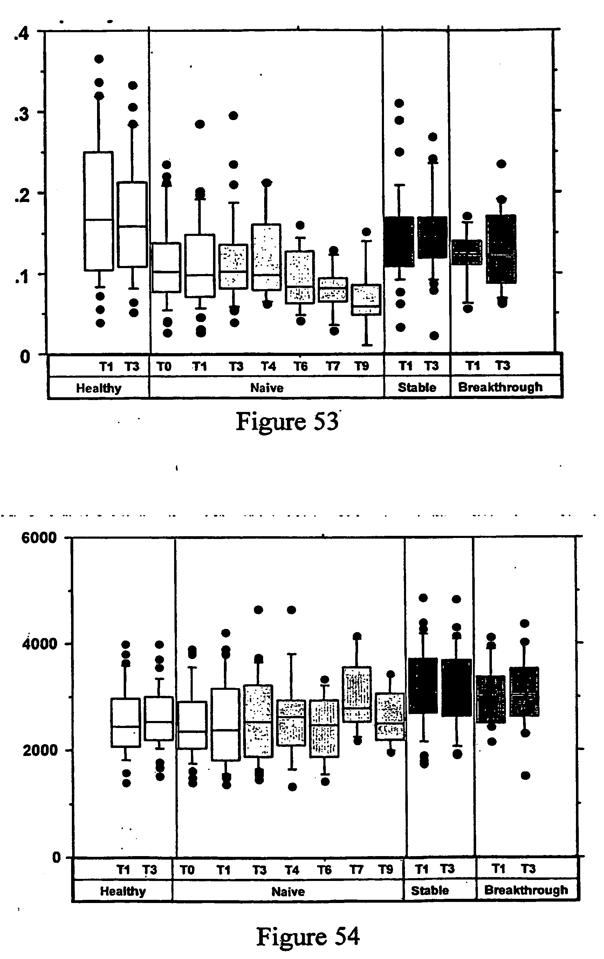

The fraction of monocytes expressing the early activation antigen CD69 was highest in the Healthy group and lowest for the untreated Naive group (Figure 53 and Table 69). Long-term Avonex treated subjects in both the Stable and Breakthrough groups approached the levels of Healthy subjects. This was not observed in the limited number of Naive subjects that continued at three and six months.

Table 69: (Var: CYT-16261)

|  | NvH | SvH | BvH | SvN | BvN | SvB |
|---|---|---|---|---|---|---|
| P* | 4.39 | 1.31 | 1.81 | 2.57 | 0.72 | 0.77 |
| ES** | 0.90 | 0.41 | 0.66 | 0.64 | 0.29 | 0.39 |
| ^MR | 0.64 | 0.84 | 0.72 | 0.76 | 0.88 | 0.86 |
| *Neg log p-value, **Effect size<br>^ Mean ratio: MS/H; N/S,B; B/S | | | | | | |

### NK cell antigens

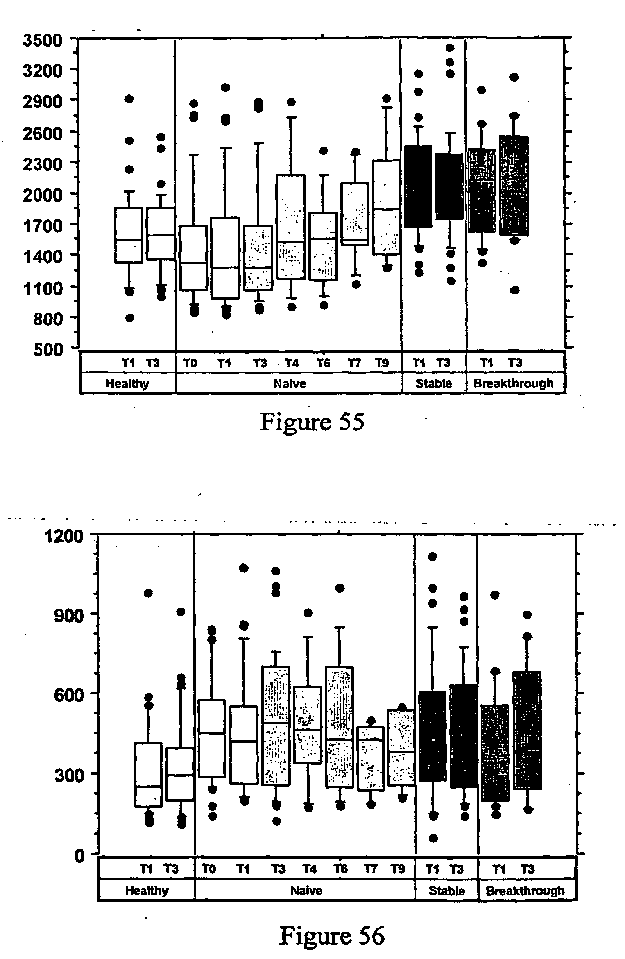

CD56 (NCAM, neural cell adhesion molecule) expression on NK cells was highest in the Avonex treated Stable and Breakthrough groups (Figures 54 and 55 and Table 70 and
Table 71). The antigen was measured in two assays with different gating strategies. Both are displayed in the figure and give consistent results. CD56 was about 15-30% greater in the Stable and Breakthrough groups than the untreated Naïve and Healthy subjects.
NKB1 expression on NK cells was 30-40% lower in the Healthy group (Figure 56 and Table 72). There was no difference among the MS groups or with drug treatment NKB1 (aka KIR, killer cell inhibitory receptor) is expressed on a subset of natural killer cells and a small subset of T cells.

Table 70:

|  | NvH | SvH | BvH | SvN | BvN | SvB |
|---|---|---|---|---|---|---|
| P* | 0.32 | 3.77 | 1.66 | 4.12 | 1.83 | 0.44 |
| ES** | 0.14 | 0.87 | 0.65 | 0.93 | 0.71 | 0.25 |
| ^MR | 0.97 | 1.21 | 1.15 | 0.80 | 0.84 | 0.95 |

Table 71:

|  | NvH | SvH | BvH | SvN | BvN | SvB |
|---|---|---|---|---|---|---|
| P* | 0.75 | 4.73 | 3.30 | 5.39 | 3.30 | 0.01 |
| ES** | 0.29 | 1.02 | 1.06 | 1.14 | 1.10 | 0.01 |

(continued)

|  | NvH | SvH | BvH | SvN | BvN | SvB |
|---|---|---|---|---|---|---|
| ^MR | 0.91 | 1.29 | 1.29 | 0.71 | 0.71 | 1.00 |

*Neg log p-value, **Effect size
^ Mean ratio MS/H; N/S,B; B/S

Table 72: (Var: CYST- 18610)

|  | NvH | SvH | BvH | SvN | BvN | SvB |
|---|---|---|---|---|---|---|
| P* | 2.36 | 2.31 | 1.08 | 0.07 | 0.18 | 0.23 |
| ES** | 0.68 | 0.67 | 0.52 | 0.05 | 0.13 | 0.16 |
| ^MR | 1.41 | 1.44 | 1.32 | 0.98 | 1.06 | 0.92 |

*Neg log p-value, **Effect size
^Mean ratio: MS/H; N/S,B; B/S

**Intracellular cytokines.**

The frequency of IL-2, IL-4, IL-10, IFN$\gamma$, and TNF$\alpha$ producing CD4 and CD8 T cells *ex vivo* without or with PMA plus ionomycin stimulation were measured in all four groups at all time points. TNF$\alpha$ producing B cells and monocytes were also monitored. Without stimulation these cells produce very low levels of cytokines. With the assay background we feel that the frequency of cytokine producing cells without stimulation is too low to be measured accurately. Therefore the data from unstimulated PBMC is not discussed in this example.

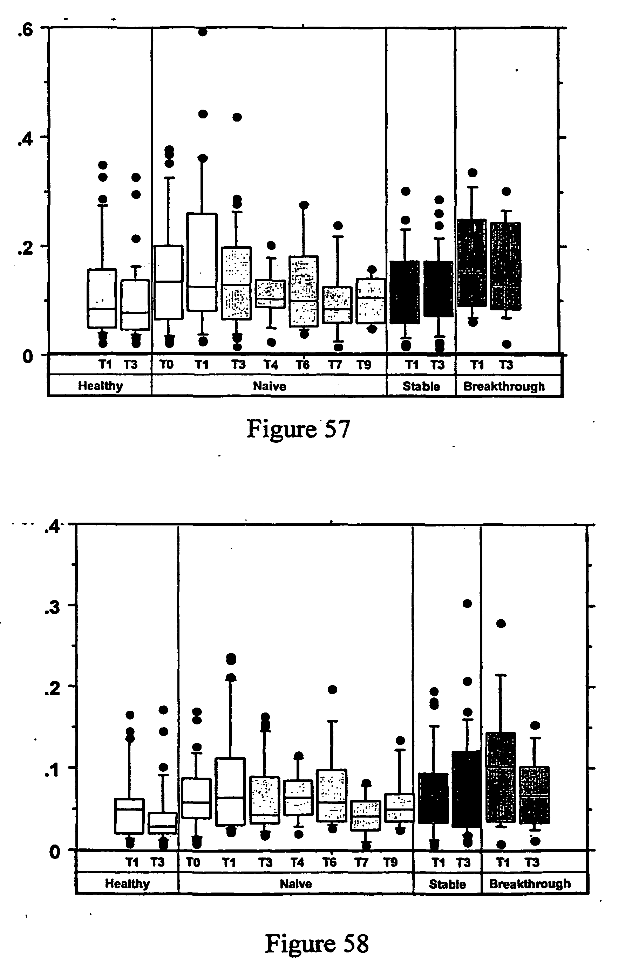

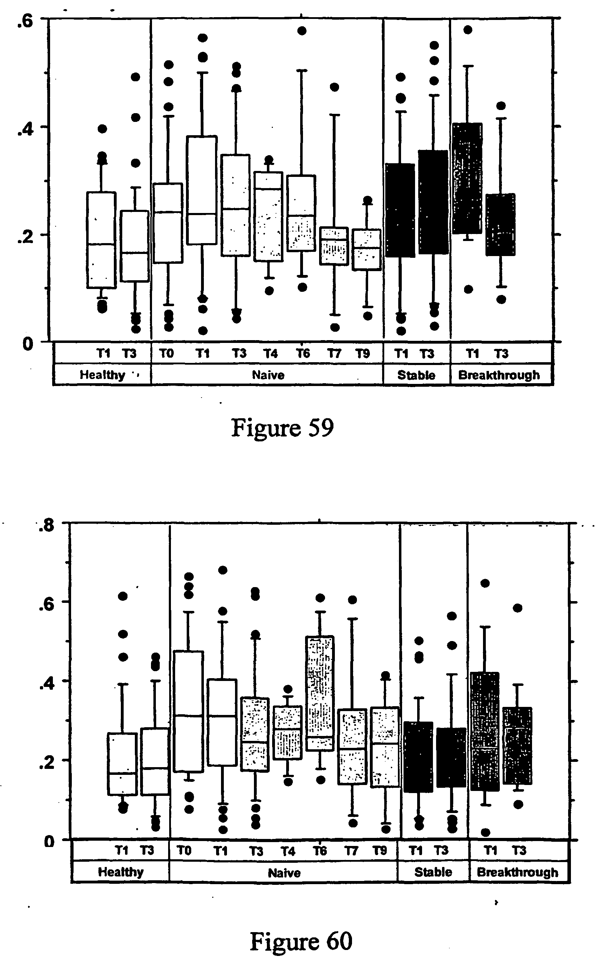

The frequency of IFN$\gamma$ producing CD8 T cells (Figure 57 and Table 73) and the frequency IL-2 producing CD4 and CD8 T cells (Figure 58 and 59 and Table 74 and Table 75) was higher in all three MS groups compared to Healthy control). There were no significant differences between Naive and Avonex treated MS subjects or between Stable and Breakthrough subjects. The frequency of TNF$\alpha$ producing B cells was also higher in all three MS groups at steady state (Figure 60 and Table 76). Also, the Naive group had higher frequency of TNF$\alpha$ producing B cells as compared to Avonex treated subjects. Even though the frequency of TNF$\alpha$ producing B cells was not statistically different between Stable and Breakthrough group, it was slightly higher in Breakthrough subjects.

Table 73: (Var: CYT- 16615, fraction of CD8 T cells)

|  | NvH | SvH | BvH | SvN | BvN | SvB |
|---|---|---|---|---|---|---|
| P* | 1.72 | 0.43 | 1.66 | 1.18 | 0.00 | 1.20 |
| ES** | 0.49 | 0.18 | 0.66 | 0.37 | 0.05 | 0.54 |
| ^MR | 1.45 | 1.13 | 1.50 | 1.29 | 0.96 | 1.34 |

*Neg log p-value, **Effect size
^ Mean ratio: MS/H; N/S,B; B/S

Table 74: (Var: CYT- 16731 fraction of CD8 T cells)

|  | NvH | SvH | BvH | SvN | BvN | SvB |
|---|---|---|---|---|---|---|
| P* | 2.19 | 2.01 | 2.20 | 0.02 | 0.29 | 0.31 |
| ES** | 0.58 | 0.54 | 0.81 | 0.00 | 0.20 | 0.20 |
| ^MR | 1.58 | 1.57 | 1.82 | 1.00 | 0.87 | 1.16 |

*Neg log p-value, **Effect size
^ Mean ratio: MS/H; N/S,B; B/S

Table 75: (Var: CYT- 16694 fraction of CD4 T cells)

|  | NvH | SvH | BvH | SvN | BvN | SvB |
|---|---|---|---|---|---|---|
| **P*** | 1.93 | 1.63 | 1.97 | 0.12 | 0.11 | 0.23 |
| **ES**** | 0.52 | 0.45 | 0.70 | 0.06 | 0.10 | 0.17 |
| **^MR** | 1.32 | 1.28 | 1.40 | 1.03 | 0.95 | 1.09 |

*Neg log p-value, **Effect size
^Mean ratio: MS/H; N/S,B; B/S

Table 76: (Var: CYT-16914 fraction ofB cells)

|  | NvH | SvH | BvH | SvN | BvN | SvB |
|---|---|---|---|---|---|---|
| **P*** | 3.08 | 0.01 | 0.73 | 3.05 | 0.70 | 0.71 |
| **ES**** | 0.77 | 0.02 | 0.44 | 0.75 | 0.32 | 0.42 |
| **^MR** | 1.55 | 1.01 | 1.29 | 1.53 | 1.20 | 1.28 |

*Neg log p-value, **Effect size
^Mean ratio: MS/H; N/SB; B/S

**T cell subsets**

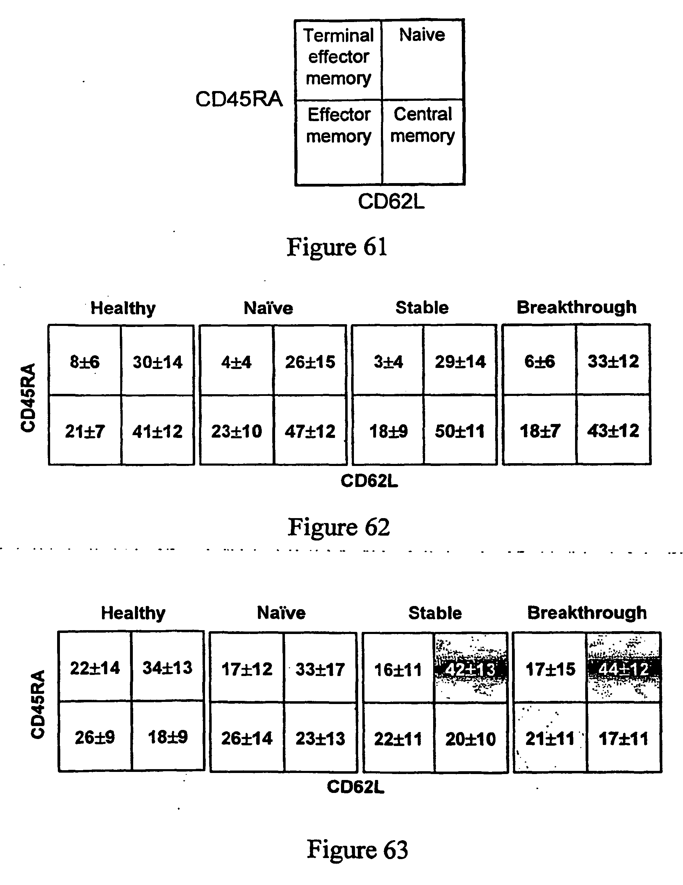

CD4 and CD8 T cells can be divided into different subsets in multiple ways. One is based on the expression of CD45RA and CD62L which can be used to divide the cells into four populations: naïve T cells, which have not seen antigen before, and three memory cell populations which have **(Figure 61)** (48, 98, 115). Means for each of the cohorts are presented in **Figures 62 and 63.** For CD4 T cells, there were modest differences among the cohorts, Naive and Stable groups had a higher % of central memory cells (CD45RA negative, CD62L positive) and lower % of terminal effector memory (CD45RA positive, CD62L negative) compared to Healthy. Breakthrough had lower % of central memory CD4 T cells compared to Stable; and, Breakthrough had higher % of naive cells (double positive) compared to Naive MS group. For CD8 T cells, long term Avonex treatment increased the % of naive (double positive) cells. Both the Stable and Breakthrough groups were more than the Healthy and Naive groups. This was the strongest and most consistent difference for these populations. The altered distribution of these populations suggests a change in T cell homeostasis. These may reflect changes in apoptosis or migration of the different cell types.

**Immunoassays**

Multiple differences were observed for specific serum analytes in the between group comparisons. **Table 77**provides summary results for all analytes that had a p-value <0.05 in at least one comparison. Several consistent and strong differences were observed for the Healthy vs MS comparisons.

## Table 77: Immunoassay differences – Between group comparison at steady state

| | | [-log(p-value)] | | | | | | Effect Size | | | | | | Mean Ratios | | | | | |
|---|---|---|---|---|---|---|---|---|---|---|---|---|---|---|---|---|---|---|---|
| | | H v MS | | | v N | | | H v MS | | | v N | | | H v MS | | | v N | | |
| IA ID | Assay | HvN | HvS | HvB | SvN | BvN | SvB | HvN | HvS | HvB | SvN | BvN | SvB | HvN | HvS | HvB | SvN | BvN | SvB |
| 51 | MXA* | ND | ND | ND | 6.7 | 3.0 | 2.1 | NA | NA | NA | 1.1 | 1.4 | 0.7 | NA | NA | NA | 0.12 | 0.06 | 1.90 |
| 20 | MIP-1A | 4.7 | 3.1 | 1.7 | 0.6 | 0.9 | 0.2 | 1.1 | 0.8 | 0.7 | 0.2 | 0.4 | 0.1 | 0.65 | 0.72 | 0.75 | 0.91 | 0.87 | 1.05 |
| 3 | S-CD14 | 3.5 | 7.0 | 6.6 | 0.3 | 0.6 | 0.3 | 0.9 | 1.2 | 1.6 | 0.2 | 0.3 | 0.2 | 1.58 | 1.71 | 1.84 | 0.92 | 0.86 | 1.07 |
| 39 | IL-6R | 3.4 | 4.0 | 1.2 | 0.5 | 0.2 | 0.7 | 0.6 | 0.7 | 0.5 | 0.2 | 0.2 | 0.3 | 0.66 | 0.61 | 0.70 | 1.08 | 0.94 | 1.15 |
| 14 | sV-CAM | 2.9 | 4.3 | 6.1 | 0.4 | 2.1 | 1.5 | 0.8 | 1.0 | 1.7 | 0.2 | 0.8 | 0.7 | 1.44 | 1.56 | 2.01 | 0.93 | 0.72 | 1.29 |
| 16 | MCP-1 | 2.9 | 3.4 | 3.0 | 0.5 | 0.9 | 0.2 | 0.7 | 0.8 | 0.9 | 0.2 | 0.5 | 0.2 | 0.68 | 0.61 | 0.56 | 1.12 | 1.23 | 0.92 |
| 38 | sIL-2R | 2.3 | 1.3 | 0.2 | 0.4 | 1.1 | 0.5 | 0.6 | 0.4 | 0.2 | 0.2 | 0.5 | 0.3 | 0.70 | 0.78 | 0.92 | 0.89 | 0.76 | 1.18 |
| 44 | MMP-9 | 2.2 | 3.7 | 1.0 | 2.1 | 0.2 | 0.9 | 0.5 | 0.7 | 0.5 | 0.5 | 0.1 | 0.4 | 0.57 | 0.42 | 0.53 | 1.37 | 1.08 | 1.27 |
| 42 | MMP-3 | 1.9 | 1.2 | 0.7 | 0.5 | 0.4 | 0.0 | 0.6 | 0.4 | 0.4 | 0.2 | 0.2 | 0.0 | 0.60 | 0.70 | 0.70 | 0.87 | 0.87 | 1.00 |
| 1 | TIMP-1 | 1.8 | 3.0 | 0.9 | 0.1 | 0.3 | 0.7 | 0.5 | 0.6 | 0.4 | 0.0 | 0.2 | 0.3 | 0.70 | 0.69 | 0.80 | 1.02 | 0.88 | 1.16 |
| 47 | β-NGF | 1.8 | 0.4 | 2.3 | 1.2 | 0.2 | 1.6 | 0.6 | 0.2 | 0.9 | 0.5 | 0.1 | 0.7 | 1.58 | 1.12 | 1.72 | 1.41 | 0.92 | 1.53 |
| 11 | E-SELECTIN | 1.5 | 1.0 | 0.5 | 0.2 | 0.2 | 0.1 | 0.5 | 0.4 | 0.3 | 0.1 | 0.2 | 0.1 | 1.26 | 1.20 | 1.17 | 1.05 | 1.08 | 0.98 |
| 50 | NT-3 | 1.3 | 1.5 | 1.2 | 0.5 | 0.4 | 0.0 | 0.5 | 0.5 | 0.6 | 0.2 | 0.3 | 0.1 | 2.18 | 2.85 | 3.38 | 0.77 | 0.64 | 1.19 |
| 22 | RANTES | 1.1 | 2.7 | 1.2 | 1.9 | 0.2 | 0.7 | 0.3 | 0.5 | 0.5 | 0.4 | 0.1 | 0.3 | 1.48 | 3.62 | 1.65 | 0.41 | 0.90 | 0.46 |
| 6 | LIF | 0.5 | 2.4 | 0.4 | 0.5 | 0.1 | 0.9 | 0.2 | 0.7 | 0.2 | 0.3 | 0.1 | 0.5 | 0.77 | 0.55 | 0.83 | 1.41 | 0.92 | 1.52 |
| 21 | MIP-1B | 0.5 | 1.7 | 3.6 | 0.3 | 1.2 | 1.8 | 0.2 | 0.6 | 1.1 | 0.2 | 0.6 | 0.6 | 0.85 | 0.72 | 0.48 | 1.18 | 1.76 | 0.67 |
| 12 | sICAM-1 | 0.6 | 1.5 | 1.4 | 0.5 | 0.4 | 0.0 | 0.2 | 0.4 | 0.6 | 0.2 | 0.2 | 0.0 | 1.22 | 1.54 | 1.50 | 0.79 | 0.81 | 0.97 |
| 10 | PAI-TOTAL | 0.2 | 1.4 | 0.2 | 0.7 | 0.0 | 0.7 | 0.2 | 0.4 | 0.1 | 0.3 | 0.0 | 0.4 | 0.87 | 0.70 | 0.91 | 1.26 | 0.96 | 1.30 |
| 48 | BDNF | 0.2 | 1.4 | 0.3 | 1.1 | 0.1 | 0.6 | 0.1 | 0.4 | 0.2 | 0.4 | 0.1 | 0.3 | 0.94 | 0.80 | 0.91 | 1.17 | 1.03 | 1.13 |
| 7 | NT-PROBNP | 0.7 | 1.3 | 0.8 | 0.5 | 0.4 | 0.1 | 0.3 | 0.4 | 0.4 | 0.2 | 0.2 | 0.1 | 1.55 | 1.99 | 2.33 | 0.78 | 0.67 | 1.17 |
| 17 | MCP-2 | 1.1 | 0.7 | 1.0 | 2.8 | 2.8 | 0.3 | 0.4 | 0.3 | 0.5 | 0.7 | 0.9 | 0.2 | 0.85 | 1.15 | 1.26 | 0.73 | 0.67 | 1.09 |
| 18 | MCP-4 | 0.8 | 0.7 | 0.1 | 2.3 | 0.8 | 0.5 | 0.3 | 0.3 | 0.1 | 0.5 | 0.3 | 0.3 | 1.45 | 0.77 | 0.94 | 1.88 | 1.54 | 1.22 |
| 31 | IL-8 | 0.8 | 0.7 | 0.7 | 1.6 | 0.1 | 1.0 | 0.3 | 0.2 | 0.3 | 0.4 | 0.1 | 0.4 | 0.66 | 1.41 | 0.60 | 0.47 | 1.10 | 0.43 |
| 15 | IP-10 | 0.1 | 0.3 | 1.1 | 0.8 | 3.2 | 2.0 | 0.0 | 0.1 | 0.5 | 0.3 | 1.0 | 0.8 | 0.98 | 1.09 | 1.36 | 0.90 | 0.72 | 1.25 |
| 23 | IFN-α | 0.7 | 0.9 | 0.7 | 0.2 | 1.8 | 0.9 | 0.2 | 0.3 | 0.4 | 0.0 | 0.4 | 0.4 | 1.46 | 1.53 | 0.63 | 0.96 | 2.31 | 0.41 |
| 9 | PAI-1 ACTIVE | 0.1 | 0.0 | 0.7 | 0.2 | 1.9 | 0.7 | 0.1 | 0.0 | 0.3 | 0.1 | 0.6 | 0.4 | 1.06 | 0.99 | 0.71 | 1.08 | 1.49 | 0.72 |

(p <0.05 for at least one comparisons). * MXA – Not measured for healthy group, but expect very low levels, similar to Naïve group

**Soluble CD14.**

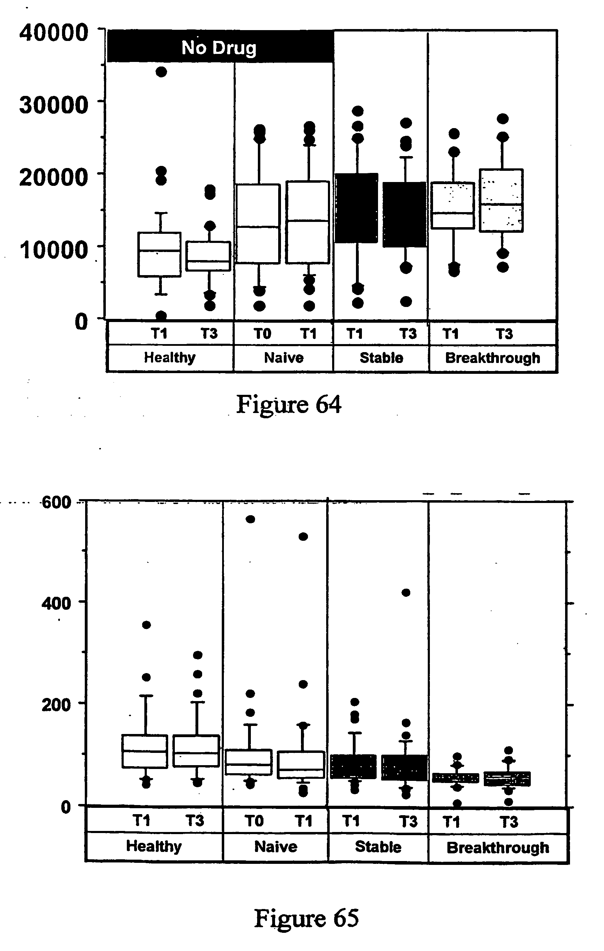

The soluble form of CD14, the monocyte surface glycoprotein (Accession No. TDHUM4) was measured by immunoassay and detected in the 2D serum proteome. The immunoassay showed a strong difference between the Healthy and MS subjects **(Figure 64 and Table 79)**. Soluble CD14 was at 60 to 80 % higher levels in the Naive, Stable and Breakthrough groups compared with the Healthy group.

One sCD14 peptide was detected in the 2D serum proteome. The peptide had a reasonable ID score (32). From the immunoassay data, the concentration of the protein was on the order of 10 ng/mL, which would be on the very low side for detection in a three-fraction 2D proteome analysis. As with the immunoassay, higher levels of sCD14 were found in the Stable and Breakthrough groups compared to the Healthy group **(Table 78)**.

**Table 78: sCD14**

|  | HvsS | HvsB | HvsN | SvsN | BvsN | SvsB |
|---|---|---|---|---|---|---|
| **P** | 2.97 | 2.43 | 1.20 | 0.72 | 1.11 | 0.48 |
| **ES** | 0.82 | 1.08 | 0.46 | 0.32 | 0.61 | 0.34 |
| **MR** | 1.12 | 1.18 | 1.07 | 0.96 | 0.91 | 1.05 |
| VAR: SE-LC-PT-0000422780 (DL) | | | | | | |

Our cytometry analysis showed CD 14 monocytes decreased in response to a pharmacodynamic effect of Avonex.

Table 79: (Var: IA-3 (pg/mL))

|  | NvH | SvH | BvH | SvN | BvN | SvB |
|---|---|---|---|---|---|---|
| **P*** | 3.46 | 7.04 | 6.56 | 0.31 | 0.57 | 0.32 |
| **ES**** | 0.88 | 1.16 | 1.57 | 0.17 | 0.34 | 0.17 |
| **^MR** | 1.58 | 1.71 | 1.84 | 0.92 | 0.86 | 1.07 |
| *Neg log p-value, **Effect size<br>^ Mean ratio: MS/H; N/S,B; B/S | | | | | | |

## Chemokines

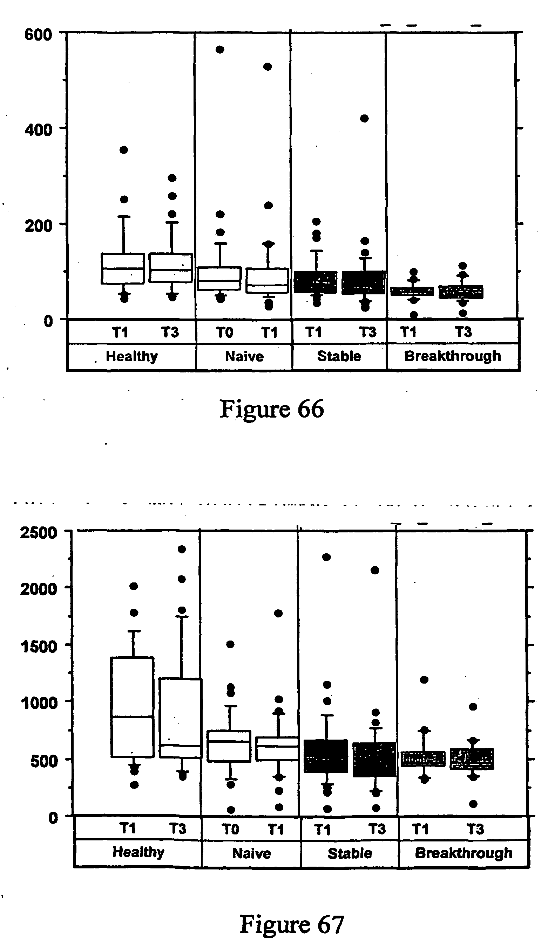

Macrophage inflammatory proteins-MIP-1α and MIP-1β are inducible CC chemokines that play a role in regulating host response to pathogens and which can contribute to disease pathophysiology. MIP-1α was 25-35% lower in MS subjects compared with Healthy controls **(Figure 65 and Table 80).** There were no significant differences among the MS groups. MIP-1β was 15-50% lower in MS subjects than Healthy controls **(Figure 66 and Table 81).** The lowest level is observed for the Breakthrough group, which was 30 % lower than the Stable group. The MIP-1β trend is H > N, S > B.

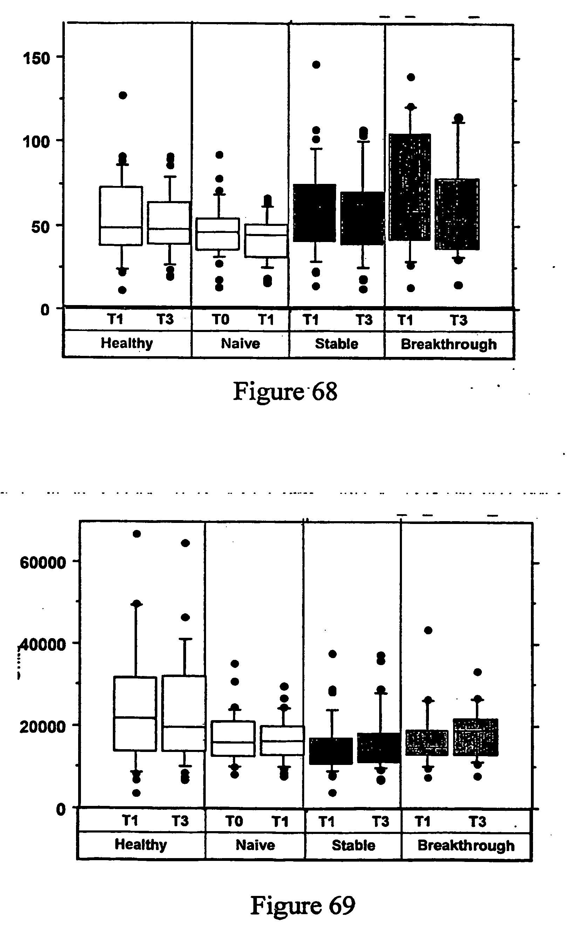

Monocyte chemoattractant proteins (MCPs) are CC chemokines that attract monocytes, activated T cells and NK cells. MCP-1 (CCL2) has been implicated in many inflammatory and autoimmune diseases. It was 30-40% lower in the MS groups compared with Healthy subjects **(Figure 67 and Table 82).** MCP-2 (CCL8) was lowest in the untreated Naive subjects. It was significantly lower, by about 30% compared with the Stable and Breakthrough groups **(Figure 68 and Table 83).** It was not significantly lower in the Naive to Healthy comparison.

Table 80: (Var: IA-20 (pg/mL))

|  | NvH | SvH | BvH | SvN | BvN | SvB |
|---|---|---|---|---|---|---|
| **P*** | 4.72 | 3.08 | 1.66 | 0.61 | 0.87 | 0.18 |
| **ES**** | 0.88 | 1.16 | 1.57 | 0.24 | 0.43 | 0.14 |
| **^MR** | 0.65 | 0.72 | 0.75 | 0.91 | 0.87 | 1.05 |
| *Neg log p-value, **Effect size<br>^ Mean ratio: MS/H; N/S,B; B/S | | | | | | |

Table 81: (Var: IA-21 (pg/mL))

|  | NvH | SvH | BvH | SvN | BvN | SvB |
|---|---|---|---|---|---|---|
| **P*** | 0.52 | 1.65 | 3.64 | 0.34 | 1.25 | 1.81 |
| **ES**** | 0.23 | 0.56 | 1.15 | 0.21 | 0.59 | 0.60 |

(continued)

|  | NvH | SvH | BvH | SvN | BvN | SvB |
|---|---|---|---|---|---|---|
| ^MR | 0.85 | 0.72 | 0.48 | 1.18 | 1.76 | 0.67 |
| *Neg log p-value, **Effect size<br>^ Mean ratio: MS/H; N/S,B; B/S | | | | | | |

Table 82: (Var: IA-16)

|  | NvH | SvH | BvH | SvN | BvN | SvB |
|---|---|---|---|---|---|---|
| P* | 2.85 | 3.42 | 3.04 | 0.45 | 0.91 | 0.21 |
| ES** | 0.71 | 0.82 | 0.93 | 0.22 | 0.47 | 0.15 |
| ^MR | 0.68 | 0.61 | 0.56 | 1.12 | 1.23 | 0.92 |
| *Neg log p-value, **Effect size<br>^ Mean ratio: MS/H; N/S,B; B/ | | | | | | |

Table 83: (Var: IA-17)

|  | NvH | SvH | BvH | SvN | BvN | SvB |
|---|---|---|---|---|---|---|
| P* | 1.08 | 0.74 | 1.04 | 2.80 | 2.80 | 0.32 |
| ES** | 0.42 | 0.33 | 0.51 | 0.74 | 0.94 | 0.19 |
| ^MR | 0.85 | 1.15 | 1.26 | 0.73 | 0.67 | 1.09 |
| *Neg log p-value, **Effect size<br>^ Mean ratio: MS/H; N/S,B; B/ | | | | | | |

**Soluble IL6-R.** The soluble form of the IL6 receptor (sCD126) was 30-40% lower in the MS subjects **Figure 69 and Table 84.** The results reached statistical significance for the comparisons to the Naive and Stable group, but not the Breakthrough group.

Table 84: (Var: IA-39)

|  | NvH | SvH | BvH | SvN | BvN | SvB |
|---|---|---|---|---|---|---|
| P* | 3.40 | 3.98 | 1.22 | 0.48 | 0.24 | 0.65 |
| ES** | 0.65 | 0.73 | 0.49 | 0.20 | 0.17 | 0.32 |
| ^MR | 0.66 | 0.61 | 0.70 | 1.08 | 0.94 | 1.15 |
| *Neg log p-value, **Effect size<br>^ Mean ratio: MS/H; N/S,B; B/S | | | | | | |

**Matrix metalloproteinases and their inhibitors**

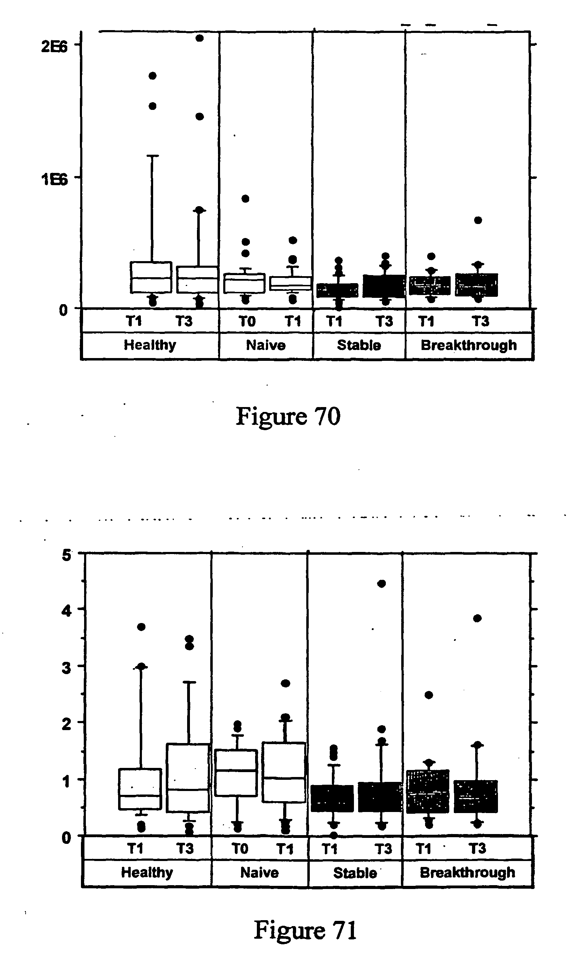

Seven Matrix metalloproteinases (MMP-1, MMP-2, MMP-3, MMP-8, MMP-9, MMP-10, MMP-13) and two inhibitors (TIMP-1 and TIMP-2) were measured in this study. There were cross group differences for MMP-9, MMP-3 and TIMP-1 **(Table 77,** above). MMP-9 was 60 to 40% lower in the MS subjects than the Healthy group **(Figure 70 and Table 85)**, with the comparisons to the Naive and Stable groups being significant. The distributions for the Healthy group were skewed toward the high end. TIMP-1 was 20 to 30% lower in MS subjects than the Healthy group **(Figure 71 and Table 86),** with the comparisons to the Naive and Stable groups being significant. It wa highest for the untreated Naive group and lowest for the treated group.

Table 85: (Var: IA-44)

|  | NvH | SvH | BvH | SvN | BvN | SvB |
|---|---|---|---|---|---|---|
| P* | 2.22 | 3.71 | 1.04 | 2.09 | 0.22 | 0.88 |
| ES** | 0.48 | 0.67 | 0.46 | 0.54 | 0.13 | 0.42 |
| ^MR | 0.57 | 0.42 | 0.53 | 1.37 | 1.08 | 1.27 |

*Neg log p-value, **Effect size
^ Mean ratio: MS/H; N/S,B; B/S

Table 86: (Var: IA-1)

|  | NvH | SvH | BvH | SvN | BvN | SvB |
|---|---|---|---|---|---|---|
| P* | 1.85 | 2.97 | 0.92 | 0.10 | 0.34 | 0.69 |
| ES** | 0.55 | 0.64 | 0.37 | 0.04 | 0.23 | 0.34 |
| ^MR | 0.70 | 0.69 | 0.80 | 1.02 | 0.88 | 1.16 |

*Neg log p-value, **Effect size
^ Mean ratio: MS/H; N/S,B; B/S

**Mass spectrometry**

Multiple additional differences were observed for analytes in the between group comparisons with the mass spectrometry platforms. Some of the identified proteins are discussed below. Emphasis is on the 2D serum proteome, which had the most interesting cross group results.

Several proteins have differences that largely refl4ct **Drug vs no Drug** and are discussed below. Differences largely associate with the Breakthrough group are discussed separately

**Mac-2-binding glycoprotein precursor (Mac2BP) (Accession No. A47161).** A drug effect was observed for Mac2BP. Higher levels were found by DeepLook analysis in Stable and in Breakthrough groups compared to both Healthy and Naive groups **(Table 87).** Seven peptides mapped to this protein, giving strong confidence in its identity. About half of the components tracked consistently across the comparisons, as shown below for two representative variables. Mac2BP was not detected in the 1D proteome.

**Table 87: Mac2BP**

|  | HvsS | HvsB | HvsN | SvsN | BvsN | SvsB |  | HvsS | HvsB | HvsN | SvsN | BvsN | SvsB |
|---|---|---|---|---|---|---|---|---|---|---|---|---|---|
| P | 1.36 | 1.68 | 0.25 | 2.23 | 1.93 | 1.00 |  | 2.78 | 1.55 | 0.10 | 4.01 | 1.69 | 0.54 |
| ES | 0.52 | 0.93 | 0.15 | 0.72 | 1.04 | 0.68 |  | 0.78 | 0.88 | 0.06 | 1.00 | 0.98 | 0.44 |
| MR | 1.23 | 1.76 | 0.93 | 0.76 | 0.53 | 1.43 |  | 1.34 | 1.64 | 0.97 | 0.73 | 0.59 | 1.22 |
| VAR: SE-LC-PT-0000426299 (2D) | | | | | | | VAR: SE-LC-PT-0000419844 (2D) | | | | | | |
| P= Neg log p-value, ES= Effect size, MR= Mean ratio: 2nd/1st | | | | | | | | | | | | | |

**Alpha-2-plasmin inhibitor (A2AP) (Accession No. P08697).** A2AP was found by DeepLook analysis to be decreased in Breakthrough group compared to Healthy, Naive and Stable group **(Table 88).** Several components (24) corresponding to A2AP were detected, of which about 40% (9) changed significantly. Four variables are indicated below. A2AP was also detected in the 1D proteome (15 variables), and 20% of the variables showed a slight decrease in the Breakthrough group compared to the Stable and Healthy groups, which is consistent with the DeepLook results.

**Table 88: A2AP**

| | HvsS | HvsB | HvsN | SvsN | BvsN | SvsB | | HvsS | HvsB | HvsN | SvsN | BvsN | SvsB |
|---|---|---|---|---|---|---|---|---|---|---|---|---|---|
| P | 0.60 | 3.19 | 0.30 | 1.11 | 3.16 | 3.85 | | 0.01 | 3.15 | 0.13 | 0.12 | 2.96 | 3.32 |
| ES | 0.28 | 0.84 | 0.17 | 0.44 | 0.86 | 0.88 | | 0.00 | 0.79 | 0.08 | 0.08 | 0.79 | 0.83 |
| MR | 1.24 | 0.51 | 0.90 | 0.73 | 1.75 | 0.41 | | 1.00 | 0.64 | 0.96 | 0.96 | 1.51 | 0.64 |
| VAR: SE-LC-PT-0000422099 (2D) | | | | | | | | SE-LC-PT-0000423949 (2D) | | | | | |

| | HvsS | HvsB | HvsN | SvsN | BvsN | SvsB | | HvsS | HvsB | HvsN | SvsN | BvsN | SvsB |
|---|---|---|---|---|---|---|---|---|---|---|---|---|---|
| P | 0.07 | 3.47 | 0.07 | 0.15 | 3.15 | 3.67 | | 0.91 | 2.27 | 0.14 | 0.57 | 2.48 | 3.82 |
| ES | 0.05 | 0.84 | 0.05 | 0.09 | 0.80 | 0.87 | | 0.37 | 0.92 | 0.08 | 0.27 | 0.95 | 1.36 |
| MR | 1.02 | 0.68 | 0.98 | 0.96 | 1.44 | 0.66 | | 0.95 | 1.13 | 0.99 | 1.04 | 0.88 | 1.19 |
| VAR: SE-LC-PT-0000420547 (2D) | | | | | | | | VAR: SE-LC-PT-0000419739 (2D) | | | | | |

| P= Neg log p-value, ES= Effect size, MR= Mean ratio: 2nd/1st |
|---|

Attractin-2; mahogany protein (Accession No. NP_036202.2). Our DeepLook analysis, as well as our 1D proteomics analysis, found differential levels of Attractin/mahogany protein in Stable and Breakthrough groups compared to Healthy group. Several components were identified to be decreased in both Stable and Breakthrough groups compared to Healthy group **(Table 89)**. The variables corresponding to this protein are shown below:

In the DeepLook analysis seven variables are identified and 6 (85%) were differentially changed. Most variables are decreased in the Stable and Breakthrough groups compared to Healthy and Naive groups. The variables corresponding to this protein are shown below. In the 1D proteome, which had different sample sets, two variables were detected with both showing a significant increase in the Stable group compared to Naive group.

**Table 89:**

| | HvsS | HvsB | HvsN | SvsN | BvsN | SvsB | | HvsS | HvsB | HvsN | SvsN | BvsN | SvsB |
|---|---|---|---|---|---|---|---|---|---|---|---|---|---|
| P | 1.31 | 0.52 | 0.22 | 0.67 | 0.67 | 1.15 | | 1.54 | 0.07 | 0.85 | 4.44 | 0.58 | 0.66 |
| ES | 0.57 | 0.51 | 0.15 | 0.34 | 0.58 | 0.94 | | 0.53 | 0.06 | 0.36 | 1.07 | 0.41 | 0.45 |
| MR | 0.86 | 1.18 | 0.96 | 1.11 | 0.81 | 1.37 | | 0.92 | 0.99 | 1.05 | 1.15 | 1.06 | 1.08 |
| VAR: SE-LC-PT-0000418145 (DL) | | | | | | | | SE-LC-PT-0000423111 (DL) | | | | | |

| | HvsS | HvsB | HvsN | SvsN | BvsN | SvsB | | HvsS | HvsB | HvsN | SvsN | BvsN | SvsB |
|---|---|---|---|---|---|---|---|---|---|---|---|---|---|
| P | 1.02 | 0.98 | 0.15 | 1.44 | 1.30 | 0.07 | | 1.91 | 2.44 | 0.02 | 2.03 | 2.52 | 0.57 |
| ES | 0.40 | 0.50 | 0.09 | 0.51 | 0.65 | 0.05 | | 0.66 | 1.00 | 0.01 | 0.72 | 1.10 | 0.40 |
| MR | 0.92 | 0.91 | 1.02 | 1.11 | 1.12 | 0.99 | | 0.92 | 0.87 | 1.00 | 1.09 | 1.15 | 0.95 |
| VAR: SE-LC-PT-0000424017 (DL) | | | | | | | | VAR: SE-LC-PT-0000420701 (DL) | | | | | |

| | HvsS | HvsB | HvsN | SvsN | BvsN | SvsB | | HvsS | HvsB | HvsN | SvsN | BvsN | SvsB |
|---|---|---|---|---|---|---|---|---|---|---|---|---|---|
| P | 1.91 | 2.44 | 0.02 | 2.03 | 2.52 | 0.57 | | 2.67 | 1.18 | 0.19 | 3.69 | 1.65 | 0.30 |

(continued)

| | HvsS | HvsB | HvsN | SvsN | BvsN | SvsB | | HvsS | HvsB | HvsN | SvsN | BvsN | SvsB |
|---|---|---|---|---|---|---|---|---|---|---|---|---|---|
| ES | 0.66 | 1.00 | 0.01 | 0.72 | 1.10 | 0.40 | | 0.76 | 0.53 | 0.11 | 0.95 | 0.70 | 0.22 |
| MR | 0.92 | 0.87 | 1.00 | 1.09 | 1.15 | 0.95 | | 0.88 | 0.91 | 1.02 | 1.16 | 1.12 | 1.03 |
| VAR: SE-LC-PT-0000420701 (DL) | | | | | | | VAR: SE-LC-PT-0000422360 (DL) | | | | | | |
| P= Neg log p-value, ES= Effect size, MR= Mean ratio: 2nd/1st | | | | | | | | | | | | | |

**Paraoxonase 1 (Accession No. P27169)** PON1, or arylesterase 1, was found at significantly lower levels in Stable group compared to Breakthrough and Naive groups (Table 90). Twenty four variables corresponding to this protein are detected by DeepLook, of which 62% (15) changed significantly. Four representative variables are shown below. PON1 was detected in the 1D proteome with 14 components. 42% (6) of the variables differ significantly in the Stable group compared to the Naive group following drug treatment, and 28% (4) of the variables differ in the Stable group compared to the Healthy group.

**TABLE 90: PON1**

| | HvsS | HvsB | HvsN | SvsN | BvsN | SvsB | | HvsS | HvsB | HvsN | SvsN | BvsN | SvsB |
|---|---|---|---|---|---|---|---|---|---|---|---|---|---|
| P | 0.56 | 1.08 | 0.73 | 1.63 | 0.05 | 2.51 | | 0.52 | 1.07 | 0.70 | 1.44 | 0.03 | 2.24 |
| ES | 0.26 | 0.47 | 0.32 | 0.56 | 0.03 | 0.94 | | 0.25 | 0.47 | 0.31 | 0.52 | 0.02 | 0.81 |
| MR | 0.93 | 1.12 | 1.11 | 1.19 | 0.99 | 1.20 | | 0.95 | 1.10 | 1.09 | 1.15 | 0.99 | 1.16 |
| **VAR** SE-LC-PT-0000424059 (DL) | | | | | | | SE-LC-PT- 0000422636 (DL) | | | | | | |

| | HvsS | HvsB | HvsN | SvsN | BvsN | SvsB | | HvsS | HvsB | HvsN | SvsN | BvsN | SvsB |
|---|---|---|---|---|---|---|---|---|---|---|---|---|---|
| P | 1.10 | 0.20 | 0.48 | 1.77 | 0.22 | 1.44 | | 0.85 | 0.03 | 0.69 | 2.08 | 0.57 | 0.51 |
| ES | 0.43 | 0.14 | 0.24 | 0.60 | 0.13 | 0.70 | | 0.36 | 0.03 | 0.31 | 0.66 | 0.32 | 0.35 |
| MR | 0.92 | 1.03 | 1.07 | 1.16 | 1.04 | 1.12 | | 0.92 | 0.99 | 1.08 | 1.17 | 1.09 | 1.08 |
| VAR SE-LC-PT- 0000417742 (DL) | | | | | | | VAR: SE-LC-PT- 0000415638 (DL) | | | | | | |
| P= Neg log p-value, ES= Effect size, MR= Mean ratio: 2nd/1st | | | | | | | | | | | | | |

**Tysabri related analytes**

Human monoclonal antibodies against VLA-4 (natalizumab, Tysabri) block T cell migration across the blood brain barrier and are a potential therapy for MS (75). Consideration of assay that directly relate to the inflammatory cell adhesion and migration pathway of VLA-4 was not part of the study set-up. However the study does have three cytometry assays and one immunoassay that are directly relevant. CD49d (aka VLA-4, alpha-4-integrin) was measured on both CD4 and CD8 T cells simultaneously with CD45RA. CD49d is expressed as a heterodimer with either of two beta subunits beta-1-integrin (CD29) or beta-7-integin. CD29 was also measured on CD4 and CD8 T cells. CD29 can complex with any one of six alpha subunits (CD49a-f) so results may not be related to the target. The alpha-4 beta-1 integrin complex (CD49d/CD29, VLA4) binds to VCAM-1, CD106, vascular cell adhesion molecule-1, which is present on endothelium and strongly up-regulated following cytokine stimulation. The soluble form, sVCAM was measured by immunoassay. Both within group and between group comparisons are considered here for these assays.

Within group comparisons for the short-term pharmacodynamic effect are presented in Table 91 for the cytometry assays. The most differences were observed for the Naïve group. CD29 expression on CD8 T cells was increased about two-fold 34 hours post injection for the Naïve group. It is not increased for the long-term Avonex treated subjects. It is also not increased on CD4 T cells. No differences were observed for the cell count ratios for CD29 subpopulations (data in e-files). Some modest changes in the distribution of CD49d CD4 T cell and CD8 T cell subsets were observed short term post dosing. The count ratio (CR) variables should be considered, since the absolute T cells counts decrease short-term post injection

Between group comparisons for the longer-term effect are presented in Table 92 for the cytometry assays. In general differences and are modest in terms of p-values and magnitude.

### Table 91: Within Group Comparisons for CD49d and CD29 assays

| CYT ID | Cell type | Cell Population | Property* | [-log(P-values)] | | | | Mean ratios | | | |
|---|---|---|---|---|---|---|---|---|---|---|---|
| | | | | N T0-1vT2 | N T3vT2 | S T1-3vT2 | B T1-3vT2 | N T0-1vT2 | N T3vT2 | S T1-3vT2 | B T1-3vT2 |
| 17335 | CD8 T cells | CD3pCD4n | CD29 int | 1.62 | 1.69 | 0.24 | 0.55 | 1.71 | 1.96 | 1.12 | 0.74 |
| 17341 | | CD3pCD4nCD29p | CD29 int | 4.37 | 2.13 | 0.31 | 0.29 | 2.13 | 1.64 | 1.15 | 0.86 |
| 17344 | CD4 T cells | CD3pCD4p | CD29 int | 0.07 | 0.28 | 0.15 | 0.07 | 1.01 | 1.04 | 0.99 | 0.99 |
| 17485 | CD4 T cells | CD4p | CD49d int | 0.69 | 2.88 | 0.63 | 1.91 | 1.03 | 1.09 | 1.02 | 1.06 |
| 17491 | | CD4pCD45RAnCD49dp | CD49d int | 0.86 | 1.41 | 0.57 | 0.06 | 1.04 | 1.06 | 1.01 | 1.00 |
| 17497 | | CD4pCD45RApCD49dp | CD49d int | 2.45 | 4.19 | 1.90 | 2.02 | 1.10 | 1.16 | 1.09 | 1.07 |
| 16023 | | CD4pCD45RAnCD49dn/CD4p | CR | 0.34 | 0.81 | 0.40 | 0.61 | 0.98 | 0.95 | 0.97 | 0.98 |
| 16025 | | CD4pCD45RAnCD49dp/CD4p | CR | 0.61 | 1.73 | 0.07 | 3.62 | 0.99 | 1.04 | 1.00 | 1.12 |
| 16027 | | CD4pCD45RApCD49dn/CD4p | CR | 2.24 | 0.17 | 0.09 | 1.32 | 1.07 | 1.01 | 1.02 | 0.88 |
| 16029 | | CD4pCD45RApCD49dp/CD4p | CR | 0.11 | 0.71 | 0.12 | 0.21 | 0.97 | 0.92 | 1.02 | 0.89 |
| 16022 | | CD4pCD45RAnCD49dn | CT | 1.43 | 2.12 | 2.83 | 1.54 | 0.91 | 0.88 | 0.86 | 0.90 |
| 16024 | | CD4pCD45RAnCD49dp | CT | 1.89 | 0.04 | 3.20 | 0.09 | 0.92 | 1.00 | 0.88 | 1.05 |
| 16026 | | CD4pCD45RApCD49dn | CT | 0.09 | 0.65 | 1.03 | 2.33 | 0.99 | 0.94 | 0.91 | 0.82 |
| 16028 | | CD4pCD45RApCD49dp | CT | 0.96 | 0.69 | 0.73 | 1.12 | 0.89 | 0.91 | 0.90 | 0.85 |
| 17512 | CD8 T cells | CD8pCD45RAnCD49dp | CD49d int | 1.60 | 0.55 | 1.31 | 0.55 | 0.95 | 0.97 | 0.97 | 0.96 |
| 17521 | | CD8pCD45RApCD49dp | CD49d int | 0.00 | 0.08 | 0.24 | 0.65 | 1.00 | 0.99 | 0.99 | 0.95 |
| 16039 | | CD8pCD45RAnCD49dn/CD8p | CR | 0.98 | 0.84 | 0.67 | 0.01 | 1.20 | 1.20 | 1.10 | 1.04 |
| 16041 | | CD8pCD45RAnCD49dp/CD8p | CR | 1.70 | 0.17 | 1.26 | 0.24 | 0.93 | 0.98 | 0.94 | 1.08 |
| 16045 | | CD8pCD45RApCD49dn/CD8p | CR | 2.24 | 0.45 | 0.44 | 0.14 | 1.26 | 1.08 | 1.05 | 1.04 |
| 16047 | | CD8pCD45RApCD49dp/CD8p | CR | 0.39 | 0.45 | 0.16 | 0.31 | 0.96 | 0.95 | 1.01 | 0.90 |
| 16038 | | CD8pCD45RAnCD49dn | CT | 0.33 | 0.19 | 0.54 | 0.40 | 1.09 | 1.07 | 0.91 | 0.84 |
| 16040 | | CD8pCD45RAnCD49dp | CT | 6.04 | 1.96 | 6.09 | 2.18 | 0.76 | 0.85 | 0.79 | 0.87 |
| 16044 | | CD8pCD45RApCD49dn | CT | 0.61 | 0.03 | 1.70 | 0.72 | 1.09 | 0.99 | 0.87 | 0.81 |
| 16046 | | CD8pCD45RApCD49dp | CT | 3.41 | 2.18 | 2.81 | 2.14 | 0.80 | 0.83 | 0.84 | 0.75 |

Int = intensity, CT = absolute cell count, CR = count ratio, Mean ratio = T2/other

## Table 92: Between Group Comparisons for CD49d and CD29 assays

| CYT ID | Cell Type | Cell Population | Property | [-log(p-value)] | | | | | | Mean ratios | | | | | |
|---|---|---|---|---|---|---|---|---|---|---|---|---|---|---|---|
| | | | | HvN-SS | HvS-SS | HvB-SS | SvN-SS | BvN-SS | SvB-SS | HvN | HvS | HvB | SvN-SS | BvN-SS | SvB-SS |
| 17335 | CD8 T cells | CD3pCD4n | CD29 int | 0.94 | 0.17 | 0.18 | 0.76 | 0.51 | 0.05 | 0.69 | 0.91 | 0.89 | 0.76 | 0.78 | 0.97 |
| 17341 | | CD3pCD4nCD29p | CD29 int | 1.83 | 0.19 | 0.03 | 1.94 | 1.34 | 0.13 | 0.69 | 1.09 | 1.02 | 0.63 | 0.68 | 0.93 |
| 17344 | CD4 T cells | CD3pCD4p | CD29 int | 0.11 | 0.76 | 0.28 | 0.55 | 0.17 | 0.21 | 1.02 | 1.08 | 1.05 | 0.94 | 0.97 | 0.97 |
| 17485 | CD4 T cells | CD4p | CD49d int | 0.25 | 1.20 | 0.89 | 0.72 | 0.54 | 0.03 | 0.97 | 0.92 | 0.92 | 1.05 | 1.05 | 1.00 |
| 17491 | | CD4pCD45RAnCD49dp | CD49d int | 1.34 | 2.40 | 0.48 | 0.29 | 0.33 | 0.80 | 0.94 | 0.92 | 0.97 | 1.02 | 0.97 | 1.05 |
| 17497 | | CD4pCD45RApCD49dp | CD49d int | 0.02 | 1.74 | 1.37 | 1.55 | 1.18 | 0.16 | 1.00 | 0.91 | 0.89 | 1.11 | 1.13 | 0.98 |
| 16023 | | CD4pCD45RAnCD49dn /CD4p | CR | 1.26 | 1.92 | 0.58 | 0.19 | 0.24 | 0.54 | 1.21 | 1.26 | 1.14 | 0.96 | 1.07 | 0.90 |
| 16025 | | CD4pCD45RAnCD49dp /CD4p | CR | 0.61 | 0.08 | 0.75 | 0.76 | 1.53 | 0.62 | 1.06 | 0.99 | 0.91 | 1.07 | 1.17 | 0.92 |
| 16027 | | CD4pCD45RApCD49dn /CD4p | CR | 1.20 | 0.79 | 0.04 | 0.19 | 0.84 | 0.56 | 0.81 | 0.84 | 0.99 | 0.96 | 0.83 | 1.17 |
| 16029 | | CD4pCD45RApCD49dp /CD4p | CR | 0.52 | 0.15 | 0.26 | 0.43 | 1.01 | 0.69 | 0.84 | 0.94 | 1.12 | 0.89 | 0.75 | 1.19 |
| 16022 | | CD4pCD45RAnCD49dn | CT | 1.47 | 1.90 | 0.47 | 0.17 | 0.39 | 0.59 | 1.26 | 1.32 | 1.12 | 0.96 | 1.13 | 0.85 |
| 16024 | | CD4pCD45RAnCD49dp | CT | 0.32 | 0.07 | 0.68 | 0.41 | 1.10 | 0.53 | 1.07 | 0.98 | 0.86 | 1.09 | 1.24 | 0.88 |
| 16026 | | CD4pCD45RApCD49dn | CT | 0.90 | 0.54 | 0.24 | 0.18 | 0.38 | 0.16 | 0.80 | 0.83 | 0.90 | 0.96 | 0.89 | 1.08 |
| 16028 | | CD4pCD45RApCD49dp | CT | 0.41 | 0.30 | 0.08 | 0.14 | 0.34 | 0.28 | 0.80 | 0.84 | 0.93 | 0.95 | 0.86 | 1.11 |
| 17512 | CD8 T cells | CD8pCD45RAnCD49dp | CD49d int | 0.29 | 0.82 | 0.11 | 0.27 | 0.06 | 0.29 | 0.98 | 0.96 | 0.99 | 1.02 | 0.99 | 1.03 |
| 17521 | | CD8pCD45RApCD49dp | CD49d int | 0.44 | 0.29 | 0.76 | 0.09 | 0.34 | 0.40 | 0.96 | 0.97 | 0.93 | 0.99 | 1.04 | 0.95 |
| 16039 | | CD8pCD45RAnCD49dn /CD8p | CR | 1.20 | 1.95 | 1.40 | 0.09 | 0.26 | 0.22 | 1.41 | 1.46 | 1.63 | 0.96 | 0.86 | 1.11 |
| 16041 | | CD8pCD45RAnCD49dp /CD8p | CR | 0.44 | 0.85 | 1.12 | 1.79 | 1.87 | 0.37 | 1.07 | 0.89 | 0.82 | 1.21 | 1.31 | 0.92 |
| 16045 | | CD8pCD45RApCD49dn /CD8p | CR | 0.05 | 1.09 | 0.97 | 1.12 | 0.99 | 0.03 | 0.98 | 1.39 | 1.42 | 0.70 | 0.69 | 1.02 |
| 16047 | | CD8pCD45RApCD49dp /CD8p | CR | 0.55 | 0.41 | 0.09 | 0.12 | 0.26 | 0.18 | 0.91 | 0.93 | 0.97 | 0.97 | 0.93 | 1.05 |
| 16038 | | CD8pCD45RAnCD49dn | CT | 1.82 | 1.02 | 0.58 | 0.76 | 0.25 | 0.11 | 1.54 | 1.23 | 1.31 | 1.25 | 1.18 | 1.06 |
| 16040 | | CD8pCD45RAnCD49dp | CT | 0.13 | 1.74 | 1.54 | 2.15 | 2.22 | 0.33 | 0.97 | 0.71 | 0.62 | 1.36 | 1.55 | 0.88 |
| 16043 | | CD8pCD45RAp/CD8p | CT | 0.62 | 0.50 | 0.71 | 1.55 | 1.48 | 0.28 | 0.93 | 1.06 | 1.10 | 0.88 | 0.84 | 1.04 |
| 16046 | | CD8pCD45RApCD49dp | CT | 0.88 | 1.49 | 1.18 | 0.35 | 0.46 | 0.14 | 0.81 | 0.72 | 0.67 | 1.13 | 1.20 | 0.94 |

\* Int = intensity, CT = absolute cell count, CR = count ratio, Mean ratio = $2^{nd}/1^{st}$.

**Soluble VCAM**

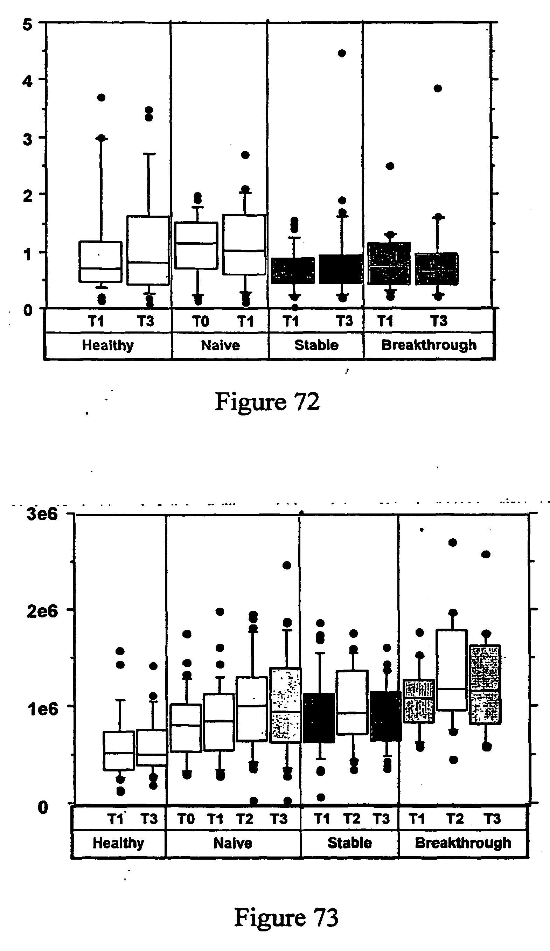

sVCAM-1 (CD106) showed many interesting differences. The most striking differences were in the cross group comparisons. Soluble VCAM was higher in all MS groups compared with the Healthy group **(Figure 73 and Table 93).** The order of groups was B > S, N (Drug) > N (No Drug) > H, with the Breakthrough group being two-fold higher than the healthy group. The 30% difference between the Breakthrough and Stable groups is significant and was observed at both 6-days and 34 hours post injection.

In addition to the long-term Avonex effect, there was also a modest 10-20% increase short-term post dosing. The biggest difference was for the Naive group when the first go on drug.

Table 93: (Var: IA-0014 (pg/mL))

|        | NvH  | SvH  | BvH  | SvN  | BvN  | SVB  |
|--------|------|------|------|------|------|------|
| **P***   | 2.93 | 4.30 | 6.10 | 0.38 | 2.11 | 1.48 |
| **ES****  | 0.76 | 0.96 | 1.66 | 0.17 | 0.82 | 0.66 |
| **^MR**   | 1.44 | 1.56 | 2.01 | 0.93 | 0.72 | 1.29 |

*Neg log p-value, **Effect size
^Mean ratio: MS/H; N/S,B; B/S

## Stable vs Breakthrough Comparisons

here we focus on differences that may distinguish responder and non responders to Avonex. Emphasis is placed on the Stable to Breakthrough comparison. The Breakthrough to Naïve and Healthy comparisons are also informative.

## Immunoassays

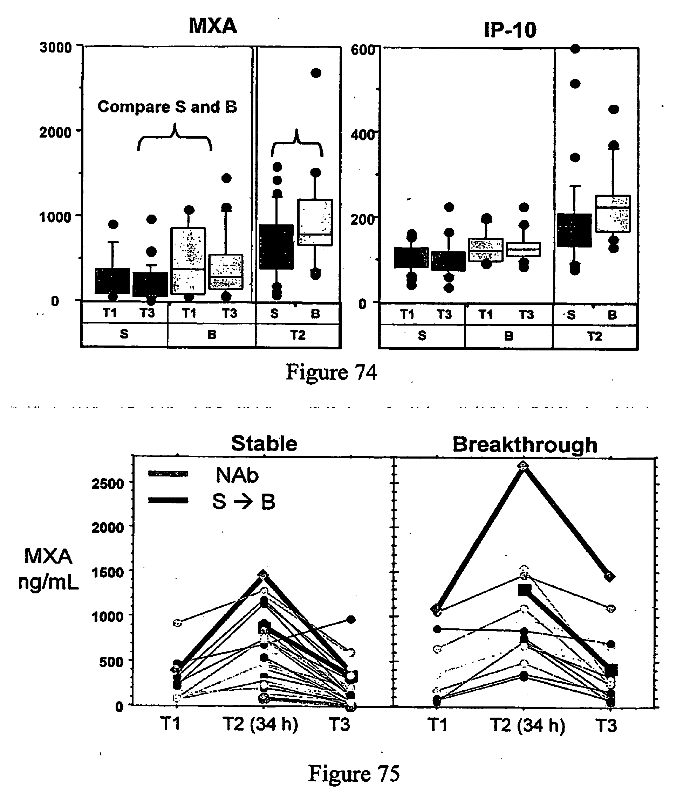

**MXA and IP-10,** two interferon inducible proteins, were higher in the Breakthrough group than the Stable group **(Figure 74 and Table 94).** The difference was significant for both at the steady state comparison and the results were consistent at the 34-hour post injection comparison. The mean difference was almost two-fold for MXA at steady state and 25% higher for IP-10. **Figure 75** shows the MXA results on a subject-by-subject basis. Two subjects were enrolled first in the Stable cohort and then in the Breakthrough cohort. Their levels of MXA are higher as Breakthrough subjects.

**Table 94**: **(var: IA** 51, 15)

|        | MXA |  |  | IP-10 |  |
|--------|------|------|---|------|------|
|        | **34 Hr** | **SS** |  | **34 Hr** | **SS** |
| **P***   | 1.24 | 2.05 |  | 0.71 | 2.05 |
| **ES****  | 0.67 | 0.72 |  | 0.36 | 0.76 |
| **^MR**   | 1.46 | 1.90 |  | 1.19 | 1.25 |
| *Neg log p-value, **Effect size ^ Mean ratio B/S | | | | | |

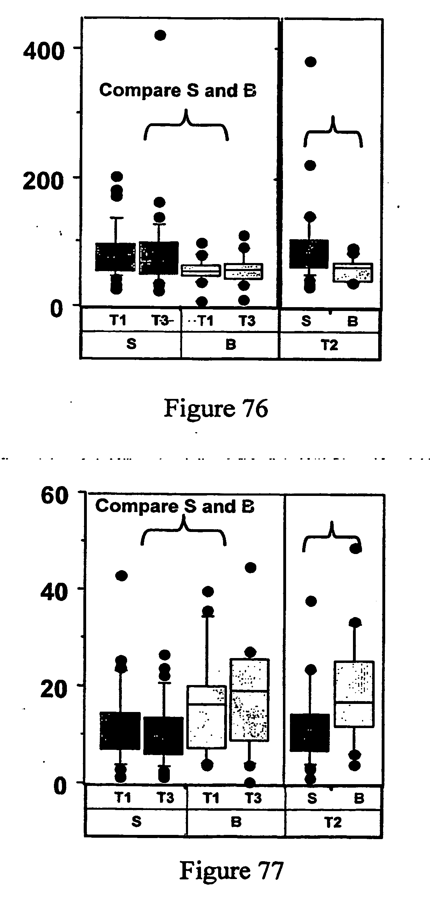

**MIP1β** was 30 to 40% lower in the Breakthrough group than the Stable group **(Figure 76 abd Table 95A and B).** The result is significant at both the steady state and 34-hour timepoints. Both groups had significantly lower levels than the Healthy group.

Table 95A (Var: IA-21)

|        | SvB |  |
|--------|------|------|
|        | **6 D** | **34 Hr** |
| **P***   | 1.81 | 2.74 |
| **ES****  | 0.60 | 0.71 |
| **^MR**   | 0.67 | 0.62 |
| Neg log p-value, **Effect size^ Mean ratio B/S | | |

Table 95B (Var: IA-21)

|  | NvH | SvH | BvH | SvN | BvN |
|---|---|---|---|---|---|
|  |  |  |  |  |  |
| P* | 0.52 | 1.65 | 3.64 | 0.34 | 1.25 |
| ES** | 0.23 | 0.56 | 1.15 | 0.21 | 0.59 |
| ^MR | 0.85 | 0.72 | 0.48 | 1.18 | 1.76 |
| ^Mean ratio: MS/H; N/S,B; B/S |  |  |  |  |  |

Beta-NGF is a nerve growth factor structurally related to BDNF, NT-3 and NT-4. It plays a crucial role in the development and preservation of the sensory and sympathetic nervous systems. Beta-NGF also acts as a growth and differentiation factor for B Cells and enhances B-cell survival. Beta-NGF is 50 to 60% higher in the Breakthrough group than the Stable group at both steady state and short-term post injection **(Figure 77 and Table 96).**

Table 96:

|  | 34 Hr | SS |
|---|---|---|
| P* | 1.63 | 1.61 |
| ES** | 0.81 | 0.71 |
| ^MR | 1.60 | 1.53 |
| *Neg log p-value, **Effect size ^ Mean ratio B/S | | |

**Mass spectrometry**

Apolipoproteins. The apolipoprotein family was highly represented among the variables detected in the 2D serum proteome. The 13 apolipoproteins identified accounted for 6.3% (1030/13297) of the total components. Apolipoproteins are one of the most abundant types of proteins in serum, and they are further enriched by our depletion protocol for albumin, immunoglobulin etc. Some are also large, with many potential tryptic peptides to detect

The Stable vs Breakthrough comparison showed five apolipoprotein differences by accession number, corresponding to four proteins, which are summarized in **Table 97.** The magnitude of the differences was modest at about 20% decrease (ApoB, ApoA1) or increase (ApoH, ApoA) in Breakthrough subjects. Many of the variables that differ in this comparison were also different in the Breakthrough comparisons Naive and Healthy subjects.

**Table 97: Apolipoprotein changes differences in the Stable vs Breakthrough comparison**

|  | Components | | | | | | |
|---|---|---|---|---|---|---|---|
| Lipoprotein, Accession # | Total | P< 0.05 | Trend | % Diff | Trend | <Exp Ratio>* | <MR> Trend |
| ApoB-100, LPHUB | 503 | 83 | 71 | 17 | Down | 0.87 | 0.73 |
| ApoB-100, P04114 | 54 | 18 | 18 | 33 | Down | 0.77 | 0.77 |
| ApoA1, P02647 | 172 | 21 | 15 | 12 | Down | 0.83 | 0.72 |
| ApoH, NBHU | 47 | 10 | 6 | 21 | Up | 1.21 | 1.29 |
| ApoA-IV, P06726 | 28 | 7 | 6 | 25 | Up | 1.31 | 1.47 |
| *<Exp Ratio> average of all components with p< 0.05, <MR> average of components with main trend | | | | | | | |

**Alpha-1-antichymotrypsin precursor (ACT) (Accession No. P01011, ITHUC).** Higher levels of ACT were observed in the Breakthrough group compared to Healthy, Naive and Stable groups. ACT was found at differential levels in our DeepLook analysis. Multiple variables (58) matched this protein with two accession numbers, which provides strong confidence in its identity. About one-third (18) of the components show consistent results in the HvB comparison. Although ACT was detected in the 1D proteome, between group differences are not observed. Data for two variables from the DeepLook experiment corresponding to ACT is presented in **Table 98.**

**Table 98: ACT**

| | HvsS | Hvs B | HvsN | SvsN | BvsN | SvsB | | HvsS | Hvs B | HvsN | SvsN | BvsN | SvsB |
|---|---|---|---|---|---|---|---|---|---|---|---|---|---|
| **P** | 0.19 | 3.03 | 0.02 | 0.15 | 2.80 | 2.50 | | 0.41 | 3.01 | 0.03 | 0.36 | 2.92 | 1.80 |
| **ES** | 0.11 | 1.01 | 0.01 | 0.09 | 0.94 | 0.90 | | 0.21 | 0.98 | 0.02 | 0.19 | 0.97 | 0.66 |
| **MR** | 1.05 | 1.43 | 1.01 | 0.96 | 0.70 | 1.36 | | 1.11 | 1.45 | 1.01 | 0.91 | 0.70 | 1.31 |
| VAR: SE-LC-PT-0000425203 (DL) | | | | | | | VAR: SE-LC-PT- SE-LC-PT-0000424567 (DL) | | | | | | |
| P= Neg log p-value, ES= Effect sizes, Mar= Mean ratio: 2nd/1st | | | | | | | | | | | | | |

Nucleobindin 1 precursor (CALNUC) (Accession No. Q02818) CALNUC levels were lower in the Breakthrough group compared to Healthy, Naïve and Stable groups **(Table 99).** The two variables corresponding to this protein, which are different charge states of the same peptide, are shown below.

**Table 99: CALNUC**

| | HvsS | Hvs B | HvsN | SvsN | BvsN | SvsB | | HvsS | Hvs B | HvsN | SvsN | BvsN | SvsB |
|---|---|---|---|---|---|---|---|---|---|---|---|---|---|
| **P** | 0.95 | 4.18 | 0.07 | 0.92 | 4.93 | 2.12 | | 1.05 | 3.95 | 0.09 | 0.99 | 4.46 | 1.69 |
| **ES** | 0.38 | 0.95 | 0.05 | 0.38 | 1.11 | 0.62 | | 0.41 | 0.92 | 0.06 | 0.40 | 1.05 | 0.54 |
| **MR** | 0.86 | 0.68 | 0.98 | 1.15 | 1.45 | 0.79 | | 0.86 | 0.71 | 0.98 | 1.15 | 1.38 | 0.83 |
| VAR: SE-LC-PT-0000421500 (DL) | | | | | | | VAR: SE-LC-PT-0000418172 (DL) | | | | | | |
| P= Neg log p-value, ES= Effect size, MR= Mean ratio: 2nd/1st | | | | | | | | | | | | | |

**Acute phase serum amyloid A protein (A-SAA) (serum amyloid A-2) (Accession No. I39456).** Two variables out of four detected, corresponding to A-SAA, were lower in the Breakthrough group compared to Healthy and Stable groups **(Table 100).** These two variables had the same amino acid sequence and one of them showed post-translational modifications. A-SAA was also detected in the 1D proteome, with 1 variable changing, out of 1 detected, in the Breakthrough group as well.

**Table 100: A-SAA**

| | HvsS | Hvs B | HvsN | SvsN | BvsN | SvsB | | HvsS | Hvs B | HvsN | SvsN | BvsN | SvsB |
|---|---|---|---|---|---|---|---|---|---|---|---|---|---|
| **P** | 0.23 | 2.20 | 0.53 | 0.26 | 1.13 | 2.03 | | 0.37 | 2.44 | 0.16 | 0.11 | 1.37 | 1.85 |
| **ES** | 0.19 | 1.08 | 0.39 | 0.20 | 0.62 | 0.84 | | 0.23 | 0.78 | 0.11 | 0.08 | 0.54 | 0.58 |
| **MR** | 0.86 | 0.37 | 0.72 | 0.83 | 1.93 | 0.43 | | 0.80 | 0.39 | 0.88 | 1.10 | 2.25 | 0.49 |
| VAR: SE-LC-PT-0000419136 (DL) | | | | | | | VAR: SE-LC-PT-0000419132 (DL) | | | | | | |
| P= Neg log p-value, ES= Effect size, Mar= Mean ratio: 2nd/1st | | | | | | | | | | | | | |

**Constitutively expressed Serum amyloid A protein (C-SAA) (serum amyloid A-4) (Accession No. P35542).** Lower levels of Amyloid A protein were identified by DeepLook analysis in the Breakthrough group compared to Healthy, Naïve and Stable groups. Four variables that differ, out of 13 detected (one third) are presented in **Table 101.** In the 1D proteome, this protein was also detected with 6 variables, of which only 1 showed differential levels in the Naïve group. This difference between 1D and 2D analysis may reflect the higher sensitivity and better sample set of the DeepLook analysis.

**Table 101: C-SAA**

| | HvsS | Hvs B | HvsN | SvsN | BvsN | SvsB | | HvsS | Hvs B | HvsN | SvsN | BvsN | SvsB |
|---|---|---|---|---|---|---|---|---|---|---|---|---|---|
| **P** | 0.28 | 3.11 | 0.57 | 0.19 | 2.13 | 2.41 | | 0.06 | 1.93 | 0.22 | 0.12 | 2.19 | 1.82 |
| **ES** | 0.16 | 0.83 | 0.27 | 0.12 | 0.70 | 0.74 | | 0.04 | 0.88 | 0.14 | 0.08 | 0.92 | 0.76 |
| **MR** | 0.89 | 0.49 | 0.83 | 0.92 | 1.68 | 0.55 | | 1.01 | 0.78 | 1.03 | 1.02 | 1.32 | 0.78 |
| VAR: SE-LC-PT-0000413711 (DL) | | | | | | | | VAR: SE-LC-PT-0000426473 (DL) | | | | | |

| | HvsS | Hvs B | B HvsN | SvsN | BvsN | SvsB | | HvsN | Hvs B | HvsN | SvsN | BvsN | SvsB |
|---|---|---|---|---|---|---|---|---|---|---|---|---|---|
| **P** | 0.33 | 0.99 | 0.04 | 0.37 | 0.83 | 1.62 | | 0.85 | 1.32 | 0.63 | 0.17 | 0.77 | 0.58 |
| **ES** | 0.17 | 0.48 | 0.03 | 0.19 | 0.41 | 0.69 | | 0.63 | 0.83 | 0.50 | 0.16 | 0.59 | 0.53 |
| **MR** | 1.04 | 0.88 | 0.99 | 0.95 | 1.13 | 0.84 | | 0.78 | 0.68 | 0.82 | 1.05 | 1.20 | 0.87 |
| VAR: SE-LC-PT-0000418796 (DL) | | | | | | | | VAR: SE-LC-PT-0000426169 (DL) | | | | | |

| P= Neg log p-value, ES= Effect size, Mar= Mean ratio: 2nd/1St |
|---|

C-reactive protein, pentraxin-related (CRP) (Accession No. NP_000558.1). Lower levels of CRP were found in the Breakthrough group compared to Healthy, Naive and Stable groups (Table 102). Three variables are identified corresponding to this protein that changed significantly, out of three detected by DeepLook analysis. Two of them are different charge states of the same peptide (shown below). CRP was not readily monitored in the serum 1D proteome on our platform due to overlapping components. Our immunoassay for CRP did not show significant changes in the Breakthrough group, which could be attributed to: 1) antigenic alterations in CRP that prevent the recognition of the native protein by I.A (87); 2) modified forms of CRP that can be detected by proteomics only; 3) forms of the protein resulting from alternative processing pathways that Proteomics only is able to detect.

**Table 102: CRP**

| | HvsS | Hvs B | HvsN | SvsN | BvsN | SvsB | | HvsS | Hvs B | HvsN | SvsN | BvsN | SvsB |
|---|---|---|---|---|---|---|---|---|---|---|---|---|---|
| **P** | 0.05 | 4.47 | 0.17 | 0.17 | 2.21 | 2.28 | | 0.78 | 0.32 | 1.89 | 0.35 | 2.41 | 1.15 |
| **ES** | 0.04 | 1.46 | 0.13 | 0.13 | 0.84 | 0.81 | | 0.41 | 0.25 | 0.82 | 0.23 | 0.91 | 0.49 |
| **MR** | 1.02 | 0.61 | 0.95 | 0.93 | 1.55 | 0.60 | | 1.56 | 0.84 | 1.92 | 1.23 | 2.30 | 0.54 |
| VAR: SE-LC-PT-0000428147 (DL) | | | | | | | | VAR: SE-LC-PT-0000425445 (DL) | | | | | |

| | HvsS | Hvs B | HvsN | SvsN | BvsN | SvsB |
|---|---|---|---|---|---|---|
| **P** | 0.77 | 1.80 | 0.76 | 0.09 | 0.31 | 0.12 |
| **ES** | 0.36 | 0.69 | 0.36 | 0.06 | 0.18 | 0.08 |
| **MR** | 1.29 | 1.36 | 1.24 | 0.96 | 0.91 | 1.05 |
| VAR: SE-LC-PT-SE-LC-PT-0000421260 (DL) | | | | | |

| P= Neg log p-value, ES= Effect size, MR= Mean ratio: 2nd/1st |
|---|

**Gelsolin isoform b (accession No. NP_937895.1).** Higher levels of Gelsolin were found in the Breakthrough group than in Healthy, Naive and Stable groups **(Table 103).** Nine variables corresponding to this protein were detected by DeepLook analysis, out of which four differ significantly (shown below). In the1D proteome, six variables were detected representing three different peptide sequences. Two of the three peptides showed a slight increase in the Naive group compared to Healthy group, and a slight decrease in Naive group compared to Stable group.

**Table 103: Gelsolin**

| | HvsS | Hvs B | HvsN | SvsN | BvsN | SvsB | | HvsS | Hvs B | HvsN | SvsN | BvsN | SvsB |
|---|---|---|---|---|---|---|---|---|---|---|---|---|---|
| **P** | 0.01 | 4.37 | 0.27 | 0.29 | 3.82 | 4.44 | | 0.24 | 2.54 | 0.14 | 0.05 | 2.04 | 2.58 |
| **ES** | 0.01 | 1.48 | 0.20 | 0.22 | 1.63 | 1.57 | | 0.13 | 0.76 | 0.09 | 0.03 | 0.68 | 0.86 |
| **MR** | 0.99 | 1.71 | 1.09 | 1.10 | 0.64 | 1.72 | | 0.96 | 0.79 | 0.97 | 01.01 | 1.23 | 0.82 |
| VAR: SE-LC-PT-0000417589 (DL) | | | | | | | | VAR: SE-LC-PT-0000421916 (DL) | | | | | |

| | HvsS | Hvs B | HvsN | SvsN | BvsN | SvsB | | HvsS Hvs | B | HvsN | SvsN | BvsN | SvsB |
|---|---|---|---|---|---|---|---|---|---|---|---|---|---|
| **P** | 0.69 | 0.72 | 0.74 | 0.03 | 1.41 | 1.38 | | 0.16 | 0.94 | 0.34 | 0.09 | 1.47 | 1.13 |
| **ES** | 0.31 | 0.47 | 0.33 | 0.02 | 0.79 | 0.72 | | 0.09 | 0.52 | 0.18 | 0.06 | 0.77 | 0.55 |
| **MR** | 0.93 | 1.12 | 0.93 | 1.00 | 0.84 | 1.20 | | 0.98 | 1.12 | 0.97 | 0.99 | 0.86 | 1.15 |
| VAR SE-LC-PT-0000414905 (DL) | | | | | | | | VAR: SE-LC-PT-0000426718 (DL) | | | | | |
| *P: Neg log p-value, ES: Effect size, MR: Mean ratio = 2nd/1st | | | | | | | | | | | | | |

Alpha-2-HS-glycoprotein precursor (Fetuin-A) (accession No. P02765) in urine. Differential levels of Fetuin-A were observed in the urine proteome. Six variables were detected corresponding to this protein, of which five differ **(Table 104).** The differences among variables are very consistent. Lower levels were detected in the Breakthrough group compared to the Healthy group, in the Breakthrough steady state compared to Naive and Stable groups, and also, at the latest time point after Avonex treatment (6D) in the Breakthrough group compared to the Naive and Stable group at those later points. The consistency of this findings make Fetuin-A a very interesting potential urine biomarker of Breakthrough subjects.

## Table 104: Fetuin-A

| | HvsN | Hvs S | HvsB | SvN SS | BvN SS | SvB SS | | HvsN | Hvs S | HvsB | SvN SS | BvN SS | SvB SS |
|---|---|---|---|---|---|---|---|---|---|---|---|---|---|
| VAR: UR-LC-PT-0000408293 | | | | | | | VAR: UR-LC-PT-0000409585 | | | | | | |
| **P** | 1.14 | 0.85 | 2.90 | 0.40 | 3.01 | 5.40 | | 0.90 | 1.16 | 3.36 | 0.05 | 1.92 | 2.05 |
| **ES** | 0.45 | 0.33 | 0.90 | 0.19 | 0.85 | 0.95 | | 0.29 | 0.38 | 0.65 | 0.08 | 0.77 | 0.71 |
| **MR** | 0.66 | 0.76 | 0.31 | 0.87 | 2.14 | 0.41 | | 0.72 | 0.68 | 0.33 | 1.06 | 2.15 | 0.50 |
| VAR: UR-LC-PT-0000410032 | | | | | | | VAR: UR-LC-PT-0000408158 | | | | | | |
| **P** | 0.98 | 0.60 | 3.97 | 0.31 | 2.94 | 4.46 | | 1.44 | 1.32 | 5.98 | 0.47 | 2.51 | 6.53 |
| **ES** | 0.31 | 0.22 | 0.67 | 0.15 | 0.85 | 0.79 | | 0.52 | 0.40 | 0.91 | 0.25 | 0.74 | 1.00 |
| **MR** | 0.70 | 0.80 | 0.32 | 0.88 | 2.18 | 0.41 | | 0.59 | 0.71 | 0.28 | 0.83 | 2.10 | 0.39 |

**\*P: Neg log p-value, ES: Effect size, MR: Mean ratio = 2nd/1st**

**AMBP protein precursor [Contains: Alpha-1-microglobulin] (accession No.** P02760). This protein is detected in the urine proteome with 28 variables, of which 20 are significantly different. Of particular interest are the 6 variables shown below **(Table 105).** These variables showed consistent lower levels in the Breakthrough group compared to Healthy, Naïve and Stable groups in steady states, as well as at 24h and 6D following Avonex injection.

## Table 105: AMBP

| | HvsN | Hvs S | HvsB | SvN SS | BvN SS | SvB SS | | HvsN | Hvs S | HvsB | SvN SS | BvN SS | SvB SS |
|---|---|---|---|---|---|---|---|---|---|---|---|---|---|
| VAR: UR-LC-PT-0000409624 | | | | | | | VAR: UR-LC-PT-0000408146 | | | | | | |
| P | 0.01 | 0.25 | 2.17 | 0.21 | 2.36 | 2.54 | | 0.18 | 0.16 | 1.77 | 0.05 | 1.07 | 2.00 |
| ES | 0.01 | 0.15 | 0.79 | 0.15 | 0.69 | 0.88 | | 0.09 | 0.09 | 0.78 | 0.01 | 0.59 | 0.82 |
| MR | 1.01 | 0.90 | 0.46 | 1.12 | 2.18 | 0.51 | | 0.94 | 0.95 | 0.54 | 0.99 | 1.74 | 0.57 |
| VAR: UR-LC-PT-0000409619 | | | | | | | VAR: UR-LC-PT-0000410129 | | | | | | |
| P | 0.19 | 0.57 | 2.86 | 0.18 | 1.93 | 2.30 | 0.01 | 0.06 | 1.72 | 0.07 | 1.36 | 2.21 | 0.01 |
| ES | 0.08 | 0.23 | 0.70 | 0.14 | 0.68 | 0.90 | 0.03 | 0.04 | 0.72 | 0.07 | 0.64 | 0.83 | 0.03 |
| MR | 0.92 | 0.83 | 0.44 | 1.11 | 2.11 | 0.53 | 1.02 | 0.98 | 0.55 | 1.05 | 1.88 | 0.56 | 1.02 |
| VAR: UR-LC-PT-0000408876 | | | | | | | VAR: UR-LC-PT-0000407540 | | | | | | |
| P | 0.20 | 0.32 | 2.02 | 0.02 | 1.52 | 2.35 | | 0.34 | 0.07 | 1.64 | 0.48 | 2.83 | 6.05 |
| ES | 0.13 | 0.17 | 0.82 | 0.02 | 0.66 | 0.84 | | 0.25 | 0.02 | 0.95 | 0.25 | 1.02 | 1.05 |
| MR | 0.90 | 0.88 | 0.44 | 1.02 | 2.03 | 0.50 | | 0.80 | 0.98 | 0.23 | 0.81 | 3.41 | 0.24 |

P= Neg log p-value, ES= Effect size, MR= Mean ratio: $2^{nd}/1^{st}$

REFERENCES

1. Alam J, Goelz S, Rioux P, Scaramucci J, Jones W, et al. 1997. Comparative pharmacokinetics and pharmacodynamics of two recombinant human interferon beta-1a a (IFN beta-1 a) products administered intramuscularly in healthy male and female volunteers. Pharm.Res. 14: 546

2. Arnason BG. 1996. Interferon beta in multiple sclerosis. Clin Immunol Immunopathol 81: 1

3. Askanas V EW. 1998. Sporadic inclusion-body myositis and its similarities to Alzheimer disease brain. Recent approaches to diagnosis and pathogenesis, and relation to aging. Scand J Rheumatol. 27: 389

4. Bar-Or A, Oliveira EM, Anderson DE, Hafler DA. 1999. Molecular pathogenesis of multiple sclerosis. J Neuroimmunol 100: 252

5. Basham T, Smith W, Lanier L, Morhenn V, Merigan T. 1984. Regulation of expression of class II major histocompatibility antigens on human peripheral blood monocytes and Langerhans cells by interferon. Hum Immunol 10: 83

6. Bates DM, Pinheiro JC. 1998. Computational methods for multilevel models availalble at the CiteSeer Scientific Literature Digital Library website

7. Baxevanis CN, Reclos GJ, Arsenis P, Anastasopoulos E, Katsiyiannis A, et al. 1989. Decreased expression of HLA-DR antigens on monocytes in patients with multiple sclerosis. J Neuroimmunol 22: 177

8. Baxevanis CN, Reclos GJ, Papamichail M. 1990. Decreased HLA-DR antigen expression on monocytes causes impaired suppressor cell activity in multiple sclerosis. J Immunol 144: 4166

9. Beavis AJ, Pennline KJ. 1996. Allo-7: a new fluorescent tandem dye for use in flow cytometry 3. Cytometry 24: 390

10. Becker CH, Hastings CA, Norton SM. 2002. Mass spectrometric quantification of chemical mixture components, Patent No. 6,835,927

11. Benjamini Y, Hochber Y. 1995. Controlling the False Discovery Rate: A practical and powerful approach to multiple testing. Journal of the Royal Statistical Society, Series B 57:289

12. Bielekova B, Martin R. 2004. Development of biomarkers in multiple sclerosis. Brain 127: 1463

13. Bitsch A, Bahner D, Wachter C, Elitok E, Bogumil T, et al. 2004. Interferon beta-1b modulates serum sVCAM-1 levels in primary progressive multiple sclerosis. Acta Neurol Scand 110: 386

14. Bjartmar C, Trapp BD. 2001. Axonal and neuronal degeneration in multiple sclerosis: mechanisms and functional consequences. Curr Opin Neurol 14: 271

15. Blair RC, Troendle JF, Beck RW. 1996. Control of familywise errors in multiple endpoint assessments via stepwise permutation tests. 15: 1107

16. Bofill M, Borthwick NJ. 2000. CD38 in health and disease. Chem Immunol 75: 218

17. Boylan MT, Crockard AD, Duddy ME, Armstrong MA, McMillan SA, Hawkins SA. 2001. Interferon-beta1a administration results in a transient increase of serum amyloid A protein and C-reactive protein: comparison with other markers of inflammation 1. 75: 191

18. Brettschneider J, Ecker D, Bitsch A, Bahner D, Bogumil T, et al. 2002. The macrophage activity marker sCD14 is increased in patients with multiple sclerosis and upregulated by interferon beta-1b. J Neuroimmunol 133: 193

19. Brod SA, Nelson LD, Khan M, Wolinsky JS. 1997. IFN-beta 1b treatment of relapsing multiple sclerosis has no effect on CD3-induced inflammatory or counterregulatory anti-inflammatory cytokine secretion ex vivo after nine months. Int J Neurosci 90: 135

20. Buttmann M, Merzyn C, Rieckmann P. 2004. Interferon-beta induces transient systemic IP-10/CXCL10 chemokine release in patients with multiple sclerosis. J Neuroimmunol 156: 195

21. Calabresi PA, Prat A, Biernacki K, Rollins. J, Antel JP. 2001. T lymphocytes conditioned with Interferon beta induce membrane and soluble VCAM on human brain endothelial cells. J Neuroimmunol 115: 161

22. Campbell SJ FM, Clements JM, Wells G, Miller KM, Perry VH, Anthony DC. 2004. Reduction of excitotoxicity and associated leukocyte recruitment by a broad-spectrum matrix metalloproteinase inhibitor J Neurochem. 89: 1378

23. Chaudhuri A, Behan PO. 2004. Multiple sclerosis is not an autoimmune disease. Arch Neurol 61: 1610

24. Chieux V, Chehadeh W, Hautecoeur P, Harvey J, Wattre P, Hober D. 2001. Increased levels of antiviral MxA protein in peripheral blood of patients with a chronic disease of unknown etiology. 65: 301

25. Chitnis T, Imatola J, Khoury SJ. 2005. Therapeutic Strategies To Prevent Neurodegeneration And PromoteRegeneration In Multiple Sclerosis. Current Drug Targets - Immune, Endocrine & Metabolic Disorders, In press

26. Chung TF SJ, McKee A, Fine RE, Schreiber BM, Liang JS, Johnson RJ. 2000. Serum amyloid A in Alzheimer's disease brain is predominantly localized to myelin sheaths and axonal membrane. Amyloid 7: 105

27. Comabella M, Imitola J, Weiner HL, Khoury SJ. 2002. Interferon-beta treatment alters peripheral blood monocytes chemokine production in MS patients. J Neuroimmunol 126:205

28. Crucian B, Dunne P, Friedman H, Ragsdale R, Pross S, Widen R. 1996. Detection of altered T helper 1 and T helper 2 cytokine production by peripheral blood mononuclear cells in patients with multiple sclerosis utilizing intracellular cytokine detection by flow cytometry and surface marker analysis. Clin Diagn Lab Immunol 3: 411

29. Daveau M JL, Soury E, Olivier E, Masson S, Lyoumi S, Chan P, Hiron M, Lebreton JP, Husson A, Jegou S, Vaudry H, Salier JP. 1998. Hepatic and extra-hepatic transcription of inter-alpha-inhibitor family genes under normal or acute inflammatory conditions in rat. Arch Biochem Biophys. 350: 315

30. Davis PD, Feather-Henigan KD, Hines K. 2002. Assay of peroxidase activity, USA Patent No. 6, 432, 662

31. Deaglio S, Mallone R, Baj G, Donati D, Giraudo G, et al. 2001. Human CD38 and its ligand CD31 define a unique lamina propria T lymphocyte signaling pathway. Faseb J 15: 580

32. Deaglio S, Mehta K, Malavasi F. 2001. Human CD38: a (r)evolutionary story of enzymes and receptors. Leuk Res 25: 1

33. Deisenhammer F, Reindl M, Harvey J, Gasse T, Dilitz E, Borger T. 1999. Bioavailability of interferon beta 1b in MS patients with and without neutralizing antibodies. Neurology 52: 1239

34. Dietz LJ, Dubrow RS, Manian BS, Sizto NL. 1996. Volumetric capillary cytometry: a new method for absolute cell enumeration. Cytometry 23: 177

35. Dziegielewska KM AN, Lovell D, Nicol SC, Muller-Esterl W, Saunders NR. 1992. Fetuin-a new acute phase protein in the adult and in the fetus. Folia Histochem Cytobiol. 30: 187

36. Ferrante P, Fusi ML, Saresella M, Caputo D, Biasin M, et al. 1998. Cytokine production and surface marker expression in acute and stable multiple sclerosis: altered IL-12 production and augmented signaling lymphocytic activation molecule (SLAM)-expressing lymphocytes in acute multiple sclerosis. J Immunol 160: 1514

37. Franciotta D, Zardini E, Bergamaschi R, Andreoni L, Cosi V. 2003. Interferon gamma and interleukin 4 producing T cells in peripheral blood of multiple sclerosis patients undergoing immunomodulatory treatment. J Neurol Neurosurg Psychiatry 74: 123

38. Furlan R, Bergami A, Lang R, Brambilla E, Franciotta D, et al. 2000. Interferon-beta treatment in multiple sclerosis patients decreases the number of circulating T cells producing interferon-gamma and interleukin-4. J Neuroimmunol 111: 86

39. Genc K, Dona DL, Reder AT. 1997. Increased CD80(+) B cells in active multiple sclerosis and reversal by interferon bets-1b therapy. J Clin Invest 99: 2664

40. Geroldi D MP, Bianchi M, Di Vito C, Reino M, Bertona M, Emanuele E. 2005. Genetic association of alpha2-Heremans-Schmid glycoprotein polymorphism with late-onset Alzheimer's disease in Italians. Neurosci Lett. [Epub ahead of print]

41. Giorelli M, De Blasi A, Defazio G, Avolio C, Iacovelli L, et al. 2002. Differential regulation of membrane bound and soluble ICAM 1 in human endothelium and blood mononuclear cells: effects of interferon beta-1a. Cell Commun Adhes 9: 259

42. Gniadek P, Aktas O, Wandinger KP, Bellmann-Strobl J, Wengert O, et al. 2003. Systemic IFN-beta treatment induces apoptosis of peripheral immune cells in MS patients. J Neuroimmunol 137: 187

43. Goldstein D, Sielaff KM, Storer BE, Brown RR, Datta SP, et al. 1989. Human biologic response modification by interferon in the absence of measurable serum concentrations: a comparative trial of subcutaneous and intravenous interferon-beta serine. *J Natl Cancer Inst* 81: 1061

44. Gomes AC, Jonsson G, Mjornheim S, Olsson T, Hillert J, Grandien A.2003. Upregulation of the apoptosis regulators cFLIP, CD95 and CD95 ligand in peripheral blood mononuclear cells in relapsing-remitting multiple sclerosis. *J Neuroimmunol* 135: 126

45. Graber J, Zhan M, Ford D, Kursch F, Francis G, et al. 2005. Interferon-beta-1 a induces increases in vascular cell adhesion molecule: implications for its mode of action in multiple sclerosis. *J Neuroimmunol* 161: 169

46. Hafler DA, Slavik JM, Anderson DA, O'Connor KC, De Jager P, Baecher-Allan CM. 2005. Multiple Sclerosis. *Immunol Rev* 204: 208

47. Hafler DA, Weiner HL. 1995. Immunologic mechanisms and therapy in multiple sclerosis. *Immunol Rev* 144: 75

48. Hamann D, Baars PA, Rep MH, Hooibrink B, Kerkhof Garde SR, et al. 1997. Phenotypic and functional separation of memory and effector human CD8+ T cells. *J Exp Med* 186: 1407

49. Hirsch RL, Johnson KP. 1986. The effects of long-term administration of recombinant alpha-2 interferon on lymphocyte subsets, proliferation, and suppressor cell function in multiple sclerosis. J Interferon Res 6: 171

50. Holm S. 1979. A simple sequentially rejective multiple test procedure. In Scand J Stat, pp. 65

51. Hussien Y, Sanna A, Soderstrom M, Link H, Huang YM. 2004. Multiple sclerosis: expression of CD1a and production of IL-12p70 and IFN-gamma by blood mononuclear cells in patients on combination therapy with IFN-beta and glatiramer acetate compared to monotherapy with IFN-beta. Mult Scler 10: 16

52. Ishikawa R, Biron CA. 1993. IFN induction and associated changes in splenic leukocyte distribution. J Immunol 150: 3713

53. Jensen J, Krakauer M, Sellebjerg F. 2005. Cytokines and adhesion molecules in multiple sclerosis patients treated with interferon-beta1b. Cytokine 29: 24

54. Kanai Y TO, Kanai Y, Miura K, Kurosawa Y. 1993. Novel autoimmune phenomena induced in vivo by a new DNA binding protein Nuc: a study on MRL/n mice. Immunol Lett. 39: 83

55. Kantor AB. 2002. Comprehensive phenotyping and biological marker discovery. Dis Markers 18: 91

56. Kantor AB, .Alters SE, Cheal K, Dietz LJ. 2004. Immune systems biology: immunoprofiling of cells and molecules. Biotechniques 36: 520

57. Kantor AB, Wang W, Lin H, Govindarajan H, Anderle M, et al. 2004. Biomarker discovery by comprehensive phenotyping for autoimmune diseases. Clin Immunol 111: 186

58. Kastrukoff LF, Morgan NG, Zecchini D, White R, Petkau AJ, et al. 1999. Natural killer cells in relapsing-remitting MS: effect of treatment with interferon beta-1B. Neurology 52: 351

59. Kilinc M, Saatci-Cekirge I, Karabudak R. 2003. Serial analysis of soluble intercellular adhesion molecule-1 level in relapsing-remitting multiple sclerosis patients during IFN-beta1b treatment. J Interferon Cytokine Res 23: 127

60. Killestein J, Rep MH, Barkhof F, Roos MT, Ader HJ, et al. 2001. Active MRI lesion appearance in MS patients is preceded by fluctuations in circulating T-helper 1 and 2 cells. J Neuroimmunol 118: 286

61. Komgold R, Blank KJ, Murasko DM. 1953. Effect of interferon on thoracic duct lymphocyte output: induction with either poly I:poly C or vaccinia virus. J Immunol 130:2236

62. Kracke A, von Wussow P, A1 Masri AN, Dalley G, Windhagen A, Heidenreich F. 2000. Mx proteins in blood leukocytes for monitoring interferon beta-1b therapy in patients with MS. Neurology 54: 193

63. Landmann R, Muller B, Zimmerli W. 2000. CD14, new aspects of ligand and signal diversity. Microbes Infect 2: 295

64. Licastro F PS, Davis LJ, Caputo L, Tagliabue J, Savorani G, Cucinotta D, Annoni G. 2001. Alpha-1-antichymotrypsin and oxidative stress in the peripheral blood from patients with probable Alzheimer disease: a short-term longitudinal study. Alzheimer Dis Assoc Disord. 15: 51

65. Ling PD, Warren MK, Vogel SN. 1985. Antagonistic effect of interferon-beta on the interferon-gamma-induced expression of Ia antigen in murine macrophages. J Immunol 135: 1857

66. Liu JS, Amaral TD, Brosnan CF, Lee SC. 1998. IFNs are critical regulators of IL-1 receptor antagonist and IL-1 expression in human microglia. J Immunol 161: 1989

67. Liu Z, Pelfrey CM, Cotleur A, Lee JC, Rudick RA. 2001. Immunomodulatory effects of interferon beta-1a in multiple sclerosis. J Neuroimmunol 112: 153

68. Lucchinetti C, Bruck W, Parisi J, Scheithauer B, Rodriguez M, Lassmann H. 2000. Heterogeneity of multiple sclerosis lesions: implications for the pathogenesis of demyelination. Ann Neurol 47: 707

69. Mainou-Fowler T, Dignum HM, Proctor SJ, Summerfield GP. 2004. The prognostic value of CD38 expression and its quantification in B cell chronic lymphocytic leukemia (B-CLL). Leuk Lymphoma 45: 455

70. Malucchi S, Sala A, Gilli F, Bottero R, Di Sapio A, et al. 2004. Neutralizing antibodies reduce the efficacy of betaIFN during treatment of multiple sclerosis. Neurology 62: 2031

71. Marckmann S, Wiesemann E, Hilse R, Trebst C, Stangel M, Windhagen A. 2004. Interferon-beta up-regulates the expression of co-stimulatory molecules CD80, CD86 and CD40 on monocytes: significance for treatment of multiple sclerosis. Clin Exp Immunol 138: 499

72. Marhaug G DS. 1994. Serum amyloid A: an acute phase apolipoprotein and precursor of AA amyloid. Baillieres Clin Rheumatol. 8: 553

73. Matusevicius D, Kivisakk P, Navikas VV, Tian W, Soderstrom M, et al. 1998. Influence of IFN-beta1b (Betaferon) on cytokine mRNA profiles in blood mononuclear cells and plasma levels of soluble VCAM-1 in multiple sclerosis. Eur J Neurol 5: 265

74. Maury CP TA, Raunio P. 1983. The acute phase response and its relation to amyloid A degrading activity in serum of patients with rheumatoid arthritis undergoing arthroplasty. Eur J Clin Invest. 13: 73

75. Miller DH, Khan OA, Sheremata WA, Blumhardt LD, Rice GP, et al. 2003. A controlled trial of natalizumab for relapsing multiple sclerosis. N Engl J Med 348: 15

76. Molnarfi N, Hyka-Nouspikel N, Gruaz L, Dayer JM, Burger D. 2005. The production of IL-1 receptor antagonist in IFN-beta-stimulated human monocytes depends on the activation of phosphatidylinositol 3-kinase but not of STAT1. J Immunol 174: 2974

77. Moody MD, Van Arsdell SW, Murphy KP, Orencole SF, Burns C. 2001. Array-based ELISAs for high-throughput analysis of human cytokines. Biotechniques 31: 186

78. Mujumdar RB, Ernst LA, Mujumdar SR, Lewis CJ, Waggoner AS. 1993. Cyanine dye labeling reagents: sulfoindocyanine succinimidyl esters. Bioconjug Chem 4: 105

79. Noronha A, Toscas A, Jensen MA. 1993. Interferon beta decreases T cell activation and interferon gamma production in multiple sclerosis. J Neuroimmunol 46: 145

80. Norton SM, Winkler J, Dietz LJ. 2000. Cell enumeration and characterization in Microvolume Laser Scanning Cytometry:A multicolor image processing package. Proc.SPIE-Int.Soc.Opt.Eng. 3921: 20

81. O'Connor KC, Bar-Or A, Hafler DA. 2001. The neuroimmunology of multiple sclerosis: possible roles of T and B lymphocytes in immunopathogenesis. J Clin Immunol 21: 81

82. Oh SK, Luhowskyj S, Witt P, Ritch P, Reitsma D, et al. 1994. Quantitation of interferon-induced Mx protein in whole blood lysates by an immunochemiluminescent assay: elimination of protease activity of cell lysates in toto. J Immunol Methods 176: 79

83. Pachner AR, Bertolotto A, Deisenhammer F. 2003. Measurement of MxA mRNA or protein as a biomarker of IFNbeta bioactivity: detection of antibody-mediated decreased bioactivity (ADB). Neurology 61: S24

84. Perini P, Wadhwa M, Buttarello M, Meager A, Facchinetti A, et al. 2000. Effect of IFNbeta and anti-IFNbeta antibodies on NK cells in multiple sclerosis patients. J Neuroimmunol 105: 91

85. Peterson JW, Trapp BD. 2005. Neuropathobiology of multiple sclerosis. Neurol Clin 23: 107

86. Pogue SL, Preston BT, Stalder J, Bebbington CR, Cardarelli PM. 2004. The receptor for type I IFNs is highly expressed on peripheral blood B cells and monocytes and mediates a distinct profile of differentiation and activation of these cells. J Interferon Cytokine Res 24: 131

87. Potempa LA MB, Laurent P, Zemel ES, Gewurz H. 1983. Antigenic, electrophoretic and binding alterations of human C-reactive protein modified selectively in the absence of calcium. Mol Immunol. 20: 1165

88. R Development Core Team 2005. R: A language and environment for statistical computing. R Foundation for Statistical Computing Website

89. Ray S LP, Ochieng J. 2003. Members of the cystatin superfamily interact with MMP-9 and protect it from autolytic degradation without affecting its gelatinolytic activities. Biochim Biophys Acta 1652: 91

90. Rep MH, Schrijver HM, van Lopik T, Hintzen RQ, Roos MT, et al. 1999. Interferon (IFN)-beta treatment enhances CD95 and interleukin 10 expression but reduces interferon-gamma producing T cells in MS patients. J Neuroimmunol 96: 92

91. Rieckmann P, Kruse N, Nagelkerken L, Beckmann K, Miller D, et al. 2005. Soluble vascular cell adhesion molecule (VCAM) is associated with treatment effects of Interferon beta-1b in patients with Secondary Progressive Multiple Sclerosis. J Neurol 252:526

92. Rieckmann P, O'Connor P, Francis GS, Wetherill G, Alteri E. 2004. Haematological effects of interferon-beta-1a (Rebif) therapy in multiple sclerosis. Drug Saf 27: 745

93. Roederer M, Kantor AB, Parks DR, Herzenberg LA. 1996. Cy7PE and Cy7APC: bright new probes for immunofluorescence 436. Cytometry 24: 191

94. Roy S, Anderle M, Hua L, Becker C. 2004. Differential expression profiling of serum proteins and metabolites for biomarker discovery. Int J. of Mass Spectrometry 238: 163

95. Roy S, Becker C. 2005. Quantification of proteins and metabolites by mass spectrometry without isotype labeling. In Methods in Molecular Biology: An overview of recent developments in proteomics using quantitative mass spectrometry, ed. S Sechi: Humana Press

96. Rudick RA. 2001. Contemporary immunomodulatory therapy for multiple sclerosis. J Nearoophtalmol 21:284

97. Rudick RA, Carpenter CS, Cookfair DL, Tuohy VK, RansohoffRM. 1993. In vitro and in vivo inhibition of mitogen-driven T-cell activation by recombinant interferon beta. Neurology 43: 2080

98. Sallusto F, Lenig D, Forster R, Lipp M, Lanzavecchia A. 1999. Two subsets of memory T lymphocytes with distinct homing potentials and effector functions. Nature 401: 708

99. Schif-Zuck S WJ, Netzer N, Zohar Y, Meiron M, Wildbaum G, Karin N. 2005. Targeted overexpression of IL-18 binding protein at the central nervous system overrides flexibility in functional polarization of antigen-specific Th2 cells. J Immunol. 174:4307

100. Schmidt R SH, Fazekas F, Kapeller P, Roob G, Lechner A, Kostner GM, Hartung HP. 2000. MRI cerebral white matter lesions and paraoxonase PON1 polymorphisms : three-year follow-up of the austrian stroke prevention study. Arterioscler Thromb Vasc Biol. 20:1811

101. Sega S, Wraber B, Mesec A, Horvat A, Ihan A. 2004. IFN-betala and IFN-beta1b have different patterns of influence on cytokines. Clin Neurol Neurosurg 106: 255

102. Smith ME. 2001. Phagocytic properties of microglia in vitro: implications for a role in multiple sclerosis and EAE. Microsc Res Tech 54: 81

103. Soilu-Hanninen M, Salmi A, Salonen R. 1995. Interferon-beta downregulates expression of VLA-4 antigen and antagonizes interferon-gamma-induced expression of HLA-DQ on human peripheral blood monocytes. J Neuroimmunol 60: 99

104. Sospedra M, Martin R. 2005. Immunology of multiple sclerosis. Annu Rev Immunol 23: 683

105. Southwick PL, Ernst LA, Tauriello EW, Parker SR, Mujumdar RB, et al. 1990. Cyanine dye labeling reagents-carboxymethylindocyanine succinimidyl esters 166. Cytometry 11: 418

106. Spear GT, Paulnock DM, Jordan RL, Meltzer DM, Merritt JA, Borden EC. 1987. Enhancement of monocyte class I and II histocompatibility antigen expression in man by in vivo beta-interferon. Clin Exp Immunol 69: 107

107. Struyf S, Van Collie E, Paemen L, Put W, Lenaerts JP, et al. 1998. Synergistic induction of MCP-1 and -2 by IL-lbeta and interferons in fibroblasts and epithelial cells. JLeukoc Biol 63: 364

108. Sturzebecher S, Maibauer R, Heuner A, Beckmann K, Aufdembrinke B. 1999. Pharmacodynamic comparison of single doses of IFN-betala and IFN-beta1b in healthy volunteers. 19: 1257

109. Stuve O, Prod'homme T, Youssef S, Dunn S, Neuhaus O, et al. 2004. Statins as potential therapeutic agents in multiple sclerosis. Curr Neurol Neurosci Rep 4: 237

110. Szalai AJ NS, Hu XZ, Barnum SR. 2002. Experimental allergic encephalomyelitis is inhibited in transgenic mice expressing human C-reactive protein. J Immunol 168: 5792

111. Sztein MB, Steeg PS, Johnson HM, Oppenheim JJ. 1984. Regulation of human peripheral blood monocyte DR antigen expression in vitro by lymphokines and recombinant interferons. J Clin Invest 73: 556

112. Tanaka J SK. 1994. Localization and characterization of gelsolin in nervous tissues: gelsolin is specifically enriched in myelin-forming cells. J Neurosci. 14: 1038

113. Towbin H, Schmitz A, Jakschies D, von Wussow P, Horisberger MA. 1992. A whole blood immunoassay for the interferon-inducible human Mx protein. 12: 67

114. Trojano M, Defazio G, Avolio C, Paolicelli D, Giuliani F, et al. 2000. Effects of rIFN-beta-1b on serum circulating ICAM-1 in relapsing remitting multiple sclerosis and on the membrane-bound ICAM-1 expression on brain microvascular endothelial cells. J Neurovirol 6 Suppl 2: S47

115. Tussey L, Speller S, Gallimore A, Vessey R. 2000. Functionally distinct CD8+ memory T cell subsets in persistent EBV infection are differentiated by migratory receptor expression. Eur J Immunol 30: 1823

116. Uhlar CM WA. 1999. Serum amyloid A, the major vertebrate acute-phase reactant. Eur J Biochem. 265: 501

117. Vallittu AM, Halminen M, Peltoniemi J, Ilonen J, Julkunen I, et al. 2002. Neutralizing antibodies reduce MxA protein induction in interferon-beta-1a-treated MS patients. Neurology 58: 1786

118. Van Weyenbergh J, Lipinski P, Abadie A, Chabas D, Blank U, et al. 1998. Antagonistic action of IFN-beta and IFN-gamma on high affinity Fc gamma receptor expression in healthy controls and multiple sclerosis patients. J Immunol 161: 1568

119. Venables WN, Ripley BD. 2002. Modern Applied Statistics with S: Springer

120. von Wussow P, Jakschies D, Hochkeppel HK, Fibich C, Penner L, Deicher H. 1990. The human intracellular Mx-homologous protein is specifically induced by type I interferons. 20: 2015

121. Walton ID, Dietz LJ, Frescatore RL, Chen J, Winkles J, et al. 2000. Microvolume laser scanning cytometry platform for biological marker discovery. Presented at Proc.SPIE-Int.Soc.Opt.Eng.

122. Wang W, Zhou H, Lin H, Roy S, Shaler TA, et al. 2003. Quantification of proteins and metabolites by mass spectrometry without isotopic labeling or spiked standards. Anal Chem 75: 4818

123. Waubant E, Gee L, Miller K, Stabler G, Goodkin D. 2001. IFN-betala may increase serum levels of TIMP-1 in patients with relapsing-remitting multiple sclerosis. J Interferon Cytokine Res 21: 181

124. Waubant E, Goodkin D, Bostrom A, Bacchetti P, Hietpas J, et al. 2003. IFNbeta lowers MMP-9/TMP-1 ratio, which predicts new enhancing lesions in patients with SPMS. Neurology 60: 52

125. Waubant E, Goodkin DE, Gee L, Bacchetti P, Sloan R, et al. 1999. Serum MMP-9 and TIMP-1 levels are related to MRI activity in relapsing multiple sclerosis. Neurology 53: 1397

126. Williams GJ, Witt PL. 1998. Comparative study of the pharmacodynamic and pharmacologic effects of Betaseron and AVONEX. J Interferon Cytokine Res 18: 967

127. Woodroofe N, Cross AK, Harkness K, Simpson JE. 1999. The role of chemokines in the pathogenesis of multiple sclerosis. Adv Fxp Med Biol 468: 135

128. Yamada T WA, Yamaguchi T, Itoh Y, Kawai T. 1997. Automated measurement of a constitutive isotype of serum amyloid A/SAA4 and comparison with other apolipoproteins. J Clin Lab Anal. 11: 363

129. Youssef S, Stuve O, Patarroyo JC, Ruiz PJ, Radosevich JL, et al. 2002. The HMG-CoA se inhibitor, atorvastatin, promotes a Th2 bias and reverses paralysis in central nervous system autoimmune disease. Nature 420: 78

130. Zhou H, Kantor AB, Becker C. 1005. Differential metobolic profiling for biomarker discovery. In Metabolome Analyses: Strategies in Systems Biology, ed. S Vaidyanathan, GG Harrigan, T Goodacre: Springer

While various embodiments of the present invention have been described in detail, it is apparent that modifications and adaptations of those embodiments will occur to those skilled in the art. It is to be expressly understood, however, that such modifications and adaptations are within the scope of the present invention, as set forth in the following claims.

**Claims**

1. A method of monitoring interferon (IFN)-beta therapy In a human subject, the subject having been administered an IFN-beta therapeutic composition, the method comprising evaluating in a biological sample whether administration of the IFN-beta therapeutic composition causes a transient change in a monocyte-associated variable, wherein the monocyte-assodated variable is monocyte expression of an HLA Class II HLA-DP, wherein the subject is a multiply sclerosis (MS) patient on established IFN-beta therapy, and wherein a transient change in the monocyte-associated variable indicates efficacious treatment of multiple sclerosis.

2. The method of Claim 1, wherein the change in the monocyte-associated variable is an increase and occurs within about 1 to about 2 days after administration of the therapeutic composition.

3. The method of Claim 1, wherein the change in the monocyte-associated variable is reversed at about 6 days after administration of the therapeutic composition.

4. The method of any of Claims 1 to 3, wherein two or more, three or more, or four or more monocyte-associated variables are evaluated, and wherein the further monocyte-associated variables are selected from the group consisting of absolute monocyte count; monocyte/leukocyte ratio; and monocyte expression of CCR5, CD11b. CD38, CD40, CD54, CD64, CD69, CD86, TLR2, TLR4; or MCP2 (monocyte chemoattractant protein 2).

5. The method of any of Claims 1 to 4, wherein the change comprises an increase in monocyte expression of HLA-DP protein of at least 10%.

6. The method of any of Claims 1 to 5, wherein the change comprises an increase in two or more, or three or more HLA Class II, the HLA Class II further comprising DR, DQ or PAN (HLA-DP, DR and DQ).

7. The method of any one of Claims 1 to 6, wherein the expression of the HLA Class II is an increase in expression and wherein the increase is of at least about 30%, at least about 40%, at least about 50%, at least about 60%, at least about 65%, at least about 70%, or at least about 75%.

8. The method of any one of Claims 1 to 6, wherein the expression of the HLA Class II is an increase in expression and wherein the increase is of between about 30% and about 120%, or between about 50% and about 75%.

9. The method of any of Claims 1-8, wherein the change in the monocyte-associated variable is detected by cytometry, immunoassay, mass spectrometry, or a method of quantifying nucleic acids.

10. The method of any of Claims 1-9, wherein the therapeutic composition comprises an interferon beta variant, homologue, or fragment thereof, wherein said variant, homologue, or fragment has a similar activity as interferon beta 1 a.

11. The method of Claim 10, wherein said interferon beta homologue is derivatized by pegylation.

12. The method of Claim 1, wherein the therapeutic composition comprises AVONEX® or BETASERON®.

13. The method of Claim 1, wherein the expression of the HLA Class II molecule has an effect size of at least about 0.7, at least about 0.8, at least about 0.9, at least about 1, at least about 1.1, at least about 1.2, at least about 1.3, at least about 1.4, at least about 1.5, at least about 1.6, at least about 1.7, at least about 1.8, at least about 1.9, at least about 2, al least about 2.1, at least about 2.2, at least about 2.3, at least about 2.4, at least about 2.5, at least about 2.6, or at least about 2.8.

**Patentansprüche**

1. Verfahren zur Überwachung einer Interferon (IFN)-beta-Therapie bei einem humanen Subjekt, wobei dem Subjekt eine therapeutische Zusammensetzung von IFN-beta verabreicht wird, wobei das Verfahren das Beurteilen in einer biologischen Probe, ob die Verabreichung der therapeutischen Zusammensetzung von IFN-beta eine vorübergehende Veränderung einer mit Monozyten in Verbindung stehenden Variablen verursacht, umfasst, wobei die mit Monozyten in Verbindung stehende Variable die Monozytenexpression von einem HLA-Klasse-II-HLA-DP ist, wobei das Subjekt ein Multiple Sklerose (MS)-Patient in einer etablierten IFN-beta-Therapie ist, und wobei eine vorübergehende Veränderung der mit Monozyten in Verbindung stehenden Variablen eine wirksame Behandlung von Multipler Sklerose anzeigt.

2. Verfahren nach Anspruch 1, wobei die Veränderung der mit Monozyten in Verbindung stehenden Variablen eine Zunahme ist und diese innerhalb von etwa 1 bis etwa 2 Tagen nach der Verabreichung der therapeutischen Zusammensetzung erfolgt.

3. Verfahren nach Anspruch 1, wobei die Veränderung der mit Monozyten in Verbindung stehenden Variablen etwa 6 Tage nach der Verabreichung der therapeutischen Zusammensetzung rückläufig ist.

4. Verfahren nach einem der Ansprüche 1 bis 3, wobei zwei oder mehr, drei oder mehr oder vier oder mehr mit Monozyten in Verbindung stehende Variablen beurteilt werden und wobei die weiteren, mit Monozyten in Verbindung stehenden Variablen aus der Gruppe von der absoluten Monozytenzahl; dem Monozyten/Leukozyten-Verhältnis; und der Monozytenexpression von CCR5, CD11b, CD38, CD40, CD54, CD64, CD69, CD86, TLR2, TLR4; oder MCP2 (Monocyte Chemoattractant Protein 2) ausgewählt sind.

5. Verfahren nach einem der Ansprüche 1 bis 4, wobei die Veränderung eine Zunahme der Monozytenexpression des HLA-DP-Proteins von mindestens 10 % umfasst.

6. Verfahren nach einem der Ansprüche 1 bis 5, wobei die Veränderung eine Zunahme von zwei oder mehr, oder drei oder mehr HLA-Klasse-II umfasst, wobei das HLA-Klasse-II ferner DR, DQ oder PAN (HLA-DP, DR und DQ) umfasst.

7. Verfahren nach einem der Ansprüche 1 bis 6, wobei die Expression des HLA-Klasse-II eine Zunahme der Expression ist und wobei die Zunahme mindestens etwa 30 %, mindestens etwa 40 %, mindestens etwa 50 %, mindestens etwa 60 %, mindestens etwa 65 %, mindestens etwa 70 % oder mindestens etwa 75 % beträgt.

8. Verfahren nach einem der Ansprüche 1 bis 6, wobei die Expression des HLA-Klasse-II eine Zunahme der Expression ist und wobei die Zunahme zwischen etwa 30 % und etwa 120 % oder zwischen etwa 50 % und etwa 75 % liegt.

9. Verfahren nach einem der Ansprüche 1-8, wobei die Veränderung der mit Monozyten in Verbindung stehenden Variablen durch Zytometrie, einen Immunoassay, Massenspektrometrie oder ein Verfahren zur quantitativen Bestimmung von Nucleinsäuren detektiert wird.

**10.** Verfahren nach einem der Ansprüche 1-9, wobei die therapeutische Zusammensetzung eine Variante von Interferon-beta, ein Homologon oder ein Fragment hiervon umfasst, wobei die Variante, das Homologon oder das Fragment eine ähnliche Aktivität wie Interferon-beta-1a aufweisen.

**11.** Verfahren nach Anspruch 10, wobei das Interferon-beta-Homologon durch PEGylierung derivatisiert ist.

**12.** Verfahren nach Anspruch 1, wobei die therapeutische Zusammensetzung AVONEX® oder BETASERON® umfasst.

**13.** Verfahren nach Anspruch 1, wobei die Expression des HLA-Klasse-II-Moleküls eine Wirkungsgröße von mindestens etwa 0,7, mindestens etwa 0,8, mindestens etwa 0,9, mindestens etwa 1, mindestens etwa 1,1, mindestens etwa 1,2, mindestens etwa 1,3, mindestens etwa 1,4, mindestens etwa 1,5, mindestens etwa 1,6, mindestens etwa 1,7, mindestens etwa 1,8, mindestens etwa 1,9, mindestens etwa 2, mindestens etwa 2,1, mindestens etwa 2,2, mindestens etwa 2,3, mindestens etwa 2,4, mindestens etwa 2,5, mindestens etwa 2,6 oder mindestens etwa 2,8 aufweist.

**Revendications**

**1.** Procédé de surveillance d'un traitement par interféron (IFN)-bêta chez un sujet humain, le sujet s'étant vu administrer une composition thérapeutique d'IFN-bêta, le procédé comprenant le fait d'évaluer dans un échantillon biologique si l'administration de la composition thérapeutique d'IFN-bêta provoque un changement transitoire dans une variable associée aux monocytes, dans lequel la variable associée aux monocytes est l'expression monocytaire d'HLA-DP, un HLA de classe II, dans lequel le sujet est un patient atteint de sclérose en plaques (SP) suivant un traitement IFN-bêta établi, et dans lequel un changement transitoire dans la variable associée aux monocytes indique un traitement efficace de la sclérose en plaques.

**2.** Procédé selon la revendication 1, dans lequel le changement dans la variable associée aux monocytes est une augmentation et a lieu dans une période d'environ 1 à environ 2 jours après administration de la composition thérapeutique.

**3.** Procédé selon la revendication 1, dans lequel le changement dans la variable associée aux monocytes est inversé environ 6 jours après administration de la composition thérapeutique.

**4.** Procédé selon l'une quelconque des revendications 1 à 3, dans lequel deux ou plus, trois ou plus, ou quatre ou plus variables associées aux monocytes sont évalués, et dans lequel les variables associées aux monocytes supplémentaires sont choisies parmi le groupe constitué par la numération absolue de monocytes ; le rapport monocyte/leucocyte ; et l'expression monocytaire de CCR5, CD11b, CD38, CD40, CD54, CD64, CD69, CD86, TLR2, TLR4 ; ou MCP2 (protéine chimio-attractive monocytaire 2).

**5.** Procédé selon l'une quelconque des revendications 1 à 4, dans lequel le changement comprend une augmentation de l'expression monocytaire de la protéine HLA-DP d'au moins 10 %.

**6.** Procédé selon l'une quelconque des revendications 1 à 5, dans lequel le changement comprend une augmentation dans deux ou plus ou trois ou plus HLA de classe II, les HLA de classe II comprenant en outre DR, DQ ou PAN (HLA-DP, DR et DQ).

**7.** Procédé selon l'une quelconque des revendications 1 à 6, dans lequel l'expression de HLA de classe II est une augmentation dans l'expression et dans lequel l'augmentation est d'au moins environ 30 %, au moins environ 40 %, au moins environ 50 %, au moins environ 60 %, au moins environ 65 %, au moins environ 70 % ou au moins environ 75 %.

**8.** Procédé selon l'une quelconque des revendications 1 à 6, dans lequel l'expression de HLA de classe II est une augmentation dans l'expression et dans lequel l'augmentation est d'entre environ 30 % et environ 120 %, ou d'entre environ 50 % en environ 75 %.

**9.** Procédé selon l'une quelconque des revendications 1 à 8, dans lequel le changement dans la variable associée aux monocytes est détecté par cytométrie, immunodosage, spectrométrie de masse ou un procédé de quantification d'acides nucléiques.

**10.** Procédé selon l'une quelconque des revendications 1 à 9, dans lequel la composition thérapeutique comprend un variant d'interféron bêta, un homologue ou un fragment de ce dernier, dans lequel ledit variant, homologue, ou fragment a une activité similaire à l'interféron bêta 1a.

**11.** Procédé selon la revendication 10, dans lequel ledit homologue d'interféron bêta est dérivatisé par pégylation.

**12.** Procédé selon la revendication 1, dans lequel la composition thérapeutique comprend de l'AVONEX® ou du BETASERON®.

**13.** Procédé selon la revendication 1, dans lequel l'expression de la molécule HLA de classe II a une ampleur d'effet d'au moins environ 0,7, au moins environ 0,8, au moins environ 0,9, au moins environ 1, au moins environ 1,1, au moins environ 1,2, au moins environ 1,3, au moins environ 1,4, au moins environ 1,5, au moins environ 1,6, au moins environ 1,7, au moins environ 1,8, au moins environ 1,9, au moins environ 2, au moins environ 2,1, au moins environ 2,2, au moins environ 2,3, au moins environ 2,4, au moins environ 2,5, au moins environ 2,6 ou au moins environ 2,8.

Figure 1

Figure 2

Figure 3A

Figure 3B

Figure 4A

Figure 4B

Figure 5A

Figure 5B

Figure 6A

Figure 6B

Figure 7

LC-MS Metabolome Identification Process

Figure 8

EP 1 929 297 B1

Figure 9

Figure 10

Figure 11A          Figure 11B          Figure 11C

87

Figure 12

Figure 13

Figure 14

Figure 15A

Figure 15B

Figure 16

Figure 17

Figure 18

Figure 19

Figure 20

Figure 21

Figure 22

Figure 23

Figure 24

Figure 25

Figure 26

Figure 27

Figure 28

Figure 29

Figure 30

Figure 31

Figure 32

Figure 33

Figure 34

Figure 35

Figure 36

Figure 37

Figure 38

Figure 39

## C-Reactive Protein

Figure 40

## α-1-anti-chymotrypsin (Pep)

Figure 41

## Peptide 1

## Peptide 2

## Peptide 3

Figure 42          Figure 43          Figure 44

Figure 45

Figure 46

Figure 47

Figure 48

Figure 49

Figure 50

Figure 51

Figure 52

Figure 53

Figure 54

Figure 55

Figure 56

Figure 57

Figure 58

Figure 59

Figure 60

CD45RA

| Terminal effector memory | Naive |
|---|---|
| Effector memory | Central memory |

CD62L

Figure 61

|  | Healthy | | Naïve | | Stable | | Breakthrough | |
|---|---|---|---|---|---|---|---|---|
| CD45RA | 8±6 | 30±14 | 4±4 | 26±15 | 3±4 | 29±14 | 6±6 | 33±12 |
|  | 21±7 | 41±12 | 23±10 | 47±12 | 18±9 | 50±11 | 18±7 | 43±12 |

CD62L

Figure 62

|  | Healthy | | Naïve | | Stable | | Breakthrough | |
|---|---|---|---|---|---|---|---|---|
| CD45RA | 22±14 | 34±13 | 17±12 | 33±17 | 16±11 | 42±13 | 17±15 | 44±12 |
|  | 26±9 | 18±9 | 26±14 | 23±13 | 22±11 | 20±10 | 21±11 | 17±11 |

CD62L

Figure 63

Figure 64

Figure 65

Figure 66

Figure 67

Figure 68

Figure 69

Figure 70

Figure 71

Figure 72

Figure 73

Figure 74

Figure 75

Figure 76

Figure 77

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6835927 B
- US 6432662 B

- US 60722172 B

**Non-patent literature cited in the description**

- **STÜRZEBECHER et al.** *J. Interferon & Cytokine Res.,* 1999, vol. 19, 1257-64
- **SHAPIRO et al.** *Journal of Neuroimmunology,* 2003, vol. 144, 116-124
- **BOYLAN et al.** *Immunology letter,* 2001, vol. 75, 191-197
- **LUBLIN ; REINGOLD.** *Neurology,* 1996, vol. 46 (4), 907-11
- **MAULIK et al.** Molecular Biotechnology: Therapeutic Applications and Strategies. Wiley-Liss, Inc, 1997
- **HUMBLET ; DUNBAR.** Animal Reports in Medicinal Chemistry. Academic Press, 1993, vol. 28, 275-283
- **MERRIFIELD.** *Methods Enzymol.,* 1997, vol. 289, 3-13
- **WADE et al.** *Australas Biotechnol.,* 1993, vol. 3 (6), 332-336
- **WONG et al.** *Experientia,* 1991, vol. 47 (11-12), 1123-1129
- **CAREY et al.** *Ciba Found Symp.,* 1991, vol. 158, 187-203
- **PLANE et al.** *Biologicals,* 1990, vol. 18 (3), 147-157
- **BODANSZKY.** *Int. J. Pept. Protein Res.,* 1985, vol. 25 (5), 449-474
- **H. DUGAS ; C. PENNEY.** *BIOORGANIC CHEMISTRY,* 1981, 54-92
- **ALTSCHUL, S.F. ; MADDEN, T.L. ; SCHAAFFER, A.A. ; ZHANG, J. ; ZHANG, Z. ; MILLER, W. ; LIPMAN, DJ.** Gapped BLAST and PSI-BLAST: a new generation of protein database search programs. *Nucleic Acids Res.,* 1997, vol. 25, 3389
- **ALAM J ; GOELZ S ; RIOUX P ; SCARAMUCCI J ; JONES W et al.** Comparative pharmacokinetics and pharmacodynamics of two recombinant human interferon beta-1a a (IFN beta-1 a) products administered intramuscularly in healthy male and female volunteers. *Pharm.Res.,* 1997, vol. 14, 546
- **ARNASON BG.** Interferon beta in multiple sclerosis. *Clin Immunol Immunopathol,* 1996, vol. 81, 1

- **ASKANAS V EW.** Sporadic inclusion-body myositis and its similarities to Alzheimer disease brain. Recent approaches to diagnosis and pathogenesis, and relation to aging. *Scand J Rheumatol.,* 1998, vol. 27, 389
- **BAR-OR A ; OLIVEIRA EM ; ANDERSON DE ; HAFLER DA.** Molecular pathogenesis of multiple sclerosis. *J Neuroimmunol,* 1999, vol. 100, 252
- **BASHAM T ; SMITH W ; LANIER L ; MORHENN V ; MERIGAN T.** Regulation of expression of class II major histocompatibility antigens on human peripheral blood monocytes and Langerhans cells by interferon. *Hum Immunol,* 1984, vol. 10, 83
- **BATES DM ; PINHEIRO JC.** *Computational methods for multilevel models availalble at the CiteSeer Scientific Literature Digital Library website,* 1998
- **BAXEVANIS CN ; RECLOS GJ ; ARSENIS P ; ANASTASOPOULOS E ; KATSIYIANNIS A et al.** Decreased expression of HLA-DR antigens on monocytes in patients with multiple sclerosis. *J Neuroimmunol,* 1989, vol. 22, 177
- **BAXEVANIS CN ; RECLOS GJ ; PAPAMICHAIL M.** Decreased HLA-DR antigen expression on monocytes causes impaired suppressor cell activity in multiple sclerosis. *J Immunol,* 1990, vol. 144, 4166
- **BEAVIS AJ ; PENNLINE KJ.** Allo-7: a new fluorescent tandem dye for use in flow cytometry 3. *Cytometry,* 1996, vol. 24, 390
- **BENJAMINI Y ; HOCHBER Y.** Controlling the False Discovery Rate: A practical and powerful approach to multiple testing. *Journal of the Royal Statistical Society, Series B,* 1995, vol. 57, 289
- **BIELEKOVA B ; MARTIN R.** Development of biomarkers in multiple sclerosis. *Brain,* 2004, vol. 127, 1463
- **BITSCH A ; BAHNER D ; WACHTER C ; ELITOK E ; BOGUMIL T et al.** Interferon beta-1b modulates serum sVCAM-1 levels in primary progressive multiple sclerosis. *Acta Neurol Scand,* 2004, vol. 110, 386

- **BJARTMAR C ; TRAPP BD.** Axonal and neuronal degeneration in multiple sclerosis: mechanisms and functional consequences. *Curr Opin Neurol,* 2001, vol. 14, 271
- **BLAIR RC ; TROENDLE JF ; BECK RW.** *Control of familywise errors in multiple endpoint assessments via stepwise permutation tests,* 1996, vol. 15, 1107
- **BOFILL M ; BORTHWICK NJ.** CD38 in health and disease. *Chem Immunol,* 2000, vol. 75, 218
- **BOYLAN MT ; CROCKARD AD ; DUDDY ME ; ARMSTRONG MA ; MCMILLAN SA ; HAWKINS SA.** *Interferon-beta1a administration results in a transient increase of serum amyloid A protein and C-reactive protein: comparison with other markers of inflammation,* 2001, vol. 1 (75), 191
- **BRETTSCHNEIDER J ; ECKER D ; BITSCH A ; BAHNER D ; BOGUMIL T et al.** The macrophage activity marker sCD14 is increased in patients with multiple sclerosis and upregulated by interferon beta-1b. *J Neuroimmunol,* 2002, vol. 133, 193
- **BROD SA ; NELSON LD ; KHAN M ; WOLINSKY JS.** IFN-beta 1b treatment of relapsing multiple sclerosis has no effect on CD3-induced inflammatory or counterregulatory anti-inflammatory cytokine secretion ex vivo after nine months. *Int J Neurosci,* 1997, vol. 90, 135
- **BUTTMANN M ; MERZYN C ; RIECKMANN P.** Interferon-beta induces transient systemic IP-10/CXCL10 chemokine release in patients with multiple sclerosis. *J Neuroimmunol,* 2004, vol. 156, 195
- **CALABRESI PA ; PRAT A ; BIERNACKI K ; ROLLINS. J ; ANTEL JP.** T lymphocytes conditioned with Interferon beta induce membrane and soluble VCAM on human brain endothelial cells. *J Neuroimmunol,* 2001, vol. 115, 161
- **CAMPBELL SJ FM ; CLEMENTS JM ; WELLS G ; MILLER KM ; PERRY VH ; ANTHONY DC.** Reduction of excitotoxicity and associated leukocyte recruitment by a broad-spectrum matrix metalloproteinase inhibitor. *J Neurochem.,* 2004, vol. 89, 1378
- **CHAUDHURI A ; BEHAN PO.** Multiple sclerosis is not an autoimmune disease. *Arch Neurol,* 2004, vol. 61, 1610
- **CHIEUX V ; CHEHADEH W ; HAUTECOEUR P ; HARVEY J ; WATTRE P ; HOBER D.** *Increased levels of antiviral MxA protein in peripheral blood of patients with a chronic disease of unknown etiology,* 2001, vol. 65, 301
- **CHITNIS T ; IMATOLA J ; KHOURY SJ.** Therapeutic Strategies To Prevent Neurodegeneration And Promote Regeneration In Multiple Sclerosis. *Current Drug Targets - Immune, Endocrine & Metabolic Disorders,* 2005
- **CHUNG TF SJ ; MCKEE A ; FINE RE ; SCHREIBER BM ; LIANG JS ; JOHNSON RJ.** Serum amyloid A in Alzheimer's disease brain is predominantly localized to myelin sheaths and axonal membrane. *Amyloid,* 2000, vol. 7, 105
- **COMABELLA M ; IMITOLA J ; WEINER HL ; KHOURY SJ.** Interferon-beta treatment alters peripheral blood monocytes chemokine production in MS patients. *J Neuroimmunol,* 2002, vol. 126, 205
- **CRUCIAN B ; DUNNE P ; FRIEDMAN H ; RAGSDALE R ; PROSS S ; WIDEN R.** Detection of altered T helper 1 and T helper 2 cytokine production by peripheral blood mononuclear cells in patients with multiple sclerosis utilizing intracellular cytokine detection by flow cytometry and surface marker analysis. *Clin Diagn Lab Immunol,* 1996, vol. 3, 411
- **DAVEAU M JL ; SOURY E ; OLIVIER E ; MASSON S ; LYOUMI S ; CHAN P ; HIRON M ; LEBRETON JP ; HUSSON A ; JEGOU S.** Hepatic and extra-hepatic transcription of inter-alpha-inhibitor family genes under normal or acute inflammatory conditions in rat. *Arch Biochem Biophys,* 1998, vol. 350, 315
- **DEAGLIO S ; MALLONE R ; BAJ G ; DONATI D ; GIRAUDO G et al.** Human CD38 and its ligand CD31 define a unique lamina propria T lymphocyte signaling pathway. *Faseb J,* 2001, vol. 15, 580
- **DEAGLIO S ; MEHTA K ; MALAVASI F.** Human CD38: a (r)evolutionary story of enzymes and receptors. *Leuk Res,* 2001, vol. 25, 1
- **DEISENHAMMER F ; REINDL M ; HARVEY J ; GASSE T ; DILITZ E ; BORGER T.** Bioavailability of interferon beta 1b in MS patients with and without neutralizing antibodies. *Neurology,* 1999, vol. 52, 1239
- **DIETZ LJ ; DUBROW RS ; MANIAN BS ; SIZTO NL.** Volumetric capillary cytometry: a new method for absolute cell enumeration. *Cytometry,* 1996, vol. 23, 177
- **DZIEGIELEWSKA KM AN ; LOVELL D ; NICOL SC ; MULLER-ESTERL W ; SAUNDERS NR.** Fetuin-a new acute phase protein in the adult and in the fetus. *Folia Histochem Cytobiol.,* 1992, vol. 30, 187
- **FERRANTE P ; FUSI ML ; SARESELLA M ; CAPUTO D ; BIASIN M et al.** Cytokine production and surface marker expression in acute and stable multiple sclerosis: altered IL-12 production and augmented signaling lymphocytic activation molecule (SLAM)-expressing lymphocytes in acute multiple sclerosis. *J Immunol,* 1998, vol. 160, 1514
- **FRANCIOTTA D ; ZARDINI E ; BERGAMASCHI R ; ANDREONI L ; COSI V.** Interferon gamma and interleukin 4 producing T cells in peripheral blood of multiple sclerosis patients undergoing immunomodulatory treatment. *J Neurol Neurosurg Psychiatry,* 2003, vol. 74, 123

- **FURLAN R ; BERGAMI A ; LANG R ; BRAMBILLA E ; FRANCIOTTA D et al.** Interferon-beta treatment in multiple sclerosis patients decreases the number of circulating T cells producing interferon-gamma and interleukin-4. *J Neuroimmunol,* 2000, vol. 111, 86
- **GENC K ; DONA DL ; REDER AT.** Increased CD80(+) B cells in active multiple sclerosis and reversal by interferon bets-1b therapy. *J Clin Invest,* 1997, vol. 99, 2664
- **GEROLDI D MP ; BIANCHI M ; DI VITO C ; REINO M ; BERTONA M ; EMANUELE E.** Genetic association of alpha2-Heremans-Schmid glycoprotein polymorphism with late-onset Alzheimer's disease in Italians. *Neurosci Lett.,* 2005
- **GIORELLI M ; BLASI A ; DEFAZIO G ; AVOLIO C ; IACOVELLI L et al.** Differential regulation of membrane bound and soluble ICAM 1 in human endothelium and blood mononuclear cells: effects of interferon beta-1a. *Cell Commun Adhes,* 2002, vol. 9, 259
- **GNIADEK P ; AKTAS O ; WANDINGER KP ; BELLMANN-STROBL J ; WENGERT O et al.** Systemic IFN-beta treatment induces apoptosis of peripheral immune cells in MS patients. *J Neuroimmunol,* 2003, vol. 137, 187
- **HIRSCH RL ; JOHNSON KP.** The effects of long-term administration of recombinant alpha-2 interferon on lymphocyte subsets, proliferation, and suppressor cell function in multiple sclerosis. *J Interferon Res,* 1986, vol. 6, 171
- **HOLM S.** A simple sequentially rejective multiple test procedure. *Scand J Stat,* 1979, 65
- **HUSSIEN Y ; SANNA A ; SODERSTROM M ; LINK H ; HUANG YM.** Multiple sclerosis: expression of CD1a and production of IL-12p70 and IFN-gamma by blood mononuclear cells in patients on combination therapy with IFN-beta and glatiramer acetate compared to monotherapy with IFN-beta. *Mult Scler,* 2004, vol. 10, 16
- **ISHIKAWA R ; BIRON CA.** IFN induction and associated changes in splenic leukocyte distribution. *J Immunol,* 1993, vol. 150, 3713
- **JENSEN J ; KRAKAUER M ; SELLEBJERG F.** Cytokines and adhesion molecules in multiple sclerosis patients treated with interferon-beta1b. *Cytokine,* 2005, vol. 29, 24
- **KANAI Y TO ; KANAI Y ; MIURA K ; KUROSAWA Y.** Novel autoimmune phenomena induced in vivo by a new DNA binding protein Nuc: a study on MRL/n mice. *Immunol Lett.,* 1993, vol. 39, 83
- **KANTOR AB.** Comprehensive phenotyping and biological marker discovery. *Dis Markers,* 2002, vol. 18, 91
- **KANTOR AB ; ALTERS SE ; CHEAL K ; DIETZ LJ.** Immune systems biology: immunoprofiling of cells and molecules. *Biotechniques,* 2004, vol. 36, 520
- **KANTOR AB ; WANG W ; LIN H ; GOVINDARAJAN H ; ANDERLE M et al.** Biomarker discovery by comprehensive phenotyping for autoimmune diseases. *Clin Immunol,* 2004, vol. 111, 186
- **KASTRUKOFF LF ; MORGAN NG ; ZECCHINI D ; WHITE R ; PETKAU AJ et al.** Natural killer cells in relapsing-remitting MS: effect of treatment with interferon beta-1B. *Neurology,* 1999, vol. 52, 351
- **KILINC M ; SAATCI-CEKIRGE I ; KARABUDAK R.** Serial analysis of soluble intercellular adhesion molecule-1 level in relapsing-remitting multiple sclerosis patients during IFN-beta1b treatment. *J Interferon Cytokine Res,* 2003, vol. 23, 127
- **KILLESTEIN J ; REP MH ; BARKHOF F ; ROOS MT ; ADER HJ et al.** Active MRI lesion appearance in MS patients is preceded by fluctuations in circulating T-helper 1 and 2 cells. *J Neuroimmunol,* 2001, vol. 118, 286
- **KOMGOLD R ; BLANK KJ ; MURASKO DM.** Effect of interferon on thoracic duct lymphocyte output: induction with either poly I:poly C or vaccinia virus. *J Immunol,* 1953, vol. 130, 2236
- **KRACKE A ; WUSSOW P ; MASRI AN ; DALLEY G ; WINDHAGEN A ; HEIDENREICH F.** Mx proteins in blood leukocytes for monitoring interferon beta-1b therapy in patients with MS. *Neurology,* 2000, vol. 54, 193
- **LANDMANN R ; MULLER B ; ZIMMERLI W.** CD14, new aspects of ligand and signal diversity. *Microbes Infect,* 2000, vol. 2, 295
- **LICASTRO F PS ; DAVIS LJ ; CAPUTO L ; TAGLIABUE J ; SAVORANI G ; CUCINOTTA D ; ANNONI G.** Alpha-1-antichymotrypsin and oxidative stress in the peripheral blood from patients with probable Alzheimer disease: a short-term longitudinal study. *Alzheimer Dis Assoc Disord.,* 2001, vol. 15, 51
- **LING PD ; WARREN MK ; VOGEL SN.** Antagonistic effect of interferon-beta on the interferon-gamma-induced expression of Ia antigen in murine macrophages. *J Immunol,* 1985, vol. 135, 1857
- **LIU JS ; AMARAL TD ; BROSNAN CF ; LEE SC.** IFNs are critical regulators of IL-1 receptor antagonist and IL-1 expression in human microglia. *J Immunol,* 1998, vol. 161, 1989
- **LIU Z ; PELFREY CM ; COTLEUR A ; LEE JC ; RUDICK RA.** Immunomodulatory effects of interferon beta-1a in multiple sclerosis. *J Neuroimmunol,* 2001, vol. 112, 153
- **LUCCHINETTI C ; BRUCK W ; PARISI J ; SCHEITHAUER B ; RODRIGUEZ M ; LASSMANN H.** Heterogeneity of multiple sclerosis lesions: implications for the pathogenesis of demyelination. *Ann Neurol,* 2000, vol. 47, 707

- **MAINOU-FOWLER T ; DIGNUM HM ; PROCTOR SJ ; SUMMERFIELD GP.** The prognostic value of CD38 expression and its quantification in B cell chronic lymphocytic leukemia (B-CLL. *Leuk Lymphoma,* vol. 45, 455
- **MALUCCHI S ; SALA A ; GILLI F ; BOTTERO R ; DI SAPIO A et al.** Neutralizing antibodies reduce the efficacy of betaIFN during treatment of multiple sclerosis. *Neurology,* 2004, vol. 62, 2031
- **MARCKMANN S ; WIESEMANN E ; HILSE R ; TREBST C ; STANGEL M ; WINDHAGEN A.** Interferon-beta up-regulates the expression of co-stimulatory molecules CD80, CD86 and CD40 on monocytes: significance for treatment of multiple sclerosis. *Clin Exp Immunol,* 2004, vol. 138, 499
- **MARHAUG G DS.** Serum amyloid A: an acute phase apolipoprotein and precursor of AA amyloid. *Baillieres Clin Rheumatol.,* 1994, vol. 8, 553
- **MATUSEVICIUS D ; KIVISAKK P ; NAVIKAS VV ; TIAN W ; SODERSTROM M et al.** Influence of IFN-beta1b (Betaferon) on cytokine mRNA profiles in blood mononuclear cells and plasma levels of soluble VCAM-1 in multiple sclerosis. *Eur J Neurol,* 1998, vol. 5, 265
- **MAURY CP TA ; RAUNIO P.** The acute phase response and its relation to amyloid A degrading activity in serum of patients with rheumatoid arthritis undergoing arthroplasty. *Eur J Clin Invest.,* 1983, vol. 13, 73
- **MILLER DH ; KHAN OA ; SHEREMATA WA ; BLUMHARDT LD ; RICE GP et al.** A controlled trial of natalizumab for relapsing multiple sclerosis. *N Engl J Med,* 2003, vol. 348, 15
- **MOLNARFI N ; HYKA-NOUSPIKEL N ; GRUAZ L ; DAYER JM ; BURGER D.** The production of IL-1 receptor antagonist in IFN-beta-stimulated human monocytes depends on the activation of phosphatidylinositol 3-kinase but not of STAT1. *J Immunol,* 2005, vol. 174, 2974
- **MOODY MD ; ARSDELL SW ; MURPHY KP ; ORENCOLE SF ; BURNS C.** Array-based ELISAs for high-throughput analysis of human cytokines. *Biotechniques,* 2001, vol. 31, 186
- **MUJUMDAR RB ; ERNST LA ; MUJUMDAR SR ; LEWIS CJ ; WAGGONER AS.** Cyanine dye labeling reagents: sulfoindocyanine succinimidyl esters. *Bioconjug Chem,* 1993, vol. 4, 105
- **NORONHA A ; TOSCAS A ; JENSEN MA.** Interferon beta decreases T cell activation and interferon gamma production in multiple sclerosis. *J Neuroimmunol,* 1993, vol. 46, 145
- **NORTON SM ; WINKLER J ; DIETZ LJ.** Cell enumeration and characterization in Microvolume Laser Scanning Cytometry:A multicolor image processing package. *Proc.SPIE-Int.Soc.Opt.Eng.,* 2000, vol. 3921, 20
- **O'CONNOR KC ; BAR-OR A ; HAFLER DA.** The neuroimmunology of multiple sclerosis: possible roles of T and B lymphocytes in immunopathogenesis. *J Clin Immunol,* 2001, vol. 21, 81
- **OH SK ; LUHOWSKYJ S ; WITT P ; RITCH P ; REITSMA D et al.** Quantitation of interferon-induced Mx protein in whole blood lysates by an immunochemiluminescent assay: elimination of protease activity of cell lysates in toto. *J Immunol Methods,* 1994, vol. 176, 79
- **PACHNER AR ; BERTOLOTTO A ; DEISENHAMMER F.** Measurement of MxA mRNA or protein as a biomarker of IFNbeta bioactivity: detection of antibody-mediated decreased bioactivity (ADB. *Neurology,* 2003, vol. 61, 24
- **PERINI P ; WADHWA M ; BUTTARELLO M ; MEAGER A ; FACCHINETTI A et al.** Effect of IFN-beta and anti-IFNbeta antibodies on NK cells in multiple sclerosis patients. *J Neuroimmunol,* 2000, vol. 105, 91
- **PETERSON JW ; TRAPP BD.** Neuropathobiology of multiple sclerosis. *Neurol Clin,* 2005, vol. 23, 107
- **POGUE SL ; PRESTON BT ; STALDER J ; BEBBINGTON CR ; CARDARELLI PM.** The receptor for type I IFNs is highly expressed on peripheral blood B cells and monocytes and mediates a distinct profile of differentiation and activation of these cells. *J Interferon Cytokine Res,* 2004, vol. 24, 131
- **POTEMPA LA MB ; LAURENT P ; ZEMEL ES ; GEWURZ H.** Antigenic, electrophoretic and binding alterations of human C-reactive protein modified selectively in the absence of calcium. *Mol Immunol.,* 1983, vol. 20, 1165
- R: A language and environment for statistical computing. *R Foundation for Statistical Computing Website,* 2005
- **RAY S LP ; OCHIENG J.** Members of the cystatin superfamily interact with MMP-9 and protect it from autolytic degradation without affecting its gelatinolytic activities. *Biochim Biophys Acta,* 2003, vol. 1652, 91
- **REP MH ; SCHRIJVER HM ; LOPIK T ; HINTZEN RQ ; ROOS MT et al.** Interferon (IFN)-beta treatment enhances CD95 and interleukin 10 expression but reduces interferon-gamma producing T cells in MS patients. *J Neuroimmunol,* 1999, vol. 96, 92
- **RIECKMANN P ; KRUSE N ; NAGELKERKEN L ; BECKMANN K ; MILLER D et al.** Soluble vascular cell adhesion molecule (VCAM) is associated with treatment effects of Interferon beta-1b in patients with Secondary Progressive Multiple Sclerosis. *J Neurol,* 2005, vol. 252, 526
- **RIECKMANN P ; O'CONNOR P ; FRANCIS GS ; WETHERILL G ; ALTERI E.** Haematological effects of interferon-beta-1a (Rebif) therapy in multiple sclerosis. *Drug Saf,* 2004, vol. 27, 745

- **ROEDERER M ; KANTOR AB ; PARKS DR ; HERZENBERG LA.** Cy7PE and Cy7APC: bright new probes for immunofluorescence 436. *Cytometry,* 1996, vol. 24, 191
- **ROY S ; ANDERLE M ; HUA L ; BECKER C.** Differential expression profiling of serum proteins and metabolites for biomarker discovery. *Int J. of Mass Spectrometry,* 2004, vol. 238, 163
- Quantification of proteins and metabolites by mass spectrometry without isotype labeling. **ROY S ; BECKER C.** Methods in Molecular Biology: An overview of recent developments in proteomics using quantitative mass spectrometry. Humana Press, 2005
- **RUDICK RA.** Contemporary immunomodulatory therapy for multiple sclerosis. *J Nearoophtalmol,* 2001, vol. 21, 284
- **RUDICK RA ; CARPENTER CS ; COOKFAIR DL ; TUOHY VK ; RANSOHOFFRM.** In vitro and in vivo inhibition of mitogen-driven T-cell activation by recombinant interferon beta. *Neurology,* 1993, vol. 43, 2080
- **SALLUSTO F ; LENIG D ; FORSTER R ; LIPP M ; LANZAVECCHIA A.** Two subsets of memory T lymphocytes with distinct homing potentials and effector functions. *Nature,* 1999, vol. 401, 708
- **SCHIF-ZUCK S WJ ; NETZER N ; ZOHAR Y ; MEIRON M ; WILDBAUM G ; KARIN N.** Targeted overexpression of IL-18 binding protein at the central nervous system overrides flexibility in functional polarization of antigen-specific Th2 cells. *J Immunol.,* 2005, vol. 174, 4307
- **SCHMIDT R SH ; FAZEKAS F ; KAPELLER P ; ROOB G ; LECHNER A ; KOSTNER GM ; HARTUNG HP.** MRI cerebral white matter lesions and paraoxonase PON1 polymorphisms : three-year follow-up of the austrian stroke prevention study. *Arterioscler Thromb Vasc Biol.,* vol. 20, 1811
- **SEGA S ; WRABER B ; MESEC A ; HORVAT A ; IHAN A.** IFN-betala and IFN-beta1b have different patterns of influence on cytokines. *Clin Neurol Neurosurg,* 2004, vol. 106, 255
- **SMITH ME.** Phagocytic properties of microglia in vitro: implications for a role in multiple sclerosis and EAE. *Microsc Res Tech,* 2001, vol. 54, 81
- **SOILU-HANNINEN M ; SALMI A ; SALONEN R.** Interferon-beta downregulates expression of VLA-4 antigen and antagonizes interferon-gamma-induced expression of HLA-DQ on human peripheral blood monocytes. *J Neuroimmunol,* 1995, vol. 60, 99
- **SOSPEDRA M ; MARTIN R.** Immunology of multiple sclerosis. *Annu Rev Immunol,* 2005, vol. 23, 683
- **SOUTHWICK PL ; ERNST LA ; TAURIELLO EW ; PARKER SR ; MUJUMDAR RB et al.** Cyanine dye labeling reagents-carboxymethylindocyanine succinimidyl esters 166. *Cytometry,* 1990, vol. 11, 418
- **SPEAR GT ; PAULNOCK DM ; JORDAN RL ; MELTZER DM ; MERRITT JA ; BORDEN EC.** Enhancement of monocyte class I and II histocompatibility antigen expression in man by in vivo beta-interferon. *Clin Exp Immunol,* 1987, vol. 69, 107
- **STRUYF S ; COLLIE E ; PAEMEN L ; PUT W ; LENAERTS JP et al.** Synergistic induction of MCP-1 and -2 by IL-lbeta and interferons in fibroblasts and epithelial cells. *JLeukoc Biol,* 1998, vol. 63, 364
- **STURZEBECHER S ; MAIBAUER R ; HEUNER A ; BECKMANN K ; AUFDEMBRINKE B.** Pharmacodynamic comparison of single doses of IFN-betala and IFN-beta1b in healthy volunteers. *Pharmacodynamic comparison of single doses of IFN-betala and IFN-beta1b in healthy volunteers,* 1999, vol. 19, 1257
- **STUVE O ; PROD'HOMME T ; YOUSSEF S ; DUNN S ; NEUHAUS O et al.** Statins as potential therapeutic agents in multiple sclerosis. *Curr Neurol Neurosci Rep,* 2004, vol. 4, 237
- **SZALAI AJ NS ; HU XZ ; BARNUM SR.** Experimental allergic encephalomyelitis is inhibited in transgenic mice expressing human C-reactive protein. *J Immunol,* 2002, vol. 168, 5792
- **SZTEIN MB ; STEEG PS ; JOHNSON HM ; OPPENHEIM JJ.** Regulation of human peripheral blood monocyte DR antigen expression in vitro by lymphokines and recombinant interferons. *J Clin Invest,* 1984, vol. 73, 556
- **TANAKA J SK.** Localization and characterization of gelsolin in nervous tissues: gelsolin is specifically enriched in myelin-forming cells. *J Neurosci.,* 1994, vol. 14, 1038
- **TOWBIN H ; SCHMITZ A ; JAKSCHIES D ; WUSSOW P ; HORISBERGER MA.** *A whole blood immunoassay for the interferon-inducible human Mx protein,* vol. 12, 67
- **TROJANO M ; DEFAZIO G ; AVOLIO C ; PAOLICELLI D ; GIULIANI F et al.** Effects of rIFN-beta-1b on serum circulating ICAM-1 in relapsing remitting multiple sclerosis and on the membrane-bound ICAM-1 expression on brain microvascular endothelial cells. *J Neurovirol,* 2000, vol. 6 (2), 47
- **TUSSEY L ; SPELLER S ; GALLIMORE A ; VESSEY R.** Functionally distinct CD8+ memory T cell subsets in persistent EBV infection are differentiated by migratory receptor expression. *Eur J Immunol,* 2000, vol. 30, 1823
- **UHLAR CM WA.** Serum amyloid A, the major vertebrate acute-phase reactant. *Eur J Biochem.,* 1999, vol. 265, 501
- **VALLITTU AM ; HALMINEN M ; PELTONIEMI J ; ILONEN J ; JULKUNEN I et al.** Neutralizing antibodies reduce MxA protein induction in interferon-beta-1a-treated MS patients. *Neurology,* 2002, vol. 58, 1786

- **WEYENBERGH J ; LIPINSKI P ; ABADIE A ; CHABAS D ; BLANK U et al.** Antagonistic action of IFN-beta and IFN-gamma on high affinity Fc gamma receptor expression in healthy controls and multiple sclerosis patients. *J Immunol,* 1998, vol. 161, 1568
- **VENABLES WN ; RIPLEY BD.** Modern Applied Statistics with S. Springer, 2002
- **WUSSOW P ; JAKSCHIES D ; HOCHKEPPEL HK ; FIBICH C ; PENNER L ; DEICHER H.** *The human intracellular Mx-homologous protein is specifically induced by type I interferons,* 1990, vol. 20, 2015
- **WALTON ID ; DIETZ LJ ; FRESCATORE RL ; CHEN J ; WINKLES J et al.** Microvolume laser scanning cytometry platform for biological marker discovery. *Proc.SPIE-Int.Soc.Opt.Eng,* 2000
- **WANG W ; ZHOU H ; LIN H ; ROY S ; SHALER TA et al.** Quantification of proteins and metabolites by mass spectrometry without isotopic labeling or spiked standards. *Anal Chem,* 2003, vol. 75, 4818
- **WAUBANT E ; GEE L ; MILLER K ; STABLER G ; GOODKIN D.** IFN-betala may increase serum levels of TIMP-1 in patients with relapsing-remitting multiple sclerosis. *J Interferon Cytokine Res,* 2001, vol. 21, 181
- **WAUBANT E ; GOODKIN D ; BOSTROM A ; BACCHETTI P ; HIETPAS J et al.** IFNbeta lowers MMP-9/TMP-1 ratio, which predicts new enhancing lesions in patients with SPMS. *Neurology,* 2003, vol. 60, 52
- **WAUBANT E ; GOODKIN DE ; GEE L ; BACCHETTI P ; SLOAN R et al.** Serum MMP-9 and TIMP-1 levels are related to MRI activity in relapsing multiple sclerosis. *Neurology,* 1999, vol. 53, 1397
- **WILLIAMS GJ ; WITT PL.** Comparative study of the pharmacodynamic and pharmacologic effects of Betaseron and AVONEX. *J Interferon Cytokine Res,* 1998, vol. 18, 967
- **WOODROOFE N ; CROSS AK ; HARKNESS K ; SIMPSON JE.** The role of chemokines in the pathogenesis of multiple sclerosis. *Adv Fxp Med Biol,* 1999, vol. 468, 135
- **YAMADA T WA ; YAMAGUCHI T ; ITOH Y ; KAWAI T.** Automated measurement of a constitutive isotype of serum amyloid A/SAA4 and comparison with other apolipoproteins. *J Clin Lab Anal.,* 1997, vol. 11, 363
- **YOUSSEF S ; STUVE O ; PATARROYO JC ; RUIZ PJ ; RADOSEVICH JL et al.** The HMG-CoA se inhibitor, atorvastatin, promotes a Th2 bias and reverses paralysis in central nervous system autoimmune disease. *Nature,* 2002, vol. 420, 78
- Differential metobolic profiling for biomarker discovery. **ZHOU H ; KANTOR AB ; BECKER C.** Metabolome Analyses: Strategies in Systems Biology. Springer, 2005

SEQUENCE LISTING

<110> PPD, Inc. PPD Biomarker Discovery Sciences LLC

<120> Biomarkers for Multiple Sclerosis and Methods of Use Thereof

<130> 5453-1-PCT

<150> 60/722,172
<151> 2005-09-29

<160> 4

<170> PatentIn version 3.3

<210> 1
<211> 187
<212> PRT
<213> Homo sapiens

<400> 1

```
Met Thr Asn Lys Cys Leu Leu Gln Ile Ala Leu Leu Leu Cys Phe Ser
1               5                   10              15

Thr Thr Ala Leu Ser Met Ser Tyr Asn Leu Leu Gly Phe Leu Gln Arg
            20          25                  30

Ser Ser Asn Phe Gln Cys Gln Lys Leu Leu Trp Gln Leu Asn Gly Arg
        35          40                  45

Leu Glu Tyr Cys Leu Lys Asp Arg Met Asn Phe Asp Ile Pro Glu Glu
    50              55                  60

Ile Lys Gln Leu Gln Gln Phe Gln Lys Glu Asp Ala Ala Leu Thr Ile
65          70                  75                  80

Tyr Glu Met Leu Gln Asn Ile Phe Ala Ile Phe Arg Gln Asp Ser Ser
            85                  90                  95

Ser Thr Gly Trp Asn Glu Thr Ile Val Glu Asn Leu Leu Ala Asn Val
            100                 105                 110

Tyr His Gln Ile Asn His Leu Lys Thr Val Leu Glu Glu Lys Leu Glu
        115                 120                 125

Lys Glu Asp Phe Thr Arg Gly Lys Leu Met Ser Ser Leu His Leu Lys
    130                 135                 140

Arg Tyr Tyr Gly Arg Ile Leu His Tyr Leu Lys Ala Lys Glu Tyr Ser
145                 150                 155                 160

His Cys Ala Trp Thr Ile Val Arg Val Glu Ile Leu Arg Asn Phe Tyr
                165                 170                 175

Phe Ile Asn Arg Leu Thr Gly Tyr Leu Arg Asn
            180                 185
```

<210> 2
<211> 19
<212> PRT
<213> Homo sapiens

<400> 2

```
Lys Cys Ser Tyr Thr Glu Asp Ala Gln Cys Ile Asp Gly Thr Ile Glu
1               5                   10                  15

Val Pro Lys
```

<210> 3
<211> 21
<212> PRT
<213> Homo sapiens

<400> 3

```
    Ala Thr Phe Gly Cys His Asp Gly Tyr Ser Leu Asp Gly Pro Glu Glu
    1               5                   10                  15

    Ile Glu Cys Thr Lys
                20
```

<210> 4

<211> 20
<212> PRT
<213> Homo sapiens

<400> 4

```
    Thr Phe Tyr Glu Pro Gly Glu Glu Ile Thr Tyr Ser Cys Lys Pro Gly
    1               5                   10                  15

    Tyr Val Ser Arg
                20
```